# FASCICULE DE BREVET EUROPEEN

(11) **EP 1 487 804 B1**
(45) Date de publication et mention de la délivrance du brevet: **26.02.2014**
(21) Numéro de dépôt: 03744396.7
(22) Date de dépôt: 07.03.2003
(51) Int. Cl.: C07D 231/56, C07D 401/04, C07D 405/04, C07D 409/04, C07C 7/08, A61K 31/416, A61P 25/02, A61P 35/00, A61P 37/00

(54) **DERIVES D'AMINOINDAZOLES COMME INHIBITEURS DE PROTEINE-KINASE**
AMINOINDAZOLDERIVATE ALS PROTEINKINASE-INHIBITOREN
AMINOINDAZOLE DERIVATIVES AS PROTEIN-KINASE INHIBITORS

(30) Priorité: 11.03.2002 FR 0202997
(43) Date de publication de la demande: 22.12.2004
(73) Titulaire: Aventis Pharma S.A., 92160 Antony (FR)
(72) Inventeur: DUTRUC-ROSSET, Gilles, F-75012 Paris (FR); LESUISSE, Dominique, F-93100 Montreuil (FR); ROONEY, Thomas, F-91400 Orsay (FR); HALLEY, Franck, F-92370 Chaville (FR)
(74) Mandataire: Bonnet, Maiwenn
(86) Numéro de dépôt international: PCT/FR2003/000752
(87) Numéro de publication internationale: WO 2003/078403

(56) Documents cités:
- WO-A-01/85726
- DE-A- 2 458 965
- DE-A- 2 622 761
- DE-B- 1 280 878
- FR-A- 2 111 641
- US-A- 3 133 081
- US-A- 3 316 207
- US-A- 3 720 671
- DAIDONE, GIUSEPPE ET AL: "Synthesis, crystallographic studies and biological evaluation of some 2-substituted 3-indazolyl-4(3H)-quinazolinones and 3-indazolyl-4(3H)-benzotriazinones" HETEROCYCLES (1996), 43(11), 2385-2396, XP002221089
- RAFFA, DEMETRIO ET AL: "Synthesis and antiproliferative activity of novel 3-(indazol-3-yl)-quinazolin-4(3H)-one and 3-(indazol-3- yl)benzotriazin-4(3H)-one derivatives" ARCHIV DER PHARMAZIE (WEINHEIM, GERMANY) (1999), 332(9), 317-320, XP002221090
- KORBONITS, DEZSO ET AL: "Ring transformation of 3-(2-aminoaryl)-1,2,4-oxadiazoles into 3-acylaminoindazoles;extension of the Boulton-Katritzy scheme" J. CHEM. SOC., PERKIN TRANS. 1 (1982), (3), 759-66, XP002221091
- VIVONA, NICOLO ET AL: "Mononuclear heterocyclic rearrangements. Part 12. Rearrangement of 1,2,4-oxadiazoles into indazoles" J. HETEROCYCL. CHEM. (1979), 16(4), 783-4, XP002221092
- DATABASE CHEMCATS [en ligne] Chemical Abstract Service; XP002221093 & "LaboTest Stock" 2 janvier 2002 (2002-01-02) , LABOTEST, NIEDERSCHOMA, GERMANY

## Description

La présente invention concerne l'utilisation de dérivés d'aminoindazoles de formule (I): 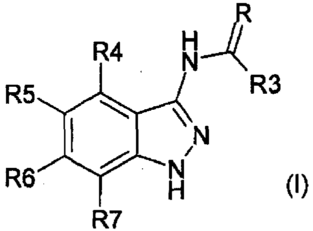 ou leurs sels pharmaceutiquement acceptables comme inhibiteur de kinase.

WO 01/85726 décrit des dérivés d'aminoindazoles 5-(1,1-dioxo-1λ⁶-isothiazolidin-2-yl)-substitués qui présentent une activité inhibitrice de kinases CDK.

L'invention a pour objet l'utilisation des dérivés d'aminoindazoles de formule (I) et leurs sels pharmaceutiquement acceptables pour la préparation de compositions pharmaceutiques destinées à prévenir et traiter les maladies pouvant résulter d'une activité anormale de kinases comme par exemple celles impliquées dans les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer, les compositions pharmaceutiques contenant les nouveaux dérivés d'aminoindazoles et leurs sels pharmaceutiquement acceptables et les dérivés nouveaux d'aminoindazoles et leurs sels pharmaceutiquement acceptables.

La présente invention concerne des dérivés d'aminoindazoles de formule (I) dans laquelle :
R est soit O, S ou NH
R3 est un radical (1-6C)alkyle, aryle(1-6C)alkyle, hétéroaryle, hétéroaryle(1-6C)alkyle, aryle ou hétéroaryle fusionné à un cycloalkyle (1-10C), hétérocycle, cycloalkyle, adamantyle, polycycloalkyles, alkényle, alkynyle ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R4, R5, R6 et R7 sont indépendamment l'un de l'autre choisis parmi les radicaux suivant hydrogène, halogène, CN, N02, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle , adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO2NR10R11, -O-SO₂R10, -SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ; à l'exception de 3-acetamido-indazole, N-(1H-indazol-3-yl)-butanamide, N-(1H-indazol-3-yl)-phenylacetamide, N-(1H-indazol-3-yl)-benzhydrylacetamide, 5-amino-3-acetamido-indazole, N-(6-chloro-1H-indazol-3-yl)-2,2,2-trifluoroacetamide, N-(6-chloro-1H-indazol-3-yl)-2-furancarboxamide, N-(6-chloro-1H-indazol-3-yl)-2-thiophenecarboxamide, N-(5-nitro-1H-indazol-3-yl)-acetamide ;
leurs racémiques, énantiomères, diastéréoisomères et leurs mélanges, leurs tautomères et leurs sels pharmaceutiquement acceptables.

Plus particulièrement, la présente invention concerne des dérivés d'aminoindazoles de formule (I) dans laquelle :
R est soit O, S ou NH
R3 est un radical (1-6C)alkyle, aryle(1-6C)alkyle, hétéroaryle, hétéroaryle(1-6C)alkyle, cycloalkyle, alkényle, alkynyle ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R4 et R7 sont hydrogène ;
R5, R6 sont indépendamment l'un de l'autre choisis parmi les radicaux suivant hydrogène, halogène, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, cycloalkyle, hétérocycle, alkényle, alkynyle, adamantyle, polycycloalkyle ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO2NR10R11, -O-SO₂R10, -SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
à l'exception de 3-acetamido-indazole, N-(1H-indazol-3-yl)-butanamide, N-(1H-indazol-3-yl)-phenylacetamide, N-(1H-indazol-3-yl)-benzhydrylacetamide, 5-amino-3-acetamido-indazole, N-(6-chloro-1H-indazol-3-yl)-2,2,2-trifluoroacetamide, N-(6-chloro-1H-indazol-3-yl)-2-furancarboxamide, N-(6-chloro-1H-indazol-3-yl)-2-thiophenecarboxamide,
N-(5-nitro-1H-indazol-3-yl)-acetamide ;
leurs racémiques, énantiomères, diastéréoisomères et leurs mélanges, leurs tautomères et leurs sels pharmaceutiquement acceptables.

Et de manière préférée, la présente invention concerne des dérivés d'aminoindazoles de formule (I) dans laquelle :
R est O
R4 et R7 sont H
R3 est un radical (1-6C)alkyle, aryle(1-6C)alkyle, hétéroaryle, hétéroaryle(1-6C)alkyle, hétérocycle, cycloalkyle, alkényle, ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R5 et R6 sont indépendamment l'un de l'autre choisis parmi les radicaux suivant hydrogène, halogène, CN, N02, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO2NR10R11, -O-SO₂R10, - SO₂-O-R10, aryle, hétéroaryle, formyle, trifluorométhyle, trifluorométhoxy;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
à l'exception de 3-acetamido-indazole, N-(1H-indazol-3-yl)-butanamide, N-(1H-indazol-3-yl)-phenylacetamide, N-(1H-indazol-3-yl)-benzhydrylacetamide, 5-amino-3-acetamido-indazole, N-(6-chloro-1H-indazol-3-yl)-2,2,2-trifluoroacetamide, N-(6-chloro-1H-indazol-3-yl)-2-furancarboxamide, N-(6-chloro-1H-indazol-3-yl)-2-thiophenecarboxamide, N-(5-nitro-1H-indazol-3-yl)-acetamide ;
leurs racémiques, énantiomères, diastéréoisomères et leurs mélanges, leurs tautomères et leurs sels pharmaceutiquement acceptables.

Dans les définitions précédentes et celles qui suivent, les radicaux alkyle(1-6C) contiennent 1 à 6 atomes de carbone en chaîne droite ou ramifiée ; les radicaux alkényles contiennent 2 à 6 atomes de carbone et une à 3 doubles liaisons conjuguées ou non en chaîne droite ou ramifiée ; les radicaux alkynyles contiennent 2 à 6 atomes de carbone et 1 à 3 triples liaisons conjuguées ou non en chaîne droite ou ramifiée ; les radicaux aryles sont choisis parmi phényle, naphtyle ou indényle et peuvent être substitué par un ou plusieurs halogènes ; les radicaux hétéroaryles contiennent 3 à 10 chaînons, contenant éventuellement un ou plusieurs hétéroatomes choisis parmi oxygène, soufre et azote en particulier, thiazolyle, thiényle, pyrrolyle, pyridinyle, furyle, imidazolyle, oxazolyle, pyrazinyle, tetrazolyle ; le radical halogène est soit, chlore, iode, fluor, brome ; les radicaux polycycloalkyles sont choisis parmi adamantyle, quinuclidinyle, bornanyle, norbornanyle, bornenyle, norbornenyle ; les radicaux hétéroaryles fusionnés à un cycloalkyle (1-10C) sont choisi parmi indanyle, isochromanyle, chromanyle, 1,2,3,4-tétrahydroisoquinolyle, 1,2,3,4-tétrahydroquinolyle ; les radicaux hétérocycles contiennent 1 à 2 hétéroatomes choisis parmi oxygène, soufre, azote et représentent en particulier piperidinyle, morpholinyle, pyrrolidinyle, imidazolidinyle, pyrrazolidinyle, isothiazolidinyle, thiazolidinyle, isoxazolidinyle, oxazolidinyle, piperazinyle.

Les composés de formule (I) présentent un ou plusieurs carbones asymétriques et peuvent donc se présenter sous forme d'isomères, de racémique, d'énantiomères et de diastéréoisomères; ceux-ci font également partie de l'invention ainsi que leurs mélanges.

Parmi les composés de formule (I) utiles selon l'invention on peut citer les composés suivants:
Acide (2Z) 4-[(6-chloro-1H-indazol-3-yl)amino]-4-oxo-2-buténoïque
Acide (2E) 4-[(6-chloro-1H-indazol-3-yl)amino]-4-oxo-2-buténoïque
(2E) 4-[(6-chloro-1H-indazol-3-yl)amino]-4-oxo-2-buténoate d'éthyle
(2Z) 4-[(6-chloro-1H-indazol-3-yl)amino]-4-oxo-2-buténoate d'éthyle
Acide 4-[(6-chloro-1H-indazol-3-yl)amino]-4-oxo-2-butanoïque
Acide (2Z) 4-[(5-bromo-1H-indazol-3-yl)amino]-4-oxo-2-buténoïque
Acide (2E) 4-[(6-chloro-1H-indazol-3-yl)amino]-4-oxo-2-buténoïque
Acide (2E) 4-[(5-bromo-1H-indazol-3-yl)amino]-4-oxo-2-buténoïque
Acide (2Z) 4-[(6-chloro-1H-indazol-3-yl)amino]-4-oxo-2-buténoïque
Acide 4-[(5-bromo-1H-indazol-3-yl)amino]-4-oxo-2-butanoïque
(2E) N-(6-chloro-1H-indazol-3-yl)-2-buténamide
(2Z) N-(6-ehloro-1H-indazol-3-yl)-2-buténamide
N-(6-chloro-1H-indazol-3-yl)-3-buténamide, chlorhydrate
4-[(6-chloro-1H-indazol-3-yl)amino]-4-oxo-2-butanoate de méthyle
N-(6-chloro-1H-indazol-3-yl)-acétamide
N-(6-chloro-1H-indazol-3-yl)-butanamide
(2E) N-(6-bromo-1H-indazol-3-yl)-2-buténamide
(2E) N-(5-méthyl-1H-indazol-3-yl)-2-buténamide
(2Z) N-(6-bromo-1H-indazol-3-yl)-2-buténamide
(2Z) N-(5-méthyl-1H-indazol-3-yl)-2-buténamide
N-(6-chloro-1H-indazol-3-yl)-2-popanamide
(2E) N-[6-(trifluorométhyl)-1H-indazol-3-yl]-2-buténamide
(2Z) N-[6-(trifluorométhy)1-1H-indazol-3-yl]-2-buténamide
4-[[6-(trifluorométhyl)-1H-indazol-3-yl]amino]-4-oxo-butanoate d'éthyle
(2E) N-[5-(trifluorométhyl)-1H-indazol-3-yl]-2-buténamide
(2Z) N-[5-(trifluorométhyl)-1H-indazol-3-yl]-2-buténamide
N-[5-chloro-1H-indazol-3-yl]-2-butanamide
N-[4-chloro-1H-indazol-3-yl]-butanamide
N-[6-(trifluorométhyl)-1H-indazol-3-yl]-butanamide
N-[6-chloro-1H-indazol-3-yl]-propénamide
N-[5-(trifluorométhyl)-1H-indazol-3-yl]-butanamide
N-[5-nitro-1H-indazol-3-yl]-butanamide
N-[6-bromo-1H-indazol-3-yl]-butanamide
N-[6-(3-pyridyl)-1H-indazol-3-yl]-butanamide
N-[4-iodo-1H-indazol-3-yl]-butanamide
N-[6-phenyl-1H-indazol-3-yl]-butanamide
N-[6-bromo-5,7-dinitro-1H-indazol-3-yl]-butanamide
N-[6-bromo-7-nitro-1H-indazol-3-yl]-butanamide
N-[6-bromo-5-nitro-1H-indazol-3-yl]-butanamide
N-[6-(furan-3-yl)-1H-indazol-3-yl]-butanamide
N-[6-[4-(phenylmethoxy)phenyl]-1H-indazol-3-yl]-butanamide
N-[6-(4-hydroxyphenyl)-1H-indazol-3-yl]-butanamide
N-[6-chloro-1H-indazol-3-yl]-benzenamide
N-[6-(3,5-difluorophenyl)-1H-mdazol-3-yl]-butanamide
N-[6-(3-thiophenyl)-1H-indazol-3-yl] butanamide
N-[6-chloro-1H-indazol-3-yl]-2-thiophenacétamide
N-[5-[[3-(fluorophenyl)sulfonyl]amino]-1H-indazol-3-yl]-benzamide
N-[6-(2-chlorophenyl)-1H-indazol-3-yl]-butanamide
N-[6-(2-chloro-4-hydroxyphenyl)-1H-indazol-3-yl]-butanamide
N-[6-(4-ethylphenyl)-1H-indazol-3-yl]-butanamide
N-[6-(4-ethenylphenyl)-1H-indazol-3-yl]-butanamide
N-[6-(4-pyridyl)-1H-indazol-3-yl]-butanamide
N-[6-(phenylmethyl)-1H-indazol-3-yl]-butanamide
N-[6-(4-aminophenyl)-1H-indazol-3-yl]-butanamide
N-[6-(1-morpholino)-1H-indazol-3-yl]-butanamide
N-[6-[(4-phenylethynyl)phenyl]-1H-indazol-3-yl]-butanamide
N-[6-(2-propenyl)-1H-indazol-3-yl]-butanamide
N-[5-amino-1H-indazol-3-yl]-butanamide
N-[6-bromo-5-chloro-1H-indazol-3-yl]-butanamide
N-[6-chloro-5-bromo-1H-indazol-3-yl]-butanamide
N-[6-chloro-5-nitro-1H-indazol-3-yl]-butanamide
N-[6-(4-hydroxyphenyl)-5-bromo-1H-indazol-3-yl]-butanamide
N-[6-(4-hydroxyphenyl)-5-(phenylamino)-1H-indazol-3-yl]-butanamide
N-[6-(4-hydroxy-phenyl)-5-(2-phenylethenyl)-1H-indazol-3-yl]-butanamide
N-[6-(4-hydroxyphenyl)-5-phenylcarbonyl-1H-indazol-3-yl]-butanamide
N-[6-(4-hydroxyphenyl)-5-[3-(dimethylamino)-propynyl]-1H-indazol-3-yl]-butanamide
N-[6-chloro-1H-indazol-3-yl]-3-thiophenecarboxamide
N-[6-chloro-1H-indazol-3-yl]-2-pyridineacetamide
N-[6-chloro-1H-indazol-3-yl]-3-pyridinecarboxamide
N-[6-chloro-1H-indazol-3-yl]-benzenacetamide
N-[6-chloro-1H-indazol-3-yl]-benzenepropanamide
N-[6-chloro-1H-indazol-3-yl]-3-pyridineacétamide
N-[6-chloro-1H-indazol-3-yl]-2-chloro-acetamide
N-[6-chloro-1H-indazol-3-yl]-4-morpholineacétamide
N-[6-chloro-1H-indazol-3-yl]-1-piperazineacétamide
N-[6-chloro-1H-indazol-3-yl]-4-[(2-méthoxyéthyl)amino]-cyclohexanecarboxamide
4-amino-N-[6-chloro-1H-indazol-3-yl]-1-piperidinecarboxamide
N-[6-chloro-1H-indazo1-3-yl]-4-morpholinylcarboxamide
leurs racémiques, énantiomères, diastéréoisomères et leurs mélanges, leurs tautomères et leurs sels pharmaceutiquement acceptables,
et plus particulièrement les composés suivants :
   Acide (2Z) 4-[(6-chloro-1H-indazol-3-yl)amino]-4-oxo-2-buténoïque
   (2E) 4-[(6-chloro-1H-indazol-3-yl)amino]-4-oxo-2-buténoate d'éthyle
   Acide 4-[(6-chloro-1H-indazol-3-yl)amino]-4-oxo-2-butanoïque
   Acide (2Z) 4-[(5-bromo-1H-mdazol-3-yl)amino]-4-oxo-2-buténoïque
   Acide (2E) 4-[(6-chloro-1H-indazol-3-yl)amino]-4-oxo-2-buténoïque
   Acide 4-[(5-bromo-1H-indazol-3-yl)amino]-4-oxo-2-butanoïque
   (2E) N-(6-chloro-1H-indazol-3-yl)-2-buténamide
   N-(6-chloro-1H-indazol-3-yl)-3-buténamide, chlorhydrate
   4-[(6-chloro-1H-indazol-3-yl)amino]-4-oxo-2-butanoate de méthyle
   N-(6-chloro-1H-indazol-3-yl)-acétamide
   N-(6-chloro-1H-indazol-3-yl)-butanamide
   (2E) N-(6-bromo-1H-indazol-3-yl)-2-buténamide
   (2E) N-(5-méthyl-1H-indazol-3-yl)-2-buténamide
   N-(6-chloro-1H-indazol-3-yl)-2-popanamide
   (2E) N-[6-(trifluorométhyl)-1H-indazol-3-yl]-2-buténamide
   4-[[6-(trifluorométhyl)-1H-indazol-3-yl]amino]-4-oxo-butanoate d'éthyle
   (2E) N-[5-(trifluorométhyl)-1H-indazol-3-yl]-2-buténamide
   N-[5-chloro-1H-indazol-3-yl]-2-butanamide
   N-[4-chloro-1H-indazol-3-yl]-butanamide
   N-[6-(trifluorométhyl)-1H-indazol-3-yl]-butanamide
   N-[6-chloro-1H-indazol-3-yl]-propénamide
   N-[5-(trifluorométhyl)-1H-indazol-3-yl]-butanamide
   N-[5-nitro-1H-indazol-3-yl]-butanamide
   N-[6-bromo-1H-indazol-3-yl]-butanamide
   N-[6-chloro-1-[[2-(triméthylsilyl)éthoxy]méthyl]-1H-indazol-3-yl]-butanamide
   N-[6-(3-pyridinyl)-1H-indazol-3-yl]-butanamide
   N-[4-iodo-1H-indazol-3-yl]-butanamide
   N-[6-phenyl-1H-indazol-3-yl]-butanamide
   N-[6-bromo-5,7-dinitro-1H-indazol-3-yl]-butanamide
   N-[6-bromo-7-nitro-1H-indazol-3-yl]-butanamide
   N-[6-bromo-5-nitro-1H-indazol-3-yl]-butanamide
   N-[(6-furan-3-yl)-1H-indazol-3-yl]-butanamide
   N-[6-[4-(phenylmethoxy)phenyl]-1H-indazol-3-yl]-butanamide
   N-[6-(4-hydroxy-phenyl)-1H-indazol-3-yl]-butanamide
   N-[6-chloro-1H-indazol-3-yl]-benzenamide
   N-[[6-(3,5-difluorophenyl)-1H-indazol-3-yl]]-butanamide
   N-[6-(3-thienyl)-1H-indazol-3-yl] butanamide
   N-[6-chloro-1H-indazol-3-yl]-2-thiophenacétamide
   N-[5-[[(3-fluorophenyl)sulfonyl]amino]-1H-indazol-3-yl]-benzamide
   N-[6-(2-phényléthyl)-1H-indazol-3-yl]-butanamide
   N-(6,7-difluoro-1H-indazol-3-yl)-butanamide:
   N-[6-(4-méthoxyphényl)-1H-indazol-3-yl]-butanamide
   N-[6-(4-méthylthiophényl)-1H-indazol-3-yl]-butanamide
   N-[6-(4-trifluorométhoxyphényl)-1H-indazol-3-yl]-butanamide
   N-[(6-(1-propènyl)-1H-indazol-3-yl]-butanamide
   N-[6-chloro-1H-indazol-3-yl]-2-pyridinecarboxamide
   N-[6-(4-fluorophényl)-1H-indazol-3-yl]-butanamide
   N-[6-[4-(1,1-diméthyléthyl)phényl]-1H-indazol-3-yl]-butanamide
   N-[6-bromo-7-amino-1H-indazol-3-yl]-butanamide
   N-[6-[4-(trifluorométhyl)phényl]-1H-indazol-3-yl]-butanamide :
   N-[6-(4-méthylphényl)-1H-indazol-3-yl]-butanamide
   N-[6-(3,5-dichlorophényl)-1H-indazol-3-yl]-butanamide
   N-[6-chloro-1H-indazol-3-yl]-3,5-dichlorobenzamide
   N-[6-(4-chlorophényl)-1H-indazol-3-yl]-butanamide
   N-[6-chloro-1H-indazol-3-yl]-benzenepropanamide, trifluoroacétate
   N-[6-chloro-1H-indazol-3-yl]-benzenepropanamide
   N-[[6-(4-éthylphényl)-1H-indazol-3-yl]]-butanamide :
   N-[6-(4-pyridinyl)-1H-indazol-3-yl]-butanamide :
   N-(5-amino-lH-mdazol-3-yl)-butanamide :
   N-(5-bromo-6-chloro-1H-indazol-3-yl)-butanamide :
   N-(6-chloro-1H-indazol-3-yl)-2-thiophenecarboxamide :
   N-(6-chloro-1H-indazol-3-yl)-2-méthylpropylamide :
   4-Chloro-N-(6-chloro-1H-indazol-3-yl)-butanamide :
   N-(5-phényl-6-chloro-1H-indazol-3-yl)-butanamide
   N-[5-bromo-6-[4-(phénylméthoxy)phényl]-1H-indazol-3-yl]-butanamide
   N-[5-bromo-6-(4-hydroxyphényl)-1H-indazol-3-yl]-butanamide
   N-[[6-(4-nitrophényl)-1H-indazol-3-yl]]-butanamide :
   N-[6-(2-chlorophényl)-1H-indazol-3-yl]-butanamide :
   N-[6-[3-(phénylméthoxy)phényl]-1H-indazol-3-yl]-butanamide
   N-[6-(3-hydroxyphényl)-1H-indazol-3-yl]-butanamide
   N-[6-chloro-5-(4-pyridinyl)-1H-indazol-3-yl]-butanamide
   N-[6-chloro-5-(3-furanyl)-1H-indazol-3-yl]-butanamide
   N-[6-[2-chloro-4-(phénylméthoxy)-phényl]-1H-indazol-3-yl]-butanamide :
   N-[6-(2-chloro-4-hydroxyphényl)-1H-indazol-3-yl]-butanamide :
   N-[5,6-dibromo-1H-indazol-3-yl]-butanamide :
   N-[6-chloro-1H-indazol-3-yl]-2,2,3,3,4,4,4-heptafluoro-butanamide :
   N-[6-chloro-5-(4-fluorophényl)-1H-indazol-3-yl]-butanamide
   N-[[6-(4-aminophényl)-1H-indazol-3-yl]]-butanamide :
   N-[6-[4-(diméthylamino)phényl]-1H-indazol-3-yl]-butanamide :
   N-(6-chloro-1H-indazol-3-yl)-4-méthyl-1-pipérazineacétamide
   N-(6-chloro-1H-indazol-3-yl)-1-pipéridineacétamide
   N-(6-chloro-1H-indazol-3-yl)-4-morpholineacétamide
   N-(6-chloro-1H-indazol-3-yl)-1H-1,2,4-triazole-1-acétamide
   N-(6-chloro-1H-indazol-3-yl)-2-(cyclohexylamino)-acétamide
   2-[(phénylméthyl)amino]-N-(6-chloro-1H-indazol-3-yl)-acétamide
   N-(6-chloro-1H-indazol-3-yl)-1H-azepine-1-acétamide
   N-(6-chloro-1H-indazol-3-yl)-1-pipérazineacétamide
   N-(6-chloro-1H-indazol-3-yl)-2-[[3-(diméthylamino)propyl]amino]-acétamide
   N-(6-chloro-1H-indazol-3-yl)-thiomorpholine-4-acétamide
   N-(6-chloro-1H-indazol-3-yl)-1-pyrrolidineacétamide
   N-(6-chloro-1H-indazol-3-yl)-2-[[2-(diméthylamino)éthyl]amino]-acétamide
   N-(6-chloro-1H-indazol-3-yl)-1-cyclopropylaminoacétamide, trifluoroacétate
   N-(6-chloro-1H-indazol-3-yl)-1-cyclopropylaminoacétamide
   N-(6-chloro-1H-indazol-3-yl)-2-(2-diéthylamino-éthylamino)-acétamide, tris trifluoroacétate
   N-(6-chloro-1H-indazol-3-yl)-2-(2-diéthylamino-éthylamino)-acétamide,
   N-[5,6-diphényl-1H-indazol-3-yl]-butanamide :
   N-[6-chloro-5-(4-méthylphényl)- 1H-indazol-3-yl]-butanamide
   N-[5-phényl-6-[4-(phénylméthoxy)phényl]-1H-indazol-3-yl]-butanamide :
   N-[5-phényl-6-(4-hydroxyphényl)-1H-indazol-3-yl]-butanamide
   N-[6-chloro-5-(4-pyridinyl)-1H-indazol-3-yl]-butanamide :
   N-[5-(4-aminophényl)-6-chloro-1H-indazol-3-yl]-butanamide :
   N-[6-chloro- 5-(4-éthylphényl)-1H-indazol-3-yl]-butanamide
   N-[6-chloro-5-[4-(phénylméthoxy)phényl]- 1H-indazol-3-yl]-butanamide
   N-[6-chloro-5-(4-hydroxyphényl)- 1H-indazol-3-yl]-butanamide
   N-[5,6-bis[4-(phénylméthoxy)phényl]-1H-indazol-3-yl]-butanamide
   N-[5,6-bis (4-hydroxyphényl)-1H-indazol-3-yl]-butanamide
   N-[5-(3-furanyl)-6-[4-(phénylméthoxy)phényl]-1H-indazol-3-yl]-butanamide
   N-[5-(3-furanyl)-6-([4-hydroxyphényl)-1H-indazol-3-yl]-butanamide
   N-[5-(4-éthylphényl)-6-[4-(phénylméthoxy)phényl]-1H-indazol-3-yl]-butanamide
   N-[5-(4-éthylphényl)-6-(4-hydroxyphényl)-1H-indazol-3-yl]-butanamide
   N-[5-(3-pyridinyl)-6-[4-(phénylméthoxy)phényl]-1H-indazol-3-yl]-butanamide
   N-[5-(3-pyridinyl)-6-(4-hydroxyphényl)-1H-indazol-3-yl]-butanamide
   N-[5-(2-furanyl)-6-[4-(phénylméthoxy)phényl]-1H-indazol-3-yl]-butanamide:
   N-[5-(2-furanyl)-6-(4-hydroxyphényl)-1H-indazol-3-yl]-butanamide
   N-(5-bromo-6-chloro-7-nitro-1H-indazol-3-yl)-butanamide :
   N-(5-bromo-6,7-difluoro-1H-indazol-3-yl)-butanamide
   N-[6-(4-cyanophényl)-1H-indazol-3-yl]-butanamide :
   N-(6,7-difluoro-5-nitro-1H-indazol-3-yl)-butanamide :
   N-(6,7-difluoro-5-phényl-1H-indazol-3-yl)-butanamide
   N-[6-(6-hydroxy-pyridin-3-yl)- 1H-indazol-3-yl]-butanamide
   N-[6-(3,4-dihydroxy-phényl)-1H-indazol-3-yl]-butanamide, trifluoroacétate
   N-[6-(3,4-dihydroxy-phényl)-1H-indazol-3-yl]-butanamide
   N-[7-fluoro-5-nitro-6-[2-(phényléthyl)amino]-1H-indazol-3-yl]-butanamide
   N-(7-fluoro-5-nitro-6-morpholino-1H-indazol-3-yl)-butanamide
   N-(7-fluoro-5-amino-6-morpholino-1H-indazol-3-yl)-butanamide
   N-(5-bromo-7-fluoro -6-morpholino-1H-indazol-3-yl)-butanamide
   N-[7-fluoro-6-(trifluorométhyl)-1H-indazol-3-yl]-butanamide
   N-(6-bromo-4,5,7-trifluoro-1H-indazol-3-yl)-butanamide
   N-[6-(6-amino-pyridin-3-yl)-1H-indazol-3-yl]-butanamide, difluoroacétate
   N-[6-(6-amino-pyridin-3-yl)-1H-indazol-3-yl]-butanamide
   2-chloro-N-(6,7-difluoro-1H-indazol-3-yl)-acétamide
   N-(6,7-difluoro-1H-indazol-3-yl)-1-pipéridineacétamide
   son racémique, ses énantiomères, tautomères ainsi que leurs sels pharmaceutiquement acceptables,

L'invention concerne également les compositions pharmaceutiques contenant en tant que principe actif un dérivé de formule (I) pour lequel soit
R est soit O, S ou NH
R3 est un radical (1-6C)alkyle, aryle(1-6C)alkyle, hétéroaryle, hétéroaryle(1-6C)alkyle, aryle ou hétéroaryle fusionné à un cycloalkyle (1-10C), hétérocycle, cycloalkyle, adamantyle, polycycloalkyles, alkényle, alkynyle ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R4, R5, R6 et R7 sont indépendamment l'un de l'autre choisis parmi les radicaux suivant hydrogène, halogène, CN, N02, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle , adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
leurs racémiques, énantiomères, diastéréoisomères et leurs mélanges, leurs tautomères et leurs sels pharmaceutiquement acceptables.

Plus particulièrement, la présente invention concerne l'utilisation des dérivés d'aminoindazoles de formule (I) dans laquelle :
R est soit O, S ou NH
R3 est un radical (1-6C)alkyle, aryle(1-6C)alkyle, hétéroaryle, hétéroaryle(1-6C)alkyle, cycloalkyle, alkényle, alkynyle ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R4 et R7 sont hydrogène ;
R5, R6 sont indépendamment l'un de l'autre choisis parmi les radicaux suivant hydrogène, halogène, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, cycloalkyle, hétérocycle, alkényle, alkynyle , adamantyle, polycycloalkyle ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
leurs racémiques, énantiomères, diastéréoisomères et leurs mélanges, leurs tautomères et leurs sels pharmaceutiquement acceptables.

Et de manière préférée, la présente invention concerne l'utilisation des dérivés d'aminoindazoles de formule (I) dans laquelle :
R est O
R4 et R7 sont H
R3 est un radical (1-6C)alkyle, aryle(1-6C)alkyle, hétéroaryle, hétéroaryle(1-6C)alkyle, hétérocycle, cycloalkyle, alkényle, ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R5 et R6 sont indépendamment l'un de l'autre choisis parmi les radicaux suivant hydrogène, halogène, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, - SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
leurs racémiques, énantiomères, diastéréoisomères et leurs mélanges, leurs tautomères et leurs sels pharmaceutiquement acceptables.

Dans les définitions précédentes et celles qui suivent, les radicaux alkyles et alkyle(1-6C) contiennent 1 à 6 atomes de carbone en chaîne droite ou ramifiée ; les radicaux alkényles contiennent 1 à 6 atomes de carbone et une à 3 doubles liaisons conjuguées ou non en chaîne droite ou ramifiée ; les radicaux alkynyles contiennent 1 à 6 atomes de carbone et 1 à 3 triples liaisons conjuguées ou non en chaîne droite ou ramifiée ; les radicaux aryles sont choisis parmi phényle, naphtyle ou indényle et peuvent être substitué par un ou plusieurs halogènes ; les radicaux hétéroaryles contiennent 3 à 10 chaînons, contenant éventuellement un ou plusieurs hétéroatomes choisis parmi oxygène, soufre et azote en particulier, thiazolyle, thiényle, pyrrolyle, pyridinyle, furyle, imidazolyle, oxazolyle, pyrazinyle, tetrazolyle ; le radical halogène est soit, chlore, iode, fluor, brome ; les radicaux polycycloalkyles sont choisis parmi adamantyle, quinuclidinyle, bornanyle, norbornanyle, bornenyle, norbornenyle ; les radicaux hétéroaryles fusionnés à un cycloalkyle (1-10C) sont choisi parmi indanyle, isochromanyle, chromanyle, 1,2,3,4-tétrahydroisoquinolyle, 1,2,3,4-tétrahydroquinolyle ; les radicaux hétérocycles contiennent 1 à 2 hétéroatomes choisis parmi oxygène, soufre, azote et représentent en particulier piperidinyle, morpholinyle, pyrrolidinyle, imidazolidinyle, pyrrazolidinyle, isothiazolidinyle, thiazolidinyle, isoxazolidinyle, oxazolidinyle, piperazinyle.

Les dérives de formule (I) pour lesquels R=O peuvent être obtenus par acylation des dérivés 3-amino correspondants, soit à l'aide d'un chlorure d'acide, d'un anhydride ou bien par réaction d'un acide en présence d'un agent d'activation.

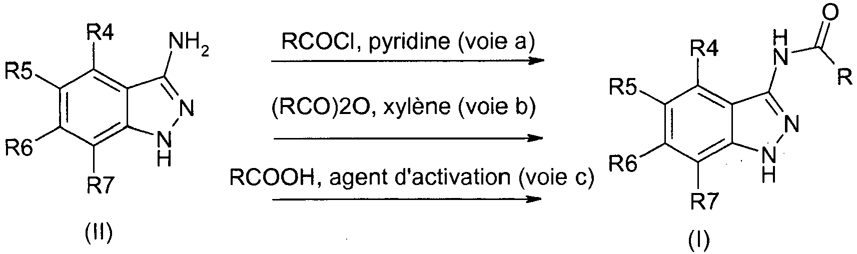

Par la voie (a) la réaction s'effectue en présence d'une base comme la pyridine, la triéthylamine, la diisopropyléthylamine ; la réaction peut débuter à 0°C et lorsque l'addition du chlorure d'acide est terminée on laisse agiter à la température ambiante (G. DAIDONE, Heterocycles, 43, (11), 2385-96, (1996) ou on chauffe si nécessaire.

Par la voie (b) la réaction peut s'effectuer au reflux d'un solvant inerte tel le xylène ou le tétrahydrofuranne (F. ALBERICIO, Synth. Commun., 31, (2), 225-32, (2001)) ou le dichlorométhane, (G. PROCTER, Tetrahedron, 51, (47), 12837-842, (1995)) ou dans l'anhydride lui-même.

Par la voie (c) la réaction s'effectue en présence d'un agent d'activation type carbodiimide seul (DCC, EDAC) (M. C. DESAI, Tetrahedron Lett., 34, 7685, (1993)) ou en présence d'hydroxybenzotriazole et de diméthylaminopyridine (J. P. GAMET, Tetrahedron, 40, 1995, (1984), K. BARLOS, J. Org. Chem., 50, 696, (1985)) ou selon les méthodes bien connues de couplage de la chimie peptidique (M. BODANSZKY, Principles of Peptide Synthesis ; Springer-Verlag, New York, NY, pages 9-58, (1984)) ou de formation de la liaison amide.

Lorsque pour (I) R3 comporte sur le carbone terminal un acide, ce dernier peut-être obtenu par condensation d'un anhydride cyclique tel l'anhydride maléique, succinique, phtalique, ou par condensation d'un chlorure d'acide ester puis saponification de ce dernier, selon le schéma suivant :

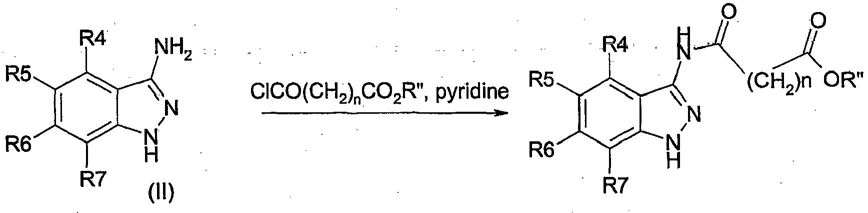

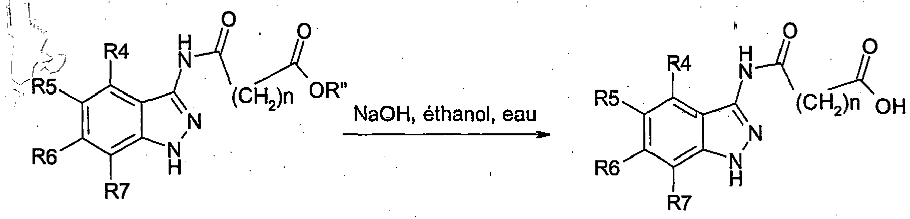

La fonction ester peut être réduite par les méthodes connues de l'homme de métier tel le tetrahydroborate de sodium dans le méthanol ou l'hydrure de lithium aluminium dans le dioxane ou le THF pour donner l'alcool correspondant selon le schéma suivant:

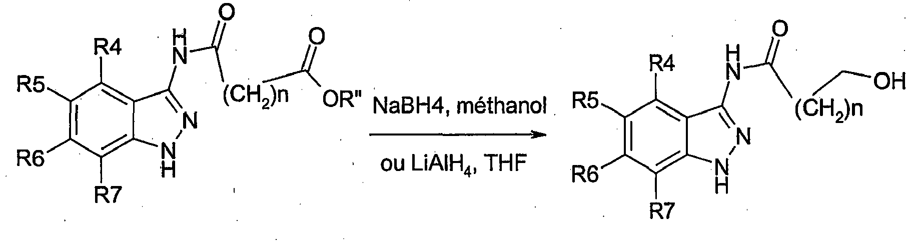

Pour les dérivés de formule (I) et pour lesquels R=S, ces derniers sont obtenus par thionation des dérivés oxo correspondants à l'aide du réactif de Lawesson (R. OLSSON, Tetrahedron Lett., 41, (41), 7947-50, (2000)) ou par traitement à l'aide du pentasulfure de phosphore dans la pyridine ou le toluène (J. VOSS, Justus Liebig Ann. Chem., 716, 209, (1968) ; O TSUGE, Chem. Lett., 1369, (1980)).

Les dérivés de formule (I) pour lesquels R=NH peuvent être obtenus par réaction des 3-amino 1H-indazoles avec un nitrile ou avec un sel de Merweein (S. PATAI, The Chemistry of amidines and imidates, J. Wiley and Sons, (1975), page 283).

Les 3-amino 1H-indazoles de formule (II) peuvent être obtenus par réaction d'un 2-fluorobenzonitrile avec de l'hydrazine, hydrate ou chlorhydrate au reflux de 2 à 18 heures dans un alcool type éthanol ou n-butanol selon (R.F. KALTENBACH, Bioorg. Med. Chem. Lett., 9,(15), 2259-62, (1999)):

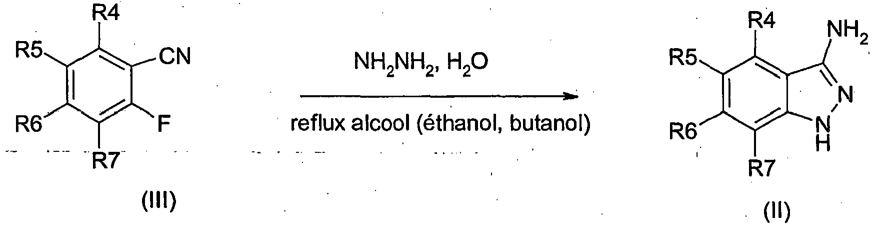

Pour les composés pour lesquels R4, R5, R6 et R7 sont indépendamment l'un de l'autre choisis parmi les radicaux suivant hydrogène, halogène, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, cycloalkyle, alkényle, alkynyle, adamantyle; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy ; peuvent être obtenus par des réactions mettant en jeu la chimie du palladium : Suzuki, (A. SUZUKI, Pure Appl. Chem. 63, 419-22, (1991), Stille (J.. STILLE, Angew. Chem. Int. Ed. 25, 508-24, (1986), Heck, (R. F. HECK, Org. React., 27, 345-90, (1982), Sonogashira, (K. SONOGASHIRA, Synthesis 777, (1977), Buckwald (S.L. BUCKWALD, Acc. Chem.Re., 31, 805, (1998) à partir des dérivés halogénés correspondants.

Pour cela il est nécessaire de protéger les fonctions réactives. Ainsi, les fonctions OH, SH, COOH, NH2 doivent être protégées avant de faire le couplage. Les groupements protecteurs sont introduits selon toutes les méthodes connues de l'homme de l'art et notamment celles décrites par T.W. GREENE, Protective groups in Organic Synthesis, J. Wiley-Interscience Publication (1991). Il est préférable de protéger l'azote en position 1 par des groupements tels que le *tert*-butoxycarbonyle ou des dérivés siliciés. On choisira de préférence un groupement silylé *tert-*butyldiméthylsilyle, triisopropylsilyle qui peuvent être éliminés par les anions fluorure ou avec l'acide acétique et plus particulièrement un groupement triméthylsilyléthoxyméthyle clivable par le fluorure de tétrabutylammonium au reflux dans des solvants tels que le tétrahydrofurane, le dioxane. (J. P. WHITTEN, J. Org. Chem., 51, 1891, (1986) ; B. H. LIPSHUTZ, Tetrahedron Lett., 4095, (1986)).

Les dérivés protégés en 1 par triméthylsilyléthoxyméthyle sont obtenus en faisant réagir le composés de départ avec le chlorure de triméthylsilyléthoxyméthyle en présence d'hydrure de sodium dans un solvant tel que le diméhylformamide à température ambiante (J. P. WHITTEN, J. Org. Chem., 51, 1891, (1986) ; M. P. EDWARDS, Tetrahedron, 42, 3723, (1986))

De même, la fonction azote 1-NH de l'indazole sera protégée par des groupements tels tosyle, carbamate, benzyle ou dérivés silylés. Par exemple dans le cas où l'on voudrait pratiquer un couplage au palladium sur un dérivé halogéné en position 6, il faudra protéger l'azote en position 1 comme montré ci-dessous (X = Cl, Br, I) :

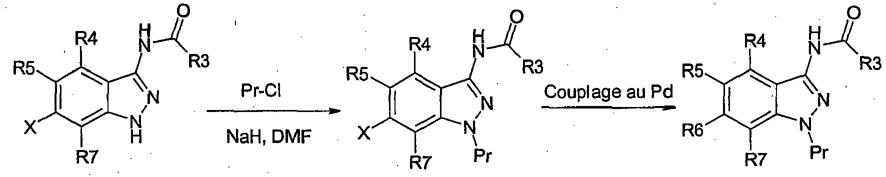

La déprotection s'effectue selon des méthodes connues par l'homme du métier et décrites par T.W. GREENE, Protective groups in Organic Synthesis, J. Wiley-Interscience Publication (1991). Par exemple, si le groupement protecteur en position 1 est un triméthylsilyléthoxyméthyle il pourra être déprotégé par réaction avec le fluorure de tétrabutylammonium comme montré ci-dessous:

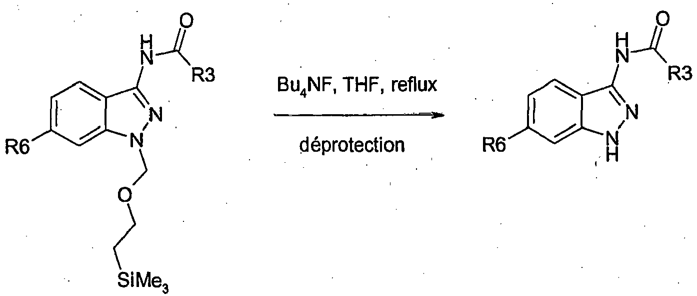

Lorsque l'un des groupements R4, R5, R6 ou R7 engagé pour le couplage utilisant la chimie du palladium contient lui-même une fonction réactive telle hydroxyle, amine, thiol, acide ou de manière générale renferme un hétéroatome, il est nécessaire de protéger ces dernières également avant d'effectuer le couplage au palladium. Ainsi par exemple une fonction phénol sera introduite sous la forme protégée (O-benzyle par exemple) à partir du dérivé chloré et l'azote en 1 étant protégé comme explicité auparavant :

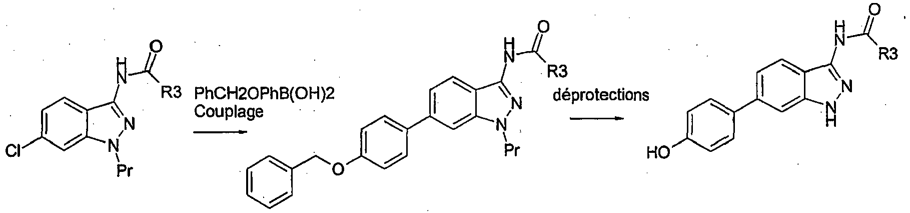

Le groupement benzyle sera ensuite éliminé par exemple par traitement à l'iodure de triméthylsilyle au reflux dans l'acétonitrile. La protection pourra également être réalisé par un groupement triméthylsilyléthoxyméthyle clivable par le fluorure de tétrabutylammonium au reflux dans des solvants tels que le tetrahydrofurane, le dioxane. (J. P. WHITTEN, J. Org. Chem., 51, 1891, (1986); B. H. LIPSHUTZ, Tetrahedron Lett., 4095, (1986)).

Certains dérivés peuvent subir des réactions de substitution électrophile, nitration, halogénation, acylation Friedel et Crafts.

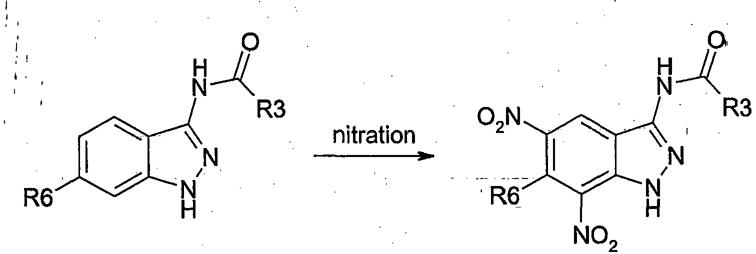

Par exemple la nitration des dérivés substitués en 6 (telle que décrite ci-dessus) peut être faite par des méthodes bien connues tel que l'acide nitrique dans l'acide acétique ou le tetrafluoroborate de nitronium dans des solvants tel l'acétonitrile, (J. L. DUFFY, J. Org. Chem., 56, 3006-09, (1991)). Bien entendu la fonction nitro peut être réduite par l'hydrogène en présence de palladium (B. BARAGATTI, Eur. J. Med, 35,(10), 949-55, (2000)), ou avec le chlorure stanneux en présence d'acide chlorhydrique, (R. P. DIXON, Org. Prep. Proced. Int., 32, (6), 573-77, (2000)), avec le sulfate ferreux en présence d'ammoniaque (S. CASTELLANO, J. Heterocycl. Chem., 37, (6), 1539-42, (2000)). La fonction amine ainsi libérée peut être acylée ou subir une diazotation pour conduire à des réaction de Sandmeyer-Gatterman (substitution par Cl, Br, I, CN, RS, OH,) (H.H. HODGSON, Chem. Rev., 40, 251-77, (1947) ; T. SUGAYA, Synthesis, 73-76, (1994) ; les dérivés diazonium (N. SUZUKI, J. Chem. Soc. Perkin Tr., 645, (1987)) ou les dérivés halogénés obtenus pouvant à nouveau donner lieu, comme précédement, à des réactions mettant en jeu la chimie du palladium.

Les composés de formule (II) de 3-amino-indazole dans laquelle : 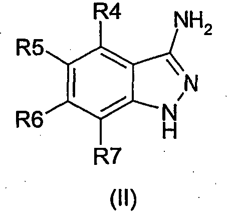
R4, R5, R6 et R7 sont indépendamment l'un de l'autre choisis parmi les radicaux suivant hydrogène, halogène, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO2NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle, adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO2NR10R11, -O-SO₂R10, -SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
leurs isomères, leurs mélanges, leurs racémiques énantiomères, diastéréoisomères, tautomères sont utiles comme intermédiaire pour la préparation de dérivés de formule générale (I).

Les composés de formule générale (II) ou leur sel pharmaceutiquement acceptable peuvent également être utilisés pour préparer un médicament et des compositions pharmaceutiques pour les mêmes indications queles composés de formule (I).

On peut citer parmi les composés de formule (II) les produits suivant :
3-amino-5-bromo-1H-indazole
3-amino-6-bromo-1H-indazole
3-amino-5-méthyl-1H-indazole
3-amino-6-(trifluorométhyl)-1H-indazole
3-amino-5-(trifluorométhyl)-1H-indazole
3-amino-4-chloro-1H-indazole
3-amino-5-nitro-1H-indazole
3-amino-6-(3-pyridinyl)-1H-indazole
3-amino-4-iodo-1H-indazole
3-amino-6-phenyl-1H-indazole
3-amino-6-bromo-5,7-dinitro-1H-indazole
3-amino-6-bromo-7-nitro-1H-indazole
3-amino-6-bromo-5-nitro-1H-indazole
3-amino-6-(furan-3-yl)-1H-indazole
3-amino-6-[4-(phenylmethoxy)phenyl]-1H-indazole
3-amino-6-(4-hydroxy-phenyl)-1H-indazole
3-amino-6-(3,5-difluorophenyl)-1H-indazole
3-amino-6-(3-thienyl)-1H-indazole
3-amino-5-[[(3-fluorophenyl)sulfonyl]amino]-1H-indazole
3-amino-6-(2-phényléthyl)-1H-indazole
3-amino-6,7-difluoro-1H-indazole
3-amino-6-(4-méthoxyphényl)-1H-indazole
3-amino-6-(4-méthylthiophényl)-1H-indazole
3-amino-6-(4-trifluorométhoxyphényl)-1H-indazole
3-amino-(6-(1-propènyl)-1H-indazole
3-amino-6-(4-fluorophényl)-1H-indazole
3-amino-6-[4-(1,1-diméthyléthyl)phényl]-1H-indazole
3-amino-6-bromo-7-amino-1H-indazole
3-amino-6-(4-méthylphényl)-1H-indazole
3-amino-6-(3,5-dichlorophényl)-1H-indazole
3-amino-6-(4-chlorophényl)-1H-indazole
3-amino-6-(4-éthylphényl)-1H-indazole
3-amino-6-(4-pyridinyl)-1H-indazole
3-amino-5-amino-1H-indazole
3-amino-5-bromo-6-chloro-1H-indazole
3-amino-5-phényl-6-chloro-1H-indazole
3-amino-5-bromo-6-[4-(phénylméthoxy)phényl]-1H-indazole
3-amino-5-bromo-6-(4-hydroxyphényl)-1H-indazole
3-amino-6-(4-nitrophényl)-1H-indazole
3-amino-6-(2-chlorophényl)-1H-indazole
3-amino-6-[3-(phénylméthoxy)phényl]-1H-indazole
3-amino-6-(3-hydroxyphényl)-1H-indazole
3-amino-6-chloro-5-(4-pyridinyl)-1H-indazole
3-amino-6-chloro-5-(3-furanyl)-1H-indazole
3-amino-6-[2-chloro-4-(phénylméthoxy)-phényl]-1H-indazole
3-amino-6-(2-chloro-4-hydroxyphényl)-1H-indazole
3-amino-5,6-dibromo-1H-indazole
3-amino-6-chloro-5-(4-fluorophényl)-1H-indazole
3-amino-6-(4-aminophényl)-1H-indazole
3-amino-6-[4-(diméthylamino)phényl]-1H-indazole
3-amino-6-chloro-1H-indazole
3-amino-5,6-diphényl-1H-indazole
3-amino-6-chloro-5-(4-méthylphényl)-1H-indazole
3-amino-5-phényl-6-[4-(phénylméthoxy)phényl]-1H-indazole
3-amino-5-phényl-6-(4-hydroxyphényl)-1H-indazole
3-amino-5-(4-aminophényl)-6-chloro-1H-indazole
3-amino-6-chloro-5-(4-éthylphényl)-1H-indazole
3-amino-6-chloro-5-[4-(phénylméthoxy)phényl]-1H-indazole
3-amino-6-chloro-5-(4-hydroxyphényl)-1H-indazole
3-amino-5,6-bis[4-(phénylméthoxy)phényl]-1H-indazole
3-amino-5,6-bis(4-hydroxyphényl)-1H-indazole
3-amino-5-(3-furanyl)-6-[4-(phénylméthoxy)phényl]-1H-indazole
3-amino-5-(3-furanyl)-6-([4-hydroxyphényl)-1H-indazole
3-amino-5-(4-éthylphényl)-6-[4-(phénylméthoxy)phényl]-1H-indazole
3-amino-5-(4-éthylphényl)-6-(4-hydroxyphényl)-1H-indazole
3-amino-5-(3-pyridinyl)-6-[4-(phénylméthoxy)phényl]-1H-indazole
3-amino-5-(3-pyridinyl)-6-(4-hydroxyphényl)-1H-indazole
3-amino-5-(2-furanyl)-6-[4-(phénylméthoxy)phényl]-1H-indazole
3-amino-5-(2-furanyl)-6-(4-hydroxyphényl)-1H-indazole
3-amino-5-bromo-6-chloro-7-nitro-1H-indazole
3-amino-5-bromo-6,7-difluoro-1H-indazole
3-amino-6-(4-cyanophényl)-1H-indazole
3-amino-6,7-difluoro-5-nitro-1H-indazole
3-amino-6,7-difluoro-5-phényl-1H-indazole
3-amino-6-(6-hydroxy-pyridin-3-yl)-1H-indazole
3-amino-6-(3,4-dihydroxy-phényl)-1H-indazole
3-amino-7-fluoro-5-nitro-6-[2-(phényléthyl)amino]-1H-indazole
3-amino-7-fluoro-5-nitro-6-morpholino-1H-indazole
3-amino-7-fluoro-5-amino-6-morpholino-1H-indazole
3-amino-5-bromo-7-fluoro-6-morpholino-1H-indazole
3-amino-7-fluoro-6-(trifluorométhyl)-1H-indazole
3-amino-6-bromo-4,5,7-trifluoro-1H-indazole
3-amino-6-(6-amino-pyridin-3-yl)-1H-indazole

Les composés de formule (I) sont isolés et peuvent être purifiés par les méthodes connues habituelles, par exemple par cristallisation, chromatographie ou extraction.

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, maléate, iséthionate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de kinase et sont ainsi utiles pour la prévention et le traitement les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, l'hypertension essentielle, les maladies cardiovasculaires athérosclérotiques, le syndrome des ovaires polycystiques, le syndrome X, l'immunodéficience et le cancer.

Leurs activités ont été déterminées en mesurant l'inhibition de la phosphorylation de la protéine tau dans les coupes de cortex de rat adulte.

Les coupes de cortex d'une épaisseur de 300µm sont préparées à partir de rats mâles OFA (Iffa-Credo) âgés de 8-10 semaines, sacrifiés par décapitation. Elles sont incubées dans 5 ml de milieu DMEM contenant du pyruvate et du glucose 4.5 g/l à 37°C pendant 40 min. Les coupes sont ensuite lavées 2 fois avec le milieu, distribuées dans des microtubes (50µl dans 500µl de milieu avec ou sans composés à tester), et incubées à 37°C sous agitation. Deux heures plus tard, l'expérience est arrêtée par centrifugation. Les coupes sont lysées, sonifiées et centrifugées à 18300g, 15 min à 4°C. La concentration en protéines du surnageant est déterminée par un dosage commercial (BCA Protein Assay , Pierce) basé sur la méthode de Lowry.

Les échantillons, dénaturés au préalable 10 min à 70°C, sont séparés sur gel vertical 4-12%Bis-Tris en présence de tampon MOPS-SDS et électrotansferrés sur membrane de nitrocellulose. L'immunomarquage est réalisé par l'anticorps monoclonal AD2 qui reconnaît spécifiquement les épitopes phosphorylés Ser396/404 de la protéine tau. Les protéines immunoréactives sont visualisées par addition d'un deuxième anticorps dirigé contre les IgG de souris et couplé à la peroxydase et d'un substrat chimioluminescent. Les autoradiogrammes obtenus sont enfin quantifiés à l'aide du logiciel 'GeneTools' de Syngene (GeneGnome, Ozyme) pour déterminer une CI50.

Lés composés de formule (I) présentent une activité très intéressante et en particulier certains composés ont une CI50 inférieure à 100 µM.

Les exemples suivants illustrent l'invention de manière non limitative.

### EXEMPLE 1

### Acide (2Z) 4-[(6-chloro-1H-indazol-3-yl)amino]-4-oxo-2-buténoïque

A 1g de 6-chloro-1H-indazole-3-amine dans 30 cm³ d'ortho-xylène, on ajoute 585 mg d'anhydride maléïque préalablement broyé. Le milieu réactionnel est chauffé au reflux à 145°C pendant 10 minutes puis refroidit avec un bain d'eau. L'insoluble est filtré et lavé successivement par 2 fois 25 cm³ d'acétate d'éthyle puis avec 2 fois 25 cm³ d'éther diisopropylique. Le solide est ensuite séché sous pression réduite (90 Pa ; 50°C) pour donner 1g d'acide 4-[(6-chloro-1H-indazol-3-yl)amino]-4-oxo-2-buténoïque (forme Z) sous forme de cristaux jaunes fondant à 230°C.

Spectre de R.M.N. ¹H (300 MHz, (CD₃)₂SO d6, δ en ppm) : 6,38 (d, J = 12 Hz : 1H) ; 6,60 (d, J = 12 Hz : 1H) ; 7,13 (dd, J = 9 et 1,5 Hz : 1H) ; 7,55 (d, J = 1,5 Hz : 1H) ; 7,94 (d, J = 9 Hz : 1H) ; 10,99 (s large : 1H) ; de 12,60 à 13,40 (mf étalé : 1H) ;.12,92 (mf : 1H).

### EXEMPLE 2

### (2E)4-[(6-chloro-1H-indazol-3-yl)amino]-4-oxo-2-buténoate d'éthyle

A 1g de 6-chloro-1H-indazole-3-amine dans 50 cm³ de dichlorométhane, on ajoute 865 mg de fumarate monoéthylique. On introduit ensuite 1.4g de chlorhydrate de 1-(3-dimethylaminopropyl)-3-ethylcarbodiimide et on agite pendant 30 minutes vers 20°C. On lave avec 50 cm³ d'eau distillée puis avec 50 cm³ de solution aqueuse saturée en chlorure de sodium. La phase organique est séchée sur sulfate de magnésium, filtrée puis évaporée sous pression réduite (2 kPa ; 40°C) . On obtient 2g d'une masse gommeuse couleur brique que l'on purifie par chromatographie sous pression d'argon de 50 kPa, sur une colonne de gel de silice (granulométrie 40-60µm ; diamètre 4.5 cm), en éluant par un mélange cyclohexane-acétate d'éthyle (90/10 en volumes) et en recueillant des fractions de 40 cm³. Les fractions contenant le produit attendu sont réunies puis évaporées sous pression réduite (2 kPa) à une température voisine de 40°C. On obtient après séchage (90 Pa ; 45°C), 900 mg de 4-[(6-chloro-1H-indazol-3-yl)amino]-4-oxo-2-buténoate d'éthyle , (forme E), fondant à 220°C.

Spectre de R.M.N. ¹H (300 MHz, (CD₃)₂SO d6, δ en ppm) : de 2,50 à 2,75 (mt : 4H) ; 7,07 (dd, J = 8,5 et 1,5 Hz : 1H) ; 7,52 (d, J = 1,5 Hz : 1H) ; 7,83 (d, J = 8,5 Hz : 1H) ; 11,50 (s large : 1H) ; 12,19 (mf étalé : 1H) ; 12,75 (mf : 1H).

### EXEMPLE 3

### Acide 4-[(6-chloro-1H-indazol-3-yl)amino]-4-oxo-2-butanoïque

A 500 mg de 6-chloro-1H-indazole-3-amine dans 30 cm³ d'ortho-xylène on ajoute 300 mg d'anhydride succinique. On porte le milieu réactionnel au reflux vers 145°C pendant 16 heures puis on stoppe le chauffage et on laisse refroidir jusqu'à température ambiante vers 20°C. Le milieu réactionnel est ensuite filtré sur verre fritté ; le solide est repris par 20 cm³ d'acétate d'éthyle et par 30 cm³ d'une solution à 10% d'hydrogénocarbonate de sodium. La phase organique est séchée sur sulfate de magnésium, filtrée puis évaporée selon les conditions déjà décrites. Les cristaux blancs ainsi obtenus sont agités avec 30 cm³ d'une solution à 10 % d'hydrogénocarbonate de sodium pendant 20 minutes. Un léger insoluble est éliminé par filtration et le filtrat est acidifié par de l'acide chlorhydrique 12N ; le précipité apparu est lavé par 2 fois 10 cm³ d'eau distillée, par une fois 5 cm³ d'acétone et par 2 fois 10 cm³ d'éther diisopropylique. Le solide est ensuite séché sous pression réduite vers 40°C puis purifié par chromatographie sous pression d'argon de 50 kPa, sur une colonne de gel de silice (granulométrie 40-60 µm ; diamètre 2 cm), en éluant par un mélange dichlorométhane-méthanol (99/1 en volumes) et en recueillant des fractions de 20 cm³. Les fractions contenant le produit attendu sont réunies puis évaporées selon les conditions décrites précédemment. Le produit obtenu est repris par 10 cm³ d'acétate d'éthyle, filtré sur verre fritté et rincé avec 2 fois 5 cm³ d'acétate d'éthyle puis avec 20 cm³ d'éther diéthylique. On sèche sous pression réduite pendant une nuit (90 Pa; 40°C) et on obtient ainsi 110 mg d'acide d'acide 4-[(6-chloro-1H-indazol-3-yl)amino]-4-oxo-2-butanoïque sous forme d'un solide blanc fondant à 200°C.

Spectre de R.M.N. ¹H (300 MHz, (CD₃)₂SO d6, δ en ppm) : de 2,50 à 2,75 (mt : 4H) ; 7,07 (dd, J = 8,5 et 1,5 Hz : 1H) ; 7,52 (d, J = 1,5 Hz : 1H) ; 7,83 (d, J = 8,5 Hz : 1H) ; 11,50 (s large : 1H) ; 12,19 (mf étalé: 1H) ; 12,75 (mf : 1H).

### EXEMPLE 4

### Acide (2Z) 4-[(5-bromo-1H-indazol-3-yl)amino]-4-oxo-2-buténoïque

A 500 mg de 5-bromo-1H-indazole-3-amine préparé comme décrit dans le brevet U.S. 3 133 081, dans 20 cm³ de toluène, on ajoute 350 mg d'anhydride maléïque. Le milieu est porté au reflux vers 110°C pendant 1 heure. On arrête ensuite le chauffage et on agite vers 19 °C pendant 12 heures. Le précipité apparu est filtré sur verre fritté et rincé avec 20 cm³ d'éther diisopropylique, 2 cm³ d'acétate d'éthyle et 2 cm³ de dichlorométhane. On obtient après séchage (90 Pa ; 45°C) 448 mg d' acide 4-[(5-bromo-1H-indazol-3-yl)amino]-4-oxo-2-buténoïque, forme Z, sous forme de solide jaune fondant à 172°C.

Spectre de R.M.N. ¹H (400 MHz, (CD₃)₂SO d6, δ en ppm) : 6,38 (d, J = 12 Hz : 1H) ; 6,62 (d, J = 12 Hz : 1H) ; 7,49 (mt : 2H) ; 8,15 (s large : 1H) ; 10,95 (s large : 1H) ; de 12,70 à 13,30 (mf étalé : 1H) ; 12,98 (mf : 1H).

### EXEMPLE 5

### Acide (2E) 4-[(6-chloro-1H-indazol-3-yl)amino]-4-oxo-2-buténoïque

A 280 mg de (2E) 4-[(6-chloro-1H-indazol-3-yl)amino]-4-oxo-2-buténoate d'éthyle, décrit à l'exemple 2, dans 25 cm³ d'éthanol, on ajoute 0.95 cm³ de soude 1N. Le milieu réactionnel est ensuite chauffé à 50°C pendant 2 heures puis on ajoute encore 1 équivalent de soude 1N. On maintient la température à 50°C 30 minutes de plus et on arrête le chauffage. Vers 20°C, le milieu est neutralisé avec de l'acide chlorhydrique 1N puis concentré sous pression réduite (2 kPa ; 40°C). Le solide ainsi obtenu est repris par 25 cm³ de tétrahydrofuranne, par 50 cm³ d'acétate d'éthyle et par 25 cm³ d'eau distillée. La phase organique est lavée avec 30 cm³ d'une solution aqueuse saturée en chlorure de sodium puis séchée sur sulfate de magnésium. On filtre et on évapore dans les conditions décrites précédemment. Le résidu est repris par 10 cm³ d'acétate d'éthyle, on filtre l'insoluble puis on rince avec 5 cm³ d'acétate d'éthyle et avec 10 cm³ d'éther diéthylique et on sèche sous pression réduite (90 Pa ; 50°C). On obtient 155 mg d'acide 4-[(6-chloro-1H-indazol-3-yl)amino]-4-oxo-2-buténoïque (forme E) sous forme d'un solide jaune clair fondant à 260°C.

Spectre de R.M.N. ¹H (300 MHz, (CD₃)₂SO d6, δ en ppm) : 6,75 (d, J = 15,5 Hz : 1H) ; 7,11 (dd, J = 9 et 2 Hz : 1H) ; 7,27 (d, J = 15,5 Hz : 1H) ; 7,55 (d, J = 2 Hz : 1H) ; 7,96 (d, J = 9 Hz : 1H) ; 11,13 (s large : 1H) ; de 12,40 à 13, 10 (mf étalé : 1H) ; 12,94 (mf : 1H).

### EXEMPLE 6

### Acide 4-[(5-bromo-1H-indazol-3-yl)amino]-4-oxo-2-butanoïque

A 500 mg de 5-bromo-1H-indazole-3-amine préparé comme décrit dans le brevet U.S. 3 133 081, dans 20 cm³ de toluène, on ajoute 354 mg d'anhydride succinique. Le milieu réactionnel est porté au reflux vers 110°C pendant 13 heures. Le précipité est filtré puis rincé avec 10 cm³ d'éther diisopropylique et 10 cm³ de dichlorométhane. Le produit est repris par 20 cm³ de solution aqueuse saturée en hydrogénocarbonate de sodium et on acidifie avec de l'acide chlorhydrique 5N jusqu'à pH 9/10. On filtre le précipité formé et rince avec 20 cm³ d'eau distillée puis on reprend le solide par 20 cm³ d'acétone. On évapore à sec sous pression réduite (2 kPa ; 40°C) et on obtient après séchage (90 Pa; 45°C) 270 mg d'acide 4-[(5-bromo-1H-indazol-3-yl)amino]-4-oxo-2-butanoïque sous forme d'un solide blanc fondant vers 173°C.

Spectre de R.M.N. ¹H (300 MHz, (CD₃)₂SO d6, δ en ppm) : de 2,50 à 2,75 (mt : 4H) ; 7,45 (s large : 2H) ; 8,02 (s large : 1H) ; 10,55 (mf : 1H) ; 12,83 (mf : 1H).

### EXEMPLE 7

### (2E) N-(6-chloro-1H-indazol-3-yl)-2-buténamide

A 50 mg de 6-chloro-1H-indazole-3-amine dissout dans 5 cm³ de pyridine et refroidit vers 6°C on additionne 0.67 cm³ de chlorure de crotonyle distillé. On agite 10 minutes puis on laisse remonter la température vers 19 °C pendant 22 heures. Le milieu réactionnel est ensuite concentré à sec sous pression réduite (2 kPa ; 40°C) puis le résidu est repris par 50 cm³ de tétrahydrofuranne et par 25 cm³ d'acétate d'éthyle. La phase organique est lavée avec 2 fois 50 cm³ d'eau distillée puis avec 50 cm³ de solution aqueuse saturée en chlorure de sodium. On sèche sur sulfate de magnésium, filtre sur verre fritté puis évapore dans les conditions précédemment décrites. Le résidu obtenu est purifié par chromatographie sous pression d'argon de 50 kPa, sur une colonne de gel de silice (granulométrie 40-60 µm ; diamètre 4.5 cm), en éluant par un mélange cyclohexane-acétate d'éthyle (80/20 en volumes) et en recueillant des fractions de 20 cm³. Les fractions contenant le produit attendu sont réunies puis évaporées dans les conditions déjà décrites. On obtient après séchage (90 Pa; 45°C), 100 mg de N-(6-chloro-1H-indazol-3-yl)-2-buténamide, forme E, sous forme d'un solide blanc fondant à 226 °C.

Spectre de R.M.N. ¹H (300 MHz, (CD₃)₂SO d6, δ en ppm) : 1,90 (d large, J = 7 Hz : 3H) ; 6,27 (dd, J = 15 et 1,5 Hz : 1H) ; 6,88 (dq, J = 15 et 7 Hz : 1H) ; 7,08 (dd, J = 9 et 2 Hz : 1H) ; 7,52 (d, J = 2 Hz : 1H) ; 7,92 (d, J = 9 Hz : 1H) ; 10,53 (mf : 1H) ; 12,80 (mf : 1H).

### EXEMPLE 8

### 6-chloro-1-[(1,1-dimethylethoxy)carbonyl]-1H-indazole-3-amine

A 1g de 6-chloro-1H-indazole-3-amine dans 30 cm³ de dichlorométhane, on ajoute 1.3 g de ditertiobutyle-dicarbonate et 10 mg de diméthylaminopyridine. On agite 17 heures vers 19°C. Le milieu réactionnel est évaporé à sec selon les conditions déjà décrites, puis le résidu est purifié par chromatographie sous pression d'argon de 50 kPa, sur une colonne de gel de silice (granulométrie 40-60 µm ; diamètre 4cm), en éluant par un mélange cyclohexane-acétate d'éthyle (90/10 en volumes). Les fractions contenant le produit attendu sont réunies et évaporées sous pression réduites (2 kPa, 50 °C). On obtient après séchage (90 Pa; 45°C), 1.2 g 6-chloro-1-[(1,1-dimethylethoxy)carbonyl]-1H-indazole-3-amine, produit sous forme d'un solide blanc.

Spectre de R.M.N. ¹H (400 MHz, (CD₃)₂SO d6, δ en ppm) : 1,61 (s : 9H) ; 6,43 (s large : 2H) ; 7,36 (dd, J = 9 et 1,5 Hz : 1H) ; 7,89 (d, J = 9 Hz : 1H) ; 7,97 (s large : 1H).

### N-(6-chloro-1-[(1,1-dimethylethoxy)carbonyl]-1H-indazol-3-yl)-3-buténamide

A 1 g de 6-chloro-1-[(1,1-dimethylethoxy)carbonyl]-1H-indazole-3-amine précédemment décrit, dans 40 cm³ de dichlorométhane et 1.05 cm³ de triéthylamine, on ajoute 0.45 cm³ de chlorure de crotonyle préalablement distillé. On laisse agiter vers 19°C pendant 16 heures. Le milieu est ensuite concentré sous pression réduite (20 kPa ; 40°C) . Le résidu est repris par 100 cm³ d'acétate d'éthyle et par 50 cm³ d'eau distillée. On lave ensuite la phase organique avec 50 cm³ d'une solution aqueuse saturée en chlorure de sodium. On sèche sur sulfate de magnésium, on filtre sur verre fritté puis on évapore dans les conditions déjà décrites précédemment. Le résidu est purifié par chromatographie sous pression d'argon de 50 kPa, sur gel de silice (granulométrie 40/60 µm; diamètre 3 cm), en éluant par un mélange cyclohexane-acétate d'éthyle (90/10 en volumes) et en recueillant des fractions de 15 cm³. Les fractions contenant le produit attendu sont réunies puis évaporées sous pression réduite (2 kPa ; 40°C). On obtient après séchage (90 Pa ; 45°C) 120 mg de N-(6-chloro-1-[(1,1-dimethylethoxy)carbonyl]-1H-indazol-3-yl)-3-buténamide sous forme d'un solide jaune.

Spectre de R.M.N. ¹H (300 MHz, (CD₃)₂SO d6, δ en ppm) : 1,66 (s : 9H) ; 3,28 (d large , J = 7,5 Hz : 2H) ; 5,20 (dd, J = 10,5 et 1,5 Hz : 1H) ; 5,26 (dd, J = 17 et 1,5 Hz : 1H) ; 6,02 (mt : 1H) ; 7,43 (dd, J = 9 et 2 Hz : 1H) ; 8,05 (d, J = 9 Hz : 1H) ; 8,12 (d, J = 2 Hz : 1H) ; 11,09 (mf : 1H).

### N-(6-chloro-1H-indazol-3-yl)-3-buténamide, chlorhydrate

A 240 mg de N-(6-chloro-1-[(1,1-dimethylethoxy)carbonyl]-1H-indazol-3-yl)-3-buténamide précédemment décrit, on ajoute 10 cm³ de dioxane chlorhydrique 4N. On agite vers 19°C pendant 17 heures. Le produit cristallisé est filtré sur verre fritté , rincé par 2 fois 5 cm³ d'acétate d'éthyle et par 2 fois 5 cm³ d'éther diéthylique puis séché sous pression réduite (90 Pa ; 40°C). On obtient ainsi 125 mg de N-(6-chloro-1H-indazol-3-yl)-3-buténamide sous forme de chlorhydrate et fondant à 150°C.

Spectre de R.M.N. ¹H (300 MHz, (CD₃)₂SO d6, δ en ppm) : 3,22 (d, J = 7 Hz : 2H) ; 5,17 (d large, J = 10,5 Hz : 1H) ; 5,23 (d large, J = 18 Hz : 1H) ; de 5,30 à 6,80 (mf étalé : 2H) ; 6,02 (mt : 1H) ; 7,07 (dd, J = 9 et 2 Hz : 1H) ; 7,52 (d, J = 2 Hz : 1H) ; 7,82 (d, J = 9 Hz : 1H) ; 10,50 (s large : 1H).

### EXEMPLE 9

### 4-[(6-chloro-1H-indazol-3-yl)amino]-4-oxo-2-butanoate de méthyle

A 3,5g de 4-chloro-4-oxo-butanoate de méthyle, dans 10 cm³ de dichlorométhane, on ajoute 4g de 6-chloro-1H-indazole-3-amine dans 40 cm³ de pyridine à 5 °C. On laisse revenir à 19°C pendant 19 heures. Le milieu réactionnel est évaporé dans les conditions précédemment décrites. Le résidu est repris par 75 cm³ de tétrahydrofuranne et par 75 cm³ d'acétate d'éthyle. On lave avec 3 fois 50 cm³ d'eau distillée. On sèche sur sulfate de magnésium, filtre sur verre fritté et évapore sous pression réduite (2 kPa ; 40°C). Le produit est purifié par chromatographie sous pression d'argon de 50 kPa , sur une colonne de gel de silice (granulométrie 40-60 µm ; diamètre 6 cm), en éluant par un mélange cyclohexane-acétate d'éthyle (70/30 en volumes) et en recueillant des fractions de 50 cm³. Les fractions contenant le produit attendu sont réunies puis évaporées sous pression réduite (2 kPa ; 40°C). On obtient après séchage (90 Pa ; 45°C), 3g de 4-[(6-chloro-1H-indazol-3-yl)amino]-4-oxo-2-butanoate de méthyle, sous forme de solide blanc fondant à 170°C.

Spectre de R.M.N. ¹H (300 MHz, (CD₃)₂SO d6, δ en ppm) : de 2,60 à 2,80 (mt : 4H) ; 3,63 (s : 3H) ; 7,08 (dd, J = 9 et 2 Hz : 1H) ; 7,52 (d, J = 2 Hz : 1H) ; 7,82 (d, J = 9 Hz : 1H) ; 10,52 (mf : 1H) ; 12,77 (mf étalé : 1H).

### EXEMPLE 10

### N-(6-chloro-1H-indazol-3-yl)-acétamide

A 750 mg de 6-chloro-1H-indazole-3-amine dans 10 cm³ de pyridine, on ajoute 0.32 cm³ de chlorure d'acétyle préalablement distillé après avoir refroidit le milieu réactionnel vers 3°C. Puis on laisse ensuite revenir le milieu à 19 °C pendant 48 heures. Le milieu réactionnel est évaporé à sec sous pression réduite (2 kPa ; 40°C). Le résidu est repris par 75 cm³ d'acétate d'éthyle et par 50 cm³ d'eau distillée. La phase organique est relavée avec 50 cm³ d'eau distillée puis séchée sur sulfate de magnésium, filtrée sur verre fritté et évaporée sous pression réduite. Le résidu obtenu est purifié par chromatographie sous pression d'argon de 50 kPa, sur une colonne de gel de silice (granulométrie 40-60 µm ; diamètre 4 cm), en éluant par un mélange cyclohexane-acétate d'éthyle (60/40 en volumes) et en recueillant des fractions de 35 cm³. Les fractions contenant le produit attendu sont réunies et évaporées sous pression réduite (2 kPa ; 40°C). On obtient après séchage (90 Pa ; 45°C), 700 mg de N-(6-chloro-1H-indazol-3-yl)-acétamide, fondant à 240°C.

Spectre de R.M.N. ¹H (300 MHz, (CD₃)₂SO d6, δ en ppm) : 2,13 (s : 3H) ; 7,08 (dd, J = 9 et 2 Hz : 1H); 7,52 (d, J = 2 Hz : 1H); 7,86 (d, J = 9 Hz : 1H); 10,45 (mf : 1H) ; de 12,50 à 13,10 (mf étalé : 1H).

### EXEMPLE 11

### N-(6-chloro-1H-indazol-3-yl)-butanamide

A 750 mg de 6-chloro-1H-indazole-3-amine dans 10 cm³ de pyridine, on ajoute 0.47 cm³ de chlorure de butyryle, après avoir refroidit le milieu réactionnel vers 3°C. Puis on laisse ensuite revenir le milieu à 19 °C pendant 14 heures. Le milieu réactionnel est évaporé à sec sous pression réduite (2 kPa ; 40°C). Le résidu est repris par 50 cm³ d'acétate d'éthyle, par 50 cm³ de tétrahydrofurane et par 50 cm³ d'eau distillée. La phase organique est relavée avec 50 cm³ d'eau distillée et avec 50 cm³ d'une solution aqueuse saturée en chlorure de sodium puis séchée sur sulfate de magnésium, filtrée sur verre fritté et évaporée sous pression réduite. Le résidu obtenu est purifié par chromatographie sous une pression d'argon de 50 kPa, sur une colonne de gel de silice (granulométrie 40-60 µm ; diamètre 2.5 cm), en éluant par cyclohexane-acétate d'éthyle (70/30 en volumes) et en recueillant des fractions de 25 cm³. Les fractions contenant le produit attendu sont réunies puis évaporées sous pression réduite (2 kPa ; 40 °C). On obtient après séchage (90 P ; 45°C), 200 mg de N-(6-chloro-1H-indazol-3-yl)-butanamide, sous forme d'un solide blanc fondant à 230°C.

Spectre de R.M.N. ¹H (300 MHz, (CD₃)₂SO d6, δ en ppm) : 0,98 (t, J = 7 Hz : 3H) ; 1,67 (mt : 2H) ; 2,40 (t, J = 7 Hz : 2H) ; 7,08 (dd, J = 9 et 2 Hz : 1H) ; 7,52 (d, J = 2 Hz : 1H) ; 7,84 (d, J = 9 Hz : 1H) ; 10,39 (mf : 1H) ; de 12,50 à 13,00 (mf étalé : 1H).

### EXEMPLE 12

### 6-bromo-1H-indazole-3-amine

A 10 g de 4-bromo-2-fluorobenzonitrile dans 100 cm³ d'éthanol, on additionne 7.3 cm³ d'hydrazine monohydratée. Le milieu est portée au reflux vers 78°C pendant 12 heures. On filtre ensuite le précipité apparu sur verre fritté. On obtient après séchage (90 Pa ; 45°C), 9.7 g de 6-bromo-1H-indazole-3-amine sous forme d'un solide blanc.

Spectre de R.M.N. ¹H (300 MHz, (CD₃)₂SO d6, δ en ppm) : 5,45 (s large : 2H) ; 7,03 (dd, J = 9 et 2 Hz : 1H) ; 7,43 (d, J = 2 Hz : 1H) ; 7,65 (d, J = 9 Hz : 1H) ; 11,50 (mf : 1H).

### (2E) N-(6-bromo-1H-indazol-3-yl)-2-buténamide

A 2 g de 6-bromo-1H-indazole-3-amine préparé précédemment dans 30 cm³ de pyridine, refroidit vers 3°C, on additionne 1.07 cm³ de chlorure de crotonyle. On laisse le milieu revenir vers 19°C pendant 12 heures. On évapore le milieu réactionnel sous pression réduite (2kPa ; 50°C) et on reprend le résidu par 20 cm³ d' acétate d'éthyle et par 20 cm³ d'eau distillée. La phase aqueuse est réextraite avec 20 cm³ d'acétate d'éthyle. Les phases aqueuses sont réunies puis évaporées dans les conditions décrites précédemment. Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (50/50 en volumes) et en recueillant des fractions de 15 cm³. Les fractions contenant le produit attendu, sont réunies et évaporées sous pression réduite (2 kPa ; 50°C). On obtient après séchage (90 Pa; 45°C), 130 mg de N-(6-bromo-1H-indazol-3-yl)-2-buténamide (forme E) sous forme d'un solide beige fondant à 232°C.

Spectre de R.M.N. ¹H (300 MHz, (CD₃)₂SO d6, δ en ppm) : 1,91 (dd, J = 7 et 1,5 Hz : 3H) ; 6,27 (dd, J = 15 et 1,5 Hz : 1H) ; 6,89 (dq, J = 15 et 7 Hz : 1H) ; 7,20 (dd, J = 9 et 2 Hz : 1H) ; 7,68 (d, J = 2 Hz : 1H) ; 7,87 (d, J = 9 Hz : 1H) ; 10,54 (mf: 1H) ; 12,80 (mf étalé : 1H).

### EXEMPLE 13

### (2E) N-(5-méthyl-1H-indazol-3-yl)-2-buténamide

A 560 mg de 5-méthyl-1H-indazole-3-amine préparé comme dans le brevet E.P. 90 9720 dans 30 cm³ de pyridine, on additionne 0.33 cm³ de chlorure de crotonyle. On laisse le milieu revenir vers 19°C pendant 12 heures. On évapore le milieu réactionnel sous pression réduite (2 kPa ; 50°C) et on reprend le résidu par 25 cm³ de tétrahydrofuranne, par 25 cm³ d'acétate d'éthyle et par 25 cm³ d'eau distillée. La phase organique est lavée par 2 fois 25 cm³ d'eau distillée. On sèche sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice (granulomètrie 40-60µm ; diamètre 2.5 cm), en éluant par un mélange cyclohexane-acétate d'éthyle (50/50 en volumes) et en recueillant des fractions de 20 cm³. Les fractions contenant le produit attendu, sont réunies et évaporées sous pression réduite (2 kPa ; 50°C). On obtient après séchage (90 Pa ; 45°C), 50 mg de N-(5-méthyl-1H-indazol-3-yl)-2-buténamide (forme E), sous forme d'un solide blanc fondant vers 218°C.

Spectre de R.M.N. ¹H (300 MHz, (CD₃)₂SO d6, δ en ppm) : 1,90 (d large, J = 7 Hz : 3H) ; 2,38 (s : 3H) ; 6,25 (dd, J = 15 et 1,5 Hz : 1H) ; 6,86 (dq, J = 15 et 7 Hz : 1H) ; 7,17 (dd, J = 9 et 2 Hz : 1H) ; 7,34 (d, J = 9 Hz : 1H) ; 7,56 (s large : 1H) ; 10,31 (mf : 1H) ; 12,52 (mf : 1H).

### EXEMPLE 14

### N-(6-chloro-1H-indazol-3-yl)-2-propanamide

A 750 mg de 6-chloro-1H-indazole-3-amine dans 10 cm³ de pyridine, refroidit vers 3°C, on additionne 0.39 cm³ de chlorure de propionyle. On laisse le milieu revenir vers 19°C pendant 12 heures et on évapore le milieu réactionnel sous pression réduite (2kPa ; 50°C). On reprend le résidu par 40 cm³ de tétrahydrofuranne, par 40 cm³ d'acétate d'éthyle et par 40 cm³ d'eau distillée. La phase organique est lavée par 40 cm³ d'eau distillée et par 40 cm³ d'une solution aqueuse saturée en chlorure de sodium. On sèche sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice (granulométrie 40-60 µm ; diamètre 4 cm), en éluant par un mélange cyclohexane-acétate d'éthyle (70/30 en volumes) et en recueillant des fractions de 35 cm³. Les fractions contenant le produit attendu, sont réunies et évaporées sous pression réduite (2 kPa ; 50°C). Le produit obtenu est repris par 50 cm³ d'éther diéthylique, filtré sur verre fritté puis lavé par 2 fois 10 cm³ d'éther diéthylique. On essore et après séchage (90 Pa ; 45°C), on obtient 440 mg de N-(6-chloro-1H-indazol-3-yl)-2-propanamide, sous forme d'un solide blanc fondant à 210°C.

Spectre de R.M.N. ¹H (300 MHz, (CD₃)₂SO d6, δ en ppm) : 5,66 (s : 2H) ; 7,42 (d, J = 9 Hz : 1H) ; 7,50 (d large, J = 9 Hz : 1H) ; 8,22 (s large : 1H) ; 10,86 (mf : 1H).

### EXEMPLE 15

### (2E) N-[6-(trifluorométhyl)-1H-indazol-3-yl]-2-buténamide

A 500 mg de 6-trifluorométhyl-1H-indazole-3-amine préparé comme décrit dans le brevet U.S. 3 133 081, dans 10 cm³ de pyridine, refroidit vers 10°C, et on additionne 0.23 cm³ de chlorure de crotonyle. On laisse revenir la température vers 19°C pendant 17 heures. On évapore le milieu réactionnel sous pression réduite (2 kPa ; 50°C) et on reprend le résidu par 25 cm³ de tétrahydrofuranne, par 25 cm³ d'acétate d'éthyle et par 25 cm³ d'eau distillée. La phase organique est lavée avec 25 cm³ d'eau distillée. On sèche les phases organiques réunies sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice ( granulométrie 40-60 µm ; diamètre 2 cm), en éluant par un mélange cyclohexaneacétate d'éthyle (70/30 en volumes) et en recueillant des fractions de 30 cm³. Les fractions contenant le produit attendu, sont réunies et évaporées sous pression réduite (2 kPa ; 50°C). Le résidu est repris par 30 cm³ d'éther diisopropylique, puis filtré sur verre fritté. On obtient après séchage (90 Pa; 45°C), 41 mg de N-[6-(trifluorométhyl)-1H-indazol-3-yl]-2-buténamide (forme E), sous forme d'un solide blanc fondant à 208°C.

Spectre de R.M.N. ¹H (300 MHz, (CD₃)₂SO d6, δ en ppm) : 1,91 (dd, J = 7 et 1,5 Hz : 3H) ; 6,29 (dd, J = 15 et 1,5 Hz : 1H) ; 6,91 (dq, J = 15 et 7 Hz : 1H) ; 7,35 (d large, J = 9 Hz : 1H) ; 7,83 (s large : 1H) ; 8,11 (d, J = 9 Hz : 1H) ; 10,65 (mf : 1H) ; de 12,60 à 13,50 (mf étalé : 1H).

### EXEMPLE 16

### 4-[[6-(trifluorométhyl)-1H-indazol-3-yl]amino]-4-oxo-butanoate d'éthyle

A 249 mg de 6-(trifluorométhyl)-1H-indazole-3-amine préparé comme décrit dans le brevet U.S. 3 133 081 dans 10 cm³ de pyridine, refroidit vers 10°C, on additionne 0.23 cm³ de chlorure de crotonyle. On laisse revenir la température vers 19°C pendant 17 heures. On évapore le milieu réactionnel sous pression réduite (2 kPa ; 50°C). On reprend le résidu par 25 cm³ de tétrahydrofuranne, par 25 cm³ d'acétate d'éthyle et par 25 cm³ d'eau distillée. La phase organique est lavée avec 2 fois 25 cm³ d'eau distillée. On sèche les phases organiques réunies sur sulfate de magnésium et on filtre sur verre fritté puis on évapore sous pression réduite (2 kPa ; 50°C). Le résidu est purifié par chromatographie sous pression d'argon de 50 kPa, sur colonne de gel de silice (granulométrie 40-60 µm ; diamètre 2 cm), en éluant par un mélange dichlorométhane-méthanol (98/2 en volumes) et en recueillant des fractions de 30 cm³. Les fractions contenant le produit attendu, sont réunies et évaporées sous pression réduite (2 kPa ; 50°C). On obtient après séchage (90 Pa ; 45°C), 210 mg de 4-[[6-(trifluorométhyl)-1H-indazol-3-yl]amino]-4-oxo-butanoate d'éthyle, sous forme d'un solide blanc fondant à 248°C.

Spectre de R.M.N. ¹H (300 MHz, (CD₃)₂SO d6, δ en ppm) : 1,21 (t, J = 7 Hz : 3H) ; de 2,60 à 2,80 (mt : 4H) ; 4,10 (q, J = 7 Hz : 2H) ; 7,35 (d large, J = 9 Hz : 1H) ; 7,84 (s large : 1H) ; 8,02 (d, J = 9 Hz : 1H) ; 10,61 (mf : 1H) ; de 12,60 à 13,60 (mf étalé : 1H).

### EXEMPLE 17

### (2E) N-[5-(trifluorométhyl)-1H-indazol-3-yl]-2-buténamide

A 500 mg de 5-(trifluorométhyl)-1H-indazole-3-amine préparé selon le brevet U.S. 3 133 081, dans 15 cm³ de pyridine, on additionne 0.24 cm³ de chlorure de crotonyle. On laisse agiter vers 19°C pendant 12 heures et on évapore le milieu réactionnel sous pression réduite (2 kPa; 50°C). On reprend le résidu par 25 cm³ de tétrahydrofuranne, par 25 cm³ d'acétate d'éthyle et par 25 cm³ d'eau distillée. On relave la phase organique avec 25 cm³ d'eau distillée. On sèche la phase organique sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice (granulométrie 40-60µm ; diamètre 2.5 cm), en éluant par un mélange cyclohexane-acétate d'éthyle (60/40 en volumes) et en recueillant des fractions de 25 cm³. Les fractions contenant le produit attendu, sont réunies et évaporées sous pression réduite (2 kPa ; 50°C). On obtient après séchage ( 90 Pa; 45°C), 63 mg de N-[5-(trifluorométhyl)-1H-indazol-3-yl]-2-buténamide (forme E), sous forme d'un solide écru fondant vers 242°C.

Spectre de R.M.N. ¹H (300 MHz, (CD₃)₂SO d6, δ en ppm) : 1,91 (dd, J = 7 et 1,5 Hz : 3H) ; 6,30 (dd, J = 15 et 1,5 Hz : 1H) ; 6,93 (dq, J = 15 et 7 Hz : 1H) ; 7,60 (dd, J = 9 et 2 Hz : 1H) ; 7,66 (d, J = 9 Hz : 1H) ; 8,42 (s large : 1H) ; 10,73 (mf : 1H) ; de 12,90 à 13,40 (mf étalé : 1H).

### EXEMPLE 18

### N-[5-chloro-1H-indazol-3-yl]-2-butanamide

A 500 mg de 5-chloro-1H-indazole-3-amine préparé selon le brevet E.P. 90 972 dans 25 cm³ de pyridine, refroidit vers 5°C, on additionne 0.31 cm³ de chlorure de butyryle. On laisse revenir la température vers 19°C pendant 17 heures et on évapore le milieu réactionnel sous pression réduite (2 kPa ; 50°C). On reprend le résidu par 25 cm³ de tétrahydrofuranne, par 25 cm³ d'acétate d'éthyle et par 25 cm³ d'eau distillée. La phase organique est séchée sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice ( granulométrie 40-60µm ; diamètre 2.5 cm), en éluant par un mélange cyclohexane-acétate d'éthyle (60/40 en volumes) et en recueillant des fractions de 30 cm³. Les fractions contenant le produit attendu, sont réunies et évaporées sous pression réduite (2 kPa ; 50°C). On obtient après séchage (90 Pa ; 45°C), 100 mg de N-[5-chloro-1H-indazol-3-yl]-2-butanamide, sous forme d'un solide blanc fondant à 216°C.

Spectre de R.M.N. ¹H (300 MHz, (CD₃)₂SO d6, δ en ppm) : 0,97 (t, J = 7,5 Hz : 3H) ; 1,68 (mt : 2H) ; 2,40 (t, J = 7,5 Hz : 2H) ; 7,35 (dd, J = 9 et 2 Hz : 1H) ; 7,49 (dd, J = 9 et 0,5 Hz : 1H) ; 7,86 (dd, J = 2 et 0,5 Hz : 1H) ; 10,41 (mf : 1H) ; 12,82 (mf : 1H).

### EXEMPLE 19

### N-[4-chloro-1H-indazol-3-yl]-butanamide

A 1 g de 4-chloro-1H-indazole-3-amine préparé comme décrit dans le brevet E.P. 90 972 dans 10 cm³ de pyridine, refroidit vers 10°C, on additionne 0.23 cm³ de chlorure de butyryle. On laisse revenir la température vers 19°C pendant 17 heures. On évapore le milieu réactionnel sous pression réduite (2 kPa ; 50°C). On reprend le résidu par 25 cm³ d'acétate d'éthyle et par 25 cm³ d'eau distillée. La phase organique est lavée avec 2 fois 25 cm³ d'eau distillée et avec 25 cm³ d'une solution aqueuse saturée en chlorure de sodium. On sèche la phase organique sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice (granulométrie 40-60 µm ; diamètre 4 cm), en éluant par un mélange dichlorométhane-méthanol (99/1 en volumes) et en recueillant des fractions de 30 cm³. Les fractions contenant le produit attendu, sont réunies et évaporées sous pression réduite (2 kPa ; 50°C). On obtient ainsi 80 mg de N-[4-chloro-1H-indazol-3-yl]-butanamide, sous forme d'un solide blanc fondant à 198°C.

Spectre de R.M.N. ¹H (300 MHz, (CD₃)₂SO d6, δ en ppm) : 0,98 (t large, J = 7 Hz : 3H) ; 1,66 (mt : 2H) ; 2,35 (t très large, J = 7 Hz : 2H) ; 7,15 (d large, J = 8 Hz : 1H) ; 7,34 (t, J = 8 Hz : 1H) ; 7,49 (d, J = 8 Hz : 1H) ; 9,80 (mf : 1H).

### EXEMPLE 20

### N-[6-(trifluorométhyl)-1H-indazol-3-yl]-butanamide

A 500 mg de 6-(trifluorométhyl)-1H-indazole-3-amine préparé comme dans le brevet U.S. 3 133 081 dans 5 cm³ de pyridine, refroidit vers 10°C, on additionne 0.26 cm³ de chlorure de butyryle. On laisse revenir la température vers 19°C pendant 19 heures. On évapore le milieu réactionnel sous pression réduite (2 kPa ; 50°C). On reprend le résidu par 15 cm³ d'acétate d'éthyle et par 15 cm³ d'eau distillée. La phase organique est lavée avec 15 cm³ d'eau distillée et avec 15 cm³ d'une solution aqueuse saturée en chlorure de sodium. On sèche la phase organique sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice (granulométrie 40-60 µm ; diamètre 2 cm), en éluant par un mélange cyclohexane-acétate d'éthyle (70/30 en volumes) et en recueillant des fractions de 20 cm³. Les fractions contenant le produit attendu, sont réunies et évaporées sous pression réduite (2 kPa ; 50°C). On obtient après séchage (90 Pa ; 45°C), 49 mg de N-[6-(trifluorométhyl)-1H-indazol-3-yl]-butanamide, sous forme d'un solide blanc fondant à 200°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,42 (t, J = 7,5 Hz : 2H) ; 7,34 (d large, J = 9 Hz : 1H) ; 7,82 (s large : 1H) ; 8,04 (d, J = 9 Hz : 1H) ; 10,49 (mf : 1H) ; 13,10 (mf étalé : 1H).

### EXEMPLE 21

### 6-chloro-1-[[2-(triméthylsilyl)éthoxy]méthyl]-1H-indazole-3-amine

A 478 mg d'hydrure de sodium dans 50 cm³ de diméthylformamide anhydre, on additionne une solution de 2 g de 6-chloro-1H-indazole-3-amine dans 20 cm³ de diméthylformamide. On refroidit ensuite vers 3°C pour ajouter 2.12 cm³ de chlorure de [2-(triméthylsilyl)éthoxy]méthyle dans 10 cm³ de diméthylformamide. On laisse revenir vers 19°C pendant 45 minutes puis on reprend par 250 cm3 d'acétate d'éthyle. On lave avec 3 fois 100 cm³ d'eau distillée et par 100 cm³ d'une solution aqueuse saturée en chlorure de sodium. La phase organique est séchée sur sulfate de magnésium, filtrée sur verre fritté puis évaporée sous pression réduite (2 kPa ; 40°C). Le résidu est purifié par chromatographie sous pression d'argon de 50 kPa, sur colonne de gel de silice (granulométrie 40-60 µm ; diamètre 4 cm), en éluant par un mélange cyclohexane-acétate d'éthyle (90/10 en volumes) et en recueillant des fractions de 100 cm³. Les fractions contenant le produit attendu, sont réunies et évaporées sous pression réduite (2 kPa ; 50°C). On obtient 2 g de 6-chloro-1-[[(2-triméthylsilyl)éthoxy]méthyl]-1H-indazole-3-amine sous forme d'une huile jaune.

Spectre de R.M.N. ¹H (300 MHz, (CD₃)₂SO d6, δ en ppm) : - 0,09 (s : 9H) ; 0,80 (t, J = 8 Hz : 2H) ; 3,48 (t, J = 8 Hz : 2H) ; 5,43 (s : 2H) ; 5,68 (s large : 2H) ; 7,01 (dd, J = 9 et 2 Hz : 1H) ; 7,61 (d, J = 2 Hz : 1H) ; 7,74 (d, J = 9 Hz : 1H).

### N-[6-chloro-1-[(2-triméthylsilyléthoxy)méthyl]-1H-indazol-3-yl]-propénamide

A 1 g de 6-chloro-1-[[(2-triméthylsilyl)éthoxy]méthyl]-1H-indazole-3-amine, précédemment décrit, dans 25 cm³ de dichlorométhane et 0.57 cm³ de triéthylamine, on ajoute 0.33 cm³ de chlorure d'acryloyle. Le milieu réactionnel est agité pendant 30 minutes puis on l'évapore sous pression réduite (2 kPa ; 40°C). Le résidu est repris avec 100 cm³ d'acétate d'éthyle et on lave avec 2 fois 50 cm³ d'eau distillée et avec 50 cm³ d'une solution aqueuse saturée en chlorure de sodium. La phase organique est séchée sur sulfate de magnésium , filtrée sur verre fritté puis évaporée sous pression réduite (2 kPa ; 40°C). Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (90/10 en volumes) et en recueillant des fractions de 35 cm³. Les fractions contenant le produit attendu, sont réunies et évaporées sous pression réduite (2 kPa ; 503°C). On obtient après séchage ( 90 Pa; 45°C), 160 mg de N-[6-chloro-1-[[2-(triméthylsilyl)éthoxy]méthyl]-1H-indazol-3-yl]-propénamide sous forme d'un solide blanc.

Spectre de R.M.N. ¹H (300 MHz, (CD₃)₂SO d6, δ en ppm) : - 0,08 (s : 9H) ; 0,83 (t, J = 8 Hz : 2H) ; 3,54 (t, J = 8 Hz : 2H) ; 5,68 (s : 2H) ; 5,84 (dd, J = 10,5 et 2 Hz : 1H) ; 6,35 (dd, J = 16,5 et 2 Hz : 1H) ; 6,60 (dd, J = 16,5 et 10,5 Hz : 1H) ; 7,18 (dd, J = 9 et 2 Hz : 1H) ; 7,88 (d, J = 2 Hz : 1H) ; 8,00 (d, J = 9 Hz : 1H) ; 10,40 (mf : 1H).

### N-[6-chloro-1H-indazol-3-yl]-propénamide

A 160 mg de N-[6-chloro-1-[[2-(triméthylsilyl)éthoxy]méthyl]-1H-indazol-3-yl]]-propénamide précédemment décrit, dans 10 cm³ d'éthanol, on additionne 5 cm³ d'acide chlorhydrique 5N. On chauffe le milieu vers 78°C pendant 30 minutes. On laisse ensuite revenir vers 19°C pour ajouter 6 cm³ d'hydroxyde de sodium 5N. On évapore le milieu réactionnel sous pression réduite (2 kPa ; 40°C) et on reprend le résidu par 50 cm³ d'acétate d'éthyle, par 25 cm³ de tétrahydrofuranne et par 20 cm³ d'eau distillée. On lave la phase organique avec 50 cm³ d'une solution aqueuse saturée en chlorure de sodium. On sèche ensuite sur sulfate de magnésium, on filtre sur verre fritté puis on évapore sous pression réduite (2 kPa ; 40°C). Le résidu est purifié par chromatographie sous pression d'argon de 50 kPa, sur colonne de gel de silice (granulométrie 40-60 µm ; diamètre 1.5 cm), en éluant par un mélange cyclohexane-acétate d'éthyle (50/50 en volumes) et en recueillant des fractions de 7 cm³. Les fractions contenant le produit attendu, sont réunies et évaporées sous pression réduite (2 kPa ; 50°C). Le résidu est repris par 10 cm³ de dichlorométhane, on filtre l'insoluble sur verre fritté et on lave avec 2 fois 5 cm³ de dichlorométhane. On obtient après séchage (90 Pa; 45°C), 10 mg de N-[6-chloro-1H-indazol-3-yl]-propénamide sous forme d'un solide blanc fondant à 205°C.

Spectre de R.M.N. ¹H (300 MHz, (CD₃)₂SO d6, δ en ppm) : 5,82 (dd, J = 10,5 et 2 Hz : 1H) ; 6,34 (dd, J = 17 et 2 Hz : 1H) ; 6,60 (dd, J = 17 et 10,5 Hz : 1H) ; 7,10 (dd, J = 9 et 2 Hz : 1H) ; 7,54 (d, J = 2 Hz : 1H) ; 7,95 (d, J = 9 Hz : 1H ; 10,78 (mf étalé : 1H) ; 12,86 (mf étalé : 1H).

### EXEMPLE 22

### N-[5-(trifluorométhyl)-1H-indazol-3-yl]-butanamide

A 500 mg de 5-(trifluorométhyl)-1H-indazole-3-amine préparé selon le brevet U.S. 3 133 081, dans 15 cm³ de pyridine, on refroidit vers 5°C et et on additionne 0.26 cm³ de chlorure de butyryle. On laisse le milieu réactionnel revenir vers 19 °C pendant 12 heures. On évapore le milieu réactionnel sous pression réduite (2 kPa ; 50°C). On reprend le résidu par 15 cm³ d'acétate d'éthyle et par 15 cm³ d'eau distillée. La phase organique séchée sur sulfate de magnésium, on filtre sur verre fritté puis on évapore sous pression réduite (2 kPa; 50°C). Le résidu est repris par 15 cm³ de dichlorométhane, on filtre et on sèche sous pression réduite (90 Pa; 50°C) pour obtenir 390 mg de N-[5-(trifluorométhyl)-1H-indazol-3-yl]-butanamide, sous forme d'un solide blanc cassé fondant à 230 °C.

Spectre de R.M.N. ¹H (300 MHz, (CD₃)₂SO d6, δ en ppm) : 0,97 (t, J = 7,5 Hz : 3H) ; 1,68 (mt : 2H) ; 2,43 (t, J = 7 Hz : 2H) ; 7,60 (dd, J = 9 et 1,5 Hz : 1H) ; 7,65 (d, J = 9 Hz : 1H) ; 8,34 (s large : 1H) ; 10,60 (s large : 1H) ; 13,06 (s large : 1H).

### EXEMPLE 23

### N-[5-nitro-1H-indazol-3-yl]-butanamide

A 1g de 5-nitro-1H-indazole-3-amine préparé comme décrit dans le brevet SU 742430 dans 25 cm³ de pyridine, on refroidit vers 5°C et on additionne 0.58 cm³ de chlorure de butyryle. On laisse le milieu réactionnel revenir vers 19 °C pendant 12 heures. On filtre l'insoluble présent puis on évapore le filtrat sous pression réduite (2kPa ; 50°C). On reprend le résidu par 15 cm³ d'acétate d'éthyle et par 15 cm³ d'eau distillée. La phase organique est séchée sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice ( granulométrie 40-60 µm ; diamètre 3.5 cm), en éluant par un mélange cyclohexane-acétate d'éthyle (70/30 en volumes) et en recueillant des fractions de 20 cm³. Les fractions contenant le produit attendu, sont réunies et évaporées sous pression réduite (2 kPa ; 50°C) . On obtient après séchage (90 Pa ; 50°C), 480 mg de N-[5-nitro-1H-indazol-3-yl]-butanamide sous forme d'un solide blanc.

Spectre de R.M.N. ¹H (300 MHz, (CD₃)₂SO d6, δ en ppm) : 0,98 (t, J = 7,5 Hz : 3H) ; 1,70 (mt : 2H) ; 2,46 (t, J = 7 Hz : 2H) ; 7,63 (d, J = 9 Hz : 1H) ; 8,18 (dd, J = 9 et 2 Hz : 1H) ; 9,05 (d, J = 2 Hz : 1H) ; 10,77 (mf : 1H) ; de 13,00 à 13,70 (mf étalé : 1H).

### EXEMPLE 24

### N-[6-bromo-1H-indazol-3-yl]-butanamide

A 500 mg de 6-bromo-1H-indazole-3-amine précédemment décrit dans l'exemple 12, dans 15 cm³ de pyridine, on refroidit vers 5°C et on additionne 0.24 cm³ de chlorure de butyryle. On laisse le milieu réactionnel revenir vers 19 °C pendant 50 heures. On évapore le milieu réactionnel sous pression réduite (2 kPa ; 50°C). On reprend le résidu par 15 cm³ d'acétate d'éthyle et par 15 cm³ d'eau distillée. La phase organique est séchée sur sulfate de magnésium, on filtre sur verre fritté puis on évapore sous pression réduite (2 kPa; 50°C). Le résidu est repris par 15 cm³ de dichlorométhane, filtré pour donner après séchage (90 Pa ; 50°C), 356 mg de N-[6-bromo-1H-indazol-3-yl]-butanamide, sous forme d'un solide blanc cassé fondant à 202 °C.

Spectre de R.M.N. ¹H (300 MHz, (CD₃)₂SO d6, δ en ppm) : 0,98 (t large, J = 7,5 Hz : 3H) ; 1,67 (mt : 2H) ; 2,39 (t large, J = 7 Hz : 2H) ; 7,20 (d large, J = 9 Hz : 1H) ; 7,68 (s large : 1H) ; 7,78 (d large, J = 9 Hz : 1H) ; 10,40 (mf : 1H) ; 12,75 (mf : 1H).

### EXEMPLE 25

### N-[6-chloro-1-[[2-(triméthylsilyl)éthoxy]méthyl]-1H-indazol-3-yl]]-butanamide

A 606 mg d'hydrure de sodium à 60%, dans 20 cm³ de diméthylformamide, on additionne 3g de N-(6-chloro-1H-indazol-3-yl)-butanamide précédemment décrit dans l'exemple 11, en solution dans 40 cm³ de diméthylformamide. Après avoir refroidit vers 5°C, on ajoute 2.68cm³ de chlorure de [[2-(triméthylsilyl)éthoxy]méthyle] dans 10 cm³ de diméthylformamide. On laisse la température revenir vers 21°C et agite pendant 2 heures. Le milieu réactionnel est ensuite évaporé sous pression réduite (2kPa ; 45 °C). Le résidu est repris par 200 cm³ d'acétate d'éthyle et par 100 cm³ d'eau distillée. On relave avec 2 fois 100 cm³ d'eau distillée et avec 100 cm³ d'une solution aqueuse saturée en chlorure de sodium. La phase organique est séchée sur sulfate de magnésium, filtrée sur verre fritté puis évaporée sous pression réduite (2 kPa; 50°C). Le résidu est purifié par chromatographie sous pression d'argon de 50 kPa, sur colonne de gel de silice ( granulométrie 40-60 µm ; diamètre 4.5 cm), en éluant par un mélange cyclohexane-acétate d'éthyle (80/20 en volumes) et en recueillant des fractions de 100 cm³. Les fractions contenant le produit attendu sont réunies et évaporées sous pression réduite ( 2 kPa ; 50°C) . On obtient après séchage (90 Pa ; 50°C), 3 g de N-[6-chloro-1-[[2-(triméthylsilyl)éthoxy]méthyl]-1H-indazol-3-yl]]-butanamide sous forme d'une huile jaune.

Spectre de R.M.N. ¹H (300 MHz, (CD₃)₂SO d6, δ en ppm) : - 0,08 (s : 9H) ;.0,83 (t large, J = 8 Hz : 2H) ; 0,96 (t, J = 7,5 Hz : 3H) ; 1,67 (mt : 2H) ; 2,40 (t, J = 7,5 Hz : 2H) ; 3,53 (t, J = 8 Hz : 2H) ; 5,66 (s : 2H) ; 7,16 (dd, J = 9 et 2 Hz : 1H) ; 7,86 (d, J = 2 Hz : 1H) ; 7,88 (d, J = 9 Hz : 1H) ; 10,53 (mf : 1H).

### N-[6-(3-pyridinyl)-1-[[2-(triméthylsilyl)éthoxy]méthyl]-1H-indazol-3-yl]-butanamide

A 1.5 g de N-[6-chloro-1-[[2-(triméthylsilyl)éthoxy]méthyl]-1H-indazol-3-yl]-butanamide précédemment décrit, dans 40 cm³ de dioxanne, on additionne 900 mg de diéthyl-3-pyridyl-borane, 1.86 g de fluorure de césium, 18.4 mg d'acétate de palladium et enfin 48 mg de biphényl-2-dicyclohexylphosphine-2-(N,N-diméthylamino). On chauffe ensuite vers 100°C pendant 17 heures puis on filtre sur verre fritté et on évapore sous pression réduite (2 kPa ; 50°C). Le résidu est repris par 75 cm³ d'acétate d'éthyle et par 50 cm³ d'eau distillée. La phase organique est relavée avec 50 cm³ d'eau-distillée et avec 50 cm³ d'une solution aqueuse saturée en chlorure de sodium. La phase aqueuse est séchée sur sulfate de magnésium, filtrée sur verre fritté puis évaporée sous pression réduite dans les conditions décrites précédemment. Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (50/50 en volumes) et en recueillant des fractions de 25 cm³. Les fractions contenant le produit attendu, sont réunies et évaporées sous pression réduite (2 kPa ; 50°C). On obtient après séchage (90 Pa ; 50°C), 900 mg de N-[6-(3-pyridinyl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0.09 (s : 9H) ; 0,84 (t large, J = 8 Hz : 2H) ; 0,98 (t, J = 7,5 Hz : 3H) ; 1,70 (mt : 2H) ; 2,43 (t, J = 7,5 Hz : 2H) ; 3,59 (t, J = 8 Hz : 2H) ; 5,76 (s : 2H) ; 7,52 (dd, J = 9 et 2 Hz : 1H) ; 7,55 (dd large, J = 8,5 et 4,5 Hz : 1H) ; 7,97 (d, J = 9 Hz : 1H) ; 8,09 (s large : 1H) ; 8,20 (ddd, J = 8,5 - 2,5 et 2 Hz : 1H) ; 8,63 (dd, J = 4,5 et 2 Hz : 1H) ; 9,02 (d large, J = 2,5 Hz : 1H) ; 10,51 (mf: 1H).

### N-[6-(3-pyridinyl)-1H-indazol-3-yl]-butanamide

A 900 mg de N-[6-(3-pyridinyl)-1-[[2-(trimethylsilyl)éthoxy]méthyl]-1H-indazol-3-yl]-butanamide décrit précédemment, dans 20 cm³ de tétrahydrofuranne, on additionne 13.3cm³ de fluorure de tétrabutylammonium en solution 1M dans le tétrahydrofuranne. On chauffe le milieu réactionnel vers 66°C pendant 21 heures. On arrête ensuite le chauffage et on ajoute 100 cm³ d'acétate d'éthyle. On lave avec 50 cm³ d'une solution aqueuse saturée en hydrogénocarbonate de sodium puis par 2 fois 50 cm³ d'eau distillée et par 50 cm³ d'une solution aqueuse saturée en chlorure de sodium. La phase organique est séchée sur sulfate de magnésium, filtrée sur verre frittée et évaporée sous pression réduite (2 kPa ; 50°C). Le résidu est purifié par chromatographie sous pression d'argon de 50 kPa, sur colonne de gel de silice ( (granulométrie 40-60 µm ; diamètre 2 cm), en éluant par de l'acétate d'éthyle et en recueillant des fractions de 25 cm³. Les fractions contenant le produit attendu, sont réunies et évaporées sous pression réduite (2 kPa ; 50°C). On obtient après séchage (90 Pa; 50°C), 380 mg de N-[6-(3-pyridinyl)-1H-indazol-3-yl]-butanamide, sous forme d'un produit blanc fondant à 205°C.

Spectre de R.M.N. ¹H (300 MHz, (CD₃)₂SO d6, δ en ppm) : 0,98 (t, J = 7,5 Hz : 3H) ; 1,70 (mt : 2H) ; 2,41 (t, J = 7 Hz : 2H) ; 7,42 (dd, J = 9 et 1,5 Hz : 1H) ; 7,53 (ddd, J = 8 - 5 et 0,5 Hz : 1H) ; 7,73 (s large : 1H) ; 7,92 (d large, J = 9 Hz : 1H) ; 8,18 (ddd, J = 8 - 2 et 1,5 Hz : 1H) ; 8,62 (dd, J = 5 et 2 Hz : 1H) ; 8,98 (d large, J = 1,5 Hz : 1H) ; 10,37 (mf : 1H) ; 12,80 (mf : 1H).

### EXEMPLE 26

### 4-iodo-1H-indazole-3-amine

A 2 g de 2-fluoro-6-iodobenzonitrile dans 25 cm³ d'éthanol, on ajoute 1.2 cm³ d'hydrazine monohydratée. On chauffe ensuite le milieu réactionnel au reflux vers 78°C pendant 12 heures. On laisse revenir le milieu vers 20°C puis on additionne 20 cm³ d'eau distillée afin de faire précipiter le produit. L'insoluble est filtré sur verre fritté puis rincé avec 20 cm³ d'eau distillée puis repris par 20 cm³ de dichlorométhane. La phase organique est ensuite séchée sur sulfate de magnésium et évaporée sous pression réduite (2 kPa ;45°C). Après séchage (90 Pa ; 50°C), on obtient 1.65 g de 4-iodo-1H-indazole-3-amine, sous forme de solide jaune fondant à 157°C.

Spectre de R.M.N. ¹H (300 MHz, (CD₃)₂SO d6, δ en ppm) : 5,05 (s large : 2H) ; 6,95 (dd, J = 7,5 et 8,5 Hz : 1H) ; 7,30 (dd, J = 8,5 et 1 Hz : 1H) ; 7,37 (d large, J = 7,5 Hz : 1H) ; 11,80 (mf : 1H).

### N-[4-iodo-1H-indazol-3-yl]-butanamide

A 500 mg de 4-iodo-1H-indazole-3-amine décrit précédemment dans 15 cm³ de pyridine, on refroidit vers 5°C et on aditionne 0.20 cm³ de chlorure de butyryle. On laisse le milieu réactionnel revenir vers 19 °C pendant 50 heures. On évapore le milieu réactionnel sous pression réduite (2 kPa ; 50°C). On reprend le résidu par 15 cm³ d'acétate d'éthyle, par 15 cm³ de tétrahydrofuranne et par 15 cm³ d'eau distillée. La phase organique est séchée sur sulfate de magnésium, puis filtrée sur verre fritté et on évapore sous pression réduite (2 kPa ; 50°C). Le résidu est repris par 15 cm³ de dichlorométhane et filtré. On reprend l'insoluble dans 10 cm³ de méthanol, on filtre l'insoluble et on évapore le filtrat sous pression réduite, pour obtenir après séchage ( 90 Pa ; 50°C), 70 mg de N-[4-iodo-1H-indazol-3-yl]-butanamide sous forme d'un solide écru.

Spectre de R.M.N. ¹H (300 MHz, (CD₃)₂SO d6, δ en ppm) : 0,98 (t large, J = 7,5 Hz : 3H) ; 1,68 (mt : 2H) ; 2,39 (t large, J = 7 Hz : 2H) ; 7,09 (t, J = 8 Hz : 1H) ; 7,54 (d, J = 8 Hz : 1H) ; 7,58 (d large, J = 8 Hz : 1H) ; 9,68 (s large : 1H) ; 13,08 (mf : 1H).

### EXEMPLE 27

### N-[6-phenyl)-1-[[2-(triméthylsilyl)éthoxy]méthyl]-1H-indazol-3-yl]-butanamide

A 1.5 g de N-[6-chloro-1-[[2-(triméthylsilyl)éthoxy]méthyl]-1H-indazol-3-yl]-butanamide précédemment décrit dans l'exemple 25, dans 30cm³ de dioxanne, on additionne 497 mg d'acide phénylboronique, 1.24 g de fluorure de césium, 12.35 mg d'acétate de palladium et enfin, 48 mg de biphényl-2-dicyclohexylphosphine-2-(N,N-diméthylamino). On chauffe ensuite vers 100°C pendant 18 heures puis on filtre sur verre fritté et on évapore sous pression réduite (2 kPa ; 50°C). Le résidu est rincé par 50 cm³ de tétrahydrofuranne et par 50 cm³ d'eau distillée. On reprend par 75 cm³ d'acétate d'éthyle et par 50 cm³ d'eau distillée. La phase organique est séchée sur sulfate de magnésium, filtrée sur verre fritté puis évaporée sous pression réduite dans les conditions décrites précédemment. Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (75/25 en volumes) et en recueillant des fractions de 25 cm³. Les fractions contenant le produit attendu, sont réunies et évaporées sous pression réduite (2 kPa ; 50°C). On obtient après séchage (90 Pa; 50°C), 1 g de N-[6-phenyl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,08 (s : 9H) ; 0,84 (t, J = 8 Hz : 2H) ; 0,98 (t, J = 7,5 Hz : 3H) ; 1,69 (mt : 2H) ; 2,42 (t, J = 7 Hz : 2H) ; 3,59 (t, J = 8 Hz : 2H) ; 5,74 (s : 2H) ; 7,42 (t large, J = 7,5 Hz : 1H) ; 7,47 (dd, J = 9 et 1,5 Hz : 1H) ; 7,53 (t large, J = 7,5 Hz : 2H) ; 7,79 (d large, J = 7,5 Hz : 2H) ; 7,93 (d, J = 9 Hz : 1H) ; 7,96 (s large : 1H) ; 10,48 (mf : 1H).

### N-[6-phenyl-1H-indazol-3-yl]-butanamide

A 900 mg de N-[6-phenyl)-1-[[2-(triméthylsilyl)éthoxy]méthyl]-1H-indazol-3-yl]-butanamide décrit précédemment, dans 30 cm³ de tétrahydrofuranne, on additionne 14.65 cm³ de fluorure de tétrabutylammonium en solution 1M dans le tétrahydrofuranne. On chauffe le milieu réactionnel vers 66°C pendant 16 heures. On arrête ensuite le chauffage et on ajoute 75 cm³ d'acétate d'éthyle. On lave avec 75 cm³ d'une solution aqueuse saturée en hydrogénocarbonate de sodium puis par 2 fois 75 cm³ d'eau distillée et par 50 cm³ d'une solution aqueuse saturée en chlorure de sodium. La phase organique est séchée sur sulfate de magnésium, filtrée sur verre fritté et évaporée sous pression réduite (2 kPa; 50°C). Le résidu est purifié par chromatographie sous pression d'argon de 50 kPa, sur colonne de gel de silice ( granulométrie 40-60 µm ; diamètre 3.5 cm), en éluant par de l'acétate d'éthyle et en recueillant des fractions de 35 cm³. Les fractions contenant le produit attendu, sont réunies et évaporées sous pression réduite (2 kPa ; 50°C). Le résidu est repris par 10 cm³ d'acétate d'éthyle puis on filtre sur verre fritté, on lave avec 2 fois 5 cm³ d'acétate d'éthyle et avec 20 cm³ d'éther diisopropylique. On obtient après séchage (90 Pa; 50°C), 420 mg de N-[6-phenyl-1H-indazol-3-yl]-butanamide sous forme d'un produit blanc fondant à 220°C.

Spectre de R.M.N. ¹H (300 MHz, (CD₃)₂SO d6 , δ en ppm) : 0,99 (t large, J = 7 Hz : 3H) ; 1,70 (mt : 2H) ; 2,41 (t, J = 7 Hz : 2H) ; 7,37 (dd, J = 9 et 1,5 Hz : 1H) ; 7,40 (t large, J = 7,5 Hz : 1H) ; 7,51 (t large, J = 7,5 Hz : 2H) ; 7,63 (s large : 1H) ; 7,74 (d large, J = 7,5 Hz : 2H) ; 7,98 (d, J = 9 Hz : 1H) ; 10,34 (mf : 1H) ; 12,70 (mf : 1H).

### EXEMPLE 28

### N-[6-bromo-5,7-dinitro-1H-indazol-3-yl]-butanamide

A 500 mg de N-[6-bromo-1H-indazol-3-yl]-butanamide précédemment décrit dans, l'exemple 24, dans 20 cm³ d'acétonitrile, refroidit vers 3°C, on additionne en une seule fois 470 mg de tétrafluoroborate de nitronium. On laisse revenir vers 19°C pendant 14 heures. On ajoute dans le milieu réactionnel, 15 cm³ d'acétate d'éthyle et 15 cm³ d'eau distillée. Le milieu est ensuite évaporé sous pression réduite (2 kPa ; 40°C) et le résidu est repris par 20 cm³ de dichlorométhane. On filtre l'insoluble et on le lave avec 20 cm³ d'éther diisopropylique. On obtient après séchage (90 Pa ; 45°C), 200 mg de N-[6-bromo-5,7-dinitro-1H-indazol-3-yl]-butanamide sous forme d'un solide ocre fondant à 260°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,45 (t, J = 7,5 Hz : 2H) ; 9,05 (s : 1H) ; 11,06 (mf : 1H) ; 14,04 (mf : 1H).

### EXEMPLE 29

### N-[6-bromo-7-nitro-1H-indazol-3-yl]-butanamide

A 500 mg de N-[6-bromo-1H-indazol-3-yl]-butanamide précédemment décrit dans l'exemple 24, dans 25 cm³ d'acétonitrile, refroidit vers 3°C, on additionne en une seule fois 235 ing de tétrafluoroborate de nitronium. On maintient vers 3°C pendant 1 heure puis on laisse revenir vers 19°C pendant 14 heures. On ajoute dans le milieu réactionnel, 15 cm³ d'acétate d'éthyle et 15 cm³ d'eau distillée. Le milieu est ensuite évaporé sous pression réduite (2 kPa ; 40°C) et le résidu est repris par 20 cm³ de dichlorométhane. On filtre l'insoluble et on le lave avec 20 cm³ d'éther diisopropylique. Le résidu est purifié par chromatographie sous pression d'argon de 50 kPa, sur colonne de gel de silice (granulométrie 40-60 µm ; diamètre 3.5 cm), en éluant par de l'acétate d'éthyle-cyclohexane (30/70 en volumes) et en recueillant des fractions de 35 cm³. Les fractions contenant le produit attendu, sont réunies et évaporées sous pression réduite (2 kPa ; 50°C). On obtient après séchage (90 Pa ; 50°C), 30 mg de N-[6-bromo-7-nitro-1H-indazol-3-yl]-butanamide sous forme d'un produit blanc fondant à 248°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,43 (t, J = 7,5 Hz : 2H) ; 7,54 (d large, J = 9 Hz : 1H) ; 8,13 (d, J = 9 Hz : 1H) ; 10,68 (mf : 1H) ; 13,44 (mf étalé : 1H).

### EXEMPLE 30

### N-[6-bromo-5-nitro-1H-indazol-3-yl]-butanamide

Lors de la purification par chromatographie de l'exemple 29 sous pression d'argon 50 kPa, sur colonne de gel de silice (granulomètrie 40-60 µm ; diamètre 3.5 cm), en éluant par de l'acétate d'éthyle-cyclohexane (30/70 en volumes) on recueille des fractions de 35 cm³. Les fractions contenant le produit attendu, sont réunies et évaporées sous pression réduite (2 kPa ; 50°C). On obtient après séchage (90 Pa ; 50°C), 10 mg de N-[6-bromo-5-nitro-1H-indazol-3-yl]-butanamide, sous forme d'un produit blanc fondant à 259°C.

Spectre de R.M.N. ¹H (300 MHz, (CD₃)₂SO d6, δ en ppm) : 0,96 (t, J = 7,5 Hz : 3H) ; 1,69 (mt : 2H) ; 2,43 (t, J = 7,5 Hz : 2H) ; 7,95 (s : 1H) ; 8,81 (s : 1H) ; 10,80 (mf : 1H) ; de 12,70 à 13,70 (mf étalé : 1H).

### EXEMPLE 31

### N-[6-(furan-3-yl)-1-[[2-(triméthylsilyl)éthoxy]méthyl]-1H-indazol-3-yl]-butanamide

A 1 g de N-[6-chloro-1-[[2-(triméthylsilyl)éthoxy]méthyl]-1H-indazol-3-yl]-butanamide précédemment décrit dans l'exemple 25, dans 30 cm³ de dioxanne, on additionne 457 mg d'acide furanne-3-boronique, 1.24 g de fluorure de césium, 13 mg d'acétate de palladium et enfin, 31 mg de biphényl-2-dicyclohexylphosphine-2-(N,N-diméthylamino). On chauffe ensuite vers 100°C pendant 23 heures. On ajoute encore 457 mg d'acide furanne-3-boronique, 1.24 g de fluorure de césium, 13 mg d'acétate de palladium et enfin, 31 mg de biphényl-2-dicyclohexylphosphine-2-(N,N-diméthylamino) et on poursuit le reflux pendant 7 heures. On laisse ensuite revenir vers 19°C pendant 16 heures puis on filtre sur verre fritté et on évapore sous pression réduite (2 kPa ; 50°C). Le résidu est rincé par 50 cm³ de tétrahydrofuranne et par 50 cm³ d'eau distillée. On reprend par 75 cm³ d'acétate d'éthyle et par 50 cm³ d'eau distillée. La phase organique est séchée sur sulfate de magnésium, filtrée sur verre fritté puis évaporée sous pression réduite dans les conditions décrites précédemment. Le résidu est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (80/20 en volumes) et en recueillant des fractions de 35 cm³. Les fractions contenant le produit attendu, sont réunies et évaporées sous pression réduite (2 kPa ; 50°C). On obtient 130 mg de N-[6-(furan-3-yl)-1-[[2-(triméthylsilyl)éthoxylméthyl]-1H-indazol-3-yl]-butanamide sous forme d'une huile orange.

Le spectre de masse a été réalisé en impact electronique (70eV)

EI m/z = 399 M⁺; m/z = 282 C₁₆H₁₃N₃O₂⁺; m/z = 271 C₁₅H₁₃N₃O₂⁺, m/z = 212 C₁₂H₁₀N₃O⁺; m/z = 73 C₃H₉Si⁺

### N-[6-(furan-3-yl)-1H-indazol-3-yl]-butanamide

A 120 mg de N-[6-(furan-3-yl)-1-[[2-(triméthylsilyl)éthoxy]méthyl]-1H-indazol-3-yl]-butanamide, décrit précédemment, dans 5 cm³ de tétrahydrofuranne, on additionne 1.95 cm³ de fluorure de tétrabutylammonium en solution 1M dans le tétrahydrofuranne. On chauffe le milieu réactionnel vers 66°C pendant 17 heures. On arrête ensuite le chauffage et on ajoute 50 cm³ d'acétate d'éthyle. On lave avec 50 cm³ d'une solution aqueuse saturée en hydrogénocarbonate de sodium puis par 50 cm³ d'une solution aqueuse saturée en chlorure de sodium. La phase organique est séchée sur sulfate de magnésium, filtrée sur verre fritté et évaporée sous pression réduite (2 kPa ; 50°C). Le résidu est purifié par chromatographie sous pression d'argon de 50 kPa, sur colonne de gel de silice (granulométrie 40-60 µm ; diamètre 2 cm), en éluant par acétate d'éthyle-cyclohexane (30/70 en volumes) et en recueillant des fractions de 15 cm³. Les fractions contenant le produit attendu, sont réunies et évaporées sous pression réduite (2 kPa ; 50°C). Le résidu est repris par 10 cm³ d'éther diisopropylique. On filtre sur verre fritté pour obtenir après séchage (90 Pa ; 50°C), 35 mg de N-[6-(furan-3-yl)-1H-indazol-3-yl]-butanamide sous forme d'un solide blanc fondant à 195°C.

Spectre de R.M.N. ¹H (300 MHz, (CD₃)₂SO d6, δ en ppm) : 0,98 (t large, J = 7,5 Hz : 3H) ; 1,68 (mt : 2H) ; 2,39 (t, J = 7 Hz : 2H) ; 7,06 (s large : 1H) ; 7,34 (d large, J = 9 Hz : 1H) ; 7,60 (s large : 1H) ; de 7,70 à 7,85 (mt : 2H) ; 8,27 (s large : 1H) ; 10,29 (mf : 1H) ; 12,62 (mf : 1H).

### EXEMPLE 32

### N-[6-[4-(phenylmethoxy)phenyl]-1-[[2-(triméthylsilyl)éthoxy]méthyl]-1H-indazol-3-yl]-butanamide

A 1 g de N-[6-chloro-1-[[2-(triméthylsilyl)éthoxy]méthyl]-1H-indazol-3-yl]-butanamide précédemment décrit dans l'exemple 25, dans 30cm³ de dioxanne, on additionne 930 mg d'acide 4-benzyloxyphénylboronique, 1.24 g de fluorure de césium, 13 mg d'acétate de palladium et enfin, 31 mg de biphényl-2-dicyclohexylphosphine-2-(N,N-diméthylamino). On chauffe ensuite vers 100°C pendant 5 heures. On laisse ensuite revenir vers 19°C puis on filtré sur verre fritté et on évapore sous pression réduite (2 kPa ; 50°C). Le résidu est rincé par 50 cm³ de tétrahydrofuranne et par 50 cm³ d'eau distillée. On reprend par 150 cm³ d'acétate d'éthyle, par 50 cm³ d'eau distillée et par 50 cm³ d'une solution aqueuse saturée en chlorure de sodium. La phase organique est séchée sur sulfate de magnésium, filtrée sur verre fritté puis évaporée sous pression réduite dans les conditions décrites précédemment. Le résidu est purifié par chromatographie sous pression d'argon de 50 kPa, sur colonne de gel de silice (granulomètrie 40-60 µm ; diamètre 3.5 cm), en éluant par un mélange cyclohexane-acétate d'éthyle (80/20 en volumes) et en recueillant des fractions de 35 cm³. Les fractions contenant le produit attendu, sont réunies et évaporées sous pression réduite (2 kPa ; 50°C). On obtient 1.2 g de N-[6-[4-(phenylmethoxy)phenyl]-1-[[2-(triméthylsilyl)éthoxy]méthyl]-1H-indazol-3-yl]-butanamide sous forme d'une huile orange.

Spectre de R.M.N. ¹H (300 MHz, (CD₃)₂SO d6, δ en ppm) : - 0,08 (s : 9H) ; 0,83 (t large, J = 8 Hz : 2H) ; 0,99 (t, J = 7,5 Hz : 3H) ; 1,68 (mt : 2H) ; 2,42 (t large, J = 7 Hz : 2H) ; 3,57 (t large, J = 8 Hz : 2H) ; 5,21 (s : 2H) ; 5,73 (s : 2H) ; 7,16 (d, J = 8,5 Hz : 2H) ; de 7,30 à 7,50 (mt : 4H) ; 7,51 (d large, J = 7,5 Hz : 2H) ; 7,73 (d, J = 8,5 Hz : 2H) ; de 7,85 à 7,95 (mt : 2H) ; 10,46 (mf : 1H).

### N-[6-[4-(phenylmethoxy)phenyl]-1H-indazol-3-yl]-butanamide

A 1.2 g de N-[6-[4-(phenylmethoxy)phenyl]-1-[[2-(triméthylsilyl)éthoxy]méthyl]-1H-indazol-3-yl]-butanamide, décrit précédemment, dans 30 cm³ de tétrahydrofuranne, on additionne 14 cm³ de fluorure de tétrabutylammonium en solution 1M dans le tétrahydrofuranne. On chauffe le milieu réactionnel vers 66°C pendant 17 heures. On arrête ensuite le chauffage et on ajoute 75 cm³ d'acétate d'éthyle. On lave avec 50 cm³ d'une solution aqueuse saturée en hydrogénocarbonate de sodium puis par 50 cm³ d'une solution aqueuse saturée en chlorure de sodium. La phase organique est séchée sur sulfate de magnésium, filtrée sur verre fritté et évaporée sous pression réduite (2 kPa; 50°C). Le résidu est purifié par chromatographie sous pression d'argon de 50 kPa, sur colonne de gel de silice ( granulomètrie 40-60 µm ; diamètre 3.5 cm), en éluant par cyclohexane-acétate d'éthyle (80/20 en volumes) et en recueillant des fractions de 30 cm³. Les fractions contenant le produit attendu, sont réunies et évaporées sous pression réduite (2 kPa ; 50°C). Le résidu est repris par 2 fois 2 cm³ d'éther diisopropylique. On filtre sur verre fritté pour obtenir après séchage (90 Pa; 50°C), 220 mg de N-[6-[4-(phenylmethoxy)phenyl]-1H-indazol-3-yl]-butanamide sous forme d'un solide blanc, fondant à 220°C.

Spectre de R.M.N. ¹H (300 MHz, (CD₃)₂SO d6, δ en ppm) : 1,00 (t large, J = 7,5 Hz : 3H) ; 1,69 (mt : 2H) ; 2,41 (t, J = 7 Hz : 2H) ; 5,20 (s : 2H) ; 7,15 (d, J = 8,5 Hz : 2H) ; de 7,30 à 7,50 (mt : 3H) ; 7,33 (d large, J = 9 Hz : 1H) ; 7,51 (d large, J = 7,5 Hz : 2H) ; 7,57 (s large : 1H) ; 7,68 (d, J = 8,5 Hz : 2H) ; 7,83 (d, J = 9 Hz : 1H) ; 10,31 (mf : 1H) ; 12,64 (mf : 1H).

### EXEMPLE 33

### N-[6-(4-hydroxyphenyl)-1H-indazol-3-yl]-butanamide

A 200 mg de N-[6-[4-(phenylmethoxy)phenyl]-1H-indazol-3-yl]-butanamide décrit précédemment, dans 7.5 cm³ d'acétonitrile, on additionne 0.15 cm³ de iodotriméthylsilane puis 5 cm³ de tétrahydrofuranne et on chauffe le milieu vers 82°C pendant 2 heures. On ajoute 0.15 cm³ de iodotriméthylsilane et on poursuit le chauffage pendant 17 heures. Le milieu réactionnel est ensuite évaporé à sec , sous pression réduite (2 kPa ; 40°C). Le résidu est repris par 75 cm³ d'acétate d'éthyle, puis on lave avec 2 fois 50 cm³ d'une solution aqueuse saturée en sulfate de sodium et avec 50 cm³ d'une solution saturée en chlorure de sodium. La phase organique est séchée sur sulfate de magnésium, filtrée puis évaporée sous pression réduite (2 kPa ; 45°C). ). Le résidu est purifié par chromatographie sous pression d'argon de 50 kPa, sur colonne de gel de silice (granulomètrie 40-60 µm ; diamètre 1.5 cm), en éluant par de l'acétate d'éthyle et en recueillant des fractions de 30 cm³. Les fractions contenant le produit attendu, sont réunies et évaporées sous pression réduite (2 kPa ; 50°C) . Le résidu est lavé avec 3 fois 5 cm³ d'éther diisopropylique. On le filtre sur verre fritté , et on obtient après séchage (90 Pa; 40°C), 100 mg de N-[6-(4-hydroxyphenyl)]-1H-indazol-3-yl]-butanamide sous forme d'un solide blanc fondant vers 235 °C.

Spectre de R.M.N. ¹H (300 MHz, (CD₃)₂SO d6, δ en ppm) : 0,98 (t large, J = 7,5 Hz : 3H) ; 1,68 (mt : 2H) ; 2,40 (t, J = 7,5 Hz : 2H) ; 6,88 (d, J = 8,5 Hz : 2H) ; 7,29 (d large, J = 9 Hz : 1H) ; 7,51 (s large : 1H) ; 7,55 (d, J = 8,5 Hz : 2H) ; 7,80 (d, J = 9 Hz : 1H) ; 9,56 (s large : 1H) ; 12,29 (mf : 1H).

### EXEMPLE 34

### N-[6-chloro-1H-indazol-3-yl]-benzenamide

A 1 g de 6-chloro-1H-indazole-3-amine dans 15 cm³ de pyridine, refroidit vers 3°C, on additionne 0.69 cm³ de chlorure de benzoyle. On laisse le milieu revenir vers 19°C pendant 12 heures et on évapore le milieu réactionnel sous pression réduite (2 kPa ; 50°C). On reprend le résidu par 25 cm³ d'acétate d'éthyle et par 25 cm³ d'eau distillée. La phase organique est lavée par 25 cm³ d'eau distillée et par 25 cm³ d'une solution aqueuse saturée en chlorure de sodium. On sèche sur sulfate de magnésium, on filtre sur verre fritté puis on évapore sous pression réduite (2 kPa ; 50°C). Le résidu est purifié par chromatographie sous pression d'argon de 50 kPa, sur colonne de gel de silice (granulométrie 40-60µm ; diamètre 4 cm), en éluant par un mélange dichlorométhane-méthanol (99/1 en volumes) et en recueillant des fractions de 15 cm³. Les fractions contenant le produit attendu, sont réunies et évaporées sous pression réduite (2 kPa ; 50°C). Après séchage (90 Pa ; 45°C), on obtient 990 mg de N-[6-chloro-1H-indazol-3-yl]-benzenamide, sous forme d'un solide blanc fondant à 188°C.

Spectre de R.M.N. ¹H (300 MHz, (CD₃)₂SO d6, δ en ppm) : 7,13 (dd, J = 9 et 1,5 Hz : 1H) ; de 7,50 à 7,70 (mt : 3H) ; 7,59 (s large : 1H) ; 7,82 (d, J = 9 Hz : 1H) ; 8,10 (d large, J = 7,5 Hz : 2H) ; 10,88 (mf : 1H) ; 12,95 (mf : 1H).

### EXEMPLE 35

### N-[6-(3,5-difluorophenyl)-1-[[-(triméthylsilyl)éthoxy]méthyl]-1H-indazol-3-yl]-butanamide

A 1 g de N-[6-chloro-1-[[2-(triméthylsilyl)éthoxy]méthyl]-1H-indazol-3-yl]-butanamide précédemment décrit dans l'exemple 25, dans 30 cm³ de dioxanne, on additionne 645 mg d'acide 3,4-difluoro-phénylboronique, 1.24 g de fluorure de césium, 13 mg d'acétate de palladium et enfin 31 mg de biphényl-2-dicyclohexylphosphine-2-(N,N-diméthylamino). On chauffe ensuite vers 100°C pendant 17 heures. On laisse ensuite revenir vers 19°C puis on filtre sur verre fritté et on évapore sous pression réduite (2 kPa ; 50°C). Le résidu est repris par 75 cm³ d'acétate d'éthyle et par 50 cm³ d'eau distillée. On filtre l'insoluble sur verre fritté La phase organique est séchée sur sulfate de magnésium, filtrée sur verre fritté puis évaporée sous pression réduite dans les conditions décrites précédemment. Le résidu est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (80/20 en volumes) et en recueillant des fractions de 35 cm³. Les fractions contenant le produit attendu, sont réunies et évaporées sous pression réduite (2 kPa ; 50°C). On obtient 1.1 g de N-[6-(3,5-difluorophenyl)-1-[[2-(triméthylsilyl)éthoxy]méthyl]-1H-indazol-3-yl]-butanamide sous forme d'une huile orange.

Spectre de R.M.N. ¹H (400 MHz, (CD₃)₂SO d6, δ en ppm) : - 0,08 (s : 9H) ; 0,85 (t, J = 8 Hz : 2H) ; 0,98 (t, J = 7,5 Hz : 3H) ; 1,70 (mt : 2H) ; 2,43 (t, J = 7,5 Hz : 2H) ; 3,59 (t, J = 8 Hz : 2H) ; 5,77 (s : 2H) ; 7,28 (tt, J = 9 et 2 Hz : 1H) ; 7,55 (dd, J = 9 et 2 Hz : 1H) ; 7,59 (mt : 2H) ; 7,95 (d, J = 9 Hz : 1H) ; 8,12 (s large : 1H) ; 10,53 (mf : 1H).

### N-[6-(3,5-difluorophenyl)-1H-indazol-3-yl]-butanamide

A 1.1 g de N-[6-(3,5-difluorophenyl)-1-[[2-(triméthylsilyl)éthoxy]méthyl]-1H-indazol-3-yl]-butanamide, décrit précédemment, dans 30 cm³ de tétrahydrofuranne, on additionne 14 cm³ de fluorure de tétrabutylammonium en solution 1M dans le tétrahydrofuranne. On chauffe le milieu réactionnel vers 66°C pendant 18 heures. On arrête ensuite le chauffage et on ajoute 100 cm³ d'acétate d'éthyle. On lave avec 2 fois 50 cm³ d'une solution aqueuse saturée en hydrogénocarbonate de sodium puis par 50 cm³ d'une solution aqueuse saturée en chlorure de sodium. La phase organique est séchée sur sulfate de magnésium, filtrée sur verre fritté et évaporée sous pression réduite (2 kPa; 50°C). Le résidu est purifié par chromatographie sous pression d'argon de 50 kPa, sur colonne de gel de silice (granulométrie 40-60 µm ; diamètre 3.5 cm), en éluant par cyclohexane-acétate d'éthyle (60/40 en volumes) et en recueillant des fractions de 35 cm³. Les fractions contenant le produit attendu, sont réunies et évaporées sous pression réduite (2 kPa ; 50°C). Le résidu est repris par 2 fois 5 cm³ d'éther diisopropylique. On filtre sur verre fritté et sèche sous pression réduite (90 Pa ; 50°C) pour obtenir 340 mg de N-[6-(3,5-difluorophenyl)-1H-indazol-3-yl]-butanamide sous forme d'un solide blanc fondant à 260°C.

Spectre de R.M.N. ¹H (300 MHz, (CD3)₂SO d6, δ en ppm) : 1,00 (t, J = 7 Hz : 3H) ; 1,70 (mt : 2H) ; 2,42 (t, J = 7 Hz : 2H) ; 7,27 (tt, J = 9 et 2 Hz : 1H) ; 7,43 (dd, J = 9 et 2 Hz : 1H) ; 7,52 (mt : 2H) ; 7,76 (s large : 1H) ; 7,90 (d, J = 9 Hz : 1H) ; 10,37 (mf : 1H) ; 12,83 (mf étalé : 1H).

### EXEMPLE 36

### N-[6-(3-thiophenyl)-1-[[2-(triméthylsilyl)éthoxy]méthyl]-1H-indazol-3-yl] butanamide

A 1 g de N-[6-chloro-1-[[2-(triméthylsilyl)éthoxy]méthyl]-1H-indazol-3-yl]-butanamide précédemment décrit dans l'exemple 25, dans 30 cm³ de dioxanne, on additionne 522 mg d'acide 3-thiényl-boronique, 1.24 g de fluorure de césium, 13 mg d'acétate de palladium et enfin 31 mg de biphényl-2-dicyclohexylphosphine-2-(N,N-diméthylamino). On chauffe ensuite vers 100°C pendant 2 heures. On ajoute 31 mg de biphényl-2-dicyclohexylphosphine-2-(N,N-diméthylamino) et 13 mg d'acétate de palladium et on maintient le reflux pendant 17 heures. On laisse ensuite revenir vers 19°C puis on filtre sur verre fritté et on ajoute 75 cm³ d'acétate d'éthyle et par 50 cm³ d'eau distillée. On filtre l'insoluble sur verre fritté. La phase organique est séchée sur sulfate de magnésium, filtrée sur verre fritté puis évaporée sous pression réduite dans les conditions décrites précédemment. Le résidu est purifié par chromatographie sous pression d'argon de 50 kPa, sur colonne de gel de silice ( granulométrie 40-60 µm ; diamètre 4.5 cm), en éluant par un mélange cyclohexane-acétate d'éthyle (90/10 en volumes) et en recueillant des fractions de 50 cm³. Les fractions contenant le produit attendu, sont réunies et évaporées sous pression réduite ( 2 kPa ; 50°C) . On obtient 570 mg de N-[6-(3-thiophenyl)-1-[[2-(triméthylsilyl)éthoxy]méthyl]-1H-indazol-3-yl] butanamide sous forme d'une huile jaune.

Spectre de R.M.N. ¹H (300 MHz, (CD₃)₂SO d6, δ en ppm) : - 0,08 (s : 9H) ; 0,85 (t, J = 8 Hz : 2H) ; 0,99 (t, J = 7,5 Hz : 3H) ; 1,69 (mt : 2H) ; 2,42 (t, J = 7,5 Hz : 2H) ; 3,58 (t, J = 8 Hz : 2H) ; 5,72 (s : 2H) ; 7,55 (dd, J = 8,5 et 1,5 Hz : 1H) ; 7,71 (d, J = 2 Hz : 2H) ; 7,88 (d, J = 8,5 Hz : 1H) ; 8,00 (t, J = 2 Hz : 1H) ; 8,02 (s large : 1H) ; 10,45 (mf : 1H).

### N-[6-(3-thiophenyl)-1H-indazol-3-yl]butanamide

A 570 mg de N-[6-(3-thiophenyl)-1-[[2-(triméthylsilyl)éthoxy]méthyl]-1H-indazol-3-yl]_butanamide, décrit précédemment, dans 20 cm³ de tétrahydrofuranne, on additionne 8.2 cm³ de fluorure de tétrabutylammonium en solution 1M dans le tétrahydrofuranne. On chauffe le milieu réactionnel vers 66°C pendant 18 heures. On arrête ensuite le chauffage et on ajoute 75 cm³ d'acétate d'éthyle. On lave avec 2 fois 50 cm³ d'une solution aqueuse saturée en hydrogénocarbonate de sodium puis par 50 cm³ d'une solution aqueuse saturée en chlorure de sodium. La phase organique est séchée sur sulfate de magnésium, filtrée sur verre fritté et évaporée sous pression réduite ( 2 kPa ; 50°C). Le résidu est purifié par chromatographie sous pression d'argon de 50 kPa, sur colonne de gel de silice ( granulométrie 40-60µm ; diamètre 2.5 cm), en éluant par cyclohexane-acétate d'éthyle (60/40 en volumes) et en recueillant des fractions de 20 cm³. Les fractions contenant le produit attendu, sont réunies et évaporées sous pression réduite (2 kPa ; 50°C). Le résidu est repris avec 2 fois 5 cm³ d'éther diisopropylique. On filtre sur verre fritté et on évapore sous pression réduite (2 kPa ; 40°C) pour obtenir, après séchage (90 Pa ; 50°C), 260 mg de N-[6-(3-thiophenyl)-1H-indazol-3-yl] butanamide sous forme d'un solide blanc fondant vers 198°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,40 (t, J = 7 Hz : 2H) ; 7,45 (d large, J = 9 Hz : 1H) ; de 7,60 à 7,75 (mt : 2H) ; 7,70 (s large : 1H) ; 7,82 (d, J = 9 Hz : 1H) ; 7,95 (dd, J = 3 et 1,5 Hz : 1H) ; 10,32 (mf: 1H) ; 12,66 (mf étalé : 1H).

### EXEMPLE 37

### N-[6-chloro-1H-indazol-3-yl]-2-thiophenacétamide

A 1 g de 6-chloro-1H-indazole-3-amine dans 15 cm³ de pyridine, refroidit vers 3°C, on additionne 0.73 cm³ de chlorure de 2-thiopheneacétyle. On laisse le milieu revenir vers 19°C pendant 21 heures et on évapore le milieu réactionnel sous pression réduite (2 kPa ; 50°C). On reprend le résidu par 25 cm³ d'acétate d'éthyle, par 10 cm³ de tétrahydrofuranne et par 25 cm³ d'eau distillée. La phase organique est lavée par 25 cm³ d'eau distillée et par 25 cm³ d'une solution aqueuse saturée en chlorure de sodium. On sèche sur sulfate de magnésium, on filtre sur verre fritté, rince avec 5 cm³ de diméthylformamide, puis on évapore sous pression réduite (2 kPa ; 50°C). Le résidu est purifié par chromatographie sous pression d'argon de 50 kPa, sur colonne de gel de silice (granulométrie 40-60 µm ; diamètre 4 cm), en éluant par un mélange dichlorométhane-méthanol (99/1 en volumes) et en recueillant des fractions de 15 cm³. Les fractions contenant le produit attendu, sont réunies et évaporées sous pression réduite (2 kPa ; 50°C). Après séchage (90 Pa ; 45°C), on obtient 210 mg de N-[6-chloro-1H-indazol-3-yl]-2-thiophenacétamide sous forme d'un solide blanc fondant à 210°C.

Spectre de R.M.N. ¹H (300 MHz, (CD₃)₂SO d6, δ en ppm) : 3,99 (s : 2H) ; de 6,95 à 7,10 (mt : 2H) ; 7,09 (dd, J = 9 et 2 Hz : 1H) ; 7,43 (dd, J = 5et 1,5 Hz : 1H) ; 7,53 (d, J = 2 Hz : 1H) ; 7,82 (d, J = 9 Hz : 1H) ; 10,76 (mf : 1H) ; de 12,50 à 13,20 (mf étalé : 1H).

### EXEMPLE 38 :

### N-[5-[[(3-fluorophenyl)sulfonyl]amino]-1H-indazol-3-yl]-benzamide

Le N-[5-[[(3-fluorophenyl)sulfonyl]amino]-1H-indazol-3-yl]-benzamide peut être obtenu à partir 0,45 g de N-(5-amino-1H-indazol-3-yl)-benzamide, de 10 cm³ de pyridine et de 0,35 g de chlorure de (3-fluorophenyl)sulfonyle. On obtient ainsi 0,6 g de N-[5-[((3-fluorophenyl)sulfonyl]amino]-1H-indazol-3-yl]-benzamide sous forme de solide blanc fondant à 225°C (Analyse C20 H15 F N4 03 S , % calculé C : 58,53, H : 3,68, F : 4,63, N : 13,65, O : 11,69, S : 7,81, % trouvé C : 58,38, H : 3,42, N : 13,56, S : 7,44).

R.M.N. ¹H (300 MHz, (CD3)2SO d6, δ en ppm) : 7,10 (dd, J = 9 et 2 Hz : 1H) ; 7,39 (d, J = 9 Hz : 1H) ; de 7,40 à 7,70 (mt : 7H) ; 7,42 ((s large : 1H) ; 8,07 (d large, J = 7,5 Hz : 2H) ; 10,20 (mf étalé : 1H) ; 10,72 (s large : 1H) ; 12,77 (s large : 1H).

Le N-(5-amino-1H-indazol-3-yl)-benzamide peut être obtenu à partir de 0,6 g de N-(5-nitro-1H-indazol-3-yl)-benzamide, de 21 cm³ d'éthanol, de 4,2 g de sulfate ferreux, de 6,6 cm³ d'eau et de 5,1 cm³ d'ammoniaque à 32%. On obtient ainsi 0,4 g de N-(5-amino-1H-indazol-3-yl)-benzamide sous forme d'une poudre jaune fondant à 116°C.

Le N-(5-nitro-1H-indazol-3-yl)-benzamide peut être obtenu de la manière suivante : à une solution de 0,6 g de 5-nitro-1H-indazole-3-amine et de 5 cm³ de pyridine refroidie à 0°C est ajouté goutte à goutte 0,39 cm³ de chlorure de benzoyle. Le milieu est ramené à une température voisine de 20°C et maintenu sous agitation pendant 18 heures. Après addition de 20 cm³ d'eau distillée, le milieu est extrait par 20 cm³ et 10 cm³ d'acétate d'éthyle. Les phases organiques sont réunies, séchées sur sulfate de magnésium, filtrées et concentrées par évaporation sous pression réduite. Le résidu ainsi obtenu est purifié par chromatographie sur colonne de silice avec un mélange dichlorométhane-méthanol (99-1 en volumes) comme éluant. On obtient ainsi 0,9 g de N-(5-nitro-1H-indazol-3-yl)-benzamide sous forme d'un solide orange fondant à 231°C.

### EXEMPLE 39

### N-[6-(2-phényléthyl)-1-[[2-(triméthylsilyl)éthoxy]méthyl]-1H-indazol-3-yl]-butanamide

A une solution de 0,8 cm³ de styrène dans 35 cm³ de dioxane on ajoute à la seringue 27,2 cm³ de 9-borabicyclo[3.3.1]nonane et on chauffe à 75°C pendant 1 heure. A la solution refroidie on ajoute 5,5 cm³ de soude 5N puis successivement 1g de N-[6-chloro-1-[[2-(triméthylsilyl)éthoxy]méthyl]-1H-indazol-3-yl]-butanamide préparé dans l'exemple 25 , 1,2 g de fluorure de césium, 32,2 mg de 2-dicyclohexylphosphino-2'-(N,N-diméthylamino)biphényle, 12,3 mg d'acétate de palladium et on chauffe à reflux pendant 3 heures. Après refroidissement, on ajoute 50 cm³ d'eau et 75 cm³ d'acétate d'éthyle ; la phase organique est décantée, séchée sur sulfate de magnésium, filtrée et concentrée sous pression réduite (2 kPa ; 40°C) pour donner 4,5 g de brut qui est chromatographié sous pression d'argon de 50 kPa, sur une colonne de gel de silice (granulométrie 40-60 µm ; diamètre 3.5 cm), en éluant par un mélange cyclohexane-acétate d'éthyle (75/25 en volumes). Les fractions contenant le produit attendu sont réunies puis évaporées sous pression réduite (2 kPa ; 40°C) pour donner 1,4 g de N-[6-(2-phényléthyl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,06 (s : 9H) ; 0,83 (t, J = 8 Hz : 2H) ; 0,97 (t, J = 7,5 Hz : 3H) ; 1,67 (mt : 2H) ; 2,39 (t, J = 7 Hz : 2H) ; 3,00 (mt : 4H) ; 3,53 (t, J = 8 Hz : 2H) ; 5,62 (s : 2H) ; 7,04 (d large, J = 8,5 Hz : 1H) ; de 7,15 à 7,40 (mt : 5H) ; 7,50 (s large : 1H) ; 7,74 (d, J = 8,5 Hz : 1H) ; 10,38 (mf : 1H).

EI m/z = 437 M^{+.}

m/z = 320 [M - OCH₂CH₂Si(CH₃)₃]⁺

m/z = 309 [M -C₆H₁₂OSi]^{+.}

### N-[6-(2-phényléthyl)-1H-indazol-3-yl]-butanamide

A une solution de 1,4g de *N*-[6-(2-phényléthyl)-1-[[2-(triméthylsilyl)éthoxy]méthyl]-1*H*-indazol-3-yl]-butanamide décrit précédemment dans 40 cm³ de tétrahydrofurane on ajoute 19,2 cm³ de fluorure de tétrabutylammonium en solution 1M dans le tétrahydrofurane et on chauffe au reflux pendant 18 heures. Au milieu réactionnel on ajoute 100 cm³ d'acétate d'éthyle et la phase organique est lavée successivement par 100 cm³ d'une solution saturée en hydrogénocarbonate de sodium, 100 cm³ d'eau et 50 cm³ d'une solution saturée en chlorure de sodium. La phase organique est décantée, séchée sur sulfate de magnésium, filtrée et concentrée sous pression réduite (2 kPa ; 50°C) pour donner 1,4 g de brut sous forme d'huile orangée qui est purifiée par chromatographie sous pression d'argon de 50 kPa, sur une colonne de gel de silice (granulométrie 40-60 µm ; diamètre 3.5 cm), en éluant par un mélange cyclohexane-acétate d'éthyle (30/70 en volumes). Les fractions contenant le produit attendu sont concentrées à sec pour donner 0,43 g d'une huile jaune qui après trituration dans 20 cm³ d'éther diisopropylique et filtration donne 0,34 g de solide blanc à 70 % de pureté. Après purification par HPLC-MS on obtient 0,11 g de produit qui est trituré avec 10 cm³ d'éther diisopropylique, filtré et lavé avec 5 cm³ d'éther diisopropylique ; le produit est séché sous pression réduite (90 Pa ; 40°C) pour donner 0,10 g de N-[6-(2-phényléthyl)-1H-indazol-3-yl]-butanamide sous forme de solide blanc fondant à 175°C.

Spectre de R.M.N. ¹H (300 MHz, (CD₃)₂SO d6, δ en ppm) : 0,97 (t, J = 7,5 Hz : 3H) ; 1,67 (mt : 2H) ; 2,38 (t, J = 7 Hz : 2H) ; 2,99 (mt :4H) ; 6,97 (d large, J = 9Hz : 1H) ; de 7,15 à 7,35 (mt : 5H) ; 7,20 (s large : 1H) ; 7,77 (d, J = 9 Hz : 1H) ; 10,22 (s large : 1H) ; 12,44 (s large : 1H).

### EXEMPLE 40

### 6,7-difluoro-1H-indazole-3-amine:

A 0.46 cm³ de 2,3,4-trifluorobenzonitrile dans 10 cm³ d'éthanol absolu, on additionne 0.32 cm³ d'hydrazine monohydratée. On chauffe le milieu vers 75°C pendant 17 heures puis on ajoute 10 cm³ d'acétate d'éthyle, 5 cm³ de tétrahydrofurane et 5 cm³ d'eau distillée. La phase organique est décantée et relavée avec 10 cm³ d'eau distillée puis avec 10 cm³ d'une solution aqueuse saturée en chlorure de sodium. La phase organique est décantée, séchée sur sulfate de magnésium, filtrée et concentrée à sec sous pression réduite (2 kPa ; 50°C). Le résidu obtenu est purifié par chromatographie sous pression d'argon de 50 kPa, sur une colonne de gel de silice ( granulométrie 40-60 µm ; diamètre 1.5 cm), en éluant par un mélange cyclohexane-acétate d'éthyle (50/50 en volumes). Les fractions contenant le produit attendu sont réunies puis évaporées sous pression réduite (2 kPa ; 40°C) ; on obtient après séchage (90 Pa; 40°C), 100 mg de 6,7-difluoro-1H-indazole-3-amine sous forme d'un solide blanc fondant à 183°C.

Spectre de R.M.N. ¹H (300 MHz, (CD₃)₂SO d6, δ en ppm) : 5,57 (mf : 2H) ; 6,93 (mt : 1H) ; 7,52 (ddd, J = 8,5 - 4,5 et 1 Hz : 1H) ; 12,01 (mf : 1H).

### N-(6,7-difluoro-1H-indazol-3-yl)-butanamide:

A 1 g de 6,7-difluoro-1H-indazole-3-amine décrit précédemment, dans 15 cm³ de pyridine, on ajoute 0.61 cm³ de chlorure de butyryle après avoir refroidi vers 3°Cpuis on laisse à température ambiante pendant 76 heures. Le milieu réactionnel est concentré sous pression réduite (2 kPa ; 40°C) et le résidu est repris par 25 cm³ d'acétate d'éthyle et par 25 cm³ d'eau. La phase organique est lavée avec 25 cm³ d'eau distillée puis avec 25 cm³ d'une solution aqueuse saturée en chlorure de sodium. Après séchage sur sulfate de magnésium, filtration et concentration sous pression réduite (2 kPa ; 40°C), le résidu obtenu est purifié par chromatographie sous pression d'argon de 50 kPa, sur une colonne de gel de silice (granulométrie 40-60 µm ; diamètre 3 cm), en éluant par un mélange dichlorométhane-méthanol (98/2 en volumes). Les fractions contenant le produit attendu sont réunies puis évaporées sous pression réduite (2 kPa ; 40°C) ; on obtient après séchage (90 Pa ; 40°C), 596 mg de N-(6,7-difluoro-1H-indazol-3-yl)-butanamide sous forme d'un solide blanc fondant à 191°C.

Spectre de R.M.N. ¹H (300 MHz, (CD₃)₂SO d6, δ en ppm) : 0,97 (t, J = 7,5 Hz : 3H) ; 1,67 (mt : 2H) ; 2,40 (t, J = 7 Hz : 2H) ; 7,10 (mt : 1H) ; 7,63 (dd large, J = 9 et 4,5 Hz : 1H) ; 10,47 (mf étalé : 1H) ; 13,35 (mf étalé : 1H).

### EXEMPLE 41

### N-[6-(4-méthoxyphényl)-1-[[2-(triméthylsilyl)éthoxy]méthyl]-1H-indazol-3-yl]-butanamide

A 1g de N-[6-chloro-1-[[2-(triméthylsilyl)éthoxy]méthyl]-1H-indazol-3-yl]-butanamide précédemment décrit à l'exemple 25 , dans 30 cm³ de dioxane, on additionne 900 mg d'acide 4-méthoxyphénylboronique, 1.24 g de fluorure de césium, 13.5 mg d'acétate de palladium et enfin 31 mg de 2-dicyclohexylphosphine-2'-(N,N-diméthylamino)biphényle. On chauffe ensuite vers 100°C pendant 20 heures et on laisse la température revenir vers 19°C pendant 72 heures puis on filtre le milieu réactionnel sur verre fritté et on évapore sous pression réduite (2 kPa ; 50°C). Le résidu est repris par 50 cm³ d'acétate d'éthyle et par 50 cm³ d'eau distillée. La phase organique est relavée avec 50 cm³ d'eau distillée et avec 50 cm³ d'une solution aqueuse saturée en chlorure de sodium. La phase aqueuse est séchée sur sulfate de magnésium, filtrée, puis évaporée sous pression réduite dans les conditions décrites précédemment. Le résidu est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (80/20 en volumes). Les fractions contenant le produit attendu, sont réunies et évaporées sous pression réduite (2 kPa ; 50°C) ; on obtient après séchage (90 Pa ; 50°C), 1 g de N-[6-(4-méthoxyphényl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,09 (s : 9H) ; 0,83 (t, J = 8 Hz : 2H) ; 0,97 (t, J = 7,5 Hz : 3H) ; 1,68 (mt : 2H) ; 2,41 (t, J = 7 Hz : 2H) ; 3,56 (t, J = 8 Hz : 2H) ; 3,83 (s : 3H) ; 5,72 (s large : 2H) ; 7,08 (d large, J = 8,5 Hz : 2H) ; 7,42 (d large, J = 8,5 Hz : 1H) ; 7,72 (d, J = 8,5 Hz : 2H) ; de 7,85 à 7,95 (mt : 2H) ; 10,45 (mf : 1H).

EI m/z = 437 M^{+.}

m/z = 320 [M - OCH₂CH₂Si(CH₃)₃]⁺

m/z = 309 [M - C₆H₁₂OSi]^{+.}

### N-[6-(4-méthoxyphényl)-1H-indazol-3-yl]-butanamide

A 1 g de N-[6-(4-méthoxyphényl)-1-[[2-(triméthylsilyl)éthoxy]méthyl]-1H-indazol-3-yl]-butanamide décrit précédemment, dans 30 cm³ de tétrahydrofurane, on additionne 13.6 cm³ de fluorure de tétrabutylammonium en solution 1M dans le tétrahydrofurane puis on chauffe le milieu réactionnel vers 66°C pendant 19 heures. On arrête ensuite le chauffage et on ajoute 75 cm³ d'acétate d'éthyle. On lave avec 2 fois 50 cm³ d'une solution aqueuse saturée en hydrogénocarbonate de sodium puis avec 50 cm³ d'une solution aqueuse saturée en chlorure de sodium. La phase organique est séchée sur' sulfate de magnésium, filtrée et concentrée à sec sous pression réduite (2 kPa ; 50°C). Le résidu est purifié par chromatographie sous pression d'argon de 50 kPa, sur colonne de gel de silice (granulométrie 40-60 µm ; diamètre 2.5 cm), en éluant avec un mélange acétate d'éthyle-cyclohexane (60/40 en volumes). Les fractions contenant le produit attendu sont réunies et évaporées sous pression réduite (2 kPa ; 50°C). Le résidu est repris par 10 cm³ d'éther diisopropylique, filtré et lavé avec 2 fois 5 cm³ d'éther diisopropylique puis avec 2 fois 5 cm³ d'acétate d'éthyle ; on obtient après séchage (90 Pa; 50°C), 500 mg de N-[6-(4-méthoxyphényl)-1H-indazol-3-yl]-butanamide sous forme d'un produit blanc fondant à 210°C.

Spectre de R.M.N. ¹H (300 MHz, (CD₃)₂SO d6, δ en ppm) : 0,99 (t, J = 7,5 Hz : 3H) ; 1,69 (mt : 2H) ; 2,41 (t, J = 7 Hz : 2H) ; 3,83 (s : 3H) ; 7,06 (d mt, J = 8,5 Hz : 2H) ; 7,33 (dd, J = 9 et 1,5 Hz : 1H) ; 7,56 (s large : 1H) ; 7,78 (d mt, J = 8,5 Hz : 2H) ; 7,83 (d, J = 9 Hz : 1H) ; 10,31 (mf : 1H) ; 12,62 (mf : 1H).

### Exemple 42

### N-[6-[4-(méthylthio)phényl]-1-[[2-(triméthylsilyl)éthoxy]méthyl]-1H-indazol-3-yl]-butanamide

A 1g de N-[6-chloro-1-[[2-(triméthylsilyl)éthoxy]méthyl]-1H-indazol-3-yl]-butanamide précédemment décrit à l'exemple 25, dans 30 cm³ de dioxane, on additionne 0,81 g d'acide 4-méthylthiophénylboronique à 86%, 1.24 g de fluorure de césium, 13.5 mg d'acétate de palladium et enfin 31 mg de 2-dicyclohexylphosphine-2-(N,N-diméthylamino)biphényle. On chauffe ensuite vers 100°C pendant 20 heures et on laisse la température revenir à l'ambiante pendant 72 heures puis on filtre le milieu réactionnel sur verre fritté et on évapore sous pression réduite (2 kPa ; 50°C). Le traitement et la purification s'effectuent par analogie à l'exemple 41 précédant ; on obtient ainsi 0,60 g de N-[6-(4-méthylthiophényl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,09 (s : 9H) ; 0,83 (t, J = 8 Hz : 2H) ; 0,97 (t, J = 7,5 Hz : 3H) ; 1,68 (mt : 2H) ; 2,41 (t, J = 7 Hz : 2H) ; 2,55 (s : 3H) ; 3,57 (t, J = 8 Hz : 2H) ; 5,73 (s : 2H) ; 7,40 (d large, J = 8,5 Hz : 2H) ; 7,45 (dd, J = 8,5 et 1,5 Hz : 1H) ; 7,75 (d, J = 8,5 Hz : 2H) ; 7,91 (d, J = 8,5 Hz : 1H) ; 7,94 (s : 1H) ; 10,47 (mf : 1H).

EI m/z = 455 M^{+.}

m/z = 338 [M-OCH₂CH₂Si(CH₃)₃]⁺

m/z=327 [M-C₆H₁₂OSi]^{+.}

### N-[6-(4-méthylthiophényl)-1H-indazol-3-yl]-butanamide

A 600 mg de N-[6-[4-(méthylthio)phényl]-1-[[2-(triméthylsilyl]éthoxy])méthyl]-1H-indazol-3-yl]-butanamide décrit précédemment, dans 30 cm³ de tétrahydrofurane, on additionne 7.9 cm³ de fluorure de tétrabutylammonium en solution 1M dans le tétrahydrofurane. On chauffe le milieu réactionnel vers 66°C pendant 18 heures puis on arrête ensuite le chauffage et on ajoute 75 cm³ d'acétate d'éthyle. On lave avec 2 fois 50 cm³ d'une solution aqueuse saturée en hydrogénocarbonate de sodium puis avec 50 cm³ d'une solution aqueuse saturée en chlorure de sodium. La phase organique est décantée, séchée sur sulfate de magnésium, filtrée et concentrée sous pression réduite (2 kPa ; 50°C). Le résidu est purifié par chromatographie sous pression d'argon de 50 kPa, sur colonne de gel de silice (granulométrie 40-60 µm ; diamètre 2.5 cm), en éluant par un mélange acétate d'éthyle-cyclohexane (60/40 en volumes). Les fractions contenant le produit attendu, sont réunies et évaporées sous pression réduite (2 kPa; 50°C). Le résidu est repris par 10 cm³ d'éther diisopropylique, filtré et lavé avec 2 fois 5 cm³ d'éther diisopropylique puis avec 2 fois 3 cm³ d'acétate d'éthyle ; on obtient après séchage (90 Pa ; 50°C), 320 mg de N-[6-[4-(méthylthio)phényl]-1H-indazol-3-yl]-butanamide sous forme d'un produit blanc fondant à 225°C.

Spectre de R.M.N. ¹H (300 MHz, (CD₃)₂SO d6, δ en ppm) : 0,99 (t, J = 7,5 Hz : 3H) ; 1,69 (mt : 2H) ; 2,40 (t, J = 7 Hz : 2H) ; 2,54 (s : 3H) ; 7,36 (dd, J = 9 et 1,5 Hz : 1H) ; 7,39 (d, J = 8,5 Hz : 2H) ; 7,62 (s large : 1H) ; 7,70 (d, J = 8,5 Hz : 2H) ; 7,86 (d, J = 9 Hz : 1H) ; 10,33 (mf : 1H) ; 12,69 (mf : 1H).

### EXEMPLE 43

### N-[6-[4-(trifluorométhoxy)phényl]-1-[[2-(triméthylsilyl)éthoxy]méthyl]-1H-indazol-3-yl]-butanamide

A 1g de N-[6-chloro-1-[[2-(triméthylsilyl)éthoxy]méthyl]-1H-indazol-3-yl]-butanamide précédemment décrit à l'exemple 25, dans 30 cm³ de dioxane, on additionne 840 mg d'acide 4-trifluorométhoxyphénylboronique, 1.24 g de fluorure de césium, 13.5 mg d'acétate de palladium et enfin 31 mg de 2-dicyclohexylphosphine-2-(N,N-diméthylamino)biphényle. On chauffe ensuite vers 102°C pendant 20 heures puis on laisse revenir à température ambiante et on dilue le milieu réactionnel avec 75 cm³ d'acétate d'éthyle, on filtre sur verre fritté garni de célite et on concentre à sec sous pression pression réduite (2 kPa ; 50°C). Le traitement et la purification s'effectuent par analogie à l'exemple 41 précédemment décrit On obtient ainsi 1 g de N-[6-[4-(trifluorométhoxy)phényl]-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,09 (s : 9H) ; 0,83 (t, J = 8 Hz : 2H) ; 0,98 (t, J = 7,5 Hz : 3H) ; 1,68 (mt : 2H) ; 2,42 (t, J = 7 Hz : 2H) ; 3,57 (t, J = 8 Hz : 2H) ; 5,74 (s large : 2H) ; 7,46 (dd, J = 8,5 et 1,5 Hz : 1H) ; 7,50 (d large, J = 8,5 Hz : 2H) ; 7,90 (d, J = 8,5 Hz : 2H) ; 7,94 (d, J = 8,5 Hz : 1H) ; 7,99 (s large : 1H) ; 10,49 (mf : 1H).

EI m/z = 493 M^{+.}

m/z = 376 [M-OCH₂CH₂Si(CH₃)₃]⁺

m/z = 365 [M-C₆H₁₂OSi]^{+.}

### N-[6-(4-trifluorométhoxyphényl)-1H-indazol-3-yl]-butanamide

A 1 g de N-[6-[4-(trifluorométhoxy)phényl]-1-[[2-(triméthylsilyl)éthoxy]méthyl]-1H-indazol-3-yl]-butanamide décrit précédemment, dans 30 cm³ de tétrahydrofurane, on additionne 12.1 cm³ de fluorure de tétrabutylammonium en solution 1M dans le tétrahydrofurane. On chauffe le milieu réactionnel vers 66°C pendant 18 heures puis on arrête ensuite le chauffage et on ajoute 75 cm³ d'acétate d'éthyle. On lave avec 2 fois 50 cm³ d'une solution aqueuse saturée en hydrogénocarbonate de sodium puis avec 50 cm³ d'une solution aqueuse saturée en chlorure de sodium. La phase organique est décantée, séchée sur sulfate de magnésium, filtrée puis concentrée sous pression réduite (2 kPa ; 50°C). Le résidu est purifié par chromatographie sous pression d'argon de 50 kPa, sur colonne de gel de silice (granulométrie 40-60 µm ; diamètre 2.5 cm), en éluant par un mélange acétate d'éthyle-cyclohexane (60/40 en volumes). Les fractions contenant le produit attendu, sont réunies et évaporées sous pression réduite (2 kPa ; 50°C) ; le résidu est repris par 10 cm³ d'éther diisopropylique, filtré sur verre fritté puis lavé successivement avec 10 cm³ d'éther diisopropylique puis avec 2 fois 2 cm³ d'acétate d'éthyle. On obtient après séchage (90 Pa; 50°C), 520 mg de N-[6-[4-(trifluorométhoxy)phényl]-1H-indazol-3-yl]-butanamide sous forme d'un produit blanc fondant à 234°C.

Spectre de R.M.N. ¹H (300 MHz, (CD₃)₂SO d6, δ en ppm) : 0,99 (t, J = 7,5 Hz : 3H) ; 1,69 (mt : 2H) ; 2,41 (t, J = 7 Hz : 2H) ; 7,38 (d large, J = 9 Hz : 1H) ; 7,52 (d, J = 8,5 Hz : 2H) ; 7,67 (s large : 1H) ; 7,86 (d, J = 8,5 Hz : 2H) ; 7,89 (d, J = 9 Hz : 1H) ; 10,36 (mf : 1H) ; 12,75 (mf : 1H).

### EXEMPLE 44

### N-[(6-(2-propènyl)-1-[[2-(triméthylsilyl)éthoxyl]méthyl]-1H-indazol-3-yl]-butanamide

A une solution de 1 g de N-[6-chloro-1-[[2-(triméthylsilyl)éthoxy]méthyl]-1H-indazol-3-yl)-butanamide préparé dans l'exemple 25, dans 30 cm³ de dioxane on ajoute successivement 1,24 g de fluorure de césium, 0,77 cm³ de 2-allyl-4,4,5,5-tétraméthyl-1,3,2-dioxaborolane, 31,5 mg de 2-dicyclohexylphosphino-2'-(N,N-diméthylamino)biphényle, 13,5 mg d'acétate de palladium et on chauffe au reflux pendant 18 heures. Le milieu réactionnel est filtré, repris par 2 fois 50 cm³ d'acétate d'éthyle et la phase organique est lavée successivement par 50 cm³ d'eau et 50 cm³ d'une solution saturée en chlorure de sodium. Après décantation de la phase organique, séchage sur sulfate de sodium, filtration et concentration à sec sous pression réduite (2 kPa ; 50°C) on obtient 1,3 g d'huile brune qui est purifiée par chromatographie sous pression d'argon de 50 kPa, sur colonne de gel de silice ( granulométrie 40-60 µm ; diamètre 4.5 cm), en éluant par un mélange cyclohexane-acétate d'éthyle (90/10 en volumes). Après concentration et séchage (90 Pa ; 45°C), on obtient 0,72 g de N-[(6-(2-propènyl-1-[[2-(triméthylsilyl)éthoxy]méthyl]-1H-indazol-3-yl]-butanamide sous forme d'une huile jaune à 75% de pureté.

Spectre de R.M.N. ¹H (300 MHz, (CD₃)₂SO d6, δ en ppm) : - 0,08 (s : 9H) ; 0,82 (t, J = 8 Hz : 2H) ; 0,96 (t, J = 7,5 Hz : 3H) ; 1,66 (mt : 2H) ; 2,39 (t, J = 7 Hz : 2H) ; de 3,45 à 3,60 (mt : 4H) ; de 5,05 à 5,20 (mt : 2H) ; 5,62 (s : 2H) ; 6,02 (mt : 1H) ; 6,98 (d large, J = 8,5 Hz : 1H) ; 7,45 (s large : 1H) ; 7,75 (d, J = 8,5 Hz : 1H) ; 10,38 (mf : 1H).

EI m/z = 373 M^{+.}

m/z = 256 [M -OCH₂CH₂Si(CH₃)₃]⁺

m/z = 245 [M-C₆H₁₂OSi]^{+.}

### N-[(6-(1-propényl)-1H-indazol-3-yl]-butanamide

A une solution de 0,70 g de N-[6-(1-propènyl)-1-[(2-(triméthylsilyl)éthoxy]méthyl]-1H-indazol-3-yl]-butanamide dans 25 cm³ de tétrahydrofurane on ajoute 11,2 cm³ de fluorure de tétrabutylammonium en solution 1M dans le tétrahydrofurane et on chauffe à reflux pendant 18 heures. Au milieu réactionnel on ajoute 75 cm³ d'acétate d'éthyle et on lave la phase organique successivement par 2 fois 50 cm³ d'une solution saturée en hydrogénocarbonate de sodium et par 2 fois 50 cm³ d'une solution saturée en chlorure de sodium. La phase organique est décantée, séchée sur sulfate de magnésium, filtrée et concentrée à sec sous pression réduite (2 kPa ; 50°C) pour donner 0,70 g d'un solide marron. Le brut est purifiée par chromatographie sous pression d'argon de 50 kPa, sur colonne de gel de silice (granulométrie 40-60 µm ; diamètre 2.5 cm), en éluant par un mélange cyclohexane-acétate d'éthyle (70/30 en volumes). Après concentration des fractions on obtient 0,30 g d'un mélange contenant 50% du produit attendu. Par une ultime HPLC (colonne Hypurity ; C₁₈, 5µm ; longueur 100 mm, diamètre 30 mm, éluant : méthanol-acétonitrile-eau (38/38/24 en volumes) contenant 0.05% d'acide trifluoroacétique ; débit 20 cm³/mn) et par concentration à sec des fractions, reprise dans 5 cm³ d'acétate d'éthyle, filtration et séchage (90 Pa; 45°C), on obtient 12 mg de N-[6-(1-propényl)-1H-indazol-3-yl]-butanamide sous forme de cristaux blanc fondant à 195°C.

Spectre de R.M.N. ¹H (300 MHz, (CD₃)₂SO d6, δ en ppm) : 0,97 (t large, J = 7,5 Hz : 3H) ; 1,66 (mt : 2H) ; 1,89 (d large, J = 6 Hz : 3H) ; 2,37 (t, J = 7 Hz : 2H) ; 6,38 (mt : 1H) ; 6,55 (d large, J = 16 Hz : 1H) ; 7,17 (d large, J = 8,5 Hz : 1H) ; 7,29 (s large : 1H) ; 7,69 (d, J = 9 Hz : 1H) ; 10,24 (mf : 1H) ; 12,52 (mf : 1H).

### EXEMPLE 45

### N-[6-chloro-1H-indazol-3-yl]-2-pyridinecarboxamide

A 1 g de 6-chloro-1H-indazole-3-amine dans 15 cm³ de pyridine, on additionne 4.2 cm³ de diisopropyléthylamine. Le milieu réactionnel est refroidit vers 8°C pour ajouter 1.08 g de chlorhydrate de chlorure de picolinoyle et on laisse revenir la température à l'ambiante pendant 18 heures. Le milieu réactionnel est concentré à sec sous pression réduite (2 kPa ; 40°C) puis le résidu est repris par 25 cm³ d'acétate d'éthyle et par 25 cm³ d'eau distillée. La phase organique est lavée avec 25 cm³ d'eau puis avec 25 cm³ d'une solution aqueuse saturée en chorure de sodium. Après séchage sur sulfate de magnésium, filtration et concentration sous pression réduite (2 kPa ; 40°C), le résidu est purifié par chromatographie sous pression d'argon de 50 kPa, sur colonne de gel de silice (granulométrie 40-60 µm ; diamètre 3 cm), en éluant par un mélange dichlorométhane-méthanol (99/1 en volumes). Les fractions contenant le produit attendu, sont réunies et évaporées sous pression réduite (2 kPa ; 50°C) et le résidu est repris par 2 fois 15 cm³ d'éther diisopropylique. Après filtration sur verre fritté et séchage sous pression réduite (90 Pa ; 50°C), on obtient 572 mg de N-[6-chloro-1H-indazol-3-yl]-2-pyridinecarboxamide sous forme d'un solide blanc fondant à 177°C.

Spectre de R.M.N. ¹H (300 MHz, (CD₃)₂SO d6, δ en ppm) : 7,14 (dd, J = 9 et 2 Hz : 1H) ; 7,60 (d, J = 2 Hz : 1H) ; 7,73 (ddd, J = 6,5 - 5 et 1,5 Hz : 1H) ; 7,95 (d, J = 9 Hz : 1H) ; 8,12 (t dédoublé, J = 7,5 et 2 Hz : 1H) ; 8,21 (d large, J = 7,5 Hz : 1H) ; 8,79 (d large, J = 5 Hz : 1H) ; de 10,50 à 11,40 (mf étalé : 1H) ; de 12,30 à 13,40 (mf très étalé : 1H).

### EXEMPLE 46

### N-[6-(4-fluorophényl)-1-[[2-(triméthylsilyl)éthoxy])méthyl]-1H-indazol-3-yl]-butanamide

A 1g de N-[6-chloro-1-[[2-(triméthylsilyl)éthoxy]méthyl]-1H-indazol-3-yl]-butanamide décrit à l'exemple 25, dans 30 cm³ de dioxane, on additionne 840 mg d'acide 4-fluorophénylboronique , 1.24 g de fluorure de césium, 13.5 mg d'acétate de palladium et enfin 31 mg de 2-dicyclohexylphosphine-2-(N,N-diméthylamino)biphényle. On chauffe ensuite vers 102°C pendant 22 heures puis on laisse revenir à la température ambiante. Le milieu réactionnel est repris par 75 cm³ d'acétate d'éthyle, filtré sur verre fritté garni de célite puis concentré à sec sous pression réduite (2 kPa ; 50°C). Le traitement et la purification s'effectuent par analogie à l'exemple 41. On obtient ainsi 580 mg de N-[6-(4-fluorophényl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,09 (s : 9H) ; 0,83 (t, J = 8 Hz : 2H) ; 0,97 (t, J = 7,5 Hz : 3H) ; 1,68 (mt : 2H) ; 2,41 (t, J = 7 Hz : 2H) ; 3,56 (t, J = 8 Hz : 2H) ; 5,73 (s : 2H) ; 7,35 (t, J = 9 Hz : 2H) ; 7,44 (dd large, J = 9 et 1,5 Hz : 1H) ; 7,82 (dd, J = 9 et 5,5 Hz : 2H) ; 7,92 (d, J = 9 Hz : 1H) ; 7,94 (s large : 1H) ; 10,48 (mf: 1H).

EI m/z = 427 M^{+.}

m/z = 310 [M-OCH₂CH₂Si(CH₃)₃]⁺

m/z = 299 [M - C₆H₁₂OSi]^{+.}

### N-[6-(4-fluorophényl)-1H-indazol-3-yl]-butanamide

A 580 mg de N- [6-(4-fluorophényl)-1-[[2-(triméthylsilyl)éthoxy]méthyl]-1H-indazol-3-yl]-butanamide décrit précédemment, dans 30 cm³ de tétrahydrofurane, on additionne 8.1 cm³ de fluorure de tétrabutylammonium en solution 1M dans le tétrahydrofurane et on chauffe le milieu réactionnel vers 66°C pendant 22 heures. On arrête ensuite le chauffage et on ajoute 75 cm³ d'acétate d'éthyle, lave avec 50 cm³ d'une solution aqueuse saturée en hydrogénocarbonate de sodium puis avec 50 cm³ d'une solution aqueuse saturée en chlorure de sodium. La phase organique est décantée, séchée sur sulfate de magnésium, filtrée, concentrée à sec sous pression réduite (2 kPa; 50°C). Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (40/60 en volumes). Les fractions contenant le produit attendu, sont réunies et évaporées sous pression réduite (2 kPa ; 50°C). Le résidu est repris par 10 cm³ d'éther diisopropylique, filtré et lavé successivement par 5 cm³ d'éther diisopropylique et par 2 fois 3 cm³ d'acétate d'éthyle ; on obtient après séchage (90 Pa; 50°C), 250 mg de N-[6-(4-fluorophényl)-1H-indazol-3-yl]-butanamide sous forme d'un produit blanc fondant à 232°C. Spectre de R.M.N. ¹H (300 MHz, (CD₃)₂SO d6, δ en ppm) : 0,98 (t, J = 7,5 Hz : 3H) ; 1,69 (mt : 2H) ; 2,39 (t, J = 7 Hz : 2H) ; 7,30 (d, J = 9 Hz : 1H) ; 7,32 (t, J = 9 Hz : 2H) ; 7,61 (s large : 1H) ; 7,78 (dd, J = 9 et 6 Hz : 2H) ; 7,87 (d, J = 9 Hz : 1H) ; 10,33 (mf: 1H) ; 12,70 (mf : 1H).

### EXEMPLE 47

### N-[6-[(1,1-diméthyléthyl)phényl]-1-[[2-(triméthylsilyl)éthoxy]méthyl]-1H-indazol-3-yl]-butanamide :

A 1g de N-[6-chloro-1-[[2-(triméthylsilyl)éthoxy]méthyl]-1H-indazol-3-yl]-butanamide décrit à l'exemple 25, dans 30 cm³ de dioxane, on additionne 840 mg d'acide 4-tert-butylphénylboronique, 1.24 g de fluorure de césium, 13.5 mg d'acétate de palladium et enfin 31 mg de 2-dicyclohexylphosphine-2-(N,N-diméthylamino)biphényle. On chauffe ensuite vers 102°C pendant 21 heures puis on laisse la température revenir à l'ambiante et on dilue le milieu réactionnel avec 75 cm³ d'acétate d'éthyle, filtre sur verre fritté garni de célite et concentre à sec sous pression réduite (2 kPa ; 50°C). Le traitement et la purification s'effectuent par analogie à l'exemple 41. On obtient ainsi 1.13 g de N-[6-[4-(1,1-diméthyléthyl)phényl]-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,08 (s : 9H) ; 0,83 (t, J = 8 Hz : 2H) ; 0,98 (t, J = 7,5 Hz : 3H) ; 1,35 (s : 9H) ; 1,68 (mt : 2H) ; 2,41 (t, J = 7 Hz : 2H) ; 3,57 (t, J = 8 Hz : 2H) ; 5,72 (s large : 2H) ; 7,44 (d large, J = 9 Hz : 1H) ; 7,53 (d large, J = 8 Hz : 2H) ; 7,70 (d large, J = 8 Hz : 2H) ; 7,89 (d, J = 9 Hz : 1H) ; 7,91 (s large : 1H) ; 10,46 (mf : 1H).

EI m/z = 465 M^{+.}

m/z = 348 [M-OCH₂CH₂Si(CH₃)₃]⁺

m/z = 337 [M - C₆H₁₂OSi]^{+.}

### N-[6-[4-(1,1-diméthyléthyl)phényl]-1H-indazol-3-yl]-butanamide

A 1.13 g de N-[6-[4-(1,1-diméthyléthyl)phényl]-1-[[2-(triméthylsilyl)éthoxy]méthyl]-1H-indazol-3-yl]-butanamide décrit précédemment, dans 30 cm³ de tétrahydrofurane, on additionne 14.6 cm³ de fluorure de tétrabutylammonium en solution 1M dans le tétrahydrofurane. On chauffe le milieu réactionnel vers 66°C pendant 22 heures puis on arrête ensuite le chauffage et on ajoute 75 cm³ d'acétate d'éthyle, lave la phase organique avec 75 cm³ d'une solution aqueuse saturée en hydrogénocarbonate de sodium puis avec 50 cm³ d'une solution aqueuse saturée en chlorure de sodium. La phase organique est séchée sur sulfate de magnésium, filtrée et concentrée à sec sous pression réduite (2 kPa; 50°C). Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (70/30 en volumes). Les fractions contenant le produit attendu, sont réunies et évaporées sous pression réduite (2 kPa; 50°C) ; le résidu est repris par 10 cm³ d'éther diisopropylique, filtré sur verre fritté et lavé successivement avec 10 cm³ d'éther diisopropylique puis avec 2 fois 5 cm³ d'acétate d'éthyle. On obtient après séchage (90 Pa; 50°C), 320 mg de N-[6-[4-(1,1-diméthyléthyl)phényl]-1H-indazol-3-yl]-butanamide sous forme d'un produit blanc fondant à 246°C.

Spectre de R.M.N. ¹H (300 MHz, (CD₃)₂SO d6, δ en ppm) : 0,99 (t, J = 7,5 Hz : 3H) ; 1,36 (s : 9H) ; 1,70 (mt : 2H) ; 2,41 (t, J = 7 Hz : 2H) ; 7,36 (d large, J = 9 Hz : 1H) ; 7,52 (d, J = 8,5 Hz : 2H) ; 7,61 (s large : 1H) ; 7,66 (d large, J = 8,5 Hz : 2H) ; 7,85 (d, J = 9 Hz : 1H) ; 10,32 (mf : 1H) ; 12,66 (mf : 1H).

### EXEMPLE 48

### N-[6-bromo-7-amino-1H-indazol-3-yl]-butanamide

A 510 mg de N-[6-bromo-7-nitro-1H-indazol-3-yl]-butanamide décrit à l'exemple 29, dans 20 cm³ d'éthanol et refroidit vers 5 °C, on ajoute goutte à goutte 4.25g de sulfate ferreux heptahydraté solubilisé dans 25 cm³ d'eau . La température remonte vers 28 °C, on laisse agiter pendant 30 minutes puis on ajoute 5.2 cm³ d'ammoniaque à 28% et on chauffe au reflux pendant 2 heures puis on ajoute encore 2 fois 1.5 cm³ d'ammoniaque à 28 % et on laisse agiter encore 10 minutes et on filtre à chaud sur verre fritté garni de célite. Le précipité est rincé par 20 cm³ de méthanol et on concentre à sec le filtrat sous pression réduite (2 kPa ; 40 °C). Le résidu est repris avec 50 cm³ d'acétate d'éthyle, lavé par 25 cm³ d'une solution saturée en hydrogénocarbonate de sodium puis par 25 cm³ d'une solution saturée en chlorure de sodium. La phase organique est décantée, séchée sur sulfate de magnésium et concentrée sous pression réduite (2 kPa ; 40°C). On obtient après séchage (90 Pa ; 50°C), 55 mg de N-[6-bromo-7-amino-1H-indazol-3-yl]-butanamide sous forme d'un solide mauve.

Spectre de R.M.N. ¹H (300 MHz, (CD₃)₂SO d6, δ en ppm) : 0,95 (t, J = 7,5 Hz : 3H) ; 1,65 (mt : 2H) ; 2,35 (t, J = 7 Hz : 2H) ; 5,47 (s large : 2H) ; 6,901 (d, J = 8,5 Hz : 1H) ; 7,00 (d, J = 8,5 Hz : 1H) ; 10,18 (s large : 1H) ; 12,38 (mf : 1H).

EI m/z = 296 M^{+.}

m/z = 226 [M -C₄H₆O]^{+.}

### EXEMPLE 49

### N-[6-[4-(trifluorométhyl)phényl]-1-[[2-(triméthylsilyl)éthoxy]méthyl]-1H-indazol-3-yl]-butanamide :

A 1g de N-[6-chloro-1-[[2-(triméthylsilyl)éthoxy]méthyl]-1H-indazol-3-yl]-butanamide décrit à l'exemple 25, dans 30 cm³ de dioxane, on additionne 775 mg d'acide 4-trifluorométhylphénylboronique , 1.24 g de fluorure de césium, 13.5 mg d'acétate de palladium et enfin 31 mg de 2-dicyclohexylphosphine-2-(N,N-diméthylamino)biphényle. On chauffe ensuite au reflux pendant 18 heures puis on laisse la température revenir à l'ambiante et on dilue le milieu réactionnel avec 75 cm³ d'acétate d'éthyle, filtre sur verre fritté garni de célite et on concentre à sec sous pression réduite (2 kPa ; 50°C). Le traitement et la purification s'effectuent par analogie à l'exemple 41. On obtient ainsi 1 g de N-[6-[4-(trifluorométhyl)phényl]-1-[[2-(triméthylsilyl)éthoxy]méthyl]-1H-indazol-3-yl]-butanamide sous forme d'une huile jaune à 95% de pureté.

Spectre de R.M.N. ¹H (300 MHz, (CD₃)₂SO d6, δ en ppm) : - 0,09 (s : 9H) ; 0,83 (t, J = 8 Hz : 2H) ; 0,98 (t, J = 7,5 Hz : 3H) ; 1,69 (mt : 2H) ; 2,42 (t, J = 7 Hz : 2H) ; 3,56 (t, J = 8 Hz : 2H) ; 5,75 (s : 2H) ; 7,52 (d large, J = 8,5 Hz : 1H); 7,88 (d large, J = 8,5 Hz : 2H) ; 7,97 (d, J = 8,5 Hz : 1H) ; 8,01 (d large, J = 8,5 Hz : 2H); 8,07 (s large: 1H); 10,51 (mf : 1H).

EI m/z = 477 M^{+.}

m/z = 360 [M - OCH₂CH₂Si(CH₃)₃]⁺

m/z = 349 [M - C₆H₁₂OSi]^{+.}

### N-[6-[4-trifluorométhyl)phényl]-1H-indazol-3-yl]-butanamide :

A une solution de 1g de N-[6-[4-(trifluoromethyl)phényl]-1-[(2-(triméthylsilyl)éthoxy]méthyl]-1H-indazol-3-yl]-butanamide dans 30 cm³ de tétrahydrofurane on ajoute 12,6 cm³ de fluorure de tétrabutylammonium en solution 1M dans le tétrahydrofurane et on chauffe à reflux pendant 18 heures. Le milieu réactionnel est dilué par 75 cm³ d'acétate d'éthyle et la phase organique est lavée successivement par 2 fois 50 cm³ d'une solution saturée en hydrogénocarbonate de sodium et par 2 fois 50 cm³ d'une solution saturée en chlorure de sodium. La phase organique est décantée, séchée sur sulfate de magnésium, filtrée et concentrée à sec sous pression réduite (2 kPa ; 50°C) pour donner 0,95 g d'un solide marron. Le brut est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (60/40 en volumes). Les fractions contenant le produit attendu, sont réunies et évaporées sous pression réduite (2 kPa ; 50°C) pour donner 0,60 g de cristaux crème qui sont repris dans 10 cm³ d'éther diisopropylique, filtrés et séchés sous pression réduite (90 Pa; 50°C) pour donner 0,47 g de N-[6-[4-(trifluoromethyl)phényl]-1H-indazol-3-yl]-butanamide sous forme de cristaux blancs fondant à plus de 260°C.

Spectre de R.M.N. ¹H (300 MHz, (CD₃)₂SO d6, δ en ppm) : 0,98 (t, J = 7,5 Hz : 3H); 1,69 (mt : 2H); 2,41 (t, J = 7 Hz : 2H); 7,42 (dd, J = 9 et 1,5 Hz : 1H) ; 7,73 (s large : 1H); 7,85 (d, J = 8,5 Hz : 2H) ; 7,92 (d, J = 9 Hz : 1H) ; 7,97 (d, J = 8,5 Hz : 2H); 10,37 (mf : 1H).

### EXEMPLE 50

### N-[6-(4-méthylphényl)-1-[[2-(triméthylsilyl)éthoxy]méthyl]-1H-indazol-3-yl]-butanamide :

A 1g de N-[6-chloro-1-[[2-(triméthylsilyl)éthoxy]méthyl]-1H-indazol-3-yl]-butanamide décrit à l'exemple 25, dans 30 cm³ de dioxane, on additionne 555 mg d'acide 4-méthylphénylboronique , 1.24 g de fluorure de césium, 13.5 mg d'acétate de palladium et enfin 31 mg de 2-dicyclohexylphosphine-2-(N,N-diméthylamino)biphényle puis on chauffe ensuite vers 104°C pendant 5 heures 30 minutes et on laisse la température revenir à l'ambiante pendant 16 heures. On dilue le milieu réactionnel avec 75 cm³ d'acétate d'éthyle, filtre sur verre fritté garni de célite et on concentre à sec le filtrat sous pression réduite (2 kPa ; 50°C). Le traitement et la purification s'effectuent par analogie à l'exemple 41. On obtient 1.1 g de N-[6-(4-méthylphényl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,09 (s : 9H) ; 0,83 (t, J = 8 Hz : 2H) ; 0,97(t, J = 7,5 Hz : 3H) ; 1,68 (mt : 2H) ; 2,38 (s : 3H) ; 2,41 (t, J = 7 Hz : 2H) ; 3,56 (t, J = 8 Hz : 2H); 5,73 (s : 2H); 7,32 (d large, J = 8 Hz : 2H); 7,44 (d large, J = 9 Hz : 1H) ; 7,68 (d large, J = 8 Hz : 2H) ; 7,90 (d, J = 9 Hz : 1H) ; 7,92 (s large: 1H) ; 10,46 (mf : 1H).

EI m/z = 423 M^{+.}

m/z = 306 [M - OCH₂CH₂Si(CH₃)₃]⁺

m/z = 295 [M - C₆H₁₂OSi]^{+.}

### N-[6-(4-méthylphényl)-1H-indazol-3-yl]butanamide

A 1.1 g de N-[6-(4-méthylphényl)-1-[[2-(triméthylsilyl)éthoxy]méthyl]-1H-indazol-3-yl]-butanamide décrit précédemment, dans 30 cm³ de tétrahydrofurane, on additionne 14.6 cm³ de fluorure de tétrabutylammonium en solution 1M dans le tétrahydrofurane. On chauffe le milieu réactionnel vers 66°C pendant 18 heures puis on arrête ensuite le chauffage. On ajoute au milieu réactionnel 75 cm³ d'acétate d'éthyle et on lave la phase organique avec 2 fois 50 cm³ d'une solution aqueuse saturée en hydrogénocarbonate de sodium puis avec 50 cm³ d'une solution aqueuse saturée en chlorure de sodium. La phase organique est séchée sur sulfate de magnésium, filtrée et concentrée à sec sous pression réduite (2 kPa ; 50°C). Le résidu est purifié par chromatographie sous pression d'argon de 50 kPa, sur colonne de gel de silice (granulométrie 40-60 µm ; diamètre 2.5 cm), en éluant par un mélange acétate d'éthyle-cyclohexane (70/30 en volumes). Les fractions contenant le produit attendu, sont réunies et évaporées sous pression réduite (2 kPa ; 50°C). Le résidu est repris par 10 cm³ d'éther diisopropylique, filtré et lavé successivement avec 10 cm³ d'éther diisopropylique puis avec 3 fois 3 cm³ d'acétate d'éthyle. On obtient après séchage (90 Pa ; 50°C), 500 mg de N-[6-(4-méthylphényl)-1H-indazol-3-yl]-butanamide sous forme d'un produit blanc fondant à 210°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,37 (s : 3H) ; 2,40 (t, J = 7 Hz : 2H) ; 7,31 (d, J = 8 Hz : 2H) ; 7,35 (mt : 1H) ; 7,59 (s large: 1H) ; 7,63 (d, J = 8 Hz : 2H) ; 7,85 (d, J = 9 Hz : 1H) ; 10,32 (s large : 1H); 12,65 (mf : 1H).

### EXEMPLE 51

### N-[6-bromo-1-[[2-(triméthylsilyl)éthoxy]méthyl]-1H-indazol-3-yl]-butanamide :

A une suspension de 1,1 g d'hydrure de sodium à 60 % dans l'huile dans 20 cm³ de diméthylformamide et refroidie à 0°C on ajoute goutte à goutte une solution de 6 g de N-(6-bromo-1H-indazol-3-yl)-butanamide, préparé dans l'exemple 24, dans 50 cm³ de diméthylformamide puis 4.5 cm³ d'une solution de chlorure 2-(triméthylsilyl)éthoxyméthyle dans 10 cm³ de diméthylformamide à 10°C et on laisse revenir le milieu réactionnel à température ambiante. On ajoute 100 cm³ d'acétate d'éthyle au milieu réactionnel puis on lave avec 2 fois 50 cm³ d'eau ; la phase organique est décantée, séchée sur sulfate de magnésium et concentrée à sec sous pression réduite (2 kPa ; 45°C) pour donner 6,9 g de solide. Le brut est purifié par' chromatographie sous pression d'argon de 50 kPa, sur colonne de gel de silice ( granulométrie 40-60 µm ; diamètre 3.5 cm), en éluant par un gradiant cyclohexane-acétate d'éthyle (100/0 à 90/10 en volumes). Les fractions contenant le produit attendu sont concentrées à sec pour donner 2.9 g de N-[6-bromo-1-[[2-(triméthylsilyl)éthoxy]méthyl]-1H-indazol-3-yl]-butanamide sous forme d'un solide blanc cassé.

Spectre de R.M.N. ¹H (300 MHz, (CD₃)₂SO d6, δ en ppm) : - 0,07 (s : 9H) ; 0,83 (t, J = 8 Hz : 2H) ; 0,97 (t, J = 7,5 Hz : 3H) ; 1,67 (mt : 2H) ; 2,40 (t, J = 7 Hz : 2H) ; 3,53 (t, J = 8 Hz : 2H) ; 5,67 (s : 2H) ; 7,29 (dd, J = 9 et 1,5 Hz : 1H) ; 7,82 (d, J = 9 Hz : 1H) ; 8,01 (d, J = 1,5 Hz : 1H); 10,54 (mf : 1H).

EI m/z = 411 M^{+.}

m/z = 294 [M - OCH₂CH₂Si(CH₃)₃]⁺

m/z = 283 [M - C₆H₁₂OSi]^{+.}

### N-[6-(3,5-dichlorophényl)-1-[[2-(triméthylsilyl)éthoxy]méthyl]-1H-indazol-3-yl]-butanamide :

A 1g de N-[6-bromo-1-[[2-(triméthylsilyl)éthoxy]méthyl]-1H-indazol-3-yl]-butanamide précédemment décrit dans 30 cm³ de dioxane, on additionne 0,44 g d'acide 3,5-dichlorophénylboronique, 0.64 g de carbonate de sodium en solution dans 18 cm³ d'eau et 0,186 g de tétrakis-triphénylphosphine palladium et on porte à reflux pendant 18 heures. On dilue le milieu réactionnel avec 75 cm³ d'acétate d'éthyle et 50 cm³ d'eau puis on filtre le milieu sur verre fritté garni de célite et on concentre à sec sous pression réduite (2 kPa ; 50°C). Le traitement et la purification s'effectuent par analogie à l'exemple 41. On obtient ainsi 0,90 g de N-[6-(3,5-dichlorophényl)-1-[(2-(triméthylsilyl)éthoxy]méthyl]-1H-indazol-3-yl]-butanamide sous forme d'une cire jaune.

Spectre de R.M.N. ¹H (300 MHz, (CD₃)₂SO d6, δ en ppm) : - 0,09 (s : 9H) ; 0,85 (t, J = 8 Hz : 2H) ; 0,99 (t, J = 7,5 Hz : 3H) ; 1,69 (mt : 2H) ; 2,42 (t, J = 7 Hz : 2H) ; 3,58 (t, J = 8 Hz : 2H) ; 5,77 (s : 2H) ; 7,53 (dd, J = 8,5 et 1,5 Hz : 1H) ; 7,66 (t, J = 2 Hz : 1H) ; 7,87 (d, J = 2 Hz : 2H) ; 7,95 (d, J = 8,5 Hz : 1H) ; 8,14 (s large : 1H) ; 10,51 (mf : 1H).

EI m/z = 477 M^{+.}

m/z = 360 [M - OCH₂CH₂Si(CH₃)₃]⁺

m/z = 349 [M - C₆H₁₂OSi]^{+.}

### N-[6-(3,5-dichlorophényl)-1H-indazol-3-yl]-butanamide

A 1 g de N-[6-(3,5-dichlorophényl)-1-[[2-(triméthylsilyl)éthoxy]méthyl]-1H-indazol-3-yl]-butanamide décrit précédemment, dans 20 cm³ de tétrahydrofurane, on additionne 11.2 cm³ de fluorure de tétrabutylammonium en solution 1M dans le tétrahydrofurane puis on chauffe le milieu réactionnel vers 65°C pendant 18 heures et on arrête le chauffage pour ajouter 75 cm³ d'acétate d'éthyle. La phase organique est lavée avec 2 fois 50 cm³ d'une solution aqueuse saturée en hydrogénocarbonate de sodium puis avec 50 cm³ d'une solution aqueuse saturée en chlorure de sodium. La phase organique est séchée sur sulfate de magnésium, filtrée et évaporée sous pression réduite (2 kPa ; 50°C). On obtient après séchage (90 Pa ; 50°C), 290 mg de N-[6-(3,5-dichlorophényl)-1H-indazol-3-yl]-butanamide sous forme d'un produit blanc.

Spectre de R.M.N. ¹H (300 MHz, (CD₃)₂SO d6, δ en ppm) : 0,98 (t large, J = 7,5 Hz : 3H) ; 1,69 (mt : 2H) ; 2,40 (t, J = 7 Hz : 2H) ; 7,40 (d large, J = 8,5 Hz : 1H) ; 7,63 (t, J = 2 Hz : 1H) ; 7,75 (s large : 1H); 7,81 (d, J = 2 Hz : 2H) ; 7,89 (d, J = 8,5 Hz : 1H); 10,37 (mf: 1H); de 12,70 à 12,95 (mf étalé : 1H).

EI m/z = 347 M^{+.}

m/z = 277 [M - C₄CH₆O]^{+.}

### EXEMPLE 52

### N-[6-chloro-1H-indazol-3-yl]-3,5-dichlorobenzamide

A 1g de 6-chloro-1H-indazole-3-amine dans 15 cm³ de pyridine, on ajoute 0.83 cm³ de chlorure de 3,5-dichlorobenzoyle après avoir refroidi avec un bain de glace vers 3°C puis on laisse agiter 10 minutes à cette température et on laisse revenir à température ambiante pendant 18 heures. Le milieu réactionnel est ensuite concentré à sec sous pression réduite (2kPa ; 50 °C) et le résidu est repris avec 25 cm³ d'acétate d'éthyle et 25 cm³ d'eau. Le précipité formé est filtré et on obtient après séchage (90 Pa; 50°C), 700 mg de N-[6-chloro-1H-indazol-3-yl]-3,5-dichlorobenzamide sous forme d'un solide blanc fondant vers 240 °C.

Spectre de R.M.N. ¹H (300 MHz, (CD₃)₂SO d6, δ en ppm) : 7,15 (dd, J = 8,5 et 2 Hz : 1H) ; de 7,50 à 7,65 (mt : 2H) ; 7,72 (d, J = 8,5 Hz : 1H) ; 7,79 (s large : 1H) ; 7,90 (d, J = 8,5 Hz : 1H) ; 11,06 (s large : 1H).

### EXEMPLE 53

### N-[6-(4-chlorolphényl)-1-[[2-(triméthylsilyl)éthoxy]méthyl]-1H-indazol-3-yl]-butanamide :

A 900 mg de N-[6-bromo-1-[[2-(triméthylsilyl)éthoxy]méthyl]-1H-indazol-3-yl]-butanamide décrit à l'exemple 51 , dans 40 cm³ de dioxane, on additionne 512 mg d'acide 4-chlorophénylboronique , 578 mg de carbonate de potassium, et 167 mg de tétrakis-triphényl palladium. On chauffe ensuite vers 104°C pendant 2 heures et on laisse la température revenir vers 19°C pendant 16 heures. On dilue le milieu réactionnel avec 75 cm³ d'acétate d'éthyle, filtre le milieu sur verre fritté garni de célite et concentre à sec sous pression réduite (2 kPa ; 50°C). Le résidu est repris avec 100 cm³ d'acétate d'éthyle et 50 cm³ d'eau. La phase organique est relavée par 25 cm³ d'une solution aqueuse saturée en chlorure de sodium puis décantée, séchée sur sulfate de magnésium, filtrée et évaporée sous pression réduite (2 kPa ;50 °C). Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (80/20 en volumes). Les fractions contenant le produit attendu, sont réunies et évaporées sous pression réduite (2 kPa ; 50°C) ; on obtient 600 mg de N-[6-(4-chlorophényl)-1-[[2-(triméthylsilyl)éthoxy]méthyl]-1H-indazol-3-yl]-butanamide sous forme d'une cire jaune.

Spectre de R.M.N. ¹H (300 MHz, (CD₃)₂SO d6, δ en ppm) : - 0,09 (s : 9H) ; 0,83 (t, J = 8 Hz : 2H) ; 0,97 (t, J = 7,5 Hz : 3H) ; 1,68 (mt : 2H) ; 2,41 (t, J = 7 Hz : 2H) ; 3,56 (t, J = 8 Hz : 2H) ; 5,73 (s : 2H) ; 7,46 (d large, J = 9 Hz : 1H) ; 7,58 (d, J = 8,5 Hz : 2H) ; 7,81 (d, J = 8,5 Hz : 2H) ; 7,93 (d, J = 9 Hz : 1H) ; 7,98 (s large : 1H) ; 10,49 (mf : 1H).

EI m/z = 443 M^{+.}

m/z = 326 [M - OCH₂CH₂Si(CH₃)₃]⁺

m/z = 315 [M - C₆H₁₂OSi]^{+.}

### N-[6-(4-chlorophényl)-1H-indazol-3-yl]-butanamide

A une solution de 600 mg de N-[6-(4-chlorophényl)-1-[[2-(triméthylsilyl)éthoxy]méthyl]-1H-indazol-3-yl]-butanamide préparé précédemment dans 20 cm³ de tétrahydrofurane, on additionne 9.5 cm³ de fluorure de tétrabutylammonium en solution 1M dans le tétrahydrofurane puis on chauffe le milieu réactionnel vers 65°C pendant 18 heures et on arrête le chauffage pour ajouter 40 cm³ d'acétate d'éthyle. La phase organique est lavée avec 2 fois 30 cm³ d'une solution aqueuse saturée en hydrogénocarbonate de sodium puis avec 30 cm³ d'une solution aqueuse saturée en chlorure de sodium. La phase organique est séchée sur sulfate de magnésium, filtrée et évaporée sous pression réduite (2 kPa ; 50°C). On obtient après séchage (90 Pa ; 50°C), 238 mg de N-[6-(4-chlorophényl)-1H-indazol-3-yl]-butanamide sous forme d'une poudre beige.

Spectre de R.M.N. ¹H (300 MHz, (CD₃)₂SO d6, δ en ppm) : 0,98 (t large, J = 7,5 Hz : 3H) ; 1,68 (mt : 2H) ; 2,40 (t, J = 7 Hz : 2H) ; 7,35 (d large, J = 8,5 Hz : 1H) ; 7,55 (d, J = 8,5 Hz : 2H) ; 7,64 (s large: 1H) ; 7,77 (d, J = 8,5 Hz : 2H) ; 7,88 (d, J = 8,5 Hz : 1H); 10,34 (mf : 1H).

EI m/z = 313 M^{+.}

m/z = 243 [M - C₄CH₆O]^{+.}

### EXEMPLE 54

### N-[6-chloro-1H-indazol-3-yl]-benzenepropanamide, trifluoroacétate

A 1g de 6-chloro-1H-indazole-3-amine dans 15 cm³ de pyridine, on ajoute 0.88 cm³ de chlorure d'hydrocinnamoyle après avoir refroidi vers 5°C puis on laisse revenir à température ambiante pendant 18 heures. Le milieu réactionnel est ensuite concentré à sec sous pression réduite (2 kPa ; 45 °C) puis repris avec 25 cm³ d'acétate d'éthyle , 25 cm³ d'eau et 10 cm³ de tétrahydrofurane. La phase organique décantée est lavée avec 25 cm³ d'eau puis avec 25 cm³ d'une solution aqueuse saturée en chlorure de sodium ; après séchage sur sulfate de magnésium, filtration et concentration à sec sous pression réduite (2 kPa ; 50°C), le résidu est purifié par chromatographie sous pression d'argon de 50 kPa, sur colonne de gel de silice (granulométrie 15-40 µm ; diamètre 4 cm), en éluant par un mélange dichlorométhane-méthanol (97/3 en volumes). Les fractions contenant le produit attendu, sont réunies et évaporées sous pression réduite (2 kPa ; 50°C). Le produit obtenu impur , est repurifié par HPLC (colonne Hypurity; C₁₈, 5µm ; longueur 50 mm, diamètre 21 mm, éluant : gradiant acétonitrile-eau (5/95 à 95/5 en volumes) contenant 0.05% d'acide trifluoroacétique ; débit 10 cm³/mn). Après concentration des fractions contenant l'attendu, on obtient après séchage (90 Pa; 50 °C), 200 mg de N-[6-chloro-1H-indazol-3-yl]-benzenepropanamide trifluoroacétate, sous forme d'un solide blanc fondant à 224°C.

Spectre de R.M.N. ¹H (300 MHz, (CD₃)₂SO d6, δ en ppm) : 2,73 (t, J = 7,5 Hz : 2H) ; 2,97 (t, J = 7,5 Hz : 2H) ; 7,06 (dd, J = 9 et 1,5 Hz : 1H) ; de 7,15 à 7,40 (mt : 5H) ; 7,51 (d, J = 1,5 Hz : 1H) ; 7,77 (d, J = 9 Hz : 1H) ; 10,44 (s large : 1H).

### EXEMPLE 55

### N-[6-(4-éthylphényl)-1-[[2-(triméthylsilyl)éthoxylméthyl]-1H-indazol-3-yl]-butanamide

A 1g de N-[6-chloro-1-[[2-(triméthylsilyl)éthoxy]méthyl]-1H-indazol-3-yl]-butanamide décrit à l'exemple 25, dans 30 cm³ de dioxane, on additionne 612 mg d'acide 4-éthylphénylboronique , 1.24 g de fluorure de césium, 13.5 mg d'acétate de palladium et enfin 31 mg de 2-dicyclohexylphosphine-2-(N,N-diméthylamino)biphényle puis on chauffe au reflux pendant 16 heures. On dilue le milieu réactionnel avec 50 cm³ d'acétate d'éthyle et 50 cm³ d'eau, filtre sur verre fritté garni de célite et concentre à sec le filtrat sous pression réduite (2 kPa ; 50°C). Le traitement et la purification s'effectuent par analogie à l'exemple 41. Le produit encore impur est repurifié par HPLC (colonne Hypurity ; C₁₈, 5µm ; longueur 50 mm, diamètre 21 mm, éluant : acétonitrile-eau contenant 0.05% d'acide trifluoroacétique ; débit 10 cm³/mn). Après concentration des fractions contenant l'attendu et séchage (90 Pa; 45°C), on obtient 240 mg de N-[6-(4-éthylphényl)-1-[[2-(triméthylsilyl)éthoxy]méthyl]-1H-indazol-3-yl]-butanamide sous forme d'une huile jaune.. Le produit est directement engagé dans l'étapes suivante.

### N-[[6-(4-éthylphényl)-1H-indazol-3-yl]]-butanamide :

A une solution de 240 mg de N-[6-(4-éthylphényl)-1-[[2-(triméthylsilyl)éthoxy]méthyl]-1H-indazol-3-yl]-butanamide précédemment décrit dans 20 cm³ de tétrahydrofurane, on ajoute 3.3 cm³ de fluorure de tétrabutylammonium en solution 1M dans le tétrahydrofurane. Le milieu est ensuite porté à 67°C pendant 17 heures puis on laisse revenir à température ambiante et on ajoute 50 cm³ d'acétate d'éthyle. La phase organique est lavée avec 2 fois 50 cm³ d'une solution aqueuse saturée en hydrogénocarbonate de sodium, avec 2 fois avec 50 cm³ d'eau, décantée, séchée sur sulfate de magnésium, filtrée et concentrée à sec sous pression réduite (2 kPa ; 45°C). Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (60/40 en volumes). Les fractions contenant le produit attendu, sont réunie's et évaporées sous pression réduite (2 kPa ; 50°C). Le résidu est repris avec 10 cm³ d'éther diisopropylique, filtrée sur verre fritté et lavé successivement avec 2 cm³ d'acétate d'éthyle puis avec 10 cm³ d'éther diisopropylique. Après séchage (90 Pa ; 50 °C), on obtient 80 mg de N-[6-(4-éthylphényl)-1H-indazol-3-yl]-butanamide sous forme d'un solide blanc fondant à 210 °C.

Spectre de R.M.N. ¹H (300 MHz, (CD₃)₂SO d6, δ en ppm) : 0,98 (t large, J = 7,5 Hz : 3H) ; 1,24 (t, J = 7,5 Hz : 3H) ; 1,68 (mt : 2H) ; 2,39 (t, J = 7 Hz : 2H) ; 2,67 (q, J = 7,5 Hz : 2H) ; de 7,30 à 7,40 (mt : 3H) ; 7,59 (s large : 1H) ; 7,65 (d large, J = 8 Hz : 2H) ; 7,84 (d, J = 9 Hz : 1H) ; 10,31 (mf : 1H); 12,65 (mf : 1H).

### EXEMPLE 56

### N-[6-(4,4,5,5-tétraméthyl-[1,3,2]dioxaborolan-2-yl)-1-[[2-(triméthylsilyl)éthoxy]méthyl]-1H-indazol-3-yl]-butanamide:

A 740 mg de N-[6-chloro-1-[[2-(triméthylsilyl)éthoxy]méthyl]-1H-indazol-3-yl)-butanamide décrit à l'exemple 25 , dans 25 cm³ de dioxane, on additionne 766 mg de bis(pinacolato)diborane, 917 mg de fluorure de césium puis 9.9 mg d'acétate de palladium et enfin 23.6 mg de 2-dicyclohexylphosphine-2-(N,N-diméthylamino)diphényle. Le milieu est porté au reflux pendant 20 heures puis on laisse ensuite revenir à température ambiante et on ajoute 50 cm³ d'acétate d'éthyle et 50 cm³ d'eau. Après filtration du milieu reactionnel sur verre fritté garni de célite on lave avec 2 fois 50 cm³ d'eau. La phase organique est séchée sur sulfate de magnésium, filtrée puis concentrée à sec sous pression réduite (2 kPa ; 45 °C). Le résidu est purifié par chromatographie sous pression d'argon de 50 kPa, sur colonne de gel de silice (granulométrie 40-60 µm ; diamètre 3 cm), en éluant par un mélange cyclohexane-acétate d'éthyle (80/20 en volumes). Les fractions contenant le produit attendu, sont réunies et évaporées sous pression réduite (2 kPa; 50°C) pour donner 630 mg de N-[6-(4,4,5,5-tétraméthyl-[1,3,2]dioxaborolan-2-yl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,09 (s : 9H) ; 0,82 (t, J = 8 Hz : 2H) ; 0,96 (t, J = 7,5 Hz : 3H) ; 1,34 (s : 12H) ; 1,67 (mt : 2H) ; 2,40 (t, J = 7 Hz : 2H) ; 3,50 (t, J = 8 Hz : 2H) ; 5,71 (s : 2H) ; 7,39 (d, J = 8,5 Hz : 1H) ; 7,83 (d, J = 8,5 Hz : 1H) ; 7,97 (s : 1H) ; 10,45 (mf : 1H).

EI m/z = 459 M^{+.}

m/z = 342 [M - OCH₂CH₂Si(CH₃)₃]⁺

m/z = 331 [M - C₆H₁₂OSi]^{+.}

m/z = 272 [342 - C₄H₆O]⁺

### N-[6-(4-pyridinyl)-1-[[2-(triméthylsilyl)éthoxy]méthyl]-1H-indazol-3-yl]-butanamide :

A 320 mg de N-[6-(4,4,5,5-tétraméthyl-[1,3,2]dioxaborolan-2-yl)-1-[[2-(triméthylsilyl)éthoxy]méthyl]-1H-indazol-3-yl]-butanamide précédemment décrit, dans 20 cm³ de dioxane, on ajoute 246 mg de 4-iodopyridine, 10 cm³ d'eau, 201 mg de carbonate de sodium et 69 mg de tétrakis-triphénylphosphine palladium. Le milieu est ensuite porté au reflux pendant 20 heures puis on laisse revenir à température ambiante et on ajoute 50 cm³ d'acétate d'éthyle et 50 cm³ d'eau. Les phases organiques réunies sont lavées avec 50 cm³ d'eau distillée puis avec 50 cm³ d'une solution aqueuse saturée en chlorure de sodium puis séchées sur sulfate de magnésium, filtrées et concentrées à sec sous pression réduite (2 kPa ; 45 °C). Le résidu est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (70/30 en volumes). Les fractions contenant le produit attendu, sont réunies et évaporées sous pression réduite (2 kPa ; 50°C). On obtient 280 mg de N-[6-(4-pyridinyl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,10 (s : 9H) ; 0,83 (t, J = 8 Hz : 2H) ; 0,98 (t, J = 7,5 Hz : 3H); 1,69 (mt : 2H) ; 2,42 (t, J = 7 Hz : 2H) ; 3,57 (t, J = 8 Hz : 2H) ; 5,76 (s : 2H) ; 7,57 (dd large, J = 8,5 et 1 Hz : 1H) ; 7,82 (d large, J = 6 Hz : 2H) ; 7,98 (d, J = 8,5 Hz : 1H) ; 8,16 (s large: 1H) ; 8,70 (d large, J = 6 Hz : 2H); 10,52 (mf : 1H).

EI m/z = 410 M^{+.}

m/z = 293 [M - OCH₂CH₂Si(CH₃)₃]⁺

m/z = 282 [M - C₆H₁₂OSi]^{+.}

### N-[6-(4-pyridinyl)-1H-indazol-3-yl]-butanamide :

A 280 mg de N-[6-(4-pyridinyl)-1-[[2-(triméthylsilyl)éthoxy]méthyl]-1H-indazol-3-yl]-butanamide précédemment décrit dans 20 cm³ de tétrahydrofurane, on ajoute 4.1 cm³ de fluorure de tétrabutylammonium en solution 1M dans le tétrahydrofurane puis le milieu est porté à 67°C pendant 17 heures et on laisse ensuite revenir à température ambiante pour ajouter 50 cm³ d'acétate d'éthyle et 50 cm³ d'une solution aqueuse saturée en hydrogénocarbonate de sodium. La phase organique est lavée avec 50 cm³ d'une solution aqueuse saturée en chlorure de sodium puis avec 50 cm³ d'eau, séchée sur sulfate de magnésium, filtrée et concentrée à sec sous pression réduite (2 kPa ; 45°C). Le résidu est purifié par chromatographie sous pression d'argon de 50 kPa, sur colonne de gel de silice (granulométrie 40-60 µm ; diamètre 2 cm), en éluant par de l'acétate d'éthyle. Les fractions contenant le produit attendu, sont réunies et concentrées à sec sous pression réduite (2 kPa ; 50°C). Le résidu est repris avec 10 cm³ d'éther diisopropylique, filtré sur verre fritté puis lavé successivement avec 5 cm³ d'acétate d'éthyle et avec 10 cm³ d'éther diisopropylique. Après séchage (90 Pa ; 50 °C), on obtient 60 mg de N-[6-(4-pyridinyl)-1H-indazol-3-yl]-butanamide sous forme d'un solide blanc fondant à 200 °C.

Spectre de R.M.N. ¹H (300 MHz, (CD₃)₂SO d6, δ en ppm) : 0,99 (t, J = 7,5 Hz : 3H) ; 1,69 (mt : 2H) ; 2,41 (t, J = 7 Hz : 2H) ; 7,47 (dd large, J = 9 et 1 Hz : 1H) ; 7,78 (d large, J = 6 Hz : 2H) ; 7,81 (s large: 1H) ; 7,93 (d, J = 9 Hz : 1H) ; 8,67 (d large, J = 6 Hz : 2H) ; 10,38 (mf : 1H) ; 12,84 (s large : 1H).

### EXEMPLE 57

### N-(5-amino-1H-indazol-3-yl)-butanamide :

A une solution de 2.05 g de N-[5-nitro-1H-indazol-3-yl]-butanamide décrit à l'exemple 23, dans 80 cm³ d'éthanol, on ajoute 20 g de sulfate de fer heptahydraté solubilisé dans 50 cm³ d'eau chaude; on laisse agiter 30 minutes à température ambiante et on ajoute 24 cm³ d'ammoniaque à 28 % puis on porte au reflux pendant 2 heures et on ajoute encore 10 cm³ d'ammoniaque à 28% et on agite encore 10 minutes. Le précipité est filtré à chaud sur un verre fritté garni de célite, rincé avec du méthanol jusqu'à ce que le filtrat soit incolore et on concentre à sec sous pression réduite (2 kPa ; 50°C). Le résidu est repris par 100 cm³ d'acétate d'éthyle et on lave avec 50 cm³ d'une solution aqueuse saturée en chlorure de sodium. La phase organique est décantée, séchée sur sulfate de magnésium, filtrée et concentrée à sec sous pression réduite. On obtient, après séchage (90 Pa ; 50 °C) 870 mg de N-(5-amino-1H-indazol-3-yl)-butanamide sous forme d'une poudre violette.

Spectre de R.M.N. ¹H (300 MHz, (CD₃)₂SO d6, δ en ppm) : 0,98 (t, J = 7,5 Hz : 3H) ; 1,66 (mt : 2H) ; 2,34 (t large, J = 7 Hz : 2H); 4,82 (mf : 2H) ; 6,70 (s large : 1H) ; 6,77 (dd, J = 9 et 2 Hz : 1H) ; 7,15 (d, J = 9 Hz : 1H) ; 9,92 (mf : 1H) ; 12,13 (mf : 1H).

EI m/z = 218 M^{+.}

m/z = 148 [M - C₄CH₆O]^{+.}

m/z = 43 [C₃H₇]⁺

### EXEMPLE 58

### N-[5-bromo-6-chloro-1-[[2-(triméthylsilyl)éthoxy]méthyl]-1H-indazol-3-yl]-butanamide

A 1g de N-[6-chloro-1-[[2-(triméthylsilyl)éthoxy]méthyl]-1H-indazol-3-yl]-butanamide décrit à l'exemple 25, dans 15 cm³ de chloroforme, on ajoute 0.22 cm³ de pyridine, puis additionne 0.14 cm³ de brome. On laisse agiter 24 heures à 20 °C puis on ajoute 50 cm³ de dichlorométhane et 50 cm³ d'une solution aqueuse saturée en sulfate de sodium. Après 10 minutes d'agitation, l'insoluble est éliminé par filtration sur verre fritté et la phase organique est lavée avec 50 cm³ d'une solution aqueuse saturée en chlorure de sodium. La phase organique est décantée, séchée sur sulfate de magnésium, filtrée et concentrée à sec sous pression réduite (2 kPa ; 45 °C). Le résidu est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (80/20 en volumes). Les fractions contenant le produit attendu, sont réunies et évaporées sous pression réduite (2 kPa ; 50°C). On obtient après séchage (90 Pa; 45°C), 940 mg de N-[5-bromo-6-chloro-1-[[2-(triméthylsilyl)éthoxy]méthyl]-1H-indazol-3-yl]-butanamide sous forme d'un solide blanc fondant à 130 °C.

Spectre de R.M.N ¹H (300 MHz, (CD₃)₂SO d6, δ en ppm) : - 0,08 (s : 9H) ; 0,82 (t, J = 8 Hz : 2H) ; 0,95 (t, J = 7,5 Hz : 3H) ; 1,66 (mt : 2H) ; 2,40 (t, J = 7,5 Hz : 2H) ; 3,52 (t, J = 8 Hz : 2H) ; 5,66 (s : 2H) ; 8,13 (s : 1H); 8,34 (s : 1H) ; 10,67 (s large: 1H).

### N-(5-bromo-6-chloro-1H-indazol-3-yl)-butanamide :

A 940 mg de N-[5-bromo-6-chloro-1-[[2-(triméthylsilyl)éthoxy]méthyl]-1H-indazol-3-yl]-butanamide précédemment décrit dans 30 cm³ de tétrahydrofurane, on ajoute 12.6 cm³ de fluorure de tétrabutylammonium en solution 1M dans le tétrahydrofurane. Le milieu est ensuite porté à 67°C pendant 19 heures puis on laisse ensuite revenir à température ambiante et on ajoute 50 cm³ d'acétate d'éthyle. La phase organique est lavée avec 2 fois 50 cm³ d'une solution aqueuse saturée en hydrogénocarbonate de sodium puis avec 50 cm³ d'une solution aqueuse saturée en chlorure de sodium, séchée sur sulfate de magnésium, filtrée puis concentrée à sec sous pression réduite (2 kPa ; 45°C). Le résidu est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (70/30 en volumes). Les fractions contenant le produit attendu, sont réunies et évaporées sous pression réduite (2 kPa; 50°C) et le résidu est repris avec 15 cm³ d'éther diisopropylique, filtré sur verre fritté, lavé successivement avec 5 cm³ d'acétate d'éthyle puis avec 2 fois 10 cm³ d'éther diisopropylique. Après séchage (90 Pa ; 50 °C), on obtient 460 mg de N-(5-bromo-6-chloro-1H-indazol-3-yl)-butanamide sous forme d'un solide blanc fondant à 250 °C.

Spectre de R.M.N. ¹H (300 MHz, (CD₃)₂SO d6, δ en ppm): 0,96 (t, J = 7,5 Hz : 3H) ; 1,66 (mt : 2H) ; 2,40 (t, J = 7 Hz : 2H); 7,77 (s : 1H) ; 8,29 (s : 1H) ; 10,53 (mf : 1H) ; de 12,50 à 13,20 (mf étalé : 1H).

### EXEMPLE 59

### N-(6-chloro-1H-indazol-3-yl)-2-thiophenecarboxamide :

A 1g de 6-chloro-1H-indazole-3-amine dans 15 cm³ de pyridine, on additionne 0.64 cm³ de chlorure de 2-thiophènecarbonyle après avoir refroidit vers 6°C avec un bain de glace puis on laisse revenir à température ambiante pendant 17 heures et le milieu réactionnel est concentré à sec sous pression réduite (2 kPa ; 45°C) puis le résidu est repris avec 20 cm³ d'acétate d'éthyle , 20 cm³ d'eau et avec 20 cm³ d'une solution aqueuse saturée en chlorure de sodium. La phase organique est décantée, séchée sur sulfate de magnésium, filtrée et concentrée à sec sous pression réduite (2 kPa ; 45 °C) puis le résidu est purifié par chromatographie sous pression d'argon de 50 kPa, sur colonne de gel de silice (granulométrie 15-40 µm ; diamètre 3 cm), en éluant par un mélange dichlorométhane-méthanol (99/1 en volumes). Les fractions contenant le produit attendu, sont réunies et évaporées sous pression réduite (2 kPa ; 50°C) ; on obtient après séchage (90 Pa ; 50 °C), 660 mg de N-(6-chloro-1H-indazol-3-yl)-2-thiophenecarboxamide sous forme d'un solide jaune pâle fondant à 215 °C.

Spectre de R.M.N. ¹H (300 MHz, (CD₃)₂SO d6, δ en ppm) : 7,11 (dd, J = 9 et 2 Hz : 1H) ; 7,25 (dd, J = 5 et 3,5 Hz : 1H) ; 7,58 (dd, J = 2 et 0,5 Hz : 1H) ; 7,81 (dd, J = 9 et 0,5 Hz : 1H) ; 7,90 (dd, J = 5 et 1,5 Hz : 1H) ; 8,14 (dd, J = 3,5 et 1,5 Hz : 1H) ; 10,98 (mf : 1H) ; 12,96 (mf : 1H).

### EXEMPLE 60

### N-(6-chloro-1H-indazol-3-yl)-2-méthylpropylamide :

A 1 g de 6-chloro-1H-indazole-3-amine dans 15 cm³ de pyridine, on ajoute 0.63 cm³ de chlorure d'isobutyryle après avoir refroidi le milieu vers 6 °C puis on laisse revenir à la température ambiante pendant 19 heures et on évapore à sec sous pression réduite (2 kPa ; 45 °C). Le résidu est repris avec 25 cm³ d'acétate d'éthyle et 25 cm³ d'eau ; le précipité formé est filtré sur verre fritté puis rincé avec de l'acétate d'éthyle. On obtient après séchage (90 Pa ; 50 °C), 384 mg de N-(6-chloro-1H-indazol-3-yl)-2-méthylpropylamide sous forme d'un produit blanc fondant à 238 °C.

Spectre de R.M.N. ¹H (300 MHz, (CD₃)₂SO d6, δ en ppm) : 1,17 (d, J = 7 Hz : 6H) ; 2,75 (mt : 1H); 7,08 (dd, J = 9 et 1,5 Hz : 1H); 7,52 (s large : 1H) ; 7,82 (d, J = 9 Hz : 1H) ; 10,35 (s large : 1H); 12,76 (s large : 1H).

### EXEMPLE 61

### 4-Chloro-N-(6-chloro-1H-indazol-3-yl)-butanamide :

A 1g de 6-chloro-1H-indazole-3-amine dans 15 cm³ de pyridine, on additionne 0.67 cm³ de chlorure de 4-chlorobutyryle après avoir refroidi vers 6°C dans un bain de glace puis on laisse ensuite revenir à température ambiante pendant 19 heures. Le milieu réactionnel est concentré à sec sous pression réduite (2 kPa ; 45°C) et le résidu est repris avec 25 cm³ d'acétate d'éthyle et 25 cm³ d'eau. Le précipité est filtré, rincé avec 15 cm³ d'acétate d'éthyle et 5 cm³ de dichlorométhane. La phase organique est séchée sur sulfate de magnésium, filtrée puis concentrée à sec sous pression réduite (2 kPa ; 45 °C) ; on obtient après séchage (90 Pa ; 50 °C), 343 mg de 4-chloro-N-(6-chloro-1H-indazol-3-yl)-butanamide sous forme d'un solide jaune pâle fondant à 220 °C.

Spectre de R.M.N. ¹H (300 MHz, (CD₃)₂SO d6, δ en ppm) : 2,09 (mt : 2H) ; 2,58 (t, J = 7 Hz : 2H) ; 3,74 (t, J = 7 Hz : 2H) ; 7,08 (dd large J = 9 et 1,5 Hz : 1H) ; 7,52 (d large, J = 1,5 Hz : 1H) ; 7,84 (d, J = 9 Hz : 1H) ; 10,51 (s large : 1H) ; de 12,60 à 13,10 (mf étalé: 1H).

### EXEMPLE 62

### N-[5-phényl-6-chloro-1-[[2-(triméthylsilyl)éthoxy]méthyl]-1H-indazol-3-yl]-butanamide

A 2g de N-[5-bromo-6-chloro-1-[[2-(triméthylsilyl)éthoxy]méthyl]-1H-indazol-3-yl]-butanamide décrit dans l'exemple 58, dans 180 cm³ de dioxane, on ajoute 821 mg d'acide phénylboronique, 1,14 g de carbonate de sodium dans 30 cm³ d'eau distillée et enfin 347 mg de tétrakis-triphénylphosphine palladium. On chauffe au reflux pendant 90 minutes puis on laisse revenir à 20 °C pour ajouter 100 cm³ d'acétate d'éthyle et 100 cm³ d'eau distillée. La phase organique est lavée avec 100 cm³ d'une solution aqueuse saturée en chlorure de sodium puis décantée et séchée sur sulfate de magnésium. Après filtration, le filtrat est concentré à sec sous pression réduite (2 kPa ; 50°C) et le résidu est purifié par chromatographie sous pression d'argon de 50 kPa, sur colonne de gel de silice (granulométrie 40-60 µm ; diamètre 4.5 cm), en éluant par un mélange cyclohexane-acétate d'éthyle (80/20 en volumes). Les fractions contenant le produit attendu sont réunies et évaporées sous pression réduite (2 kPa ; 50°C). On obtient ainsi après séchage (90 Pa ; 45°C), 2 g de N-[5-phényl-6-chloro-1-[[2-(triméthylsilyl)éthoxy]méthyl]-1H-indazol-3-yl]-butanamide sous forme d'une huile jaune.

Spectre de R.M.N ¹H (300 MHz, (CD₃)₂SO d6, δ en ppm) : - 0,05 (s : 9H) ; 0,85 (t, J = 8 Hz : 2H) ; 0,92 (t, J = 7,5 Hz : 3H) ; 1,63 (mt : 2H) ; 2,38 (t, J = 7,5 Hz : 2H) ; 3,56 (t, J = 8 Hz : 2H) ; 5,70 (s : 2H) ; de 7,30 à 7,55 (mt : 5H) ; 7,91 (s: 1H) ; 7,99 (s : 1H); 10,59 (s large : 1H).

EI m/z = 443 M^{+.}

m/z = 326 [M - OCH₂CH₂Si(CH₃)₃]⁺

m/z = 315 [M - C₆H₁₂OSi]^{+.}

### N-(5-phényl-6-chloro-1H-indazol-3-yl)-butanamide

A 650 mg de N-[5-phényl-6-chloro-1-[[2-(triméthylsilyl]éthoxy]méthyl]-1H-indazol-3-yl]-butanamide précédemment décrit, dans 25 cm³ de tétrahydrofurane, on ajoute 8.8 cm³ de fluorure de tétrabutylammonium en solution 1M dans le tétrahydrofurane. Le milieu est ensuite porté à 67°C pendant 20 heures puis on laisse ensuite revenir à température ambiante et on ajoute 75 cm³ d'acétate d'éthyle ; la phase organique est lavée avec 75 cm³ d'une solution aqueuse saturée en hydrogénocarbonate de sodium puis avec 2 fois 75 cm³ d'une solution aqueuse saturée en chlorure de sodium. La phase organique est séchée sur sulfate de magnésium, filtrée et évaporée à sec sous pression réduite (2 kPa ; 45°C). Le résidu est purifié par chromatographie sous pression d'argon de 50 kPa, sur colonne de gel de silice (granulométrie 40-60 µm ; diamètre 3,5 cm), en éluant par un mélange cyclohexane-acétate d'éthyle (70/30 en volumes). Les fractions contenant le produit attendu, sont réunies et évaporées sous pression réduite (2 kPa ; 50°C) et le résidu est repris avec 15 cm³ d'éther diisopropylique, filtré sur verre fritté et lavé successivement avec 10 cm³ d'éther diisopropylique et avec 5 cm³ d'acétate d'éthyle. Après séchage sous pression réduite (90 Pa ; 50 °C), on obtient 240 mg de N-(5-phényl-6-chloro-1H-indazol-3-yl)-butanamide sous forme d'un solide blanc fondant à 235 °C.

Spectre de R.M.N. ¹H (300 MHz, (CD₃)₂SO d6, δ en ppm) : 0,92 (t, J = 7,5 Hz : 3H) ; 1,63 (mt : 2H) ; 2,37 (t, J = 7 Hz : 2H) ; de 7,35 à 7,55 (mt : 5H) ; 7,66 (s : 1H) ; 7,85 (s : 1H) ; 10,47 (s large: 1H) ; 12,80 (mf : 1H).

### EXEMPLE 63

### N-[5-bromo-6-[4-(phénylméthoxy)phényl]-1-[[2-(triméthylsilyl)éthoxy]méthyl]-1H-indazol-3-yl]-butanamide

A 2.64 g de N-[6-[4-(phénylméthoxy)phényl]-1-[[2-(triméthylsilyl)éthoxy]méthyl]-1H-indazol-3-yl]-butanamide préparé dans l'exemple 32, dans 50 cm³ de chloroforme on ajoute 0,84 cm³ de pyridine puis goutte à goutte 0.52 cm³ de brome puis à nouveau 0.42 cm³ de pyridine et 0.26 cm³ de brome. Au milieu réactionnel on ajoute 50 cm³ de chlorure de méthylène et 100 cm³ d'une solution de thiosulfate de sodium à 10% ; la phase organique est décantée, lavée successivement par 50 cm³ d'une solution de thiosulfate de sodium à 10% et par 50 cm³ d'une solution saturée en chlorure de sodium, séchée sur sulfate de magnésium, filtrée et concentrée sous pression réduite (2 kPa ; 50°C) pour donner 3.4 g d'une huile qui est purifiée par chromatographie sous pression d'argon de 50 kPa, sur colonne de gel de silice (granulométrie 40-60 µm ; diamètre 3,5 cm), en éluant par un mélange cyclohexane-acétate d'éthyle (90/10 en volumes). Les fractions contenant le produit attendu, sont réunies et évaporées sous pression réduite (2 kPa; 50°C) pour donner 2.1 g de N-[5-bromo-6-[4-(phénylméthoxy)phényl]-1-[[2-(triméthylsilyl)éthoxy]méthyl]-1H-indazol-3-yl]-butanamide sous forme de poudre blanche fondant à 140°C.

Spectre de R.M.N. ¹H (300 MHz, (CD₃)₂SO d6, δ en ppm) : - 0,09 (s : 9H) ; 0,81 (t, J = 8 Hz : 2H) ; 0,96 (t, J = 7,5 Hz : 3H) ; 1,67 (mt : 2H) ; 2,42 (t large, J = 7 Hz : 2H) ; 3,53 (t, J = 8 Hz : 2H) ; 5,19 (s : 2H); 5,67 (s : 2H); 7,13 (d, J = 8,5 Hz : 2H); 7,37 (d, J = 8,5 Hz : 2H) ; 7,37 (mt : 1H) ; 7,43 (t large, J = 7,5 Hz : 2H); 7,51 (d large, J = 7,5 Hz : 2H) ; 7,70 (s : 1H) ; 8,26 (s : 1H) ; 10,61 (mf : 1H).

### N-[5-bromo-6-[4-(phénylméthoxy)phényl]-1H-indazol-3-yl]-butanamide

A 2 g de N-[5-bromo-6-[4-(phénylméthoxy)phényl]-1-[[2-(triméthylsilyl)éthoxy]méthyl]-1H-indazol-3-yl]-butanamide précédemment décrit dans 60 cm³ de tétrahydrofurane on ajoute 20 cm³ de fluorure de tétrabutylammonium en solution 1M dans le tétrahyrofurane et on porte à reflux pendant 18 heures ; après refroidissement on ajoute 75 cm³ d'acétate d'éthyle et la phase organique est lavée successivement avec 75 cm³ d'une solution saturée en hydrogénocarbonate de sodium et avec 75 cm³ d'une solution saturée en chlorure de sodium. La phase organique est décantée, séchée sur sulfate de magnésium, filtrée et concentrée à sec sous pression réduite (2,7 kPa ; 50°C) pour donner 2 g de brut qui est purifié par chromatographie sous pression d'argon de 50 kPa, sur colonne de gel de silice ( granulométrie 40-60 µm ; diamètre 3,5 cm), en éluant par un mélange cyclohexane-acétate d'éthyle (70/30 en volumes). Les fractions contenant le produit attendu sont réunies et évaporées sous pression réduite ( 2 kPa ; 50°C) et le résidu est repris avec 50 cm³ d'éther diisopropylique, filtré, lavé par 2 fois 10 cm³ d'éther diisopropylique et séché (90 Pa ; 45°C) pour donner 500 mg de N-[5-bromo-6-[4-(phénylméthoxy)phényl]-1H-indazol-3-yl]-butanamide sous forme de solide fondant à 200°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,42 (t, J = 7 Hz : 2H) ; 5,19 (s : 2H) ; 7,12 (d, J = 8,5 Hz : 2H) ; de 7,30 à 7,50 (mt : 6H) ; 7,53 (d large, J = 7,5 Hz : 2H) ; 8,22 (s : 1H) ; 10,50 (mf : 1H) ; 12,83 (mf: 1H).

### N-[5-bromo-6-(4-hydroxyphényl)-1H-indazol-3-yl]-butanamide

A 500 mg de N-[5-bromo-6-[4-(phénylméthoxy)phényl]-1H-indazol-3-yl]-butanamide préparé précédemment on ajoute 10 cm³ d'iodure de triméthylsilyle et on chauffe au reflux 4 heures. On ajoute au milieu 25 cm³ de méthanol et on rechauffe au reflux pendant 15 minutes puis on concentre le milieu réactionnel à sec sous pression réduite (2 kPa ; 50°C ). Le résidu est repris par 50 cm³ d'acétate d'éthyle et on lave avec 2 fois 50 cm³ d'une solution de thiosulfate de sodium à 10% puis avec 50 cm³ d'eau et 50 cm³ d'une solution saturée en chlorure de sodium. La phase organique est décantée, séchée sur sulfate de magnésium, filtrée puis concentrée à sec sous pression réduite pour donner 1 g de brut qui est purifié par chromatographie sous pression d'argon de 50 kPa, sur colonne de gel de silice ( granulométrie 40-60 µm ; diamètre 3,5 cm), en éluant par un mélange cyclohexane-acétate d'éthyle ( 60/40 en volumes). Les fractions contenant le produit attendu sont réunies et évaporées sous pression réduite ( 2 kPa; 50°C) et le résidu est repris avec 10 cm³ d'acétate d'éthyle, filtré, lavé par 2 fois 5 cm³ d'acétate d'éthyle et séché (90 Pa ; 45°C) pour donner 130 mg de N-[5-bromo-6-(4-hydroxyphényl)-1H-indazol-3-yl]-butanamide fondant à plus de 260°C.

Spectre de R.M.N. ¹H (300 MHz, (CD₃)₂SO d6, δ en ppm) : 0,98 (t, J = 7,5 Hz : 3H) ; 1,68 (mt : 2H) ; 2,40 (t, J = 7 Hz : 2H) ; 6,85 (d, J = 8,5 Hz : 2H) ; 7,25 (d, J = 8,5 Hz : 2H) ; 7,34 (s : 1H) ; 8,20 (s : 1H) ; 9,61 (mf : 1H) ; 10,48 (s large : 1H) ; 12,79 (mf: 1H).

### EXEMPLE 64

### N-[6-(4-nitrophényl)-1-[[2-7(triméthylsilyl)éthoxy]méthyl]-1H-indazol-3-yl]-butanamide :

A 1 g de N-[6-(4,4,5,5-tétraméthyl-[1,3,2]dioxaborolan-2-yl)-1-[[2-(triméthylsilyl)ethoxy]méthyl]-1H-indazol-3-yl]-butanamide préparé comme décrit à l'exemple 56, dans 50 cm³ de dioxane, on ajoute 790 mg de 4-bromonitrobenzene, 10 cm³ d'eau, 646 mg de carbonate de sodium et 190 mg de tétrakis-triphénylphosphine palladium. Le milieu réactionnel est ensuite porté au reflux pendant 18 heures et on laisse revenir à température ambiante puis on ajoute 75 cm³ d'acétate d'éthyle et la phase organique est lavée avec 2 fois 50 cm³ d'eau distillée puis avec 50 cm³ d'une solution aqueuse saturée en chlorure de sodium. Après séchage sur sulfate de magnésium, filtration et concentration à sec sous pression réduite ( 2 kPa ; 45 °C), le résidu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (80/20 en volumes). Les fractions contenant le produit attendu, sont réunies et évaporées sous pression réduite ( 2 kPa ; 50°C). On obtient 650 mg de N-[6-(4-nitrophényl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,10 (s : 9H) ; 0,83 (t, J = 8 Hz : 2H) ; 0,97 (t, J = 7,5 Hz : 3H) ; 1,68 (mt : 2H) ; 2,42 (t, J = 7 Hz : 2H) ; 3,57 (t, J = 8 Hz : 2H) ; 5,77 (s : 2H) ; 7,56 (dd large, J = 9 et 1,5 Hz : 1H) ; 7,99 (d, J = 9 Hz : 1H) ; 8,09 (d, J = 9 Hz : 2H) ; 8,15 (s large : 1H) ; 8,37 (d, J = 9 Hz : 2H) ; 10,56 (mf: 1H).

EI m/z = 454 M^{+.}

m/z = 337 [M - OCH₂CH₂Si(CH₃)₃]⁺

m/z = 326 [M - C₆H₁₂OSi]^{+.}

m/z = 73 [Si(CH₃)₃]⁺

### N-[[6-(4-nitrophényl)-1H-indazol-3-yl]]-butanamide :

A 650 mg de N-[6-(4-nitrophényl)-1-[[2-(triméthylsilyl)éthoxy]méthyl]-1H-indazol-3-yl]-butanamide précédemment décrit dans 30 cm³ de tétrahydrofurane, on ajoute 8.6 cm³ de fluorure de tétrabutylammonium en solution 1M dans le tétrahydrofurane puis le milieu est porté à 67°C pendant 18 heures et on laisse ensuite revenir à température ambiante pour ajouter 75 cm³ d'acétate d'éthyle et 75 cm³ d'une solution aqueuse saturée en hydrogénocarbonate de sodium. La phase organique est lavée avec 50 cm³ d'une solution aqueuse saturée en chlorure de sodium puis avec 50 cm³ d'eau, décantée, séchée sur sulfate de magnésium, filtrée et concentrée à sec sous pression réduite ( 2 kPa ; 45°C). Le résidu est purifié par chromatographie sous pression d'argon de 50 kPa, sur colonne de gel de silice (granulométrie 40-60 µm ; diamètre 2 cm), en éluant par un mélange cyclohexane-acétate d'éthyle (50/50 en volumes). Les fractions contenant le produit attendu, sont réunies et concentrées à sec sous pression réduite ( 2 kPa ; 50°C) ; le résidu est repris avec 10 cm³ d'acétate d'éthyle, filtré sur verre fritté puis lavé successivement avec 5 cm³ d'acétate d'éthyle et avec 10 cm³ d'éther diisopropylique. Après séchage ( 90 Pa ; 50 °C), on obtient 280 mg de N-[6-(4-nitrophényl)-1H-indazol-3-yl]-butanamide sous forme de cristaux jaune fondant à 250 °C.

Spectre de R.M.N. ¹H (300 MHz, (CD₃)₂SO d6, δ en ppm) : 0,98 (t, J = 7,5 Hz : 3H) ; 1,68 (mt : 2H) ; 2,41 (t, J = 7 Hz : 2H); 7,46 (dd, J = 8,5 et 1,5 Hz : 1H) ; 7,79 (s large : 1H) ; 7,94 (d, J = 8,5 Hz : 1H) ; 8,05 (d large, J = 9 Hz : 2H) ; 8,34 (d large, J = 9 Hz : 2H) ; 10,42 (mf : 1H) ; 12,87 (s large : 1H).

### EXEMPLE 65

### N-[6-(2-chlorophényl)-1-[(2-(triméthylsilyl)éthoxy]méthyl]-1H-indazol-3-yl]-butanamide :

A 1 g de N-[6-bromo-1-[[2-(triméthylsilyl)éthoxy]méthyl]-1H-indazol-3-yl]-butanamide préparé comme décrit à l'exemple 51, dans 60 cm³ de dioxane, on ajoute 853 mg d'acide 2-chlorophénylboronique, 30 cm³ d'eau, 964 mg de carbonate de sodium et 252 mg de tétrakis-triphénylphosphine palladium. Le milieu réactionnel est ensuite porté au reflux pendant 18 heures et filtré sur verre fritté garni de célite puis concentré à sec sous pression réduite (2 kPa ; 50°C). Le résidu est repris par 70 cm³ d'acétate d'éthyle et la phase organique est lavée avec 2 fois 30 cm³ d'eau distillée puis avec 30 cm³ d'une solution aqueuse saturée en chlorure de sodium. La phase organique est décantée, séchée sur sulfate de magnésium, filtré et concentrée à sec sous pression réduite ( 2 kPa; 45 °C). Le résidu obtenu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (90/10 en volumes). Les fractions contenant le produit attendu, sont réunies et évaporées sous pression réduite ( 2 kPa ; 50°C) ; on obtient 1 g de N-[6-(2-chlorophényl)-1-[[2-(triméthylsilyl)éthoxy]méthyl]-1H-indazol-3-yl]-butanamide sous forme d'une cire jaune.

Spectre de R.M.N. ¹H (300 MHz, (CD₃)₂SO d6, δ en ppm) : - 0,08 (s : 9H) ; 0,84 (t, J = 8 Hz : 2H) ; 0,99 (t, J = 7,5 Hz : 3H) ; 1,69 (mt : 2H) ; 2,43 (t, J = 7 Hz : 2H) ; 3,57 (t, J = 8 Hz : 2H) ; 5,71 (s : 2H) ; 7,19 (dd, J = 8,5 et 2 Hz : 1H) ; de 7,40 à 7,70 (mt : 4H) ; 7,74 (s large : 1H) ; 7,90 (d, J = 8,5 Hz : 1H) ; 10,51 (mf : 1H).

EI m/z = 443

m/z = 326 [M - OCH₂CH₂Si(CH₃)₃]⁺

m/z = 315 [M - C₆H₁₂OSi]^{+.}

### N-[6-(2-chlorophényl)-1H-indazol-3-yl]-butanamide:

A 900 mg de N-[6-(2-chlorophényl)-1-[[2-(triméthylsilyl)éthoxy]méthyl]-1H-indazol-3-yl]-butanamide précédement décrit dans 40 cm³ de tétrahydrofurane on ajoute 2.9 cm³ de fluorure de tétrabutylammonium en solution 1M dans le tétrahydrofurane et on chauffe à reflux pendant 18 heures. Le milieu réactionnel est dilué avec 50 cm³ d'acétate d'éthyle et la phase organique est lavée successivement avec 2 fois 30 cm³ d'une solution saturée en hydrogénocarbonate de sodium, avec 30 cm³ d'eau et avec 30 cm³ d'une solution saturée de chlorure de sodium. La phase organique est décantée, séchée sur sulfate de magnésium, filtrée puis concentrée à sec sous pression réduite (2 kPa ; 50°C). Le brut obtenu est purifié par chromatographie sous pression d'argon de 50 kPa, sur colonne de gel de silice ( granulométrie 40-60 µm; diamètre 2.5 cm), en éluant avec un mélange cyclohexane-acétate d'éthyle (90/10 en volumes). Les fractions contenant le produit attendu, sont réunies et évaporées sous pression réduite ( 2 kPa ; 50°C) ; on obtient 120 mg de N-[6-(2-chlorophényl)-1H-indazol-3-yl]-butanamide sous forme d'une meringue beige.

Spectre de R.M.N. ¹H (300 MHz, (CD₃)₂SO d6, δ en ppm) : 0,98 (t, J = 7,5 Hz : 3H) ; 1,69 (mt : 2H) ; 2,40 (t, J = 7 Hz : 2H) ; 7,09 (d large, J = 8,5 Hz : 1H) ; de 7,40 à 7,65 (mt : 5H) ; 7,83 (d, J = 8,5 Hz : 1H) ; 10,36 (s large : 1H) ; 12,74 (s large : 1H).

EI m/z = 313

m/z = 243 [M - C₄CH₆O]^{+.}

### EXEMPLE 66

### N-[6-[3-(phénylméthoxy)phényl]-1-[[2-(triméthylsilyl)éthoxy)méthyl]-1H-indazol-3-yl]-butanamide

A 1 g de N-[6-chloro-1-[[2-(triméthylsilyl)éthoxy)methyl]-1H-indazol-3-yl)-butanamide préparé dans l'exemple 25, on ajoute successivement 930 mg d'acide 3-benzyloxyphénylboronique, 1.24 g de fluorure de césium, 31.5 mg de 2-dicyclohexylphosphino-2'-(N,N-diméthylamino)diphényle, 13.5 mg d'acétate de palladium et on chauffe à reflux pendant 18 heures. Le milieu réactionnel est filtré sur verre fritté garni de célite et on ajoute 75 cm³ d'acétate d'éthyle au filtrat. La phase organique est lavée avec 25 cm³ d'une solution saturée en chlorure de sodium, décantée, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice ( granulométrie 40-60 µm ; diamètre 2.5 cm), en éluant par un gradient cyclohexane-acétate d'éthyle (90/10 à 75/25 en volumes). Les fractions contenant le produit attendu, sont réunies et évaporées sous pression réduite ( 2 kPa; 50°C) ; on obtient 1.1 g de N-[6-[3-(phénylméthoxy)phényl]-1-[[2-(triméthylsilyl)éthoxy)méthyl]-1H-indazol-3-yl]-butanamide sous forme d'huile à 80% de pureté.

Spectre de R.M.N. ¹H (300 MHz, (CD₃)₂SO d6, δ en ppm) : - 0,08 (s : 9H) ; 0,85 (t, J = 8 Hz : 2H) ; 0,99 (t, J = 7,5 Hz : 3H) ; 1,69 (mt : 2H) ; 2,42 (t, J = 7 Hz : 2H) ; 3,58 (t, J = 8 Hz : 2H) ; 5,23 (s : 2H) ; 5,75 (s : 2H) ; 7,08 (dd large, J = 8,5 et 2 Hz : 1H) ; de 7,30 à 7,50 (mt : 7H) ; 7,53 (d large, J = 7,5 Hz : 2H) ; 7,92 (d, J = 9 Hz : 1H) ; 7,96 (s large : 1H) ; 10,48 (mf: 1H).

DCI m/z = 516 [M+H]⁺

### N-[6-[3-(phénylméthoxy)phényl]-1H-indazol-3-yl]-butanamide

A 1 g de N-[6-[3-(phénylméthoxy)phenyl]-1-[[2-(triméthylsilyl)éthoxy]méthyl]-1H-indazol-3-yl]-butanamide décrit précédemment dans 20 cm³ de tétrahydrofurane on ajoute 2.9 cm³ de fluorure de tétrabutylammonium en solution 1M dans le tétrahydrofurane et on chauffe à reflux pendant 18 heures. Le milieu réactionnel est dilué avec 100 cm³ d'acétate d'éthyle et la phase organique est lavée sucessivement avec 50 cm³ d'une solution saturée en hydrogénocarbonate de sodium, avec 2 fois 50 cm³ d'eau et avec 50 cm³ d'une solution saturée en chlorure de sodium. La phase organique est décantée, séchée sur sulfate de magnésium, filtrée et concentrée sous pression réduite (2 kPa ; 50°C). Le brut obtenu est purifié par chromatographie sous pression d'argon de 50 kPa, sur colonne de gel de silice ( granulométrie 40-60 µm ; diamètre 2.5 cm), en éluant par un gradient cyclohexane-acétate d'éthyle (70/30 à' 40/60 en volumes). Les fractions contenant le produit attendu, sont réunies et évaporées sous pression réduite ( 2 kPa ; 50°C) ; on obtient 0.43 g de N-[6-[3-(phénylméthoxy)phényl]-1H-indazol-3-yl]-butanamide sous forme d'une poudre blanche.

Spectre de R.M.N. ¹H (300 MHz, (CD₃)₂SO d6, δ en ppm) : 0,98 (t, J = 7,5 Hz : 3H) ; 1,68 (mt : 2H) ; 2,40 (t, J = 7 Hz : 2H) ; 5,22 (s : 2H) ; 7,05 (dd large, J = 8,5 et 2 Hz : 1H) ; de 7,25 à 7,50 (mt : 7H) ; 7,52 (d large, J = 7,5 Hz : 2H) ; 7,63 (s large : 1H) ; 7,35 (d, J = 9 Hz : 1H) ; 10,33 (mf : 1H) ; 12,68 (mf : 1H).

EI m/z = 385

m/z = 315 [M - C₄CH₆O]^{+.}

### N-[6-(3-hydroxyphényl)-1H-indazol-3-yl]-butanamide

A 0.4 g de N-[6-[3-(phénylméthoxy)phényl]-1H-indazol-3-yl]-butanamide décrit précédemment on ajoute 10 cm³ d'iodure de triméthylsilyle et on chauffe au reflux pendant 3 heures puis on ajoute 50 cm³ de méthanol et on poursuit le reflux 15 minutes. Le milieu réactionnel est concentré à sec sous pression réduite (2 kPa; 50°C), on ajoute 50 cm³ d'acétate d'éthyle et on lave la phase organique avec 3 fois 50 cm³ d'une solution de thiosulfate de sodium à 10%. La phase organique est décantée, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (50/50 en volumes). Les fractions contenant le produit attendu, sont réunies et évaporées sous pression réduite (2 kPa ; 50°C) ; on obtient 0.18 g de N-[6-(3-hydroxyphényl)-1H-indazol-3-yl]-butanamide sous forme d'une poudre grise fondant 188°C.

Spectre de R.M.N. ¹H (300 MHz, (CD₃)₂SO d6, δ en ppm) : 0,98 (t large, J = 7,5 Hz : 3H) ; 1,68 (mt : 2H) ; 2,40 (t, J = 7 Hz : 2H) ; 6,80 (dd large, J = 8 et 2 Hz : 1H) ; 7,08 (mt : 1H) ; 7,13 (d large, J = 8 Hz : 1H) ; de 7,25 à 7,35 (mt : 2H) ; 7,55 (s large : 1H) ; 7,84 (d, J = 8,5 Hz : 1H) ; 9,55 (s large : 1H) ; 10,34 (mf: 1H) ; 12,67 (mf: 1H).

### EXEMPLE 67

### N-[6-chloro-5-(4-pyridinyl)-1-[[2-(triméthylsilyl)éthoxy]méthyl]-1H-indazol-3-yl]-butanamide

A 1 g de N-[[5-bromo-6-chloro-1-[[2-(triméthylsilyl)éthoxy]méthyl]-1H-indazol-3-yl]]-butanamide décrit précédemment dans l'exemple 58, dans 90 cm³ de dioxane on ajoute 415 mg d'acide 4-pyridylboronique puis une solution de 570 mg de carbonate de sodium dans 18 cm³ d'eau et enfin 173 mg de tétakis-triphénylphosphine palladium et on porte au reflux pendant 18 heures. On dilue le milieu réactionnel avec 75 cm³ d'acétate d'éthyle et 50 cm³ d'eau. La phase organique est décantée, lavée avec 50 cm³ d'eau et 50 cm³ d'une solution saturée en chlorure de sodium, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (60/40 en volumes). Les fractions contenant le produit attendu, sont réunies et évaporées sous pression réduite (2 kPa ; 50°C) ; on obtient 770 mg de N-[6-chloro-5-(4-pyridinyl)-1-[[2-(triméthylsilyl)éthoxy]méthyl]-1H-indazol-3-yl]-butanamide sous forme de cristaux blancs.

Spectre de R.M.N. ¹H (300 MHz, (CD₃)₂SO d6, δ en ppm) : - 0,05 (s : 9H) ; 0,85 (t, J = 8 Hz : 2H) ; 0,93 (t, J = 7,5 Hz : 3H) ; 1,63 (mt : 2H) ; 2,39 (t, J = 7 Hz : 2H) ; 3,55 (t, J = 8 Hz : 2H) ; 5,71 (s : 2H) ; 7,48 (d large, J = 6 Hz : 2H) ; 7,98 (s : 1H) ; 8,08 (s : 1H) ; 8,69 (d large, J = 6 Hz : 2H) ; 10,67 (mf : 1H).

ES m/z = 445 [M+H]⁺

### N-[6-chloro-5-(4-pyridinyl)-1H-indazol-3-yl]-butanamide

A 770 mg de N-[6-chloro-5-(4-pyridyl)-1-[[2-(triméthylsilyl)éthoxy]méthyl]-1H-indazol-3-yl]-butanamide décrit précédemment dans 30 cm³ de tétrahydrofurane on ajoute 10.4 cm³ de fluorure de térabutylammonium en solution 1M dans le tétrahydrofurane et on chauffe à reflux pendant 18 heures. On dilue le milieu réactionel avec 75 cm³ d'acétate d'éthyle et 75 cm³ d'une solution saturée en hydrogénocarbonate de sodium. La phase organique est décantée, lavée par 50 cm³ d'une solution saturée en chlorure de sodium, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice ( granulométrie 40-60 µm ; diamètre 2.5 cm), en éluant par de l'acétate d'éthyle. Les fractions contenant le produit attendu, sont réunies et évaporées sous pression réduite (2 kPa ; 50°C) ; le résidu est repris par 20 cm³ d'acétate d'éthyle, filtré, lavé par 5cm³ d'acétate d'éthyle et 20 cm³ d'éther diéthylique, séché sous pression réduite ( 90 Pa ; 45°C) pour donner 320 mg de N-[6-chloro-5-(4-pyridinyl)-1H-indazol-3-yl]-butanamide sous forme de cristaux blancs fondant à plus de 260°C.

Spectre de R.M.N. ¹H (300 MHz, (CD₃)₂SO d6, δ en ppm) : 0,93 (t, J = 7,5 Hz : 3H) ; 1,63 (mt : 2H) ; 2,38 (t, J = 7 Hz : 2H) ; 7,47 (dd, J = 5 et 1,5 Hz : 2H) ; 7,71 (s : 1H) ; 7,94 (s : 1H) ; 8,67 (dd, J = 5 et 1,5 Hz : 2H) ; 10,53 (mf : 1H) ; 12,90 (s large : 1H).

### EXEMPLE 68

### N-[6-chloro-5-(3-furanyl)- 1-[[2-(triméthylsilyl)éthoxy]méthyl]-1H-indazol-3-yl]-butanamide

A 1 g de N-[5-bromo-6-chloro-1-[[2-(triméthylsilyl)éthoxy]méthyl]-1H-indazol-3-yl]-butanamide décrit précédemment dans l'exemple 58, dans 90 cm³ de dioxane on ajoute 377 mg d'acide 3-furylboronique puis 570 mg de carbonate de sodium dans 18 cm³ d'eau et enfin 173 mg de tétakis-triphénylphosphine palladium et on porte au reflux pendant 18 heures. On dilue le milieu réactionnel avec 75 cm³ d'acétate d'éthyle et 50 cm³ d'eau. La phase organique est décantée, lavée avec 50 cm³ d'eau et 50 cm³ d'une solution saturée en chlorure de sodium, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (85/15 en volumes). Les fractions contenant le produit attendu, sont réunies et évaporées sous pression réduite (2 kPa ; 50°C); on obtient 800 mg de N-[6-chloro-5-(3-furanyl)-1-[[2-(triméthylsilyl)éthoxy]méthyl]-1H-indazol-3-yl]-butanamide sous forme d'huile incolore.

Spectre de R.M.N. ¹H (300 MHz, (CD₃)₂SO d6, δ en ppm) : - 0,06 (s : 9H) ; 0,84 (t, J = 8 Hz : 2H) ; 0,96 (t, J = 7,5 Hz : 3H) ; 1,66 (mt : 2H) ; 2,41 (t large, J = 7 Hz : 2H) ; 3,55 (t, J = 8 Hz : 2H) ; 5,69 (s : 2H) ; 6,79 (mt : 1H) ; 7,80 (t, J = 2 Hz : 1H) ; de 7,95 à 8,05 (mt : 3H) ; 10,59 (mf : 1H).

ES m/z=456 [M+Na]⁺

m/z = 434 [M+H]⁺

m/z=316 [M-OCH₂CH₂Si(CH₃)₃]⁺

### N-[6-chloro-5-(3-furanyl)-1H-indazol-3-yl]-butanamide

A 800 mg de N-[6-chloro-5-(3-furanyl)-1-[[2-(triméthylsilyl)éthoxy]méthyl]-1H-indazol-3-yl]-butanamide décrit précédemment dans 30 cm³ de tétrahydrofurane on ajoute 11 cm³ de fluorure de térabutylammonium en solution 1M dans le tétrahydrofurane et on chauffe à reflux pendant 18 heures. On dilue le milieu réactionnel avec 75 cm³ d'acétate d'éthyle et 50 cm³ d'une solution saturée en hydrogénocarbonate de sodium. La phase organique est décantée, lavée par 50 cm³ d'une solution saturée en chlorure de sodium, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (50/50 en volumes). Les fractions contenant le produit attendu, sont réunies et évaporées sous pression réduite (2 kPa ; 50°C); le résidu est repris par 25' cm³ d'éther diéthylique, filtré, lavé par 2 fois 10 cm³ d'éther diéthylique, séché sous pression réduite (90 Pa; 45°C) pour donner 220 mg de N-[6-chloro-5-(3-furanyl)-1H-indazol-3-yl]-butanamide sous forme de cristaux blancs fondant à 250°C.

Spectre de R.M.N. ¹H (300 MHz, (CD₃)₂SO d6, δ en ppm) : 0,95 (t, J = 7,5 Hz : 3H) ; 1,65 (mt : 2H) ; 2,39 (t large, J = 7 Hz : 2H) ; 6,76 (s large : 1H) ; 7,64 (s : 1H) ; 7,78 (t, J = 1,5 Hz : 1H) ; 7,95 (mt : 2H) ; 10,94 (mf: 1H) ; de 12,50 à 13,00 (mf étalé : 1H).

### EXEMPLE 69

### 1-bromo-2-chloro-4-(phénylméthoxy)-benzene:

A 480 mg d'hydrure de sodium à 60% dans l'huile, dans 10 cm³ de diméthylformamide on ajoute 2 g de 4-bromo-3-chlorophénol en solution dans 20 cm³ de diméthylformamide puis on ajoute une solution de 1.38 cm³ de chlorure de benzyle dissout dans 5 cm³ de diméthylformamide. Le milieu réactionnel est concentré sous pression réduite et repris par 100 cm³ d'acétate d'éthyle. La phase organique est lavée successivement avec 2 fois 50 cm³ d'eau et avec 50 cm³ d'une solution saturée en chlorure de sodium, décantée, séchée sur sulfate de magnésium, filtrée et concentrée à sec sous pression réduite (2 kPa ; 50°C) pour donner 3 g de brut qui est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (90/10 en volumes). Les fractions contenant le produit attendu, sont réunies et évaporées sous pression réduite ( 2 kPa ; 50°C). On obtient 2.63 g de 1-bromo-2-chloro-4-(phénylméthoxy) benzene sous forme d'une huile orangé qui cristallise.

Spectre de R.M.N. ¹H (300 MHz, (CD₃)₂SO d6, δ en ppm) : 5,16 (s : 2H) ; 6,99 (dd, J = 9 et 3 Hz : 1H) ; 7,34 (d, J = 3 Hz : 1H) ; de 7,35 à 7,55 (mt : 5H) ; 7,65 (d, J = 9 Hz : 1H).

EI m/z = 296

### N-[6-[2-chloro-4-(phénylméthoxy)-phényl]-1-[[2-(triméthylsilyl)éthoxy]méthyl]-1H-indazol-3-yl]-butanamide:

A 2 g de N-[6-(4,4,5,5-tétraméthyl-[1,3,2]dioxaborolan-2-yl)-1-[[2-(triméthylsilyl)éthoxy]méthyl]-1H-indazol-3-yl]-butanamide précédemment décrit à l'exemple 56, dans 180 cm³ de dioxane, on ajoute 1.95 g de 1-bromo-2-chloro-4-(phénylméthoxy)benzene décrit précédemment, 1.11 g de carbonate de sodium dans 36 cm³ d'eau et 347 mg de tétrakis-triphénylphosphine palladium. Le milieu est ensuite porté au reflux pendant 2 heures puis on laisse revenir à température ambiante et on ajoute 200 cm³ d'acétate d'éthyle, 100 cm³ d'eau et filtre sur verre fritté garni de célite. Les phases organiques réunies sont lavées avec 100 cm³ d'eau distillée puis avec 100 cm³ d'une solution aqueuse saturée en chlorure de sodium, séchées sur sulfate de magnésium, filtrées et concentrées à sec sous pression réduite ( 2 kPa ; 45 °C). Le résidu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (85/15 en volumes). Les fractions contenant le produit attendu, sont réunies et évaporées sous pression réduite (2 kPa ; 50°C). On obtient 1.34 g de N-[6-[2-chloro-4-(phénylméthoxy)-phényl)-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,08 (s : 9H) ; 0,83 (t, J = 8 Hz : 2H) ; 0,98 (t, J = 7,5 Hz : 3H) ; 1,69 (mt : 2H) ; 2,42 (t, J = 7 Hz : 2H) ; 3,55 (t, J = 8 Hz : 2H) ; 5,23 (s : 2H) ; 5,70 (s : 2H) ; 7,13 (dd, J = 8,5 et 2,5 Hz : 1H) ; 7,15 (d large, J = 8,5 Hz : 1H) ; 7,30 (d, J = 2,5 Hz : 1H) ; de 7,35 à 7,55 (mt : 6H) ; 7,69 (s large : 1H) ; 7,86 (d, J = 8,5 Hz : 1H) ; 10,51 (mf : 1H).

EI m/z = 549 M⁺.

m/z = 432 [M-OCH₂CH₂Si(CH₃)₃]⁺

m/z = 421 [M-C₆H₁₂OSi]^{+.}

### N-[6-[2-chloro-4-(phénylméthoxy)-phényl]-1H-indazol-3-yl]-butanamide:

A 1.3 g de N-[6-[2-chloro-4-(phénylméthoxy)phényl]-1-[[2-(triméthylsilyl)éthoxy]méthyl]-1H-indazol-3-yl]-butanamide précédemment décrit, dans 60 cm³ de tétrahydrofurane on ajoute 14.2 cm³ de fluorure de tétrabutylammonium en solution 1M dans le tétrahyrofurane et on porte à reflux pendant 18 heures ; après refroidissement on ajoute 75 cm³ d'acétate d'éthyle et la phase organique est lavée successivement avec 50 cm³ d'une solution saturée en hydrogénocarbonate de sodium et avec 50 cm³ d'une solution saturée en chlorure de sodium. La phase organique est décantée, séchée sur sulfate de magnésium, filtrée et concentrée à sec sous pression réduite (2 kPa ; 50°C) pour donner 1.8 g de brut qui est purifié par chromatographie sous pression d'argon de 50 kPa, sur colonne de gel de silice ( granulométrie 40-60 µm ; diamètre 3,5 cm), en éluant par un mélange cyclohexane-acétate d'éthyle ( 60/40 en volumes). Les fractions contenant le produit attendu sont réunies et évaporées sous pression réduite ( 2 kPa ; 50°C) et le résidu est repris avec 10 cm³ d'éther diisopropylique, filtré, lavé par 2 fois 10 cm³ d'éther diisopropylique et séché sous pression réduite (90 Pa ; 45°C) pour donner donner 600 mg de N-[6-[2-chloro-4-(phénylméthoxy)phényl]-1H-indazol-3-yl]-butanamide sous forme de cristaux blancs.

Spectre de R.M.N. ¹H (400 MHz, (CD₃)₂SO d6, δ en ppm) : 0,98 (t, J = 7,5 Hz : 3H) ; 1,68 (mt : 2H) ; 2,40 (t, J = 7 Hz : 2H) ; 5,22 (s : 2H) ; 7,06 (d large, J = 8,5 Hz : 1H) ; 7,11 (dd, J = 8,5 et 2,5 Hz : 1H) ; 7,27 (d, J = 2,5 Hz : 1H) ; de 7,35 à 7,55 (mt : 7H) ; 7,79 (d, J = 8,5 Hz : 1H) ; 10,35 (mf : 1H) ; 12,69 (mf : 1H).

EI m/z = 419 M^{+.}

m/z = 349 [M - C₄CH₆O]^{+.}

### N-[6-(2-chloro-4-hydroxyphényl)-1H-indazol-3-yl]-butanamide :

A 0.6 g de N-[6-[2-chloro-4-(phénylméthoxy)phenyl]-1H-indazol-3-yl]-butanamide décrit précédemment on ajoute 15 cm³ d'iodure de triméthylsilyle et on chauffe au reflux pendant 4 heures puis on ajoute 50 cm³ de méthanol et on poursuit le reflux 15 minutes. Le milieu réactionnel est concentré à sec sous pression réduite (2 kPa ; 50°C), on ajoute 100 cm³ d'acétate d'éthyle et on lave la phase organique avec 2 fois 50 cm³ d'une solution de thiosulfate de sodium à 10% puis avec 50 cm³ d'une solution saturée en chlorure de sodium. La phase organique est décantée, séchée sur sulfate de magnésium, filtrée et concentrée à sec. Le brut obtenu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (60/40 en volumes). Les fractions contenant le produit attendu, sont réunies et évaporées sous pression réduite (2 kPa ; 50°C) ; on obtient 0.24 g de N-[6-(2-chloro-4-hydroxyphényl)-1H-indazol-3-yl]-butanamide sous forme d'une meringue blanche.

Spectre de R.M.N. ¹H (400 MHz, (CD₃)₂SO d6, δ en ppm) : 0,90 (t, J = 7,5 Hz : 3H) ; 1,68 (mt : 2H) ; 2,40 (t, J = 7 Hz : 2H) ; 6,86 (dd, J = 8,5 et 2 Hz : 1H) ; 6,97 (d, J = 2 Hz : 1H) ; 7,05 (d large, J = 9 Hz : 1H) ; 7,30 (d, J = 8,5 Hz : 1H) ; 7,37 (s : 1H) ; 7,78 (d, J = 9 Hz : 1H) ; 10,02 (mf : 1H) ; 10,33 (s large : 1H) ; 12,65 (s large : 1H).

EI m/z = 329 M^{+.}

m/z = 259 [M - C₄CH₆O]^{+.}

### EXEMPLE 70

### N-[5,6-dibromo-1-[[2-(triméthylsilyl)éthoxy]méthyl]-1H-indazol-3-yl]-butanamide :

A 4 g de N-[6-bromo-1-[[2-(triméthylsilyl)éthoxy]méthyl]-1H-indazol-3-yl]-butanamide précédemment décrit à l'exemple 51, dans 80 cm³ de chloroforme on ajoute 3.3 cm³ de pyridine et on additionne goutte à goutte 2 cm³ de brome puis on laisse sous agitation pendant 18 heures. On dilue le milieu réactionnel avec 100 cm³ de chlorure de méthylène et la phase organique est lavée par 2 ,fois 100 cm³ d'une solution de thiosulfate de sodium à 10% puis avec 75 cm³ d'une solution saturée en chlorure de sodium. La phase organique est décantée, séchée sur sulfate de magnésium, filtrée et concentrée à sec sous pression réduite (2 kPa ; 50°C). Le brut est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (70/30 en volumes). Les fractions contenant le produit attendu, sont réunies et évaporées sous pression réduite (2 kPa ; 50°C) puis séchées (90 Pa; 45°C) pour donner 4.24 g de N-[5,6-dibromo-1-[[2-(triméthylsilyl)éthoxy]méthyl]-1H-indazol-3-yl]-butanamide sous forme d'un solide jaune fondant à 134°C.

Spectre de R.M.N. ¹H (300 MHz, (CD₃)₂SO d6, δ en ppm) : - 0,08 (s : 9H) ; 0,81 (t, J = 8 Hz : 2H) ; 0,95 (t, J = 7,5 Hz : 3H) ; 1,66 (mt : 2H) ; 2,40 (t, J = 7 Hz : 2H) ; 3,52 (t, J = 8 Hz : 2H) ; 5,66 (s : 2H) ; 8,26 (s : 1H) ; 8,33 (s : 1H) ; 10,66 (mf : 1H).

### N-[5,6-dibromo-1H-indazol-3-yl]-butanamide :

A 1 g de N-[5,6-dibromo-1-[[2-(triméthylsilyl)éthoxy]méthyl]-1H-indazol-3-yl]-butanamide précédemment décrit, dans 60 cm³ de tétrahydrofurane on ajoute 12.2 cm³ de fluorure de tétrabutylammonium en solution 1M dans le tétrahyrofurane et on porte à reflux pendant 18 heures ; après refroidissement on ajoute 75 cm³ d'acétate d'éthyle et la phase organique est lavée successivement avec 100 cm³ d'une solution saturée en hydrogénocarbonate de sodium puis avec 75 cm³ d'une solution saturée en chlorure de sodium. La phase organique est décantée, séchée sur sulfate de magnésium, filtrée et concentrée à sec sous pression réduite (2 kPa ; 50°C) pour donner 1.6 g de brut qui est purifié par chromatographie sous pression d'argon de 50 kPa, sur colonne de gel de silice (granulométrie 40-60 µm ; diamètre 3,5 cm), en éluant par un mélange cyclohexane-acétate d'éthyle ( 50/50' en volumes). Les fractions contenant le produit attendu sont réunies et évaporées sous pression réduite ( 2 kPa ; 50°C) et le résidu est repris dans 20 cm³ d'éther diisopropylique, filtré, lavé par 2 fois 10 cm³ d'éther diisopropylique et séché (90 Pa ; 45°C) pour donner 460 mg de N-[5,6-dibromo-1H-indazol-3-yl]-butanamide sous forme de cristaux bleutés fondant à 250°C.

Spectre de R.M.N. ¹H (300 MHz, (CD₃)₂SO d6, δ en ppm) : 0,95 (t, J = 7,5 Hz : 3H) ; 1,66 (mt : 2H) ; 2,39 (t, J = 7 Hz : 2H) ; 7,91 (s : 1H) ; 8,28 (s : 1H) ; 10,55 (mf : 1H) ; de 12,70 à 13,20 (mf étalé : 1H).

### EXEMPLE 71

### N-[6-chloro-1H-indazol-3-yl]-2,2,3,3,4,4,4-heptafluoro-butanamide:

A 1 g de 6-chloro-1H-indazole-3-amine dans 10 cm³ de pyridine, on ajoute 0.60 cm³ de chlorure d'heptafluorobutyryle après avoir refroidi le milieu vers 6 °C puis on laisse revenir à la température ambiante pendant 19 heures et on évapore à sec sous pression réduite (2 kPa ; 45 °C). Le résidu est repris avec 40 cm³ d'acétate d'éthyle et 20 cm³ d'eau ; le précipité formé est filtré sur verre fritté puis rincé avec 2 fois 10 cm³ de chlorure de méthylène et purifié par chromatographie sous pression d'argon de 50 kPa, sur colonne de gel de silice ( granulométrie 40-60 µm ; diamètre 3,5 cm), en éluant par un mélange cyclohexane-acétate d'éthyle ( 70/30 en volumes). Les fractions contenant le produit attendu sont réunies et évaporées sous pression réduite ( 2 kPa; 50°C) pour donner 0.77 g de N-[6-chloro-1H-indazol-3-yl]-2,2,3,3,4,4,4-heptafluoro-butanamide sous forme cotonneuse.

Spectre de R.M.N. ¹H (300 MHz, (CD₃)₂SO d6, δ en ppm) : 7,19 (dd large, J = 9 et 1,5 Hz : 1H) ; 7,62 (d, J = 9 Hz : 1H) ; 7,64 (s large : 1H) ; 12,09 (mf : 1H) ; 13,25 (mf : 1H).

EI m/z = 363 M^{+.}

m/z = 194 [M - CF₂CF₂CF₃]⁺

m/z = 166 [M - COCF₂CF₂CF₃]⁺

### EXEMPLE 72

### N-[5-(4-fluorophényl)-6-chloro-1-[[2-(triméthylsilyl)éthoxy]méthyl]-1H-indazol-3-yl]-butanamide

A 1g de N-[5-bromo-6-chloro-1-[[2-(triméthylsilyl)éthoxy]méthyl]-1H-indazol-3-yl]-butanamide décrit à l'exemple 58, dans 40 cm³ de dioxane, on ajoute 470 mg d'acide 4-fluorophénylboronique, 593 mg de carbonate de sodium dans 40 cm³ d'eau et 155 mg de tétrakis-triphénylphosphine palladium. On chauffe au reflux pendant 18 heures et on filtre le milieu réactionnel sur verre fritté garni de célite. On ajoute au filtrat 60 cm³ d'acétate d'éthyle et 50 cm³ d'eau distillée. La phase organique est lavée avec 2 fois 20 cm³ d'une solution aqueuse saturée en chlorure de sodium puis décantée et séchée sur sulfate de magnésium. Après filtration, le filtrat est concentré à sec sous pression réduite (2 kPa ; 50°C) et le résidu est purifié par chromatographie sous pression d'argon de 50 kPa, sur colonne de gel de silice ( granulométrie 40-60 µm ; diamètre 4.5 cm), en éluant par un mélange cyclohexane-acétate d'éthyle ( 90/10 en volumes). Les fractions contenant le produit attendu sont réunies et évaporées sous pression réduite (2 kPa ; 50°C). On obtient ainsi après séchage (90 Pa ; 45°C), 0.75 g de N-[5-(4-fluorophényl)-6-chloro-1-[(2-(triméthylsilyl)éthoxy]méthyl]-1H-indazol-3-yl]-butanamide sous forme d'une cire jaune.

Spectre de R.M.N. ¹H (300 MHz, (CD₃)₂SO d6, δ en ppm) : - 0,05 (s : 9H) ; 0,85 (t, J = 8 Hz : 2H) ; 0,93 (t, J = 7,5 Hz : 3H) ; 1,63 (mt : 2H) ; 2,38 (t, J = 7 Hz : 2H) ; 3,55 (t, J = 8 Hz : 2H) ; 5,70 (s : 2H) ; 7,31 (t large, J = 9 Hz : 2H) ; 7,46 (dd, J = 9 et 6 Hz : 2H) ; 7,91 (s : 1H) ; 8,00 (s : 1H) ; 10,60 (mf : 1H).

EI m/z=461 M^{+.}

m/z = 344 [M - OCH₂CH₂Si(CH₃)₃]⁺

m/z = 333 [M - C₆H₁₂OSi]^{+.}

### N-[6-chloro-5-(4-fluorophényl)-1H-indazol-3-yl]-butanamide

A 0.73 g de N-[6-chloro-5-(4-fluorophényl)-1-[[2-(triméthylsilyl)éthoxy]méthyl]-1H-indazol-3-yl]-butanamide décrit précédemment, dans 20 cm³ de tétrahydrofurane on ajoute 9.5 cm³ de fluorure de tétrabutylammonium en solution 1M dans le tétrahydrofurane et on porte à reflux pendant 18 heures ; après refroidissement on ajoute 30 cm³ d'acétate d'éthyle et la phase organique est lavée successivement avec 2 fois 20 cm³ d'une solution saturée en hydrogénocarbonate de sodium et avec 20 cm³ d'une solution saturée en chlorure de sodium. La phase organique est décantée, séchée sur sulfate de magnésium, filtrée et concentrée à sec sous pression réduite (2 kPa ; 50°C) ; le brut obtenu est purifié par chromatographie sous pression d'argon de 50 kPa, sur colonne de gel de silice ( granulométrie 40-60 µm ; diamètre 3,5 cm), en éluant par un mélange cyclohexane-acétate d'éthyle ( 70/30 en volumes). Les fractions contenant le produit attendu sont réunies et évaporées sous pression réduite ( 2 kPa; 50°C) et séchées (90 Pa ; 45°C) pour donner donner 200 mg de N-[6-chloro-(4-fluorophenyl)-1H-indazol-3-yl]-butanamide sous forme de poudre crème.

Spectre de R.M.N. ¹H (300 MHz, (CD₃)₂SO d6, δ en ppm) : 0,93 (t, J = 7,5 Hz : 3H) ; 1,63 (mt : 2H) ; 2,38 (t, J = 7 Hz : 2H) ; 7,30 (t large, J = 9 Hz : 2H) ; 7,45 (dd large, J = 9 et 6 Hz : 2H) ; 7,66 (s : 1H) ; 7,85 (s : 1H) ; 10,46 (mf: 1H) ; 12,80 (mf: 1H).

EI m/z = 331

m/z = 261 [M - C₄CH₆O]^{+.}

### EXEMPLE 73

### N-[[6-(4-aminophényl)-1H-indazol-3-yl]]-butanamide :

A 0.85 g de N-[6-(4-nitrophényl)-1H-indazol-3-yl]-butanamide décrit à l'exemple 64, dans 50 cm³ d'acide acétique on ajoute 856 mg de zinc en poudre puis 1 heure après, à nouveau 856 mg de zinc et on agite 1 heure à température ambiante. Le milieu réactionnel est filtré sur verre fritté garni de célite et le filtrat est concentré à sec sous pression réduite (2 kPa ; 50°C). Le résidu est repris avec 100 cm³ de tétrahydrofurane et 100 cm³ d'acétate d'éthyle et la phase organique est lavée successivement avec 100 cm³ d'une solution saturée en hydrogénocarbonate de sodium et avec 100 cm³ d'une solution saturée en chlorure de sodium puis séchée sur sulfate de magnésium, filtrée et concentrée à sec pour donner 500 mg de brut qui est purifié par chromatographie sous pression d'argon de 50 kPa, sur colonne de gel de silice ( granulométrie 40-60 µm ; diamètre 4.5 cm), en éluant par de l'acétate d'éthyle. Les fractions contenant le produit attendu sont réunies et le solide trituré dans 20 cm³ d'éther diéthylique, filtré, lavé avec 2 fois 5 cm³ d'éther diéthylique puis séché (90Pa ; 45°C) pour donner 200 mg de N-[6-(4-aminophényl)-1H-indazol-3-yl]-butanamide sous forme de cristaux jaunes fondant à 230°C.

Spectre de R.M.N. ¹H (300 MHz, (CD₃)₂SO d6, δ en ppm) : 0,98 (t large, J = 7,5 Hz : 3H) ; 1,68 (mt : 2H) ; 2,39 (t, J = 7 Hz : 2H) ; 5,24 (s large : 2H) ; 6,68 (d large, J = 8 Hz : 2H) ; 7,27 (d large, J = 8,5 Hz : 1H) ; 7,42 (d large, J = 8 Hz : 2H) ; 7,45 (s large : 1H) ; 7,76 (d, J = 8,5 Hz : 1H) ; 10,26 (mf : 1H) ; 12,49 (s large : 1H).

### EXEMPLE 74

### N-[6-[4-(diméthylamino)phényl]-1-[[2-(triméthylsilyl)éthoxy]méthyl]-1H-indazol-3-yl]-butanamide:

A 1 g de N-[6-(4,4,5,5-tétraméthyl-[1,3,2]dioxaborolan-2-yl)-1-[[2-(triméthylsilyl)éthoxy]méthyl]-1H-indazol-3-yl]-butanamide décrit à l'exemple 56, dans 50 cm³ de dioxane, on ajoute 785 mg de 4-bromo-N,N-diméthylaniline, 646 mg de carbonate de sodium, 10 cm³ d'eau et 196 mg de tétrakis-triphénylphosphine palladium. Le milieu est ensuite porté au reflux pendant 18 heures puis on laisse revenir à température ambiante et on ajoute 75 cm³ d'acétate d'éthyle et 75 cm³ d'eau. La phase organique est séchée sur sulfate de magnésium, filtrée et concentrée à sec sous pression réduite ( 2 kPa ; 45 °C) pour donner 1.6 g de brut qui est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (80/20 en volumes). Les fraçtions contenant le produit attendu, sont réunies et évaporées sous pression réduite ( 2 kPa ; 50°C). On obtient 170 mg de N-[6-[4-(diméthylamino)phényl]-1-[[2-(triméthylsilyl)éthoxy]méthyl]-1H-indazol-3-yl]-butanamide sous forme d'huile jaune.

Spectre de R.M.N. ¹H (400 MHz, (CD₃)₂SO d6, δ en ppm) : - 0,08 (s : 9H) ; 0,84 (t, J = 8 Hz : 2H) ; 0,98 (t, J = 7,5 Hz : 3H) ; 1,68 (mt : 2H) ; 2,40 (t, J = 7 Hz : 2H) ; 2,98 (s : 6H) ; 3,57 (t, J = 8 Hz : 2H) ; 5,71 (s : 2H) ; 6,85 (d, J = 9 Hz : 2H) ; 7,41 (d large, J = 8,5 Hz : 1H) ; 7,64 (d, J = 9 Hz : 2H) ; 7,82 (s large : 1H) ; 7,85 (d, J = 8,5 Hz : 1H) ; 10,43 (mf: 1H).
EI m/z = 452 M^{+.}

m/z = 335 [M - OCH₂CH₂Si(CH₃]⁺

m/z = 324 [M - C₆H₁₂OSi]^{+.}

### N-[6-[4-(diméthylamino)phényl]-1H-indazol-3-yl]-butanamide :

A 170 mg de N-[6-[4-(diméthylamino)phényl]-1-[[2-(triméthylsilyl)éthoxy]méthyl]-1H-indazol-3-yl]-butanamide précédemment décrit, dans 10 cm³ de tétrahydrofurane on ajoute 2.3 cm³ de fluorure de tétrabutylammonium en solution 1M dans le tétrahydrofurane et on porte à reflux pendant 18 heures ; après refroidissement on ajoute 50 cm³ d'acétate d'éthyle et la phase organique est lavée successivement avec 50 cm³ d'une solution saturée en hydrogénocarbonate de sodium et avec 50 cm³ d'une solution saturée en chlorure de sodium. La phase organique est décantée, séchée sur sulfate de magnésium, filtrée et concentrée à sec sous pression réduite (2 kPa ; 50°C) pour donner 160 mg de brut qui est purifié par chromatographie sous pression d'argon de 50 kPa, sur colonne de gel de silice ( granulométrie 40-60 µm ; diamètre 3,5 cm), en éluant par un mélange cyclohexane-acétate d'éthyle ( 70/30 en volumes). Les fractions contenant le produit attendu sont réunies et évaporées sous pression réduite ( 2 kPa ; 50°C) ; le résidu est repris avec 10 cm³ d'éther diéthylique, filtré et séché sous pression réduite (90 Pa ; 45°C) pour donner donner 40 mg de N-[6-chloro-5-(4-dméthylamino)phényl)- 1H-indazol-3-yl]-butanamide sous forme de cristaux jaune fondant à 260°C.

Spectre de R.M.N. ¹H (300 MHz, (CD₃)₂SO d6, δ en ppm) : 0,98 (t large, J = 7,5 Hz : 3H) ; 1,68 (mt : 2H) ; 2,39 (t, J = 7 Hz : 2H) ; 2,97 (s : 6H) ; 6,84 (d large, J = 8,5 Hz : 2H) ; 7,31 (d large, J = 9 Hz : 1H) ; 7,51 (s large : 1H) ; 7,58 (d large, J = 8,5 Hz : 2H) ; 7,78 (d, J = 9 Hz : 1H) ; 10,27 (s large : 1H) ; 12,52 (s large : 1H).

### EXEMPLE 75

### 2-chloro-N-(6-chloro-1H-indazol-3-yl)-acétamide

A 5 g de 6-chloro-1H-indazole-3-amine dans 300 cm³ de toluène on ajoute 5,1 g d'anhydride chloracétique et on chauffe au reflux pendant 18 heures. Le précipité apparu est filtré, lavé avec 20 cm³ de toluène puis avec 20 cm³ de chlorure de méthylène et séché sous pression réduite (90 Pa; 45°C) pour donner 5.1 g de 2-chloro-N-(6-chloro-1H-indazol-3-yl)-acétamide, sous forme de poudre grise fondant à 223°C.

Spectre de R.M.N. ¹H (300 MHz, (CD₃)₂SO d6, δ en ppm) : 4,38 (s : 2H) ; 7,11 (dd, J = 9 et 1,5 Hz : 1H) ; 7,56 (s large : 1H) ; 7,84 (d, J = 9 Hz : 1H) ; 10,87 (mf : 1H) ; 12,96 (mf : 1H).

### N-(6-chloro-1H-indazol-3-yl)-4-méthyl-1-pipérazineacétamide

A 500 mg de 2-chloro-N-(6-chloro-1H-indazol-3-yl)-acétamide décrit précédemment, dans 15 cm³ de diméthylformamide on ajoute 0.7 cm³ de N-méthylpipérazine et on chauffe à 140° pendant 2 heures puis le milieu réactionnel est concentré à sec sous pression réduite (2 kPa ; 50°C). On ajoute alors 50 cm³ d'acétate d'éthyle et 50 cm³ d'eau et la phase organique est lavée avec 50 cm³ d'une solution saturée en chlorure de sodium, séchée sur sulfate de magnésium, filtrée et concentrée à sec pour donner 0.53 g de brut qui est purifié par chromatographie sous pression d'argon de 50 kPa, sur colonne de gel de silice ( granulométrie 40-60 µm ; diamètre 2.5 cm), en éluant par un mélange chlorure de méthylène-méthanol-ammoniaque (93/7/1 en volumes). Les fractions contenant le produit attendu, sont réunies et évaporées sous pression réduite ( 2 kPa ; 50°C) ; le résidu est repris par 5 cm³ d'éther diéthylique, filtré et séché (90 Pa ; 45°C) pour donner 192 mg de N-(6-chloro-1H-indazol-3-yl)-4-méthyl-1-pipérazineacétamide sous forme de poudre beige fondant à 165°C.

Spectre de R.M.N. ¹H (300 MHz, (CD₃)₂SO d6, δ en ppm) : 2,18 (s : 3H) ; 2,40 (mf : 4H) ; 2,58 (mf : 4H) ; 3,22 (s : 2H) ; 7,09 (d large, J = 9 Hz : 1H) ; 7,53 (s large : 1H) ; 7,86 (d, J = 9 Hz : 1H) ; 10,11 (s large : 1H) ; 12,83 (s large : 1H).

### EXEMPLE 76

### N-(6-chloro-1H-indazol-3-yl)-1-pipéridineacétamide

On opère comme à l'exemple 75 à partir de 500 mg de 2-chloro-N-(6-chloro-1H-indazol-3-yl)-acétamide de 15 cm³ d'acétonitrile, de 0.61 cm³ de pipéridine. Le milieu réactionnel est chauffé au reflux pendant 2 heures puis le précipité apparu est filtré sur verre fritté et les cristaux, après reprise dans du méthanol, sont purifiés par chromatographie sous pression d'argon de 50 kPa, sur colonne de gel de silice ( granulométrie 40-60 µm ; diamètre 2.5 cm), en éluant par un mélange chlorure de méthylène-méthanol (93/7 en volumes). Les fractions contenant le produit attendu, sont réunies et évaporées sous pression réduite ( 2 kPa ; 50°C) ; le résidu est repris par 5 cm³ d'éther diéthylique, filtré et séché (90 Pa ; 45°C) pour donner 447 mg de N-(6-chloro-1H-indazol-3-yl)-1-pipéridineacétamide sous forme de poudre blanche fondant à 153°C.

Spectre de R.M.N. ¹H (300 MHz, (CD₃)₂SO d6, δ en ppm) : 1,42 (mt, 2H) ; 1,57 (mt : 4H) ; de 2,45 à 2,60 (mt : 4H) ; 3,17 (s : 2H) ; 7,08 (dd, J = 9 et 2 Hz : 1H) ; 7,52 (d, J = 2 Hz : 1H) ; 7,86 (d, J = 9 Hz : 1H) ; 10,05 (s large : 1H) ; 12,82 (mf : 1H).

### EXEMPLE 77

### N-(6-chloro-1H-indazol-3-yl)-4-morpholineacétamide

En opérant comme à l'exemple 75 à partir de 500 mg de 2-chloro-N-(6-chloro-1H-indazol-3-yl)-acétamide, de 15 cm³ d'acétonitrile, de 0.54 cm³ de morpholine. Le milieu réactionnel est chauffé au reflux pendant 2 heures puis concentré à sec sous pression réduite (2.7 kPa ; 50°C) et le brut obtenu est purifié par chromatographie sous pression d'argon de 50 kPa, sur colonne de gel de silice ( granulométrie 40-60 µm ; diamètre 2.5 cm), en éluant par de l'acétate d'éthyle. Les fractions contenant le produit attendu, sont réunies et évaporées sous pression réduite ( 2 kPa ; 50°C) puis séchées (90 Pa; 45°C) pour donner 470 mg de N-(6-chloro-1H-indazol-3-yl)-4-morpholineacétamide sous forme de poudre blanche fondant vers 82°-90°C.

Spectre de R.M.N. ¹H (300 MHz, (CD₃)₂SO d6, δ en ppm) : 2,58 (t large, J = 4 Hz : 4H) ; 3,24 (s : 2H) ; 3,66 (t large, J = 4 Hz : 4H) ; 7,09 (dd, J = 9 et 2 Hz : 1H) ; 7,53 (d, J = 2 Hz : 1H) ; 7,84 (d, J = 9 Hz : 1H) ; 10,18 (mf : 1H) ; 12,83 (mf étalé : 1H).

### EXEMPLE 78

### N-(6-chloro-1H-indazol-3-yl)-1H-1,2,4-triazole-1-acétamide

On opère comme à l'exemple 75 à partir de 500 mg de 2-chloro-N-(6-chloro-1H-indazol-3-yl)-acétamide de 15 cm³ d'acétonitrile, de 423 mg de 1,2,4 triazole, 283 mg de carbonate de potassium. Le milieu réactionnel est chauffé au reflux pendant 4 heures puis concentré à sec sous pression réduite (2 kPa ; 50°C) ; le brut obtenu est purifié par chromatographie sous pression d'argon de 50 kPa, sur colonne de gel de silice ( granulométrie 40-60 µm ; diamètre 2.5 cm), en éluant par de l'acétate d'éthyle. Les fractions contenant le produit attendu, sont réunies, évaporées sous pression réduite ( 2 kPa ; 50°C) et séchées ( 90 Pa ; 45°C) pour donner 120 mg de N-(6-chloro-1H-indazol-3-yl)-1H-1,2,4-triazole-1-acétamide sous forme de poudre blanche fondant à plus de 260°C.

Spectre de R.M.N. ¹H (300 MHz, (CD₃)₂SO d6, δ en ppm) : 5,27 (s : 2H) ; 7,10 (dd, J = 9 et 2 Hz : 1H) ; 7,55 (d, J = 2 Hz : 1H) ; 7,84 (d, J = 9 Hz : 1H) ; 8,03 (s : 1H) ; 8,60 (s : 1H) ; 11,00 (mf: 1H) ; 12,90 (mf: 1H).

DCI m/z = 294 [M+NH₄]⁺

m/z = 277 [M+H]⁺

### EXEMPLE 79

### N-(6-chloro-1H-indazol-3-yl)-2-(cyclohexylamino)-acétamide

On opère comme à l'exemple 75 à partir de 500 mg de 2-chloro-N-(6-chloro-1H-indazol-3-yl)-acétamide de 15 cm³ d'acétonitrile, de 0.7 cm³ de cyclohexylamine. Le milieu réactionnel est chauffé au reflux pendant 2 heures puis concentré à sec sous pression réduite (2 kPa ; 50°C) ; le brut obtenu est purifié par chromatographie sous pression d'argon de 50 kPa, sur colonne de gel de silice ( granulométrie 40-60 µm ; diamètre 2.5 cm), en éluant par un mélange chlorure de méthylène-méthanol-ammoniaque (97/2.5/0.25 en volumes). Les fractions contenant le produit attendu sont réunies et le résidu repris par 20 cm³ d'éther diisopropylique, filtré , séché sous pression réduite (90 Pa ; 45°C) pour donner 492 mg de N-(6-chloro-1H-indazol-3-yl)-2-(cyclohexylamino)-acétamide sous forme de poudre blanche fondant à 170°C.

Spectre de R.M.N. ¹H (300 MHz, (CD₃)₂SO d6, δ en ppm) : de 1,00 à 1,35 (mt : 5H) ; 1,56 (mt : 1H) ; 1,70 (mt : 2H) ; 1,84 (d très large, J = 12 Hz : 2H) ; 2,43 (mt : 1H) ; 3,39 (s : 2H) ; 7,09 (dd, J = 9 et 1,5 Hz : 1H) ; 7,52 (d, J = 1,5 Hz : 1H) ; 7,93 (d, J = 9 Hz : 1H) ; 12,82 (mf : 1H).

### EXEMPLE 80

### 2-[(phénylméthyl)amino]-N-(6-chloro-1H-indazol-3-yl)-acétamide

On opère comme à l'exemple 75 à partir de 500 mg de 2-chloro-N-(6-chloro-1H-indazol-3-yl)-acétamide, de 15 cm³ d'acétonitrile, de 0.67 cm³ de benzylamine. Le milieu réactionnel est chauffé au reflux pendant 1 heure et le précipité apparu est filtré, lavé par 5 cm³ d'acétonitrile et 5 cm³ de chlorure de méthylène puis purifié par chromatographie sous pression d'argon de 50 kPa, sur colonne de gel de silice ( granulométrie 40-60 µm ; diamètre 2.5 cm), en éluant par un mélange chlorure de méthylène-méthanol-ammoniaque (97/2.5/0.25 en volumes). Les fractions contenant le produit attendu sont réunies et le résidu repris par 20 cm³ d'éther diisopropylique, filtré et séché sous pression réduite (90 Pa; 45°C) pour donner 305 mg de 2-[(phénylméthyl)amino]-N-(6-chloro-1H-indazol-3-yl)-acétamide sous forme de poudre blanche et fondant à 156°C.

Spectre de R.M.N. ¹H (300 MHz, (CD₃)₂SO d6, δ en ppm) : 3,39 (s : 2H) ; 3,79 (s : 2H) ; 7,09 (dd, J = 9 et 2 Hz : 1H) ; 7,26 (t large, J = 7 Hz : 1H) ; de 7,30 à 7,45 (mt : 4H) ; 7,53 (d, J = 2 Hz : 1H) ; 7,89 (d, J = 9 Hz : 1H) ; de 10,00 à 10,60 (mf très étalé : 1H) ; 12,82 (mf : 1H).

### EXEMPLE 81

### N-(6-chloro-1H-indazol-3-yl)-1H-azepine-1-acétamide

On opère comme à l'exemple 75 à partir de 500 mg de 2-chloro-N-(6-chloro-1H-indazol-3-yl)-acétamide, de 15 cm³ d'acétonitrile, de 0.69 cm³ d'hexaméthylèneimine. Le milieu réactionnel est chauffé au reflux pendant 2 heures puis concentré à sec sous pression réduite (2 kPa ; 50°C) ; le brut est purifié par chromatographie sous pression d'argon de 50 kPa, sur colonne de gel de silice ( granulométrie 40-60 µm ; diamètre 2.5 cm), en éluant par de l'acétate d'éthyle. Les fractions contenant le produit attendu, sont réunies et le résidu repris par 10 cm³ d'éther diisopropylique, filtré et séché sous pression réduite (90 Pa; 45°C) pour donner 670 mg de N-(6-chloro-1H-indazol-3-yl)-1H-azepine-1-acétamide sous forme de meringue jaune.

Spectre de R.M.N. ¹H (300 MHz, (CD₃)₂SO d6, δ en ppm) : de 1,50 à 1,75 (mt : 8H) ; 2,77 (t, J = 5 Hz : 4H) ; 3,36 (s : 2H) ; 7,09 (dd, J = 9 et 2 Hz : 1H) ; 7,54 (d, J = 2 Hz : 1H) ; 7,90 (d, J = 9 Hz : 1H) ; 10,06 (mf: 1H); de 12,50 à 13,20 (mf étalé : 1H).

EI m/z = 306 M^{+.}

### EXEMPLE 82

### N-(6-chloro-1H-indazol-3-yl)-1-pipérazineacétamide

On opère comme à l'exemple 75 à partir de 500 mg de 2-chloro-N-(6-chloro-1H-indazol-3-yl)-acétamide, de 15 cm³ d'acétonitrile, de 528 mg de pipérazine. Le milieu réactionnel est chauffé au reflux pendant 1 heure puis concentré à sec sous pression réduite (2 kPa ; 50°C) ; le brut est purifié par chromatographie sous pression d'argon de 50 kPa, sur colonne de gel de silice ( granulométrie 40-60 µm ; diamètre 2.5 cm), en éluant par un mélange chlorure de méthylène-méthanol-ammoniaque (90/10/1 en volume). Les fractions contenant le produit attendu, sont réunies, concentrées sous pression réduite puis séchées (90 Pa ; 45°C) pour donner 380 mg de N-(6-chloro-1H-indazol-3-yl)-1-pipérazineacétamide sous forme de meringue blanche.

Spectre de R.M.N. ¹H (300 MHz, (CD₃)₂SO d6, δ en ppm) : 2,49 (mt : 4H) ; 2,77 (mt : 4H) ; 3,19 (s : 2H) ; 7,10 (dd, J = 9 et 2 Hz : 1H) ; 7,55 (d, J = 2 Hz : 1H) ; 7,86 (d, J = 9 Hz : 1 H) ; 10,10 (mf : 1H).

EI m/z = 293 M^{+.}

m/z = 99 [C₅H₁₁N₂]⁺

### EXEMPLE 83

### N-(6-chloro-1H-indazol-3-yl)-2-[[3-(diméthylamino)propyl]amino]-acétamide

On opère comme à l'exemple 75 à partir de 500 mg de 2-chloro-N-(6-chloro-1H-indazol-3-yl)-acétamide, de 15 cm³ d'acétonitrile, de 0.77 cm³ de 3-(diméthylamino)propylamine. Le milieu réactionnel est chauffé au reflux pendant 3 heures puis concentré à sec sous pression réduite (2 kPa ; 50°C) ; le brut est purifié par chromatographie sous pression d'argon de 50 kPa, sur colonne de gel de silice ( granulométrie 40-60 µm ; diamètre 2.5 cm), en éluant par un mélange chlorure de méthylène-méthanol-ammoniaque (90/10/1 en volume). Les fractions contenant le produit attendu, sont réunies, concentrées sous pression réduite puis séchées (90 Pa ; 45°C) pour donner 300 mg de N-(6-chloro-1H-indazol-3-yl)-2-[[3-(diméthylamino)propyl]amino]-acétamide sous forme de meringue marron clair.

Spectre de R.M.N. ¹H (300 MHz, (CD₃)₂SO d6, δ en ppm) : 1,69 (mt : 2H) ; 2,11 (s : 6H) ; 2,28 (t, J = 7 Hz : 2H) ; 2,60 (t large, J = 7 Hz : 2H) ; 3,35 (s : 2H) ; 7,08 (dd, J = 9 et 2 Hz : 1H) ; 7,53 (d, J = 2 Hz : 1H) ; 7,89 (d, J= 9 Hz : 1H) ; de 12,00 à 13,00, (mf très étalé : 1H).

EI : m/z = 309 M^{+.}

### EXEMPLE 84

### N-(6-chloro-1H-indazol-3-yl)-thiomorpholine-4-acétamide

On opère comme à l'exemple 75 à partir de 500 mg de 2-chloro-N-(6-chloro-1H-indazol-3-yl)-acétamide de 15 cm³ d'acétonitrile, de 0.62 cm³ de thiomorpholine. Le milieu réactionnel est chauffé au reflux pendant 2 heures, le précipité formé est filtré et le filtrat est concentré à sec sous pression réduite (2 kPa ; 50°C) ; le brut obtenu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (50/50 en volumes). Les fractions contenant le produit attendu, sont réunies et évaporées sous pression réduite ( 2 kPa ; 50°C) et séchées (90 Pa; 45°C) pour donner 560 mg de N-(6-chloro-1H-indazol-3-yl)-thiomorpholine-4-acétamide sous forme de meringue jaune pâle.

Spectre de R.M.N. ¹H (300 MHz, (CD₃)₂SO d6, δ en ppm) : 2,70 (mt : 4H) ; 2,83 (mt : 4H) ; 3,27 (s : 2H) ; 7,10 (dd, J = 9 et 2 Hz : 1H) ; 7,54 (d, J = 2 Hz : 1H) ; 7,82 (d, J = 9 Hz : 1H) ; 10,16 (mf : 1H) ; de 12,60 à 13,10 (mf étalé : 1H).

EI m/z = 310 M^{+.}

m/z=116 [C₅CH₁₀NS]⁺

### EXEMPLE 85

### N-(6-chloro-1H-indazol-3-yl)-1-pyrrolidineacétamide

On opère comme à l'exemple 75 à partir de 500 mg de 2-chloro-N-(6-chloro-1H-indazol-3-yl)-acétamide, de 15 cm³ d'acétonitrile, de 0.51 cm³ de pyrrolidine. Le milieu réactionnel est chauffé au reflux pendant 2 heures puis concentré à sec sous pression réduite (2 kPa ; 50°C) ; le brut obtenu est purifié par chromatographie sous pression d'argon de 50 kPa, sur colonne de gel de silice (granulométrie 40-60 µm ; diamètre 2 cm), en éluant par un mélange chlorure de méthylène-méthanol-ammoniaque (95/5/1 en volumes). Les fractions contenant le produit attendu, sont réunies, évaporées sous pression réduite ( 2 kPa ; 50°C) et séchées (90 Pa ; 45°C) pour donner 440 mg de N-(6-chloro-1H-indazol-3-yl)-1-pyrrolidineacétamide sous forme de poudre blanc cassé fondant à 168°C.

Spectre de R.M.N. ¹H (300 MHz, (CD₃)₂SO d6, δ en ppm) : 1,76 (mt : 4H) ; 2,64 (mt : 4H) ; 3,35 (s : 2H) ; 7,09 (dd, J = 9 et 2 Hz : 1H) ; 7,53 (d, J = 2 Hz : 1H) ; 7,84 (d, J = 9 Hz : 1H) ; 10,13 (mf : 1H) ; de 12,50 à 13,10 (mf très étalé : 1H).

### EXEMPLE 86

### N-(6-chloro-1H-indazol-3-yl)-2-[[2-(diméthylamino)éthyl]amino]-acétamide

On opère comme à l'exemple 75 à partir de 500 mg de 2-chloro-N-(6-chloro-1H-indazol-3-yl)-acétamide, de 15 cm³ d'acétonitrile, de 0.68 cm³ de N,N-diméthyléthylènediamine. Le milieu réactionnel est chauffé au reflux pendant 2 heures puis concentré à sec sous pression réduite (2 kPa ; 50°C) ; le brut est purifié par chromatographie sous pression d'argon de 50 kPa, sur colonne de gel de silice ( granulométrie 40-60 µm ; diamètre 2.5 cm), en éluant par un mélange chlorure de méthylène-méthanol-ammoniaque (95/5/1 en volumes). Les fractions contenant le produit attendu, sont réunies, concentrées sous pression réduite puis séchées (90 Pa ; 45°C) pour donner 113 mg de N-(6-chloro-1H-indazol-3-yl)-2-[[2-(diméthylamino)éthyl]amino]-acétamide sous forme de solide blanc fondant à 104°C.

Spectre de R.M.N. ¹H (300 MHz, (CD₃)₂SO d6, δ en ppm) : 2,15 (s : 6H) ; 2,34 (t, J = 6 Hz : 2H) ; 2,66 (t, J = 6 Hz : 2H) ; 3,40 (s : 2H) ; 7,08 (dd large, J = 9 et 2 Hz : 1H) ; 7,52 (s large : 1H) ; 7,90 (d, J = 9 Hz : 1H) ; de 9,50 à 10,30 (mf très étalé : 1H) ; 12,81 (mf : 1H).

### EXEMPLE 87

### N-(6-chloro-1H-indazol-3-yl)-1-cyclopropylaminoacétamide, trifluoroacétate

On opère comme à l'exemple 75 à partir de 500 mg de 2-chloro-N-(6-chloro-1H-indazol-3-yl)-acétamide, de 15 cm³ d'acétonitrile, de 0.45 cm³ de cyclopropylamine. Le milieu réactionnel est chauffé au reflux pendant 2 heures puis concentré à sec sous pression réduite (2 kPa ; 50°C) ; le brut est purifié par chromatographie sous pression d'argon de 50 kPa, sur colonne de gel de silice (granulométrie 40-60 µm ; diamètre 2 cm), en éluant par de l'acétate d'éthyle. Les fractions contenant le produit attendu, sont réunies, concentrées sous pression réduite pour donner 300 mg de produit encore impur qui est purifié par HPLC Après concentration des fractions contenant le produit attendu et séchage sous pression réduite (90 Pa ; 45°C), on obtient 140 mg de N-(6-chloro-1H-indazol-3-yl)-1-cyclopropylamino-acétamide, trifluoroacétate, sous forme de poudre blanche fondant à 218°C.

Spectre de R.M.N. ¹H (300 MHz, (CD₃)₂SO d6, δ en ppm) : de 0,70 à 0,95 (mt : 4H) ; 2,82 (mt : 1H) ; 4,15 (s: 2H) ; 7,14 (dd large, J = 9 et 2 Hz : 1H) ; 7,58 (s large : 1H) ; 7,86 (d, J = 9 Hz : 1H) ; 9,14 (mf: 2H) ; 11,08 (mf : 1H) ; 12,98 (s large : 1H).

### EXEMPLE 88

### N-(6-chloro-1H-indazol-3-yl)-2-(2-diéthylamino-éthylamino)-acétamide, tris Trifluoroacétate

On opère comme à l'exemple 75 à partir de 500 mg de 2-chloro-N-(6-chloro-1H-indazol-3-yl)-acétamide, de 15 cm³ d'acétonitrile, de 0.86 cm³ de N,N-diéthyléthylènediamine. Le milieu réactionnel est chauffé au reflux pendant 2 heures puis concentré à sec sous pression réduite (2 kPa ; 50°C) ; le brut est purifié par HPLC (colonne Kromasil ; C₈, 7µm ; longueur 350 mm, diamètre 60 mm, éluant : acétonitrile-eau (20/80 en volumes) contenant 0.1% d'acide trifluoroacétique ; débit 125 cm³/mn). Les fractions contenant le produit attendu, sont réunies, concentrées sous pression réduite (2 kPa ; 50°C) puis séchées (90 Pa ; 45°C) pour donner 870 mg de N-(6-chloro-1H-indazol-3-yl)-2-(2-diéthylamino-éthylamino)-acétamide tris trifluoroacétate sous forme de solide blanc fondant à 160°C.

Spectre de R.M.N. ¹H (400 MHz, (CD₃)₂SO d6 avec ajout de quelques gouttes de CD₃COOD d4, à une température de 363 K, δ en ppm) ; 1,24 (t, J = 7,5 Hz : 6H) ; 3,23 (q, J = 7,5 Hz : 4H) ; 3,47 (mt : 4H) ; 4,16 (s : 2H) ; 7,12 (d large, J = 8,5 Hz : 1H) ; 7,58 (s large : 1H) ; 7,87 (d, J = 8,5 Hz : 1H).

### EXEMPLE 89

### N-[5,6-diphényl-1-[[2-(triméthylsilyl)éthoxy]méthyl]-1H-indazol-3-yl]-butanamide:

A 1 g de N-[5,6-dibromo-1-[[2-(triméthylsilyl)éthoxy]méthyl]-1H-indazol-3-yl]-butanamide décrit à l'exemple 70, dans 150 cm³ de dioxane on ajoute 1.12 g d'acide phénylboronique, 1.55 g de carbonate de sodium dans 40 cm³ d'eau, 463 mg de tetrakis-triphénylphosphine palladium et on chauffe au reflux pendant 18 heures. On dilue le milieu réactionnel avec 100 cm³ d'acétate d'éthyle et avec 100 cm³ d'eau puis on filtre le milieu réactionnel sur verre fritté garni de célite. La phase organique est décantée et lavée avec 75 cm³ d'une solution saturée en chlorure de sodium, décantée, séchée sur sulfate de magnésium, filtrée et concentrée sous pression réduite (2 kPa ; 50°C) pour donner 2.6 g de brut qui est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle (80/20 en volumes). Les fractions contenant le produit attendu, sont réunies et évaporées sous pression réduite (2 kPa; 50°C) puis séchées (90 Pa; 45°C) pour donner 1.4 g de N-[5,6-diphényl-1-[[2-(triméthylsilyl)éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,06 (s : 9H) ; 0,86 (t, J = 8 Hz : 2H) ; 0,96 (t, J = 7,5 Hz : 3H) ; 1,66 (int : 2H) ; 2,42 (t large, J = 7 Hz : 2H) ; 3,59 (t, J = 8 Hz : 2H) ; 5,74 (s : 2H) ; de 7,05 à 7,35 (mt : 10H) ; 7,71 (s : 1H) ; 7,89 (s : 1H) ; 10,57 (mf : 1H).

EI m/z = 485 M^{+.}

m/z = 368 [M - OCH₂CH₂Si(CH₃)₃]⁺

m/z = 357 [M - C₆H₁₂OSi]^{+.}

### N-[5,6-diphényl-1H-indazol-3-yl]-butanamide:

A 1.5 g de N-[5,6-diphényl-1-[[2-(triméthylsilyl)éthoxy]méthyl]-1H-indazol-3-yl]-butanamide précédemment décrit dans 40 cm³ de tétrahydrofurane on ajoute 17.2 cm³ de fluorure de tétrabutylammonium en solution 1M dans le tétrahyrofurane et on porte à reflux pendant 18 heures ; après refroidissement on ajoute 75 cm³ d'acétate d'éthyle et la phase organique est lavée avec 75 cm³ d'une solution saturée en chlorure de sodium. La phase organique est décantée, séchée sur sulfate de magnésium, filtrée et concentrée à sec sous pression réduite (2 kPa ; 50°C) pour donner 1.5 g de brut qui est purifié par chromatographie sous pression d'argon de 50 kPa, sur colonne de gel de silice ( granulométrie 40-60 µm ; diamètre 3,5 cm), en éluant par un mélange cyclohexane-acétate d'éthyle ( 60/40 en volumes). Les fractions contenant le produit attendu sont réunies et évaporées sous pression réduite ( 2 kPa ; 50°C) ; le produit encore impur est purifié par HPLC (colonne Hypersil ; C18, 5µm; longueur 250 mm, diamètre 21 mm, éluant : méthanol-eau (70/30 en volumes) contenant 0.1% d'acide trifluoroacétique ; débit 10 cm³/mn) ; par concentration à sec des fractions contenant l'attendu, le résidu est repris avec 10 cm³ d'éther diisopropylique, filtré, lavé par 2 fois 5 cm³ d'éther diisopropylique et séché (90 Pa ; 45°C) pour donner 100 mg de N-[5,6-diphényl-1H-indazol-3-yl]-butanamide sous forme de cristaux blancs fondant à 210°C.

Spectre de R.M.N. ¹H (300 MHz, (CD₃)₂SO d6, δ en ppm) : 0,95 (t, J = 7,5 Hz : 3H) ; 1,65 (mt : 2H) ; 2,40 (t, J = 7 Hz : 2H) ; de 7,00 à 7,30 (mt :10H) ; 7,40 (s : 1H) ; 7,82 (s : 1H) ; 10,43 (s large : 1H) ; 12,75 (mf : 1H).

### EXEMPLE 90

### N-[6-chloro-5-(4-méthylphényl)-1-[[2-(triméthylsilyl)éthoxy]méthyl]-1H-indazol-3-yl]-butanamide

On opère comme à l'exemple 62 ; à 1 g de N-[5-bromo-6-chloro-1-[[2-(triméthylsilyl)éthoxy])méthyl]-1H-indazol-3-yl]-butanamide décrit à l'exemple 58, dans 50 cm³ de dioxane, on ajoute 456 mg d'acide 4-méthylphénylboronique, 560 mg de carbonate de sodium, 20 cm³ d'eau distillée et 155 mg de tétrakis-triphénylphosphine palladium. On chauffe au reflux pendant 90 minutes puis on laisse revenir à 20 °C puis le milieu réactionnel est filtré sur verre fritté garni de célite et au filtrat on ajoute 60 cm³ d'acétate d'éthyle. La phase organique décantée est lavée avec 30 cm³ d'une solution aqueuse saturée en chlorure de sodium puis séchée sur sulfate de magnésium. Après filtration, le filtrat est concentré à sec sous pression réduite (2 kPa ; 50°C) et le résidu est purifié par chromatographie sous pression d'argon de 50 kPa, sur colonne de gel de silice ( granulométrie 40-60 µm ; diamètre 2.5 cm), en éluant par un mélange cyclohexane-acétate d'éthyle ( 80/20 en volumes). Les fractions contenant le produit attendu sont réunies et évaporées sous pression réduite (2 kPa ; 50°C). On obtient après séchage (90 Pa ; 45°C), 880 mg de N-[6-chloro-5-(4-méthylphényl)-1-[[2-(triméthylsilyl)éthoxy]méthyl]-1H-indazol-3-yl]-butanamide sous forme d'une poudre blanc cassé.

Spectre de R.M.N. ¹H (300 MHz, (CD₃)₂SO d6, δ en ppm) : - 0,05 (s : 9H) ; 0,85 (t, J = 8 Hz : 2H) ; 0,93 (t, J = 7,5 Hz : 3H) ; 1,64 (mt : 2H) ; 2,39 (s : 3H) ; 2,39 (t, J = 7 Hz : 2H) ; 3,56 (t, J = 8 Hz : 2H) ; 5,70 (s : 2H) ; 7,30 (mt : 4H) ; 7,87 (s : 1H) ; 7,99 (s : 1H) ; 10,59 (mf : 1H)

EI m/z = 457 M^{+.}

m/z = 340 [M - OCH₂CH₂Si(CH₃)₃]⁺

m/z = 329 [M - C₆H₁₂OSi]^{+.}

### N-[6-chloro-5-(4-méthylphényl)-1H-indazol-3-yl]-butanamide

A 870 mg de N-[6-chloro-5-(4-méthylphényl)-1-[[2-(triméthylsilyl)éthoxy]méthyl]-1H-indazol-3-yl]-butanamide précédemment décrit, dans 20 cm³ de tétrahydrofurane, on ajoute 11.4 cm³ de fluorure de tétrabutylammonium en solution 1M dans le tétrahydrofurane. Le milieu est ensuite chauffé au reflux pendant 20 heures puis on laisse revenir à température ambiante et on ajoute 20 cm³ d'acétate d'éthyle ; la phase organique est lavée avec 2 fois 20 cm³ d'une solution aqueuse saturée en hydrogénocarbonate de sodium puis avec 2 fois 20 cm³ d'une solution aqueuse saturée en chlorure de sodium. La phase organique est décantée, séchée sur sulfate de magnésium, filtrée et évaporée à sec sous pression réduite ( 2 kPa ; 45°C). Le résidu est repris dans 10 cm³ d'éther diéthylique, filtré sur verre fritté puis séché sous pression réduite ( 90 Pa; 50 °C) pour donner 195 mg de N-[6-chloro-5-(4-méthylphenyl)- 1H-indazol-3-yl]-butanamide sous forme d'une poudre beige.

Spectre de R.M.N. ¹H (400 MHz, (CD₃)₂SO d6, δ en ppm) : 0,93 (t, J = 7,5 Hz : 3H) ; 1,63 (mt : 2H) ; de 2,30 à 2,50 (mt : 2H) ; 2,38 (s : 3H) ; 7,28 (mt : 4H) ; 7,64 (s : 1H) ; 7,81 (s : 1H) ; 10,45 (mf: 1H).

EI m/z=327 M^{+.}

m/z = 257 [M - C₄CH₆O]^{+.}

### EXEMPLE 91

### N-[6-[4-(phénylméthoxy)phényl-1-5-phényl-1-[[2-(triméthylsilyl)éthoxy]méthyl]-1H-indazol-3-yl]-butanamide

A 1 g de N-[5-bromo-6-[4-(phénylméthoxy)phényl]-1-[[2-(triméthylsilyl)éthoxy]méthyl]-1H-indazol-3-yl]-butanamide préparé dans l'exemple 63, dans 100 cm³ de dioxane on ajoute 306 mg d'acide phénylboronique, 427 mg de carbonate de sodium dans 30 cm³ d'eau, 248 mg de tétrakis-triphénylphosphine palladium et on chauffe au reflux pendant 18 heures. Au milieu réactionnel on ajoute 100 cm³ d'acétate d'éthyle, 100 cm³ d'eau et on filtre le milieu réactionnel sur verre fritté garni de célite. La phase organique est décantée, lavée successivement par 100 cm³ d'eau et par 100 cm³ d'une solution saturée en chlorure de sodium, séchée sur sulfate de magnésium, filtrée et concentrée sous pression réduite (2 kPa-50°C) pour donner 2.5 g d'une huile qui est purifiée par chromatographie sous pression d'argon de 50 kPa, sur colonne de gel de silice ( granulométrie 40-60 µm ; diamètre 3,5 cm), en éluant par un mélange cyclohexane-acétate d'éthyle ( 80/20 en volumes). Les fractions contenant le produit attendu, sont réunies et évaporées sous pression réduite (2 kPa ; 50°C) pour donner 1 g de N-[6-[4-(phénylméthoxy)phényl]-5-phényl-1-[[2-(triméthylsilyl]éthoxy]méthyl]-1H-indazol-3-yl]-butanamide sous forme d'une huile jaune.

Spectre de R.M.N. ¹H (300 MHz, (CD₃)₂SO d6, δ en ppm) : - 0,06 (s : 9H) ; 0,84 (t, J = 8 Hz : 2H) ; 0,94 (t, J = 7,5 Hz : 3H) ; 1,65 (mt : 2H) ; 2,40 (t large, J = 7 Hz : 2H) ; 3,57 (t, J = 8 H : 2H) ; 5,09 (s : 2H) ; 5,71 (s : 2H) ; 6,92 (d, J = 8,5 Hz : 2H) ; de 7,00 à 7,55 (mt : 10H) ; 7,08 (d, J = 8,5 Hz : 2H) ; 7,66 (s : 1H) ; 7,85 (s : 1H) ; 10,54 (mf : 1H).

EI m/z = 591 M^{+.}

m/z = 474 [M - OCH₂CH₂Si(CH₃)₃]⁺

### N-[5-phényl-6-[4-(phénylméthoxy)phényl]-1H-indazol-3-yl]-butanamide:

A 1 g de N-[6-[4-(phénylméthoxy)phényl]-5-phényl-1-[[2-(triméthylsilyl)éthoxy]méthyl]-1H-indazol-3-yl]-butanamide précédemment décrit, dans 40 cm³ de tétrahydrofurane on ajoute 10.1 cm³ de fluorure de tétrabutylammonium en solution 1M dans le tétrahydrofurane et on porte à reflux pendant 18 heures ; après refroidissement on ajoute 75 cm³ d'acétate d'éthyle et la phase organique est lavée successivement avec 100 cm³ d'une solution saturée en hydrogénocarbonate de sodium et avec 75 cm³ d'une solution saturée en chlorure de sodium. La phase organique est décantée, séchée sur sulfate de magnésium, filtrée et concentrée à sec sous pression réduite (2 kPa ; 50°C) pour donner 2 g de brut qui est purifié par chromatographie sous pression d'argon de 50 kPa, sur colonne de gel de silice ( granulométrie 40-60 µm; diamètre 3,5 cm), en éluant par un mélange cyclohexane-acétate d'éthyle ( 80/20 en volumes). Les fractions contenant le produit attendu sont réunies et évaporées sous pression réduite ( 2 kPa ; 50°C) ; le résidu est séché (90 Pa; 45°C) pour donner 650 mg de N-[5-phényl-6-[4-(phénylméthoxy)phényl]-1H-indazol-3-yl]-butanamide sous forme d'huile jaune.

Spectre de R.M.N. ¹H (300 MHz, (CD₃)₂SO d6, δ en ppm) : 0,96 (t, J = 7,5 Hz : 3H) ; 1,66 (mt : 2H) ; 2,40 (t, J = 7 Hz : 2H) ; 5,08 (s : 2H) ; 6,91 (d, J = 8,5 Hz : 2H) ; de 7,05 à 7,55 (mt : 10H) ; 7,09 (d, J = 8,5 Hz : 2H) ; 7,36 (s : 1H) ; 7,80 (s : 1H) ; 10,42 (s large : 1H) ; 12,70 (s large : 1H).

EI m/z = 461 M^{+.}

m/z = 391 [M - C₄CH₆O]^{+.}

m/z = 300 [391- C₆H₅CH₂]⁺

### N-[5-phényl-6-(4-hydroxyphényl)-1H-indazol-3-yl]-butanamide

A 650 mg de N-[5-phényl-6-[4-(phénylméthoxy)phenyl])-1H-indazol-3-yl]-butanamide préparé précédemment on ajoute 10 cm³ d'iodure de triméthylsilyle et on chauffe au reflux 3 heures. On ajoute au milieu 3 cm³ de méthanol et on réchauffe au reflux pendant 15 minutes puis on concentre le milieu réactionnel à sec sous pression réduite (2 kPa ; 50°C). Le résidu est repris par 75 cm³ d'acétate d'éthyle et on lave avec 2 fois 50 cm³ d'une solution de thiosulfate de sodium à 10% puis avec 50 cm³ d'eau et avec 50 cm³ d'une solution saturée en chlorure de sodium. La phase organique est décantée, séchée sur sulfate de magnésium, filtrée et concentrée à sec sous pression réduite pour donner 0.6 g de brut qui est purifié par chromatographie sous pression d'argon de 50 kPa, sur colonne de gel de silice ( granulométrie 40-60 µm; diamètre 3 cm), en éluant par un mélange cyclohexane-acétate d'éthyle ( 70/30 en volumes). Les fractions contenant le produit attendu sont réunies et évaporées sous pression réduite ( 2 kPa ; 50°C) ; le résidu est repris avec 10 cm³ d'éther diisopropylique, filtré, lavé par 5 cm³ d'acétate d'éthyle et par 5 cm³ d'éther diéthylique puis séché (90 Pa ; 45°C) pour donner 200 mg de N-[5-phényl-6-(4-hydroxyphényl)-1H-indazol-3-yl]-butanamide fondant à 220°C.

Spectre de R.M.N. ¹H (300 MHz, (CD₃)₂SO d6, δ en ppm) : 0,95 (t, J = 7,5 Hz : 3H) ; 1,65 (mt : 2H) ; 2,38 (t, J = 7 Hz : 2H) ; 6,63 (d, J = 8 Hz : 2H) ; 6,94 (d, J = 8 Hz : 2H) ; 7,09 (d large, J = 7,5 Hz : 2H) ; de 7,15 à 7,30 (mt : 3H) ; 7,33 (s : 1H) ; 7,77 (s : 1H) ; 9,40 (mf : 1H) ; 10,40 (s large : 1H) ; 12,67 (mf : 1H).

### EXEMPLE 92

### N-[6-chloro-5-(4,4,5,5-tétraméthyl-[1,3,2]dioxaborolan-2-yl)-1-[2-(triméthylsilyl)éthoxy]méthyl]-1H-indazol-3-yl]-butanamide:

A 7.5 g de N-[5-bromo-6-chloro-1-[[2-(triméthylsilyl)éthoxy]méthyl]-1H-indazol-3-yl]-butanamide décrit à l'exemple 58, dans 225 cm³ de dioxane, on additionne 5.11 g de bis(pinacolato)diborane, 277 mg de bis(dibenzylidèneacétone) palladium puis 2.48 g d'acétate de potassium et enfin 330 mg de tricyclohexylphosphine. Le milieu est porté au reflux pendant 18 heures puis on laisse revenir à température ambiante et on ajoute 100 cm³ d'acétate d'éthyle et 100 cm³ d'eau. La phase organique est décantée, lavée par 100 cm³ d'eau, par 100 cm³ d'une solution saturée en chlorure de sodium, séchée sur sulfate de magnésium, filtrée puis concentrée à sec sous pression réduite ( 2 kPa ; 45 °C) pour donner 11.2 g de brut qui est purifié par chromatographie sous pression d'argon de 50 kPa, sur colonne de gel de silice ( granulométrie 40-60 µm ; diamètre 3 cm), en éluant par un mélange cyclohexane-acétate d'éthyle (80/20 en volumes). Les fractions contenant le produit attendu, sont réunies et évaporées sous pression réduite (2 kPa; 50°C) ; pour donner 6.16g de N-[6-chloro-5-(4,4,5,5-tétraméthyl-[1,3,2]dioxaborolan-2-yl)-1-[[2-(triméthylsilyl)éthoxy]méthyl]-1H-indazol-3-yl]-butanamide sous forme d'une huile orange. Le produit est utilisé directement.

### N-[6-chloro-5-(4-nitrophényl)-1-[[2-(triméthylsilyl)éthoxy]méthyl]-1H-indazol-3-yl]-butanamide

A 2 g de N-[6-chloro-5-(4,4,5,5-tétraméthyl-[1,3,2]dioxaborolan-2-yl)- 1-[[2-(triméthylsilyl)éthoxy]méthyl]-1H-indazol-3-yl]-butanamide précédemment décrit, dans 100 cm³ de dioxane, on ajoute 246 mg de 4-bromonitrobenzene, 1.2 g de carbonate de sodium dans 20 cm³ d'eau et 365 mg de tétrakis-triphénylphosphine palladium. Le milieu est ensuite porté au reflux pendant 20 heures puis on laisse revenir à température ambiante et on ajoute 100 cm³ d'acétate d'éthyle et 100 cm³ d'eau. Le milieu réactionnel est filtré sur verre fritté garni de célite, la phase organique décantée, lavée avec 100 cm³ d'eau, avec 100 cm³ d'une solution aqueuse saturée en chlorure de sodium, séchée sur sulfate de magnésium, filtrée et concentrée à sec sous pression réduite ( 2 kPa ; 45 °C). Le brut est purifié par chromatographie sous pression d'argon de 50 kPa, sur colonne de gel de silice ( granulométrie 40-60 µm ; diamètre 4 cm), en éluant par un mélange cyclohexane-acétate d'éthyle (85/15 en volumes). Les fractions contenant le produit attendu sont réunies et évaporées sous pression réduite (2 kPa ; 50°C). On obtient 690 mg de N-[6-chloro-5-(4-nitrophényl)-1-[[2-(triméthylsilyl)éthoxy]méthyl]-1H-indazol-3-yl]-butanamide sous forme de cristaux jaunes.

Spectre de R.M.N. ¹H (300 MHz, (CD₃)₂SO d6, δ en ppm) : - 0,05 (s : 9H) ; 0,85 (t, J = 8 Hz : 2H) ; 0,92 (t, J = 7,5 Hz : 3H) ; 1,62 (mt : 2H) ; 2,39 (t, J = 7 Hz : 2H) ; 3,56 (t, J = 8 Hz : 2H) ; 5,72 (s : 2H) ; 7,74 (d, J = 8,5 Hz : 2H) ; 8,01 (s : 1H) ; 8,09 (s : 1H) ; 8,35 (d, J = 8,5 Hz : 2H) ; 10,70 (mf : 1H).

EI m/z = 488 M^{+.}

m/z = 371 [M - OCH₂CH₂Si(CH₃)₃]⁺

m/z = 360 [M - C₆H₁₂OSi]^{+.}

m/z = 73 [Si(CH₃)₃]⁺

### N-[6-chloro-5-(4-pyridinyl)-1H-indazol-3-yl]-butanamide :

A 3 g de N-[6-chloro-5-(4-nitrophényl)-1-[[2-(triméthylsilyl)éthoxy]méthyl]-1H-indazol-3-yl]-butanamide précédemment décrit, dans 135 cm³ de tétrahydrofurane, on ajoute 36.8 cm³ de fluorure de tétrabutylammonium en solution 1M dans le tétrahydrofurane puis le milieu est porté au reflux pendant 18 heures et on laisse ensuite revenir à température ambiante pour ajouter 100 cm³ d'acétate d'éthyle et 75 cm³ d'une solution aqueuse saturée en hydrogénocarbonate de sodium. La phase organique est décantée, lavée avec 100 cm³ d'une solution aqueuse saturée en hydrogénocarbonate de sodium puis avec 100 cm³ d'une solution saturée en chlorure de sodium, séchée sur sulfate de magnésium, filtrée et concentrée à sec sous pression réduite ( 2 kPa; 45°C). Le résidu est purifié par chromatographie sous pression d'argon de 50 kPa, sur colonne de gel de silice (granulométrie 40-60 µm ; diamètre 2 cm), en éluant par un mélange cyclohexane-acétate d'éthyle (80/20 en volumes). Les fractions contenant le produit attendu, sont réunies et concentrées à sec sous pression réduite (2 kPa ; 50°C) ; le résidu est repris avec 35 cm³ d'éther diisopropylique, filtré sur verre fritté puis lavé successivement avec 2 fois 20 cm³ d'éther diisopropylique. Après séchage ( 90 Pa ; 50 °C), on obtient 88 mg de N-[6-chloro-5-(4-nitrophényl)-1H-indazol-3-yl]-butanamide sous forme de cristaux jaunes fondant à 260°C.

Spectre de R.M.N. ¹H (300 MHz, (CD₃)₂SO d6, δ en ppm) : 0,92 (t, J = 7,5 Hz : 3H); 1,62 (mt : 2H) ; 2,37 (t, J = 7 Hz : 2H) ; 7,73 (d, J = 8 Hz : 2H) ; 7,72 (s : 1H) ; 7,96 (s : 1H) ; 8,34 (d, J = 8 Hz : 2H) ; 10,58(s large : 1H) ; de 12,50 à 13,20 (mf très étalé : 1H).

### EXEMPLE 93

### N-[5-(4-aminophényl)-6-chloro-1H-indazol-3-yl]-butanamide :

A 0.93 g de N-[6-chloro-5-(4-nitrophényl)-1H-indazol-3-yl]-butanamide précédemment décrit, dans 50 cm³ d'acide acétique on ajoute 845 mg de zinc en poudre puis 1 heure après, à nouveau 845 mg de zinc ; le milieu réactionnel est filtré sur verre fritté garni de célite et le filtrat est concentré à sec sous pression réduite (2 kPa ; 50°C). Le résidu est repris avec 100 cm³ d'acétate d'éthyle et 75 cm³ d'eau et la phase organique est décantée, lavée successivement avec 75 cm³ d'eau et avec 50 cm³ d'une solution saturée en hydrogénocarbonate de sodium, séchée sur sulfate de magnésium, filtrée et concentrée à sec pour donner 480 mg de brut qui est purifié par chromatographie sous pression d'argon de 50 kPa, sur colonne de gel de silice ( granulométrie 40-60 µm ; diamètre 4.5 cm), en éluant par un mélange cyclohexane-acétate d'éthyle (60/40 en volumes). Les fractions contenant le produit attendu sont réunies et le solide trituré dans 10 cm³ d'éther diéthylique, filtré, lavé avec 2 fois 5 cm³ d'éther diéthylique puis séché (90 Pa; 45°C) pour donner 110 mg de N-[5-(4-aminophényl)-6-chloro-1H-indazol-3-yl]-butanamide sous forme de solide ocre.

Spectre de R.M.N. ¹H (300 MHz, (CD₃)₂SO d6, δ en ppm) : 0,92 (t, J = 7,5 Hz : 3H) ; 1,63 (mt : 2H) ; 2,36 (t, J = 7 Hz : 2H) ; 5,21 (mf : 2H) ; 6,63 (d, J = 8 Hz : 2H) ; 7,06 (d, J = 8 Hz : 2H) ; 7,58 (s : 1H) ; 7,72 (s : 1H) ; 10,42 (s large : 1H) ; 12,71 (mf : 1H).

EI m/z = 328 M^{+.}

m/z = 284 [M - C₃H₆]^{+.}

m/z = 258 [M - C₄CH₆O]^{+.}

### EXEMPLE 94

### N-[6-chloro-5-(4-éthylphényl)-1-[[2-(triméthylsilyl)éthoxy]méthyl]-1H-indazol-3-yl]-butanamide

A 1 g de N-[5-bromo-6-chloro-1-[[2-(triméthylsilyl)éthoxy]méthyl]-1H-indazol-3-yl]-butanamide décrit à l'exemple 58, dans 75 cm³ de dioxane, on ajoute 504 mg d'acide 4-éthylphénylboronique, 664 mg de carbonate de sodium dans 20 cm³ d'eau distillée et 202 mg de tétrakis-triphénylphosphine palladium. On chauffe au reflux pendant 18 heures puis on laisse revenir la température à l'ambiante pour ajouter 75 cm³ d'acétate d'éthyle et 50 cm³ d'eau et le milieu réactionnel est filtré sur verre fritté garni de célite. Le filtrat est décanté et la phase organique est lavée successivement avec 50 cm³ d'eau, avec 50 cm³ d'une solution aqueuse saturée en chlorure de sodium, décantée, séchée sur sulfate de magnésium, filtrée puis concentrée à sec sous pression réduite (2 kPa ; 50°C). Le résidu est purifié par chromatographie sous pression d'argon de 50 kPa, sur colonne de gel de silice (granulométrie 40-60 µm ; diamètre 4,5 cm), en éluant par un mélange cyclohexane-acétate d'éthyle (80/20 en volumes). Les fractions contenant le produit attendu, sont réunies et évaporées sous pression réduite (2 kPa ; 50°C) puis séchées (90 Pa ; 50 °C) pour donner 1.1 g de N-[6-chloro-5-(4-éthylphényl)-1-[[2-(triméthylsilyl)éthoxy]méthyl]-1H-indazol-3-yl]-butanamide (sous forme d'une huile jaune.

Spectre de R.M.N. ¹H (300 MHz, (CD₃)₂SO d6, δ en ppm) : - 0,05 (s : 9H) ; 0,85 (t, J = 8 Hz : 2H) ; 0,93 (t, J = 7,5 Hz : 3H) ; 1,26 (t, J = 7,5 Hz : 3H) ; 1,64 (mt : 2H) ; 2,39 (t, J = 7 Hz : 2H) ; 2,69 (q, J = 7,5 Hz : 2H) ; 3,55 (t, J = 8 Hz : 2H) ; 5,70 (s : 2H) ; 7,33 (mt : 4H) ; 7,89 (s : 1H) ; 8,00 (s : 1H) ; 10,64 (mf : 1H).

EI m/z = 471 M^{+.}

m/z = 354 [M - OCH₂CH₂Si(CH₃)₃]⁺

m/z = 343 [M - C₆H₁₂OSi]^{+.}

### N-[6-chloro-5-(4-éthylphényl)-1H-indazol-3-yl]-butanamide

A 1.1 g de N-[6-chloro-5-(4-éthylphényl)-1-[[2-(triméthylsilyl)éthoxy]méthyl]-1H-indazol-3-yl]-butanamide (lot P-31335-046-2) précédemment décrit, dans 50 cm³ de tétrahydrofurane, on ajoute 14 cm³ de fluorure de tétrabutylammonium en solution 1M dans le tétrahydrofurane. Le milieu est ensuite porté au reflux pendant 18 heures puis on laisse revenir à température ambiante et on ajoute 75 cm³ d'acétate d'éthyle; la phase organique est lavée avec 2 fois 100 cm³ d'une solution aqueuse saturée en hydrogénocarbonate de sodium puis avec 75 cm³ d'une solution aqueuse saturée en chlorure de sodium. La phase organique est décantée, séchée sur sulfate de magnésium, filtrée et évaporée à sec sous pression réduite (2 kPa ; 45°C). Le résidu est purifié par chromatographie sous pression d'argon de 50 kPa, sur colonne de gel de silice (granulométrie 40-60 µm ; diamètre 3 cm), en éluant par un mélange cyclohexane-acétate d'éthyle (70/30 en volumes). Les fractions contenant le produit attendu, sont réunies et évaporées sous pression réduite (2 kPa ; 50°C) ; le résidu est repris avec 20 cm³ d'éther diisopropylique, filtré sur verre fritté et lavé 2 fois avec 10 cm³ d'éther diisopropylique. Après séchage sous pression réduite (90 Pa ; 50 °C), on' obtient 440 mg de N-(6-chloro-5-phényl-1H-indazol-3-yl)-butanamide sous forme de cristaux blancs fondant à 240 °C.

Spectre de R.M.N. ¹H (300 MHz, (CD₃)₂SO d6, δ en ppm) : 0,92 (t, J = 7,5 Hz : 3H) ; 1,24 (t, J = 7,5 Hz : 3H) ; 1,62 (mt : 2H) ; 2,37 (t, J = 7 Hz : 2H) ; 2,67 (q, J = 7,5 Hz : 2H) ; 7,31 (mt : 4H) ; 7,64 (s : 1H) ; 7,82 (s : 1H) ; 10,48 (mf : 1H) ; 12,80 (mf : 1H).

### EXEMPLE 95

### N-[6-chloro-5-[4-(phénylméthoxy)phényl]-1-[[2-(triméthylsilyl)éthoxy]méthyl]-1H-indazol-3-yl]-butanamide

A 2 g de N-[5-bromo-6-chloro-1-[[2-(triméthylsilyl)éthoxy]méthyl]-1H-indazol-3-yl]-butanamide décrit dans l'exemple 58, dans 100 cm³ de dioxane, on ajoute 1.54 g d'acide 4-benzyloxyphénylboronique, 1.32 g de carbonate de sodium dans 20 cm³ d'eau et 404 mg de tétrakis-triphénylphosphine palladium. On chauffe au reflux pendant 18 heures puis on laisse revenir la température à l'ambiante pour ajouter 75 cm³ d'acétate d'éthyle et 50 cm³ d'eau et le milieu réactionnel est filtré sur verre fritté garni de célite. Le filtrat est décanté et la phase organique est lavée successivement avec 100 cm³ d'eau puis avec 75 cm³ d'une solution aqueuse saturée en chlorure de sodium, décantée, séchée sur sulfate de magnésium, filtrée et concentrée à sec sous pression réduite (2 kPa; 50°C). Le résidu est purifié par chromatographie sous pression d'argon de 50 kPa, sur colonne de gel de silice (granulométrie 40-60 µm ; diamètre 4,5 cm), en éluant par un mélange cyclohexane-acétate d'éthyle (80/20 en volumes). Les fractions contenant le produit attendu, sont réunies et évaporées sous pression réduite (2 kPa ; 50°C) ; le résidu est repris par 50 cm³ de cyclohexane, filtré, lavé par 2 fois 25 cm³ de cyclohexane et séché sous pression réduite (90 Pa ; 50 °C) pour donner 2.35 g de N-[6-chloro-5-[4-(phénylméthoxy)phényl]-1-[[2-(triméthylsilyl)éthoxy]méthyl]-1H-indazol-3-yl]-butanamide sous forme de cristaux blancs fondant à 130°C.

Spectre de R.M.N. ¹H (300 MHz, (CD₃)₂SO d6, δ en ppm) : - 0,06 (s : 9H) ; 0,84 (t, J = 8 Hz : 2H) ; 0,92 (t, J = 7,5 Hz : 3H) ; 1,63 (mt : 2H) ; 2,38 (t, J = 7 Hz : 2H) ; 3,54 (t, J = 8 Hz : 2H) ; 5,18 (s : 2H) ; 5,69 (s : 2H) ; 7,11 (d, J = 8,5 Hz : 2H) ; de 7,30 à 7,55 (mt : 5H) ; 7,35 (d, J = 8,5 Hz : 2H) ; 7,86 (s : 1H) ; 7,98 (s : 1H) ; 10,61 (mf : 1H).

### N-[6-chloro-5-[4-(phénylméthoxy)phényl]-1H-indazol-3-yl]-butanamide

A 1.1 g de N-[6-chloro-5-[4-(phénylméthoxy)phényl]-1-[[2-(triméthylsilyl)éthoxy]méthyl]-1H-indazol-3-yl]-butanamide précédemment décrit, dans 50 cm³ de tétrahydrofurane, on ajoute 25.1 cm³ de fluorure de tétrabutylammonium en solution 1M dans le tétrahydrofurane. Le milieu est ensuite porté au reflux pendant 18 heures puis on laisse revenir à température ambiante et on ajoute 100 cm³ d'acétate d'éthyle; la phase organique est lavée avec 2 fois 100 cm³ d'une solution aqueuse saturée en hydrogénocarbonate de sodium puis avec 100 cm³ d'une solution aqueuse saturée en chlorure de sodium. La phase organique est décantée, séchée sur sulfate de magnésium, filtrée et évaporée à sec sous pression réduite (2 kPa ; 45°C). Le résidu, est purifié par chromatographie sous pression d'argon de 50 kPa, sur colonne de gel de silice (granulométrie 40-60 µm ; diamètre 4 cm), en éluant par un mélange cyclohexane-acétate d'éthyle (70/30 en volumes). Les fractions contenant le produit attendu, sont réunies et évaporées sous pression réduite (2 kPa ; 50°C) ; le résidu est repris avec 30 cm³ d'éther diisopropylique, filtré sur verre fritté et lavé 2 fois avec 20 cm³ d'éther diisopropylique. Après séchage sous pression réduite (90 Pa; 50 °C), on obtient 1.3 g de N-[6-chloro-5-[4-(phénylméthoxy)phényl]-1H-indazol-3-yl]-butanamide sous forme de cristaux blancs fondant à 230 °C.

Spectre de R.M.N. ¹H (300 MHz, (CD₃)₂SO d6, δ en ppm) : 0,93 (t, J = 7,5 Hz : 3H) ; 1,63 (mt : 2H) ; 2,37 (t, J = 7 Hz : 2H) ; 5,18 (s : 2H) ; 7,11 (d, J = 8,5 Hz : 2H) ; 7,35 (d, J = 8,5 Hz : 2H) ; 7,37 (mt : 1H) ; 7,44 (t large, J = 7,5 Hz : 2H) ; 7,51 (d large, J = 7,5 Hz : 2H) ; 7,64 (s : 1H) ; 7,81 (s : 1H) ; 10,48 (s large : 1H) ; 12,80 (mf: 1H).

### EXEMPLE 96

### N-[6-chloro-5-(4-hydroxyphényl)-1H-indazol-3-yl]-butanamide

A 1.1 g de N-[6-chloro-5-[4-(phénylméthoxy)phényl]-1H-indazol-3-yl]-butanamide préparé à l'exemple 95, on ajoute 20 cm³ d'iodure de triméthylsilyle et on chauffe au reflux 18 heures. On ajoute au milieu 30 cm³ de méthanol et on rechauffe au reflux pendant 30 minutes puis on concentre le milieu réactionnel à sec sous pression réduite (2 kPa ; 50°C ). Le résidu est repris par 100 cm³ d'acétate d'éthyle et par 100 cm³ d'eau et la phase organique est lavée avec 2 fois 75 cm³ d'une solution de thiosulfate de sodium à 10% puis avec 70 cm³ d'eau et 75 cm³ d'une solution saturée en chlorure de sodium. La phase organique est décantée, séchée sur sulfate de magnésium, filtrée puis concentrée à sec sous pression réduite pour donner 1.44 g de brut qui est purifié par chromatographie sous pression d'argon de 50 kPa, sur colonne de gel de silice ( granulométrie 40-60 µm ; diamètre 3.5 cm), en éluant par un mélange cyclohexane-acétate d'éthyle (70/30 en volumes). Les fractions contenant le produit attendu sont réunies et évaporées sous pression réduite (2 kPa ; 50°C) ; le résidu est repris avec 20 cm³, d'éther diisopropylique, filtré, lavé par 3 fois 10 cm³ d'éther diisopropylique puis séché (90 Pa ; 45°C) pour donner 210 mg de N-[6-chloro-5-(4-hydroxyphényl)-1H-indazol-3-yl]-butanamide sous forme de poudre blanche.

Spectre de R.M.N. ¹H (300 MHz, (CD₃)₂SO d6, δ en ppm) : 0,92 (t, J = 7,5 Hz : 3H) ; 1,62 (mt : 2H) ; 2,37 (t, J = 7 Hz : 2H) ; 6,84 (d, J = 8,5 Hz : 2H) ; 7,21 (d, J = 8,5 Hz : 2H) ; 7,61 (s : 1H) ; 7,77 (s : 1H) ; 9,57 (mf: 1H) ; 10,45 (s large : 1H) ; 12,76 (mf : 1H).

EI m/z = 329 M^{+.}

m/z = 259 [M - C₄CH₆O]^{+.}

### EXEMPLE 97

### N-[5,6-bis 4-[(phénylméthoxy)phényl]-1-[[2-(triméthylsilyl)éthoxy]méthyl]-1H-indazol-3-yl]-butanamide

A 1.35 g de N-[5,6-dibromo-1-[[2-(triméthylsilyl)éthoxy]méthyl]-1H-indazol-3-yl]-butanamide décrit à l'exemple 70, dans 100 cm³ de dioxane, on ajoute 1.9 g d'acide 4-benzyloxyphénylboronique, 1.63 g de carbonate de sodium dans 20 cm³ d'eau distillée et 500 mg de tétrakis-triphénylphosphine palladium. On chauffe au reflux pendant 18 heures puis on laisse revenir la température à l'ambiante pour ajouter 100 cm³ d'acétate d'éthyle et 100 cm³ d'eau et le milieu réactionnel est filtré sur verre fritté garni de célite. La phase organique est décantée, lavée avec 100 cm³ d'une solution aqueuse saturée en chlorure de sodium, décantée, séchée sur sulfate de magnésium, filtrée et concentrée à sec sous pression réduite (2 kPa ; 50°C). Le résidu est purifié par chromatographie sous pression d'argon de 50 kPa, sur colonne de gel de silice (granulométrie 40-60 µm ; diamètre 4,5 cm), en éluant par un mélange cyclohexane-acétate d'éthyle (70/30 en volumes). Les fractions contenant le produit attendu, sont réunies, évaporées sous pression réduite (2 kPa ; 50°C) et séchées (90 Pa; 50 °C) pour donner 1.96 g de N-[5,6-bis[4-(phénylméthoxy)phényl]-1-[[2-(triméthylsilyl)éthoxy]méthyl]-1H-indazol-3-yl]-butanamide sous forme d'huile orangée à 70% de pureté et utilisée telle quelle pour l'essai suivant.

Spectre de R.M.N. ¹H (300 MHz, (CD₃)₂SO d6, δ en ppm) : - 0,06 (s : 9H) ; 0,85 (t, J = 8 Hz : 2H) ; 0,96 (t, J = 7,5 Hz : 3H) ; 1,66 (mt : 2H) ; 2,41 (t large, J = 7 Hz : 2H) ; 3,57 (t, J = 8 Hz : 2H) ; 5,10 (mt : 4H) ; 5,71 (s large : 2H) ; de 6,90 à 7,00 (mt : 4H) ; 7,04 (d, J = 8,5 Hz : 2H) ; 7,11 (d, J = 8,5 Hz : 2H) ; de 7,30 à 7,55 (mt : 10H) ; 7,64 (s : 1H) ; 7,81 (s : 1H) ; 10,55 (mf : 1H).

EI m/z = 697 M^{+.}

m/z = 580 [M - OCH₂CH₂Si(CH₃)₃]⁺

m/z = 91 [C₆H₅CH₂]⁺

### N-[5,6-bis[4-(phénylméthoxy)phényl]-1H-indazol-3-yl]-butanamide

A 1.9 g de N-[5,6-bis[4-(phénylméthoxy)phényl]-1-[[2-(triméthylsilyl)éthoxy]méthyl]-1H-indazol-3-yl]-butanamide précédemment décrit, dans 100 cm³ de tétrahydrofurane, on ajoute 16.3 cm³ de fluorure de tétrabutylammonium en solution 1M dans le tétrahydrofurane. Le milieu est ensuite porté au reflux pendant 18 heures puis on laisse ensuite revenir à température ambiante et on ajoute 100 cm³ d'acétate d'éthyle; la phase organique est lavée avec 2 fois 100 cm³ d'une solution aqueuse saturée en hydrogénocarbonate de sodium puis avec 100 cm³ d'une solution aqueuse saturée en chlorure de sodium. La phase organique est décantée, séchée sur sulfate de magnésium, filtrée et évaporée à sec sous pression réduite (2 kPa ; 50°C). Le résidu est purifié par chromatographie sous pression d'argon de 50 kPa, sur colonne de gel de silice (granulométrie 40-60 µm ; diamètre 4.5 cm), en éluant par un mélange cyclohexane-acétate d'éthyle (70/30 en volumes). Les fractions contenant le produit attendu, sont réunies et évaporées sous pression réduite (2 kPa; 50°C); le résidu est repris avec 25 cm³ d'éther diisopropylique, filtré sur verre fritté et lavé 2 fois avec 20 cm³ d'éther diisopropylique. Après séchage sous pression réduite (90 Pa ; 50 °C), on obtient 700 mg de N-[5,6-bis 4-[(phénylméthoxy)phényl]-1H-indazol-3-yl]-butanamide sous forme de cristaux blancs fondant à 140 °C.

Spectre de R.M.N. ¹H (300 MHz, (CD₃)₂SO d6, δ en ppm) : 0,95 (t, J = 7,5 Hz : 3H) ; 1,65 (mt : 2H) ; 2,39 (t, J = 7 Hz : 2H) ; 5,08 (s large : 4H) ; de 6,85 à 7,00 (mt : 4H) ; 7,01 (d, J = 9 Hz : 2H) ; 7,09 (d, J = 9 Hz : 2H) ; de 7,30 à 7,55 (mt : 10H) ; 7,33 (s : 1H) ; 7,74 (s : 1H) ; 10,39 (s large : 1H) ; 12,67 (s large : 1H).

### EXEMPLE 98

### N-[5,6-bis (4-hydroxyphényl)-1H-indazol-3-yl]-butanamide

A 700 mg de N-[(5,6-bis[4-(phénylméthoxy)phenyl]-1H-indazol-3-yl]-butanamide préparé dans l'exemple 97, on ajoute 10 cm³ d'iodure de triméthylsilyle et on chauffe au reflux 18 heures. On ajoute au milieu 30 cm³ de méthanol et on rechauffe au reflux pendant 15 minutes puis on concentre le milieu réactionnel à sec sous pression réduite (2 kPa ; 50°C ). Le résidu est repris par 75 cm³ d'acétate d'éthyle et la phase organique est lavée 2 fois avec 75 cm³ d'une solution de thiosulfate de sodium à 10% puis avec 75 cm³ d'une solution saturée en chlorure de sodium. La phase organique est décantée, séchée sur sulfate de magnésium, filtrée puis concentrée à sec sous pression réduite (2 kPa; 50°C) pour donner 900 mg de brut qui est purifié par chromatographie sous pression d'argon de 50 kPa, sur colonne de gel de silice ( granulométrie 40-60 µm ; diamètre 3 cm), en éluant par un mélange cyclohexane-acétate d'éthyle (60/40 en volumes). Les fractions contenant le produit attendu sont réunies et évaporées sous pression réduite (2 kPa ; 50°C) et le résidu est repris avec 20 cm³ d'éther diisopropylique, filtré, lavé par 3 cm³ d'acétate d'éthyle puis par 2 fois 10 cm³ d'éther diisopropylique et séché (90 Pa; 45°C) pour donner 220 mg de N-[5,6-bis(4-hydroxyphényl)-1H-indazol-3-yl]-butanamide sous forme de poudre blanche fondant à 180°C.

Spectre de R.M.N. ¹H (300 MHz, (CD₃)₂SO d6, δ en ppm) : 0,95 (t, J = 7,5 Hz : 3H) ; 1,65 (mt : 2H) ; 2,38 (t, J = 7 Hz : 2H) ; de 6,55 à 6,70 (mt : 4H) ; 6,86 (d, J = 9 Hz : 2H) ; 6,94 (d, J = 9 Hz : 2H) ; 7,28 (s : 1H) ; 7,68 (s : 1H) ; 9,34 (mf : 2H) ; 10,36 (s large : 1H) ; 12,60 (mf : 1H).

### EXEMPLE 99

### N-[5-(3-furanyl)-6-[4-(phénylméthoxy)phényl]-1-[[2-(triméthylsilyl)éthoxy]méthyl]-1H-indazol-3-yl]-butanamide

A 1.25 g de N-[5-bromo-6-[4-(phénylméthoxy)phényl]-1-[[2-(triméthylsilyl)éthoxy]méthyl]-1H-indazol-3-yl]-butanamide préparé dans l'exemple 63, dans 125 cm³ de dioxane on ajoute 353 mg d'acide 3-furylboronique, 624 mg de carbonate de sodium dans 25 cm³ d'eau, 311 mg de tétrakis-triphénylphosphine palladium et on chauffe au reflux pendant 18 heures. Au milieu réactionnel on ajoute 100 cm³ d'acétate d'éthyle et 75 cm³ d'eau et on filtre le milieu réactionnel sur verre fritté garni de célite. La phase organique est décantée, lavée successivement par 75 cm³ d'eau et par 75 cm³ d'une solution saturée de chlorure de sodium, séchée sur sulfate de magnésium, filtrée et concentrée sous pression réduite (2 kPa ; 50°C) pour donner 2.6 g d'une huile qui est purifiée par chromatographie sous pression d'argon de 50 kPa, sur colonne de gel de silice (granulométrie 40-60 µm ; diamètre 4 cm), en éluant par un mélange cyclohexane-acétate d'éthyle (80/20 en volumes). Les fractions contenant le produit attendu, sont réunies et évaporées sous pression réduite (2 kPa ; 50°C) pour donner 1 g de N-[5-(3-furanyl)-6-[4-(phénylméthoxy)phényl]-1-[[2-(triméthylsilyl)éthoxy]méthyl]-1H-indazol-3-yl]-butanamide sous forme de cristaux couleur crème.

Spectre de R.M.N. ¹H (300 MHz, (CD₃)₂SO d6, δ en ppm) : - 0,07 (s : 9H) ; 0,83 (t, J = 8 Hz : 2H) ; 0,97 (t, J = 7,5 Hz : 3H) ; 1,68 (mt : 2H) ; 2,42 (t, J = 7 Hz : 2H) ; 3,55 (t, J = 8 Hz : 2H) ; 5,15 (s : 2H) ; 5,68 (s : 2H) ; 6,07 (mt : 1H) ; 7,04 (d, J = 8,5 Hz : 2H) ; 7,19 (d, J = 8,5 Hz : 2H) ; de 7,30 à 7,55 (mt : 6H) ; 7,55 (t, J = 2 Hz : 1H) ; 7,61 (s : 1H) ; 7,91 (s : 1H) ; 10,53 (mf : 1H).

EI m/z = 581 M^{+.}

m/z = 464 [M - OCH₂CH₂Si(CH₃)₃]⁺

### N-[5-(3-furanyl)-6-[4-(phénylméthoxy)phényl]-1H-indazol-3-yl]-butanamide

A 1 g de N-[5-(3-furanyl)-6-[4-(phénylméthoxy)phényl]-1-[[2-(triméthylsilyl)éthoxy]méthyl]-1H-indazol-3-yl]-butanamide précédemment décrit, dans 75 cm³ de tétrahydrofurane on ajoute 10.3 cm³ de fluorure de tétrabutylammonium en solution 1M dans le tétrahyrofurane et on porte à reflux pendant 18 heures ; après refroidissement on ajoute 75 cm³ d'acétate d'éthyle et la phase organique est lavée successivement avec 100 cm³ d'une solution saturée en hydrogénocarbonate de sodium et avec 75 cm³ d'une solution saturée en chlorure de sodium. La phase organique est décantée, séchée sur sulfate de magnésium, filtrée et concentrée à sec sous pression réduite (2 kPa; 50°C) pour donner 1.4 g de brut qui est purifié par chromatographie sous pression d'argon de 50 kPa, sur colonne de gel de silice (granulométrie 40-60 µm ; diamètre 4 cm), en éluant par un mélange cyclohexane-acétate d'éthyle (70/30 en volumes). Les fractions contenant le produit attendu sont réunies et évaporées sous pression réduite (2 kPa ; 50°C) ; le résidu est séché (90 Pa; 45°C) pour donner 530 mg de N-[5-(3-furanyl)-6-[4-(phénylméthoxy)phényl]-1H-indazol-3-yl]-butanamide sous forme d'huile jaune.

Spectre de R.M.N. ¹H (300 MHz, (CD₃)₂SO d6, δ en ppm) : 0,98 (t, J = 7,5 Hz : 3H) ; 1,68 (mt : 2H) ; 2,41 (t, J = 7 Hz : 2H) ; 5,15 (s : 2H) ; 6,07 (s large : 1H) ; 7,02 (d, J = 8,5 Hz : 2H) ; 7,20 (d, J = 8,5 Hz : 2H) ; de 7,30 à 7,60 (mt : 6H) ; 7,31 (s : 2H) ; 7,86 (s : 1H) ; 10,36 (mf : 1H) ; 12,66 (mf : 1H).

EI m/z = 451 M^{+.}

m/z = 381 [M - C₄H₆O]^{+.}

### N-[5-(3-furanyl)-6-([4-hydroxyphényl)-1H-indazol-3-yl]-butanamide

A 500 mg de N-[5-(3-furanyl)-6-[4-(phénylméthoxy)phényl]-1H-indazol-3-yl)]-butanamide décrit précédemment, on ajoute 10 cm³ d'iodure de triméthylsilyle et on chauffe au reflux 18 heures. On ajoute au milieu 25 cm³ de méthanol et on rechauffe au reflux pendant 10 minutes puis on concentre le milieu réactionnel à sec sous pression réduite (2 kPa ; 50°C ). Le résidu est repris par 75 cm³ d'acétate d'éthyle et 50 cm³ de tétrahydrofurane puis la phase organique est lavée avec 2 fois 100 cm³ d'une solution de thiosulfate de sodium à 10% puis avec 75 cm³ d'une solution saturée en chlorure de sodium. La phase organique est décantée, séchée sur sulfate de magnésium, filtrée puis concentrée à sec sous pression réduite pour donner 950 mg de brut qui est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (60/40 en volumes). Les fractions contenant le produit attendu sont réunies et évaporées sous pression réduite (2 kPa ; 50°C) ; le résidu est repris avec 5 cm³ d'acétate d'éthyle, filtré, lavé par 1 cm³ d'acétate d'éthyle puis par 20 cm³ d'éther diéthylique et séché (90 Pa ; 45°C) pour donner 10 mg de N-[5-(3-furanyl)-6-(4-hydroxyphényl)-1H-indazol-3-yl]-butanamide sous forme de poudre blanche fondant à 185°C.

Spectre de R.M.N. ¹H (300 MHz, (CD₃)₂SO d6, δ en ppm) : 0,97 (t, J = 7,5 Hz : 3H) ; 1,67 (mt : 2H) ; 2,40 (t, J = 7 Hz : 2H) ; 6,07 (s large : 1H) ; 6,75 (d, J = 8,5 Hz : 2H) ; 7,06 (d, J = 8,5 Hz : 2H) ; 7,27 (s large : 2H) ; 7,52 (mt : 1H) ; 7,83 (s : 1H) ; de 9,40 à 9,65 (mf : 1H) ; 10,33 (mf : 1H) ; de 12,50 à 12,75 (mf : 1H).

DCI m/z = 362 [M+H]⁺

### EXEMPLE 100

### N-[5-(4-éthylphényl)-6-[4-(phénylméthoxy)phényl]-1-[[2-(triméthylsilyl)éthoxy]méthyl]-1H-indazol-3-yl]-butanamide

A 1 g de N-[5-bromo-6-[4-(phénylméthoxy)phényl]-1-[[2-(triméthylsilyl)éthoxy]méthyl]-1H-indazol-3-yl]-butanamide préparé dans l'exemple 63, dans 100 cm³ de dioxane on ajoute 379 mg d'acide 4-éthylphénylboronique, 428 mg de carbonate de sodium dans 30 cm³ d'eau, 259 mg de tétrakis-triphénylphosphine palladium et on chauffe au reflux pendant 18 heures. Au milieu réactionnel on ajoute 100 cm³ d'acétate d'éthyle et 100 cm³ d'eau et on filtre le milieu réactionnel sur verre fritté garni de célite. La phase organique est décantée, lavée successivement par 75 cm³ d'eau et par 75 cm³ d'une solution saturée de chlorure de sodium, séchée sur sulfate de magnésium, filtrée et concentrée sous pression réduite (2 kPa ; 50°C) pour donner 1.7 g d'une huile qui est purifiée par chromatographie sous pression d'argon de 50 kPa, sur colonne de gel de silice (granulométrie 40-60 µm ; diamètre 3.5 cm), en éluant par un mélange cyclohexane-acétate d'éthyle (80/20 en volumes). Les fractions contenant le produit attendu, sont réunies et évaporées sous pression réduite (2 kPa; 50°C) pour donner 850 mg de N-[5-(4-éthylphényl)-6-[4-(phénylméthoxy)phényl]-1-[[2-(triméthylsilyl)éthoxy]méthyl]-1H-indazol-3-yl]-butanamide sous forme de cristaux gris.

Spectre de R.M.N. ¹H (300 MHz, (CD₃)₂SO d6, δ en ppm) : - 0,07 (s : 9H) ; 0,84 (t, J = 8 Hz : 2H) ; 0,94 (t, J = 7,5 Hz : 3H) ; 1,18 (t, J = 7,5 Hz : 3H) ; 1,65 (mt : 2H) ; 2,40 (t, J = 7 Hz : 2H) ; 2,58 (q, J = 7,5 Hz : 2H) ; 3,57 (t, J = 8 Hz : 2H) ; 5,10 (s : 2H) ; 5,70 (s : 2H) ; 6,93 (d, J = 8,5 Hz : 2H) ; 7,02 (d, J = 8,5 Hz : 2H) ; 7,09 (d, J = 8,5 Hz : 2H) ; 7,11 (d, J = 8,5 Hz : 2H) ; de 7,30 à 7,50 (mt : 5H) ; 7,63 (s : 1H) ; 7,82 (s : 1H) ; 10,50 (mf : 1H).

EI m/z = 619 M^{+.}

m/z = 502 [M - OCH₂CH₂Si(CH₃)₃]⁺

m/z = 91 [C₆H₅CH₂]⁺

### N-[5-(4-éthylphényl)-6-[4-(phénylméthoxy)phényl]-1H-indazol-3-yl]-butanamide

A 850 mg de N-[5-(4-éthylphényl)-6-[4-(phénylméthoxy)phényl]-1-[[2-(triméthylsilyl)éthoxy]méthyl]-1H-indazol-3-yl]-butanamide précédemment décrit, dans 50 cm³ de tétrahydrofurane on ajoute 8.3 cm³ de fluorure de tétrabutylammonium en solution 1M dans le tétrahyrofurane et on porte à reflux pendant 18 heures ; après refroidissement on ajoute 75 cm³ d'acétate d'éthyle et la phase organique est lavée successivement avec 2 fois 100 cm³ d'une solution saturée en hydrogénocarbonate de sodium et avec 75 cm³ d'une solution saturée en chlorure de sodium. La phase organique est décantée, séchée sur sulfate de magnésium, filtrée et concentrée à sec sous pression réduite (2 kPa ; 50°C) pour donner 1.5 g de brut qui est purifié par chromatographie sous pression d'argon de 50 kPa, sur colonne de gel de silice (granulométrie 40-60 µm ; diamètre 3 cm), en éluant par un mélange cyclohexane-acétate d'éthyle (70/30 en volumes). Les fractions contenant le produit attendu sont réunies et évaporées sous pression réduite (2 kPa ; 50°C) ; le résidu séché (90 Pa; 45°C) pour donner 660 mg de N-[5-(4-éthylphényl)-6-[4-(phénylméthoxy)phényl]-1H-indazol-3-yl]-butanamide sous forme de cristaux gris.

Spectre de R.M.N. ¹H (300 MHz, (CD₃)₂SO d6, δ en ppm) : 0,95 (t, J = 7,5 Hz: 3H) ; 1,17 (t, J = 7,5 Hz : 3H) ; 1,65 (mt : 2H) ; 2,39 (t, J = 7 Hz : 2H) ; 2,58 (q, J = 7,5 Hz : 2H) ; 5,08 (s : 2H) ; 6,90 (d, J = 8,5 Hz : 2H) ; 7,00 (d, J = 8,5 Hz : 2H) ; 7,08 (d, J = 8,5 Hz : 4H) ; de 7,30 à 7,50 (mt : 5H) ; 7,34 (s : 1H) ; 7,76 (s : 1H) ; 10,36 (mf : 1H) ; 12,66 (mf : 1H).

EI m/z = 489 M^{+.}

m/z = 419 [M - C₄CH₆O]⁺

### N-[5-(4-éthylphényl)-6-(4-hydroxyphényl)-1H-indazol-3-yl]-butanamide

A 600 mg de N-[5-(4-éthylphényl)-6-[4-(phénylméthoxy)phényl]-1H-indazol-3-yl]-butanamide décrit précédemment, on ajoute 10 cm³ d'iodure de triméthylsilyle et on chauffe au reflux 18 heures. On ajoute au milieu 30 cm³ de méthanol et on rechauffe au reflux pendant 5 minutes puis on concentre le milieu réactionnel à sec sous pression réduite (2 kPa ; 50°C ). Le résidu est repris par 100 cm³ d'acétate d'éthyle et la phase organique est lavée avec 2 fois 100 cm³ d'une solution de thiosulfate de sodium à 10% puis avec 75 cm³ d'une solution saturée en chlorure de sodium. La phase organique est décantée, séchée sur sulfate de magnésium, filtrée puis concentrée à sec sous pression réduite pour donner 650 mg de brut qui est purifié par chromatographie sous pression d'argon de 50 kPa, sur colonne de gel de silice ( granulométrie 40-60 µm; diamètre 3 cm), en éluant par un mélange cyclohexane-acétate d'éthyle (50/50 en volumes). Les fractions contenant le produit attendu sont réunies et évaporées sous pression réduite (2 kPa ; 50°C) ; le résidu repris avec 15 cm³ d'éther diisopropylique, filtré, lavé par 5 cm³ d'acétate d'éthyle puis par 10 cm³ d'éther diisopropylique et séché (90 Pa; 45°C) pour donner 180 mg de N-[5-(4-éthylphényl)-6-(4-hydroxyphényl)-1H-indazol-3-yl]- butanamide sous forme de cristaux crème fondant à 225°C.

Spectre de R.M.N. ¹H (300 MHz, (CD₃)₂SO d6, δ en ppm) : 0,95 (t, J = 7,5 Hz : 3H) ; 1,17 (t, J = 7,5 Hz : 3H) ; 1,65 (mt : 2H) ; 2,39 (t, J = 7 Hz : 2H) ; 2,58 (q, J = 7,5 Hz : 2H) ; 6,63 (d, J = 8,5 Hz : 2H) ; 6,95 (d, J = 8,5 Hz : 2H) ; 7,00 (d, J = 8,5 Hz: 2H) ; 7,08 (d, J = 8,5 Hz : 2H); 7,31 (s : 1H); 7,74 (s: 1H); 9,36 (mf: 1H); 10,35 (s large : 1H); 12,61 (mf: 1H).

### EXEMPLE 101

### N-[5-(3-pyridinyl)-6-[4-(phénylméthoxy)phényl]-1-[[2: (triméthylsilyl)éthoxy]méthyl]-1H-indazol-3-yl]-butanamide

A 1 g de N-[5-bromo-6-[4-(phénylméthoxy)phényl]-1-[[2-(triméthylsilyl)éthoxy]méthyl]-1H-indazol-3-yl]-butanamide préparé dans l'exemple 63, dans 100 cm³ de dioxane on ajoute 371 mg de 3-pyridyldiéthylborane, 428 mg de carbonate de sodium dans 30 cm³ d'eau, 258 mg de tétrakis-triphénylphosphine palladium et on chauffe au reflux pendant 18 heures. Au milieu réactionnel on ajoute 100 cm³ d'acétate d'éthyle, 100 cm³ d'eau et on filtre le milieu réactionnel sur verre fritté garni de célite. La phase organique est décantée, lavée successivement par 75 cm³ d'eau et par 75 cm³ d'une solution saturée en chlorure de sodium, séchée sur sulfate de magnésium, filtrée et concentrée sous pression réduite (2 kPa ; 50°C) pour donner 1.6 g d'une huile qui est purifiée par chromatographie sous pression d'argon de 50 kPa, sur colonne de gel de silice (granulométrie 40-60 µm ; diamètre 3.5 cm), en éluant par un mélange cyclohexane-acétate d'éthyle (70/30 en volumes). Les fractions contenant le produit attendu, sont réunies et évaporées sous pression réduite (2 kPa; 50°C) pour donner 700 mg de N-[5-(3-pyridinyl)-6-[4-(phénylméthoxy)phényl]-1-[[2-(triméthylsilyl)éthoxy]méthyl])-1H-indazol-3-yl]-butanamide sous forme d'huile jaune.

Spectre de R.M.N. ¹H (300 MHz, (CD₃)₂SO d6, δ en ppm): - 0,05 (s : 9H) ; 0,85 (t, J = 8 Hz : 2H) ; 0,96 (t, J = 7,5 Hz : 3H) ; 1,66 (mt : 2H) ; 2,42 (t, J = 7 Hz : 2H) ; 3,58 (t, J = 8 Hz : 2H) ; 5,11 (s : 2H) ; 5,73 (s : 2H) ; 6,96 (d, J = 8,5 Hz : 2H) ; 7,10 (d, J = 8,5 Hz : 2H) ; de 7,30 à 7,55 (mt : 5H); 7,31 (dd large, J = 7,5 et 5 Hz : 1H) ; 7,50 (ddd, J = 7,5 - 2,5 et 2 Hz : 1H); 7,72 (s : 1H) ; 7,92 (s : 1H) ; 8,31 (d large, J = 2,5 Hz : 1H) ; 8,43 (dd, J = 5 et 2 Hz : 1H) ; 10,57 (mf : 1H).

EI m/z = 592 M^{+.}

m/z = 475 [M - OCH₂CH₂Si(CH₃)₃]⁺

m/z = 91 [C₆H₅CH₂]⁺

### N-[5-(3-pyridinyl)-6-[4-(phénylméthoxy)phényl]-1H-indazol-3-yl]-butanamide

A 700 mg de N-[5-(3-pyridinyl)-6-[4-(phénylméthoxy)phényl]-1-[[2-(triméthylsilyl)éthoxy]méthyl]-1H-indazol-3-yl]-butanamide précédemment décrit, dans 50 cm³ de tétrahydrofurane on ajoute 7.1 cm³ de fluorure de tétrabutylammonium en solution 1M dans le tétrahydrofurane et on porte à reflux pendant 18 heures ; après refroidissement on ajoute 75 cm³ d'acétate d'éthyle et la phase organique est lavée successivement avec 2 fois 75 cm³ d'une solution saturée en hydrogénocarbonate de sodium puis avec 50 cm³ d'une solution saturée en chlorure de sodium. La phase organique est décantée, séchée sur sulfate de magnésium, filtrée et concentrée à sec sous pression réduite (2 kPa ; 50°C) pour donner 850 mg de brut qui est purifié par chromatographie sous pression d'argon de 50 kPa, sur colonne de gel de silice (granulométrie 40-60 µm; diamètre 3 cm), en éluant par un mélange cyclohexane-acétate d'éthyle (70/30 en volumes). Les fractions contenant le produit attendu sont réunies et évaporées sous pression réduite ( 2 kPa; 50°C); le résidu séché (90 Pa; 45°C) pour donner 460 mg de N-[5-(3-pyridinyl)-6-[4-(phénylméthoxy)phényl]-1H-indazol-3-yl]-butanamide sous forme de cristaux crème.

Spectre de R.M.N. ¹H (300 MHz, (CD₃)₂SO d6, δ en ppm) : 0,95 (t, J = 7,5 Hz : 3H) ; 1,66 (mt: 2H) ; 2,40 (t, J = 7 Hz : 2H) ; 5,08 (s : 2H) ; 6,93 (d, J = 8,5 Hz : 2H) ; 7,09 (d, J = 8,5 Hz : 2H) ; 7,29 (dd large, J = 7,5 et 4,5 Hz : 1H) ; de 7,30 à 7,55 (mt : 6H) ; 7,40 (s : 1H) ; 7,86 (s : 1H) ; 8,28 (d large, J = 2 Hz : 1H) ; 8,41 (dd, J = 4,5 et 2 Hz : 1H) ; 10,42 (mf : 1H) ; 12,76 (mf : 1H).

EI m/z = 462 M^{+.}

m/z = 392 [M - C₄CH₆O]⁺

### N-[5-(3-pyridinyl)-6-(4-hydroxyphényl)-1H-indazol-3-yl]-butanamide

A 460 mg de N-[5-(3-pyridinyl)-6-[4-(phénylméthoxy)phényl]-1H-indazol-3-yl]-butanamide décrit précédemment, on ajoute 10 cm³ d'iodure de triméthylsilyle et on chauffe au reflux 18 heures. L'insoluble est filtré, lavé par 2 fois 20 cm³ d'éther diéthylique et repris par 50 cm³ de tétrahydrofurane, 25 cm³ d'acétate d'éthyle et la phase organique est lavée avec 2 fois 100 cm³ d'une solution de thiosulfate de sodium à 10%, séchée sur sulfate de magnésium, filtrée puis concentrée à sec sous pression réduite pour donner 330 mg de brut qui est purifié par chromatographie sous pression d'argon de 50 kPa, sur colonne de gel de silice (granulométrie 40-60 µm; diamètre 2.5 cm), en éluant par un mélange cyclohexane-acétate d'éthyle (50/50 en volumes). Les fractions contenant le produit attendu sont réunies et évaporées sous pression réduite (2 kPa; 50°C) et le résidu est repris avec 10 cm³ d'éther diisopropylique, filtré, lavé par 3 fois 5 cm³ d'éther diisopropylique, par 5 cm³ d'acétate d'éthyle puis par 10 cm³ d'éther diisopropylique et séché (90 Pa ; 45°C) pour donner 90 mg de N-[5-(3-pyridinyl)-6-(4-hydroxyphényl)-1H-indazol-3-yl]-butanamide sous forme de cristaux crème fondant à 165°C.

Spectre de R.M.N. ¹H (300 MHz, (CD₃)₂SO d6, δ en ppm) : 0,96 (t, J = 7,5 Hz : 3H) ; 1,66 (mt : 2H) ; 2,40 (t, J = 7 Hz : 2H) ; 6,66 (d, J = 8,5 Hz : 2H) ; 6,96 (d, J = 8,5 Hz : 2H) ; 7,30 (dd, J = 7,5 et 4,5 Hz : 1H) ; 7,38 (s : 1H) ; 7,49 (dt, J = 7,5 et 2 Hz : 1H) ; 7,85 (s: 1H) ; 8,28 (d, J = 2 Hz : 1H) ; 8,41 (dd, J = 4,5 et 2 Hz : 1H) ; 9,46 (mf : 1H) ; 10,45 (mf : 1H) ; 12,74 (mf : 1H).

### EXEMPLE 102

### N-[5-(2-furanyl)-6-[4-(phénylméthoxy)phényl]-1-[[2-(triméthylsilyl)éthoxy]méthyl]-1H-indazol-3-yl]-butanamide

A 1.25 g de N-[5-bromo-6-[4-(phénylméthoxy)phényl]-1-[[2-(triméthylsilyl)éthoxy]méthyl]-1H-indazol-3-yl]-butanamide préparé dans l'exemple 63, dans 125 cm³ de dioxane on ajoute 353mg d'acide 2-furylboronique, 624 mg de carbonate de sodium dans 25 cm³ d'eau, 311 mg de tétrakis-triphénylphosphine palladium et on chauffe au reflux pendant 18 heures puis on ajoute 611 mg de 2-furan-2-yl-4,4,5,5-tétraméthyl-[1,3,2]dioxaborolane et on poursuit le chauffage 4 heures à reflux. Au milieu réactionnel on ajoute 75 cm³ d'acétate d'éthyle et 75 cm³ d'eau et on filtre le milieu réactionnel sur verre fritté garni de célite. La phase organique est décantée, lavée par 75 cm³ d'eau, séchée sur sulfate de magnésium, filtrée et_concentrée à sec sous-pression-réduite (2 kPa ; 50°C) pour donner 2 g d'une huile qui est purifiée par chromatographie sous pression d'argon de 50 kPa, sur colonne de gel de silice (granulométrie 40-60 µm ; diamètre 3.5 cm), en éluant par un mélange cyclohexane-acétate d'éthyle (75/25 en volumes). Les fractions' contenant le produit attendu, sont réunies et évaporées sous pression réduite (2 kPa ; 50°C) pour donner 1.20 g de N-[5-(2-furanyl)-6-[4-(phénylinéthoxy)phényl]-1-[[2-(triméthylsilyl)éthoxy]méthyl]-1H-indazol-3-yl]-butanamide sous forme de solide beige.

Spectre de R.M.N. ¹H (300 MHz, (CD₃)₂SO d6, δ en ppm) : - 0,09 (s : 9H); 0,82 (t, J = 8 Hz : 2H) ; 0,97 (t, J = 7,5 Hz : 3H) ; 1,68 (mt : 2H) ; 2,43 (t, J = 7 Hz : 2H) ; 3,55 (t, J = 8 Hz : 2H) ; 5,17 (s : 2H) ; 5,61 (d, J = 3,5 Hz : 1H) ; 5,68 (s large : 2H) ; 6,38 (dd, J = 3,5 et 1,5 Hz : 1H) ; 7,07 (d, J = 8,5 Hz : 2H) ; 7,19 (d, J = 8,5 Hz : 2H) ; 7,36 (t large, J = 7,5 Hz : 1H) ; 7,43 (t large, J = 7,5 Hz : 2H) ; 7,50 (d large, J = 7,5 Hz : 2H) ; 7,59 (s : 1H) ; 7,61 (d, J = 1,5 Hz : 1H) ; 8,20 (s : 1H) ; 10,58 (mf : 1H).

EI m/z = 581 M^{+.}

m/z = 464 [M - OCH₂CH₂Si(CH₃)₃]⁺

m/z = 91 [C₆H₅CH₂]⁺

### N-[5-(2-furanyl)-6-[4-(phénylméthoxy)phényl]-1H-indazol-3-yl]-butanamide:

A 1.20 g de N-[5-(2-furanyl)-6-[4-(phénylméthoxy)phényl]-1-[[2-(triméthylsilyl)éthoxy]méthyl]-1H-indazol-3-yl]-butanamide précédemment décrit, dans 50 cm³ de tétrahydrofurane on ajoute 12.4 cm³ de fluorure de tétrabutylammonium en solution 1M dans le tétrahyrofurane et on porte à reflux pendant 18 heures ; après refroidissement on ajoute 75 cm³ d'acétate d'éthyle et la phase organique est lavée successivement avec 2 fois 50 cm³ d'une solution saturée en hydrogénocarbonate de sodium et avec 50 cm³ d'une solution saturée en chlorure de sodium. La phase organique est décantée, séchée sur sulfate de magnésium, filtrée et concentrée à sec sous pression réduite (2 kPa ; 50°C) pour donner 1.5 g de brut qui est purifié par chromatographie sous-pression d'argon de 50 kPa, sur colonne de gel de silice (granulométrie 40-60 µm ; diamètre 3.5 cm), en éluant par un mélange cyclohexane-acétate d'éthyle (60/40 en volumes). Les fractions contenant le produit attendu sont réunies et évaporées sous pression réduite (2 kPa ; 50°C) ; le résidu séché (90 Pa; 45°C) pour donner 750 mg de N-[5-(2-furanyl)-6-[4-(phénylméthoxy)phényl]-1H-indazol-3-yl]-butanamide sous forme d'huile jaune.

Spectre de R.M.N. ¹H (300 MHz, (CD₃)₂SO d6, δ en ppm) : 0,98 (t, J = 7,5 Hz : 3H) ; 1,68 (mt : 2H) ; 2,42 (t, J = 7 Hz : 2H) ; 5,16 (s : 2H) ; 5,60 (d, J = 3,5 Hz : 1H) ; 6,37 (dd, J = 3,5 et 1,5 Hz : 1H) ; 7,06 (d, J = 8,5 Hz : 2H) ; 7,20 (d, J = 8,5 Hz : 2H) ; 7,28 (s : 1H) ; 7,36 (t large, J = 7,5 Hz : 1H) ; 7,43 (t large, J = 7,5 Hz : 2H) ; 7,51 (d large, J = 7,5 Hz : 2H) ; 7,59 (s large : 1H) ; 8,15 (s : 1H) ; 10,44 (mf : 1H) ; 12,73 (mf : 1H).

EI m/z = 451 M^{+.}

m/z = 381 [M - C₄CH₆O]^{+.}

### N-[5-(2-furanyl)-6-(4-hydroxyphényl)-1H-indazol-3-yl]-butanamide

A 750 mg de N-[(5-(2-furanyl)-6-[4-(phénylinéthoxy)phényl]-1H-indazol-3-yl)]-butanamide décrit précédemment, on ajoute 10 cm³ d'iodure de triméthylsilyle et on chauffe au reflux 18 heures. On ajoute au milieu 40 cm³ de méthanol et on rechauffe au reflux pendant 10 minutes puis on concentre le milieu réactionnel à sec sous pression réduite (2 kPa ; 50°C ). Le résidu est repris par 75 cm³ d'acétate d'éthyle et 50 cm³ de tétrahydrofurane et la phase organique est lavée avec 2 fois 75 cm³ d'une solution de thiosulfate de sodium à 10% puis avec 75 cm³ d'une solution saturée en chlorure de sodium. La phase organique est décantée, séchée sur sulfate de magnésium, filtrée puis concentrée à sec sous pression réduite pour donner 700 mg de brut qui est purifié par chromatographie sous pression d'argon de 50 kPa, sur colonne de gel de silice (granulométrie 40-60 µm ; diamètre 2.5 cm), en éluant par un mélange cyclohexane-acétate d'éthyle (50/50 en volumes). Les fractions contenant le produit attendu sont réunies et évaporées sous pression réduite (2 kPa ; 50°C) ; le résidu est repris avec 3 cm³ d'acétate d'éthyle, filtré, lavé par 2 cm³ d'acétate d'éthyle puis par 15 cm³ d'éther diisopropylique et séché (90 Pa ; 45°C) pour donner 10 mg de N-[5-(2-furanyl)-6-(4-hydroxyphényl)-1H-indazol-3-yl]-butanamide sous forme de cristaux blancs fondant à 190ºC.

Spectre de R.M.N. ¹H (300 MHz, (CD₃)₂SO d6, δ en ppm) : 0,98 (t, J = 7,5 Hz : 3H) ; 1,69 (mt : 2H) ; 2,43 (t, J = 7 Hz : 2H) ; 5,58 (d, J = 3,5 Hz : 1H) ; 6,38 (dd, J = 3,5 et 1,5 Hz : 1H) ; 6,80 (d, J = 8,5 Hz : 2H) ; 7,08 (d, J = 8,5 Hz : 2H) ; 7,26 (s : 1H) ; 7,59 (s large : 1H) ; 8,14 (s : 1H) ; 9,54 (mf : 1H) ; 10,44 (mf : 1H) ; 12,70 (mf : 1H).

### EXEMPLE 103

### N-(5-bromo-6-chloro-7-nitro-1H-indazol-3-yl)-butanamide :

A 4 g de N-(5-bromo-6-chloro-1H-indazol-3-yl)-butanamide décrit à l'exemple 58, dans 50 cm³ d'acétonitrile on ajoute 418 mg de tétrafluoroborate de nitronium à 0°C et laisse sous agitation pendant 4 heures. On ajoute au milieu réactionnel 200 cm³ d'acétate d'éthyle et 100 cm³ d'une solution saturée en hydrogénocarbonate de sodium. La phase organique est lavée par 2 fois 40 cm³ d'une solution saturée en hydrogénocarbonate de sodium puis avec 40 cm³ d'une solution saturée en chlorure de sodium. La phase organique est décantée, séchée sur sulfate de magnésium, filtrée et concentrée à sec sous pression réduite (2 kPa ; 50°C) pour donner 840 mg de brut qui est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (80/20 en volumes). Les fractions contenant le produit attendu, sont réunies et évaporées sous pression réduite (2 kPa ; 50°C) puis séchées (90 Pa ; 45°C) pour donner 20 mg de N-(5-bromo-6-chloro-7-nitro-1H-indazol-3-yl)-butanamide sous forme d'un solide jaune fondant à plus de 260°C.

Spectre de R.M.N. ¹H (300 MHz, (CD₃)₂SO d6, δ en ppm) : 0,97 (t, J = 7,5 Hz : 3H) ; 1,68 (mt : 2H) ; 2,44 (t, J = 7 Hz : 2H) ; 8,70 (s : 1H) ; 10,80 (mf: 1H) ; 13,63 (mf: 1H).

EI m/z = 360 M^{+.}

m/z = 290 [M - C₄CH₆O]^{+.}

### EXEMPLE 104

### N-(6,7-difluoro-1-[[2-(triméthylsilyl)éthoxylméthyl]-1H-indazol-3-yl)-butanamide

A 1.65 g d'hydrure de sodium à 60% dans l'huile, dans 50 cm³ de diméthylformamide on ajoute goutte à goutte une solution de 1.1 g de N-(6,7-difluoro-1H-indazol-3-yl)-butanamide préparé dans l'exemple 40, dans 180 cm³ de diméthylformamide en 3 heures. Le milieu réactionnel est concentré à sec sous pression réduite et repris avec 250 cm³ d'acétate d'éthyle et 200 cm³ d'eau ; la phase organique est décantée, lavée par 150 cm³ d'eau, séchée sur sulfate de magnésium, filtrée et concentrée à sec sous pression réduite (2 kPa ; 50°C). Le brut est purifié par chromatographie sous pression d'argon de 50 kPa, sur colonne de gel de silice ( granulométrie 40-60 µm ; diamètre 6 cm), en éluant par un mélange cyclohexane-acétate d'éthyle (80/20 en volumes). Les fractions contenant le produit attendu sont réunies et évaporées sous pression réduite (2 kPa ; 50°C) pour donner 7.3 g de N-[6,7-difluoro-1-[[2-(triméthylsilyl)éthoxy]méthyl]-1H-indazol-3-yl]-butanamide sous forme d'huile jaune.

Spectre de R.M.N. ¹H (300 MHz, (CD₃)₂SO d6, δ en ppm) : - 0,09 (s : 9H) ; 0,82 (t, J = 8 Hz : 2H) ; 0,96 (t, J = 7,5 Hz : 3H) ; 1,67 (mt : 2H) ; 2,41 (t, J = 7 Hz : 2H) ; 3,56 (t, J = 8 Hz : 2H) ; 5,66 (s : 2H) ; 7,22 (ddd, J = 11 - 9 et 7 Hz : 1H) ; 7,69 (dd large, J = 9 et 4,5 Hz : 1H) ; 10,60 (mf : 1H).

EI m/z = 369 M^{+.}

m/z = 252 [M-OCH₂CH₂Si(CH₃)₃]⁺

m/z = 241 [M-C₆H₁₂OSi]^{+.}

### N-(5-bromo-6,7-difluoro-1-[[2-(triméthylsilyl)éthoxylméthyl]-1H-indazol-3-yl)-butanamide

A 1g de N-16,7-difluoro-1-[[2-(triméthylsilyl)éthoxy]méthyl]-1H-indazol-3-yl]-butanamide précédemment décrit dans 30 cm³ de chloroforme, on ajoute 0.87 cm³ de pyridine, puis additionne 0.56 cm³ de brome et on chauffe au reflux la nuit. On ajoute au milieu réactionnel 50 cm³ de dichlorométhane et 50 cm³ d'une solution aqueuse de thiosulfate de sodium à 10%. Après 10 minutes d'agitation, l'insoluble est éliminé par filtration sur verre fritté et la phase organique est lavée avec 50 cm³ d'eau et avec 50 cm³ d'une solution saturée en chlorure de sodium. La phase organique est décantée, séchée sur sulfate de magnésium, filtrée et concentrée à sec sous pression réduite (2 kPa ; 45 °C). Le brut, 1.1 g, est purifié par chromatographie sous pression d'argon de 50 kPa, sur colonne de gel de silice (granulométrie 40-60 µm ; diamètre 3 cm), en éluant par un mélange cyclohexane-acétate d'éthyle (90/10 en volumes). Les fractions contenant le produit attendu, sont réunies et évaporées sous pression réduite (2 kPa ; 50°C). On obtient après séchage (90 Pa ; 45°C), 230 mg de N-(5-bromo-6,7-difluoro-1-[[2-(triméthylsilyl)éthoxy]méthyl]-1H-indazol-3-yl)-butanamide sous forme d'huile incolore.

Spectre de R.M.N. ¹H (300 MHz, (CD₃)₂SO d6, δ en ppm) : - 0,05 (s : 9H) ; 0,84 (t, J = 8 Hz : 2H) ; 0,95 (t, J = 7,5 Hz : 3H) ; 1,66 (mt : 2H) ; 2,43 (t, J = 7 Hz : 2H) ; 3,59 (t, J = 8 Hz : 2H) ; 5,69 (s : 2H) ; de 7,40 à 7,65 (mt : 5H) ; 7,82 (d large, J = 7 Hz : 1H) ; 10,64 (mf : 1H).

EI m/z = 447 M^{+.}

m/z = 330 [M - OCH₂CH₂Si(CH₃)₃]⁺

m/z = 319 [M-C₆H₁₂OSi]^{+.}

### N-(5-bromo-6,7-difluoro-1H-indazol-3-yl)-butanamide

A 700 mg de N-[5-bromo-6,7-difluoro-1-[[2-(triméthylsilyl)éthoxy]méthyl]-1H-indazol-3-yl]-butanamide précédemment décrit, dans 30 cm³ de tétrahydrofurane on ajoute 9.4 cm³ de fluorure de tétrabutylammonium en solution 1M dans le tétrahyrofurane et on porte à reflux pendant 18 heures ; après refroidissement on ajoute 100 cm³ d'acétate d'éthyle et 75 cm³ d'une solution saturée en hydrogénocarbonate de sodium puis la phase organique est lavée successivement avec 75 cm³ d'une solution saturée en hydrogénocarbonate de sodium et avec 75 cm³ d'une solution saturée en chlorure de sodium. La phase organique est décantée, séchée sur sulfate de magnésium et concentrée à sec sous pression réduite (2 kPa ; 50°C) pour donner 850 mg de brut qui est purifié par chromatographie sous pression d'argon de 50 kPa, sur colonne de gel de silice (granulométrie 40-60 µm ; diamètre 3 cm), en éluant par un mélange cyclohexane-acétate d'éthyle (80/20 en volumes). Les fractions contenant le produit attendu sont réunies et évaporées sous pression réduite ( 2 kPa ; 50°C) ; le résidu est repris par 8 cm³ d'éther diisopropylique, filtré, lavé par 3 cm³ d'éther diisopropylique, séché sous presson réduite (90 Pa; 45°C) pour donner 200 mg de N-(5-bromo-6,7-difluoro-1H-indazol-3-yl)-butanamide sous forme de cristaux blancs fondant à 220°C.

Spectre de R.M.N. ¹H (300 MHz, (CD₃)₂SO d6, δ en ppm) : 0,95 (t, J = 7,5 Hz : 3H) ; 1,66 (mt : 2H) ; 2,41 (t, J = 7 Hz : 2H) ; 8,03 (dd, J = 6 et 2 Hz : 1H) ; 10,58 (s large : 1H) ; 13,56 (mf: IR).

### EXEMPLE 105

### N-[6-(4-cyanophényl)-1-[[2-(triméthylsilyl)éthoxylméthyl]-1H-indazol-3-yl]-butanamide :

A 500 mg de N-[6-bromo-1-[[2-(triméthylsilyl)éthoxy]méthyl]-1H-indazol-3-yl]-butanamide préparé comme décrit à l'exemple 51, dans 100 cm³ de dioxane, on ajoute 853 mg d'acide 4-cyanophénylboronique, 15 cm³ d'eau, 1.0 g de carbonate de sodium et 314 mg de tétrakis-triphénylphosphine palladium. Le milieu réactionnel est ensuite porté au reflux pendant 4 heures et dilué avec 70 cm³ d'acétate d'éthyle et 75 cm³ d'eau. La phase organique est décantée, lavée avec 50 cm³ d'eau distillée puis avec 2 fois avec 50 cm³ d'une solution aqueuse saturée en chlorure de sodium. La phase organique est décantée, séchée sur sulfate de magnésium, filtrée et concentrée à sec sous pression réduite (2 kPa ; 45 °C). Le résidu obtenu 2.0 g est purifié par chromatographie sous pression d'argon de 50 kPa, sur colonne de gel de silice ( granulométrie 40-60 µm ; diamètre 4 cm), en éluant par un mélange cyclohexane-acétate d'éthyle (70/30 en volumes). Les fractions contenant le produit attendu, sont réunies et évaporées sous pression réduite (2 kPa ; 50°C) ; on obtient 1.0 g de N-[6-(4-cyanophényl)-1-[[2-(triméthylsilyl)éthoxy]méthyl]-1H-indazol-3-yl]-butanamide sous forme d'un solide jaune fondant à 136°C.

Spectre de R.M.N. ¹H (300 MHz, (CD₃)₂SO d6, δ en ppm) : - 0,09 (s : 9H) ; 0,83 (t, J = 8 Hz : 2H) ; 0,98 (t, J = 7,5 Hz : 3H) ; 1,69 (mt : 2H) ; 2,43 (t, J = 7 Hz : 2H) ; 3,58 (t, J = 8 Hz : 2H) ; 5,76 (s : 2H) ; 7,53 (d large, J = 8,5 Hz : 1H) ; de 7,95 à 8,05 (mt : 4H) ; 7,97 (d, J = 8,5 Hz : 1H) ; 8,11 (s : 1H) ; 10,55 (mf : 1H).

### N-[6-(4-cyanophényl)-1H-indazol-3-yl]-butanamide :

A 400 mg de N-[6-(4-cyanophényl)-1-[[2-(triméthylsilyl)éthoxy]méthyl]-1H-indazol-3-yl]-butanamide précédemment décrit dans 10 cm³ de tétrahydrofurane on ajoute 3.0 cm³ de fluorure de tétrabutylammonium en solution 1M dans le tétrahydrofurane et on chauffe à reflux pendant 18 heures. Le milieu réactionnel est dilué avec 20 cm³ d'acétate d'éthyle et la phase organique et lavée successivement avec 20 cm³ d'une solution saturée en hydrogénocarbonate de sodium, avec 2 fois 20 cm³ d'eau et avec 20 cm³ d'une solution saturée en chlorure de sodium. La phase organique est décantée, séchée sur sulfate de magnésium, filtrée puis concentrée sous pression réduite (2 kPa ; 50°C). Le brut obtenu est purifié par chromatographie sous pression d'argon de 50 kPa, sur colonne de gel de silice (granulométrie 40-60 µm ; diamètre 3 cm), en éluant avec un gradiant chlorure de méthylène-méthanol (100/0 à 98/2 en volumes). Les fractions contenant le produit attendu, sont réunies et évaporées sous pression réduite (2 kPa ; 50°C) ; on obtient 120 mg de N-[6-(4-cyanophényl)-1H-indazol-3-yl]-butanamide sous forme d'un solide fondant à 242°C.

Spectre de R.M.N. ¹H (300 MHz, (CD₃)₂SO d6, δ en ppm) : 0,98 (t, J = 7 Hz : 3H) ; 1,68 (mt : 2H) ; 2,40 (t, J = 7 Hz : 2H) ; 7,42 (d large, J = 9 Hz : 1H) ; 7,74 (s large : 1H) ; 7,92 (d, J = 9 Hz : 1H) ; 7,96 (s : 4H) ; 10,37 (mf : 1H) ; 12,81 (mf : 1H).

### EXEMPLE 106

### N-(6,7-difluoro-5-nitro-1H-indazol-3-yl)-butanamide :

A une suspension de 500 mg de N-(6,7-difluoro-1H-indazol-3-yl)-butanamide préparé dans l'exemple 40, dans 30 cm³ d'acétonitrile et refroidie à 0°C on ajoute 555 mg de tétrafluoroborate de nitronium. Après 30 minutes de réaction, on ajoute au milieu réactionnel 50 cm³ d'acétate d'éthyle et 50 cm³ d'une solution saturée en hydrogénocarbonate de sodium. La phase organique est décantée, lavée avec 50 cm³ d'eau et 50 cm³ d'une solution saturée en chlorure de sodium, séchée sur sulfate de magnésium, filtrée puis concentrée à sec sous pression réduite (2 kPa ; 50°C) pour donner 630 mg d'une huile brune. Le brut est purifié par chromatographie sous pression d'argon de 50 kPa, sur colonne de gel de silice (granulométrie 40-60 µm ; diamètre 3.5 cm), en éluant avec un mélange cyclohexane-acétate d'éthyle (70/30 en volumes). Les fractions contenant le produit attendu, sont réunies, évaporées sous pression réduite (2 kPa ; 50°C) et séchées (90 Pa, 45°C); pour donner 300 mg de N-(6,7-difluoro-5-nitro-1H-indazol-3-yl)-butanamide sous forme de cristaux jaunes fondant à 255°C.

Spectre de R.M.N. ¹H (300 MHz, (CD₃)₂SO d6, δ en ppm) : 0,96 (t, J = 7,5 Hz : 3H) ; 1,68 (mt : 2H) ; 2,45 (t, J = 7 Hz : 2H) ; 8,89 (dd, J = 6,5 et 2 Hz : 1H) ; 10,94 (mf : 1H) ; 14,05 (mf étalé : 1H).

EI m/z = 284 M^{+.}

m/z = 214 [M-C₄CH₆O]^{+.}

### EXEMPLE 107

### N-[6,7-difluoro-5-phényl-1-[[2-(triméthylsilyl)éthoxylméthyl]-1H-indazol-3-yl]-butanamide

A 1.15 g de N-(5-bromo-6,7-difluoro-1-[[2-(triméthylsilyl)éthoxy]méthyl]-1H-indazol-3-yl)-butanamide, préparé dans l'exemple 104, dans 150 cm³ de dioxane, on ajoute, 469 mg d'acide phénylboronique, 760 mg de carbonate de sodium dans 30 cm³ d'eau et 379 mg de tétrakis-triphénylphosphine palladium et on chauffe au reflux pendant 4 heures. On dilue le milieu réactionnel avec 100 cm³ d'acétate d'éthyle et 75 cm³ d'eau et on filtre sur verre fritté garni de célite. La phase organique est décantée, lavée avec 75 cm³ d'eau et avec 75 cm³ d'une solution saturée en chlorure de sodium, séchée sur sulfate de magnésium, filtrée et concentrée à sec sous pression réduite (2 kPa ; 50°C) pour donner 2 g de brut sous forme d'une huile noire. Le brut est purifié par chromatographie sous pression d'argon de 50 kPa, sur colonne de gel de silice ( granulométrie 40-60 µm ; diamètre 3.5 cm), en éluant avec un mélange cyclohexane-acétate d'éthyle (85/15 en volumes). Les fractions contenant le produit attendu, sont réunies, évaporées sous pression réduite (2 kPa ; 50°C) et séchées (90 Pa, 45°C); pour donner 1.1 g de N-[6,7-difluoro-5-phényl-1-[[2-(triméthylsilyl)éthoxy]méthyl]-1H-indazol-3-yl]-butanamide sous forme d'huile jaune.

Spectre de R.M.N. ¹H (300 MHz, (CD₃)₂SO d6, δ en ppm) : - 0,05 (s : 9H) ; 0,84 (t, J = 8 Hz : 2H) ; 0,95 (t, J = 7,5 Hz : 3H) ; 1,66 (mt : 2H) ; 2,43 (t, J = 7 Hz : 2H) ; 3,59 (t, J = 8 Hz : 2H) ; 5,69 (s : 2H) ; de 7,40 à 7,65 (mt : 5H) ; 7,82 (d large, J = 7 Hz : 1H) ; 10,64 (mf : 1H).

EI m/z = 445 M^{+.}

m/z = 328 [M - OCH₂CH₂Si(CH₃)₃]⁺

m/z = 317 [M-C₆H₁₂OSi]^{+.}

### N-(6,7-difluoro-5-phényl-1H-indazol-3-yl)-butanamide

A 1.1 g de N-[6,7-difluoro-5-phényl-1-[[2-(triméthylsilyl)éthoxy]méthyl]-1H-indazol-3-yl]-butanamide précédemment décrit, dans 50 cm³ de tétrahydrofurane on ajoute 14.8 cm³ de fluorure de tétrabutylammonium en solution 1M dans le tétrahydrofurane et on porte à reflux pendant 18 heures ; la réaction étant incomplète on rajoute 9.9 cm³ de la solution de fluorure de tétrabutylammonium et on poursuit le reflux pendant 18 heures. Après refroidissement on ajoute 100 cm³ d'acétate d'éthyle et 75 cm³ d'une solution saturée en hydrogénocarbonate de sodium; la phase' organique est décantée et lavée avec 50 cm³ d'une solution saturée en chlorure de sodium, séchée sur sulfate de magnésium, filtrée et concentrée à sec sous pression réduite (2,7 kPa ; 50°C) pour donner 1.3 g de brut qui est purifié par chromatographie sous pression d'argon de 50 kPa, sur colonne de gel de silice (granulométrie 40-60 µm ; diamètre 3.5 cm), en éluant par un mélange cyclohexane-acétate d'éthyle (70/30 en volumes). Les fractions contenant le produit attendu sont réunies et évaporées sous pression réduite (2 kPa; 50°C ; le solide obtenu est repris par 20 cm³ d'éther diisopropylique, filtré sur verre fritté, lavé par 5 cm³ d'acétate d'éthyle et 20 cm³ d'éther diisopropylique puis séché (90 Pa; 45°C) pour donner 340 mg de N-(6,7-difluoro-5-phényl)-1H-indazol-3-yl)-butanamide sous forme d'un solide blanc cotonneux fondant à 224°C.

Spectre de R.M.N. ¹H (300 MHz, (CD₃)₂SO d6, δ en ppm) : 0,95 (t, J = 7,5 Hz : 3H) ; 1,66 (mt : 2H) ; 2,41 (t, J = 7 Hz : 2H) ; de 7,40 à 7,60 (mt : 5H) ; 7,76 (d large, J = 6 Hz : 1H) ; 10,53 (mf : 1H) ; de 13,00 à 13,90 (mf étalé : 1H).

EI m/z = 284 M^{+.}

m/z = 245 [M - C₄CH₆O]^{+.}

### EXEMPLE 108

### 5-Bromo-2-[[2-(triméthylsilyl)éthoxy]méthoxy]-pyridine

A 717 mg d'hydrure de sodium à 60 % dans l'huile, dans 50 cm³ de diméthylformamide on ajoute une solution de 2.6 g de 5-bromo-2-hydroxypyridine dans 80 cm³ de diméthylformamide en 30 minutes et on laisse sous agitation 1 heure à température ambiante. Le diméthylformamide est éliminé sous pression réduite et le résidu est repris par 75 cm³ d'acétate d'éthyle et 50 cm³ d'eau ; la phase organique est décantée, lavée par deux fois 50 cm³ d'eau et 50 cm³ d'une solution saturée en chlorure de sodium, séchée sur sulfate de magnésium, filtrée et concentrée à sec sous pression réduite (2 kPa ; 50°C) pour donner une huile jaune. Le brut est purifié par chromatographie sous pression d'argon de 50 kPa, sur colonne de gel de silice ( granulométrie 40-60 µm ; diamètre 3.5 cm), en éluant par un mélange cyclohexane-acétate d'éthyle (60/40 en volumes). Les fractions contenant le produit attendu sont réunies et évaporées sous pression réduite (2 kPa ; 50°C) pour donner 2.64 g de 5-bromo-2-[[2-(triméthylsilyl)éthoxy]méthyl]-pyridine sous forme d'une huile jaune qui est utilisée telle quelle pour l'essai suivant.

Spectre de R.M.N. ¹H (300 MHz, (CD₃)₂SO d6, δ en ppm) : - 0,02 (s : 9H) ; 0,89 (t, J = 8 Hz : 2H) ; 3,73 (t, J = 8 Hz : 2H) ; 5,49 (s : 2H) ; 6,89 (d large, J = 8,5 Hz : 1H) ; 7,96 (dd, J = 8,5 et 2,5 Hz : 1H) ; 8,31 (d large, J= 2,5 Hz : 1H).

DCI m/z = 304 [M+H]⁺

### N-[6-[6-[[2-(triméthylsilyl)éthoxy]méthoxyl-pyridyl-3-yl]-1-[[2-(triméthylsilyl)éthoxylméthyl]-1H-indazol-3-yl]-butanamide

A 1 g de N-[6-(4,4,5,5-tétraméthyl-[1,3,2]dioxaborolan-2-yl)-1-[[2-(triméthylsilyl)éthoxy]méthyl]-1H-indazol-3-yl]-butanamide préparé dans l'exemple 56, dans 70 cm³ de dioxane, on ajoute 1.19 g de 5-bromo-2-[[2-(triméthylsilyl)éthoxy]méthoxy]-pyridine préparé précédemment et à la solution jaune pâle on ajoute 201 mg de tétrakis-triphénylphosphine palladium et 646 mg de carbonate de sodium dans 10 cm³ d'eau et on porte au reflux 3 heures. On dilue le milieu réactionnel avec 75 cm³ d'acétate d'éthyle et 50 cm³ d'eau et on filtre le milieu réactionnel sur verre fritté garni de célite. La phase organique est décantée, lavée avec 50 cm³ d'eau, avec 50 cm³ d'une solution saturée en chlorure de sodium, séchée sur sulfate de magnésium, filtrée et concentrée sous pression réduite (2 kPa; 50°C). Le brut obtenu est purifié par chromatographie sous pression d'argon de 50 kPa, sur colonne de gel de silice (granulométrie 40-60 µm ; diamètre 4.5 cm), en éluant par un mélange cyclohexane-acétate d'éthyle (70/30 en volumes). Les fractions contenant le produit attendu sont réunies et évaporées sous pression réduite (2 kPa ; 50°C) pour donner 0.45 g de N-[6-[6-[[2-(triméthylsilyl)éthoxy]méthoxy]-pyridyl-3-yl]-1[[2(triméthylsilyl)éthoxy]méthyl] -1H-indazol-3-yl]-butanamide sous forme d'une laque orange.

Spectre de R.M.N. ¹H (300 MHz, (CD₃)₂SO d6, δ en ppm) : - 0,09 (s : 9H) ; 0,00 (s : 9H) ; 0,83 (t, J = 8 Hz : 2H) ; 0,93 (t, J = 8 Hz : 2H) ; 0,98 (t, J = 7,5 Hz : 3H) ; 1,69 (mt : 2H) ; 2,41 (t, J = 7 Hz : 2H) ; 3,56 (t, J = 8 Hz : 2H) ; 3,79 (t, J = 8 Hz : 2H) ; 5,59 (s : 2H) ; 5,74 (s large : 2H) ; 7,02 (d, J = 8,5 Hz : 1H) ; 7,47 (d large, J = 8,5 Hz : 1H) ; 7,92 (d, J = 8,5 Hz : 1H) ; 8,00 (s large : 1H) ; 8,18 (dd, J = 8,5 et 2,5 Hz : 1H) ; 8,61 (d, J = 2,5 Hz : 1H) ; 10,53 (mf : 1H).

EI m/z = 556 M^{+.}

m/z = 439 [M - OCH₂CH₂Si(CH₃)₃]⁺

m/z = 73 [Si(CH₃)₃]⁺

### N-[6-(6-hydroxy-pyridin-3-yl)-1H-indazol-3-yl]-butanamide

A 0.73 g de N-[6-[6-[[2-(triméthylsilyl)éthoxy]méthoxy]-pyridyl-3-yl]-1[[2(triméthylsilyl)éthoxy]méthyl] -1H-indazol-3-yl]-butanamide précédemment décrit, dans 15 cm³ de tétrahydrofurane on ajoute 15.7 cm³ de fluorure de tétrabutylammonium en solution 1M dans le tétrahydrofurane et on porte à reflux pendant 18 heures ; la réaction étant incomplète on poursuit le reflux pendant 18 heures. Après refroidissement on ajoute 60 cm³ d'acétate d'éthyle et la phase organique est lavée avec 30 cm³ d'une solution saturée en hydrogénocarbonate de sodium puis avec 2 fois 30 cm³ d'eau et avec 30 cm³ d'une solution saturée en chlorure de sodium. La phase organique est décantée, séchée sur sulfate de magnésium, filtrée et concentrée à sec sous pression réduite (2 kPa; 50°C) pour donner 1.3 g de brut qui est purifié par chromatographie sous pression d'argon de 50 kPa, sur colonne de gel de silice (granulométrie 40-60 µm ; diamètre 3 cm), en éluant par un gradient chlorure de méthylène-méthanol (95/5 à 90/10 en volumes). Les fractions contenant le produit attendu sont réunies et évaporées sous pression réduite ( 2 kPa ; 50°C) ; pour donner 270 mg de produit encore impur qui est repurifié par HPLC (colonne X Terra ; C₁₈, 5µm ; longueur 100 mm, diamètre 30 mm, éluant : méthanol-eau (70/30 en volumes) contenant 0.05% d'acide trifluoroacétique ; débit 20 cm³/mn). Après concentration des fractions contenant l'attendu et séchage (90 Pa ; 45°C) on obtient 40 mg de N-[6-(6-hydroxy-pyridin-3-yl)- 1H-indazol-3-yl]-butanamide sous forme d'un solide fondant à plus de 260°C.

Spectre de R.M.N. ¹H (300 MHz, (CD₃)₂SO d6, δ en ppm) : 0,98 (t, J = 7,5 Hz : 3H) ; 1,68 (mt : 2H) ; 2,39 (t, J = 7 Hz : 2H) ; 6,47 (d, J = 9,5 Hz : 1H) ; 7,25 (d large, J = 9 Hz : 1H) ; 7,52 (s large : 1H) ; 7,77 (d, J = 2,5 Hz : 1H) ; 7,81 (d, J = 9 Hz : 1H) ; 7,90 (dd, J = 9,5 et 2,5 Hz : 1H) ; 10,30 (s large : 1H) ; 11,83 (mf : 1H) ; 12,62 (mf : 1H).

EI m/z = 296 M^{+.}

m/z = 226 [M-C₄CH₆O]^{+.}

### EXEMPLE 109

### N-[6-(2,2-diphényl-benzo[1,3]dioxol-5-yl)-1-[[2-(triméthylsilyl)éthoxylméthoxy]-1H-indazol-3-yl]-butanamide

A 1.2 g de N-[6-(4,4,5,5-tétraméthyl-[1,3,2]dioxaborolan-2-yl)-1-[[2-(triméthylsilyl)éthoxy]méthyl]-1H-indazol-3-yl]-butanamide préparé dans l'exemple 56, dans 80 cm³ de dioxane, on ajoute 1.38 g de 5-bromo-2,2-diphényl-1,3-benzodioxole préparé selon le brevet européen EP 303172A2 et à la solution jaune pâle on ajoute 242 mg de tétrakis-triphénylphosphine palladium et 688 mg de carbonate de sodium dans 10 cm³ d'eau et on porte au reflux 4 heures. On dilue le milieu réactionnel avec 50 cm³ d'acétate d'éthyle et 50 cm³ d'eau puis on filtre sur verre fritté garni de célite. Le filtrat est décanté et la phase organique est lavée avec 50 cm³ d'eau et avec 50 cm³ d'une solution saturée en chlorure de sodium, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice (granulométrie 40-60 µm ;-diamètre 4.5 cm), en éluant par un mélange cyclohexane-acétate d'éthyle (70/30 en volumes). Les fractions contenant le produit attendu sont réunies et évaporées sous pression réduite (2 kPa ; 50°C) ; pour donner 840 mg de N-[6-(2,2-diphényl-1,3-benzodioxole-5-yl)-1-[[2-(triméthylsilyl)éthoxy]méthoxy]-1H-indazol-3-yl]-butanamide sous forme d'un solide orange collant.

EI m/z = 605 M^{+.}

m/z = 488 [M - OCH₂CH₂Si(CH₃)₃]⁺

### N-[6-(3,4-dihydroxy-phényl)-1H-indazol-3-yl]-butanamide, trifluoroacétate

A 0.8 g de N-[6-(2,2-diphényl-1,3-benzodioxole-5-yl)-1-[[2-(triméthylsilyl)éthoxy]méthoxy]-1H-indazol-3-yl]-butanamide préparé précédemment, on ajoute 10 cm³ d'iodure de triméthylsilyle et on chauffe au reflux 3 heures puis on ajoute avec précaution 50 cm³ de méthanol et on poursuit le reflux 15 minutes. Le milieu réactionnel est concentré à sec sous pression réduite (2 kPa ; 50°C) et repris avec 50 cm³ d'acétate d'éthyle et 100 cm³ d'une solution de thiosulfate de sodium à 10% ; l'insoluble formé est éliminé par filtration et le filtrat est décanté. La phase organique est séchée sur sulfate de magnésium, filtrée et concentrée à sec sous pression réduite. Le brut est purifié par chromatographie sous pression d'argon de 50 kPa, sur colonne de gel de silice (granulométrie 40-60 µm ; diamètre 3 cm), en éluant par un gradient chlorure de méthylène-méthanol ammoniacal 7N (97/3 en volumes). Les fractions contenant le produit attendu sont réunies et évaporées sous pression réduite (2 kPa ; 50°C) ; pour donner 150 mg de produit encore impur qui est repurifié par HPLC (colonne X Terra ; C₁₈, 5µm ; longueur 100 mm, diamètre 30 mm, éluant :gradient acétonitrile-eau (15/85 à 45/55 en volumes) contenant 0.05% d'acide trifluoroacétique ; débit 20 cm³/mn). Après concentration des fractions contenant l'attendu et séchage (90 Pa ; 45°C) on obtient 30 mg de N-[6-(3,4-dihydroxy-phényl)-1H-indazol-3-yl]-butanamide, trifluoroacétate, sous forme d'une poudre marron fondant à 236°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,40 (t, J = 7 Hz : 2H) ; 6,85 (d, J = 8 Hz : 1H) ; 7,01 (dd, J = 8 et 2 Hz : 1H) ; 7,10 (d, J = 2 Hz : 1H) ; 7,26 (d large, J = 9 Hz : 1H) ; 7,46 (s large : 1H) ; 7,79 (d, J = 9 Hz : 1H) ; de 8,80 à 9,30 (mf étalé : 2H) ; 10,29 (s large : 1H) ; 12,57 (mf : 1H).

EI m/z = 311 M^{+.}

m/z = 241 [M-C₄CH₆O]^{+.}

### EXEMPLE 110

### N-[6-(1,3-benzodioxol-5-yl)-1-[[2-(triméthylsilyl)éthoxy]méthoxy]-1H-indazol-3-yl)-butanamide

A 1g de N-[6-chloro-1-[[2-(triméthylsilyl)éthoxy]méthyl]-1H-indazol-3-yl]-butanamide précédemment décrit , dans 30 cm³ de dioxanne, on additionne 677 g d'acide 1,3-benzodioxol-5-yl-boronique, 1.24g de fluorure de césium puis 13.5 mg d'acétate de palladium et enfm 31 mg de biphényl-2-dicyclohexylphosphine-2-(N,N-diméthylamino). On chauffe ensuite vers 94°C pendant 15 heures puis on laisse ensuite revenir à 19°C puis on filtre le milieu réactionnel sur verre fritté garni de célite. On rince avec de l'acétate d'éthyle puis on sèche la phase organique sur sulfate de magnésium, filtre et évapore le filtrat sous pression réduite (2 kPa ; 45 °C). Le résidu est purifié par chromatographie sous pression d'argon de 50 kPa, sur colonne de gel de silice (granulométrie 15-40 µm ; diamètre 3 cm), en éluant par un mélange cyclohexane-acétate d'éthyle (80/20 en volumes) ; les fractions contenant le produit attendu, sont réunies et évaporées sous pression réduite (2 kPa ; 50°C). On obtient 620 mg de N-[6-(1,3-benzodioxol-5-yl)-1-[[2-(triméthylsilyl)éthoxy]méthoxy]-1H-indazol-3-yl]-butanamide sous forme d'une huile collante jaune clair

Spectre de R.M.N. ¹H (300 MHz, (CD₃)₂SO d6, 8 en ppm) : - 0,09 (s : 9H) ; 0,83 (t, J = 8 Hz : 2H) ; 0,98 (t, J = 7,5 Hz : 3H) ; 1,68 (mt : 2H) ; 2,41 (t, J = 7 Hz : 2H) ; 3,56 (t, J = 8 Hz : 2H) ; 5,74 (s large : 2H) ; 6,10 (s : 2H) ; 7,05 (d, J = 8 Hz : 1H) ; 7,27 (dd, J = 8 et 2 Hz : 1H) ; 7,37 (d, J = 2 Hz : 1H) ; 7,42 (dd large, J = 9 et 1,5 Hz : 1H) ; 7,86 (d, J = 9 Hz : 1H) ; 7,89 (s large : 1H) ; 10,45 (mf : 1H).

EI m/z = 493 M^{+.}

m/z = 336 [M - OCH₂CH₂Si(CH₃)₃]⁺

m/z = 325 [M - C₆H₁₂OSi]^{+.}

### N-[6-(1,3-benzodioxol-5-yl)-1H-indazol-3-yl]-butanamide

A 600 mg de N-[6-(benzodioxol-5-yl)-1-[[2-(triméthylsilyl)éthoxy]méthoxy]-1H-indazol-3-yl]-butanamide précédemment décrit dans 12 cm³ de tétrahydrofuranne, on ajoute 2 cm³ de fluorure de tétrabutylammonium en solution 1M dans le tétrahydrofuranne. Le milieu est ensuite porté à 67°C pendant 16 heures. On laisse ensuite revenir à 19°C et on ajoute 60 cm³ d'acétate d'éthyle puis on lave ensuite avec 30 cm³ d'une solution aqueuse saturée en hydrogériocarbonate de sodium puis avec 2 fois 30 cm³ d'eau distillée et enfin avec 30 cm³ d'une solution aqueuse saturée en chlorure de sodium. On sèche la phase organique sur sulfate de magnésium, filtre sur verre fritté et évapore sous pression réduite (2 kPa ; 45°C). Le résidu obtenu est purifié par chromatographie sous pression d'argon de 50 kPa, sur colonne de gel de silice (granulométrie 15-40 µm ; diamètre 3 cm), en éluant par un mélange cyclohexane-acétate d'éthyle (60/40 en volumes) et en recueillant des fractions de 15 cm³. Les fractions contenant le produit attendu, sont réunies et évaporées sous pression réduite (2 kPa ; 50°C). Après séchage (90 Pa ; 50 °C), on obtient 260 mg de N-[6-(1,3-benzodioxol-5-yl)-1H-indazol-3-yl]-butanamide sous forme d'un solide blanc fondant à 240 °C.

Spectre de R.M.N. ¹H (300 MHz, (CD₃)₂SO d6, δ en ppm) : 0,98 (t, J = 7,5 Hz : 3H) ; 1,68 (mt : 2H) ; 2,40 (t, J = 7 Hz : 2H) ; 6,09 (s : 2H) ; 7,03 (d, J = 8 Hz : 1H) ; 7,21 (dd, J = 8 et 2 Hz: 1H) ; 7,30 (dd large, J = 9 et 1,5 Hz : 1H) ; 7,31 (d, J = 2 Hz : 1H) ; 7,55 (s large : 1H) ; 7,82 (d, J = 9 Hz : 1H) ; 10,31 (s large : 1H) ; 12,63 (s large : 1H).

EI m/z = 323 M^{+.}

m/z = 253 [M-C₄CH₆O]^{+.}

### EXEMPLE 111

### N-[7-fluoro-5-nitro-6-[2-(phényléthyl)amino]-1H-indazol-3-yl]-butanamide

A une solution de 500 mg de N-(6,7-difluoro-5-nitro-1H-indazol-3-yl)-butanamide préparé dans l'exemple 106 dans 10 cm³ de diméthylsulfoxide, on ajoute 1.11 cm³ de phénéthylamine et on chauffe au reflux 1 heure. Le milieu réactionnel est repris par 50 cm³ d'acétate d'éthyle et la phase organique est lavée avec 4 fois 35 cm³ d'une solution saturée en chlorure de sodium. La phase organique est décantée, séchée sur sulfate de magnésium, filtrée et concentrée à sec sous pression réduite pour donner 1.5 g d' huile. Le brut est purifié par chromatographie sous pression d'argon de 50 kPa, sur colonne de gel de silice (granulométrie 15-40 µm ; diamètre 3 cm), en éluant par un mélange cyclohexane-acétate d'éthyle (70/30 en volumes). Les fractions contenant le produit attendu, sont réunies et évaporées sous pression réduite (2 kPa ; 50°C) et le résidu est repris dans 30 cm³ d'éther diéthylique, filtré sur verre fritté, lavé par 2 fois 20 cm³ d'éther diéthylique puis séché (90 Pa ; 50 °C) pour donner 360 mg de N-[7-fluoro-5-nitro-6-[(phényléthyl)amino]-1H-indazol-3-yl]-butanamide sous forme de cristaux marron fondant à 212 °C.

Spectre de R.M.N. ¹H (300 MHz, (CD₃)₂SO d6, δ en ppm) : 0,96 (t, J = 7,5 Hz : 3H) ; 1,67 (mt : 2H) ; 2,42 (t, J = 7 Hz : 2H) ; 2,93 (t, J = 7 Hz : 2H) ; 3,76 (mt : 2H) ; 7,03 (mt : 1H) ; de 7,15 à 7,40 (mt : 5H) ; 8,85 (s : 1H) ; 10,73 (s large : 1H) ; 13,10 (mf : 1H).

EI m/z = 385 M^{+.}

m/z = 294 [M - CH₂C₆H₅]⁺

m/z = 224 [294 - C₄CH₆O]⁺

### EXEMPLE 112

### N-(7-fluoro-5-nitro=6-morpholino-1H-indazol-3-yl)-butanamide

On opère comme à l'exemple 110, à partir de 500 mg de N-(6,7-difluoro-5-nitro-1H-indazol-3-yl)-butanamide préparé dans l'exemple 106, de 10 cm³ de diméthylsulfoxide, de 0.77 cm³ de morpholine et on chauffe au reflux 1 heure. Le milieu réactionnel est repris par 75 cm³ d'acétate d'éthyle et la phase organique est lavée avec 2 fois 75 cm³ d'eau et 50 cm³ d'une solution saturée en chlorure de sodium. La phase organique est décantée, séchée sur sulfate de magnésium, filtrée et concentrée à sec sous pression réduite pour donner 1 g de solide marron. Le brut est purifié par chromatographie sous pression d'argon de 50 kPa, sur colonne de gel de silice (granulométrie 15-40 µm ; diamètre 3 cm), en éluant par un mélange cyclohexane-acétate d'éthyle (60/40 en volumes). Les fractions contenant le produit attendu, sont réunies et évaporées sous pression réduite (2 kPa ; 50°C) et le résidu est repris dans 30 cm³ d'éther diéthylique, filtré sur verre fritté, lavé par 2 fois 20 cm³ d'éther diéthylique puis séché (90 Pa ; 50 °C) pour donner 280 mg de N-(7-fluoro-5-nitro-6-morpholino-1H-indazol-3-yl)-butanamide sous forme de cristaux marron fondant à 250 °C

Spectre de R.M.N. ¹H (300 MHz, (CD₃)₂SO d6, δ en ppm) : 0,96 (t, J = 7,5 Hz : 3H) ; 1,67 (mt : 2H) ; 2,42 (t, J = 7 Hz : 2H) ; 3,16 (mt : 4H) ; 3,69 (t, J = 4 Hz : 4H) ; 8,30 (s large : 1H) ; 10,75 (mf : 1H) ; 13,68 (mf : 1H).

EI m/z = 351 M^{+.}

m/z = 334 [M-OH]⁺

### EXEMPLE 113

### N-(7-fluoro-5-amino-6-morpholino-1H-indazol-3-yl)-butanamide

A une solution de 1.1 g de N-(7-fluoro-5-nitro-1H-indazol-3-yl)-butanamide préparé dans l'exemple 112, dans 50 cm³ de méthanol, on ajoute 200 mg de palladium à 3% sur charbon et 1 g de formiate d'ammonium et on laisse agiter à température ambiante pendant 18 heures. Le milieu réactionnel est filtré sur verre fritté garni de célite et concentré à sec sous pression réduite (2 kPa; 50°C) puis purifié par chromatographie sous pression d'argon de 50 kPa, sur colonne de gel de silice ( granulométrie 15-40 µm ; diamètre 3 cm), en éluant par un mélange chlorure de méthylène-méthanol (97.5/2.5 en volumes). Les fractions contenant le produit attendu, sont réunies et évaporées sous pression réduite (2 kPa ; 50°C) et le résidu est repris dans 20 cm³ d'acétate d'éthyle, filtré sur verre fritté, lavé avec 2 fois 5 cm³ d'acétate d'éthyle et avec 10 cm³ d'éther diéthylique puis séché (90 Pa ; 50 °C) pour donner 306 mg de N-(7-fluoro-5-amino-6-morpholino-1H-indazol-3-yl)-butanamide sous forme de cristaux blanc fondant à 180 °C.

Spectre de R.M.N. ¹H (300 MHz, (CD₃)₂SO d6, δ en ppm) : 0,97 (t, J = 7,5 Hz : 3H) ; 1,67 (mt : 2H) ; 2,34 (t large, J = 7 Hz : 2H) ; 3,04 (mf étalé : 4H) ; 3,77 (mf : 4H) ; 4,95 (s large : 2H) ; 6,55 (s : 1H) ; 10,03 (mf : 1H) ; 13,57 (mf : 1H).

EI m/z = 321 M^{+.}

m/z = 306 [M-CH₃]⁺

### EXEMPLE 114

### N-(5-bromo-7-fluoro-6-morpholino-1H-indazol-3yl)-butanamide

A une suspension refroidie à 5°C de 900 mg de N-(5-amino-7-fluoro-6-morpholino-1H-indazol-3-yl)-butanamide préparé comme précédemment, dans 9 cm³ d'eau et 0.94 cm³ d'acide bromhydrique à 48% on ajoute goutte à goutte une solution de 213 mg de nitrite de sodium dans 9 cm³ d'eau et on agite à 0°C. Cette suspension est additionnée par petites portions à une solution au reflux de 482 mg de bromure cuivreux, 4.5 cm³ d'eau et 4.5 cm³ d'acide bromhydrique à 48%. Le reflux est maintenu pendant 45 minutes puis le milieu réactionnel est filtré sur verre fritté puis dissout dans 75 cm³ de tetrahydrofurane et purifié par chromatographie sous pression d'argon de 50 kPa, sur colonne de gel de silice (granulométrie 15-40 µm ; diamètre 3 cm), en éluant par un mélange cyclohexane-acétate d'éthyle (80/20 en volumes). Les fractions contenant le produit attendu sont réunies et évaporées sous pression réduite ( 2 kPa ; 50°C) et le résidu est repris dans 10 cm³ d'acétate d'éthyle, filtré et lavé par 2 fois 5 cm³ d'acétate d'éthyle et par 20 cm³ d'éther diisopropylique. Après filtration et séchage 90 Pa ; 45°C) on obtient 60 mg de N-(5-bromo-7-fluoro-6-morpholino-1H-indazol-3-yl)-butanamide sous forme de crisaux blancs fondant à 240°C.

Spectre de R.M.N. ¹H (300 MHz, (CD₃)₂SO d6, δ en ppm) : 0,96 (t, J = 7,5 Hz : 3H) ; 1,66 (mt : 2H) ; 2,38 (t, J = 7 Hz : 2H) ; 3,16 (mf : 4H) ; 3,76 (mf : 4H) ; 7,96 (s : 1H) ; 10,48 (mf : 1H) ; 13,20 (mf : 1H).

EI m/z = 384 M^{+.}

m/z = 314 [M-C₄CH₆O]^{+.}

### EXEMPLE 115

### N-[7-fluoro-6-(trifluorométhyl)-1H-indazol-3-yl]-butanamide

A 2.1 g de 7-fluoro-6-(trifluorométhyl)-1H-indazole-3-amine préparé comme dans le brevet WO0222608, dans 20 cm³ de pyridine, on ajoute 1.0 cm³ de chlorure de butyryle après avoir refroidi vers 3°C puis on laisse à température ambiante pendant 76 heures. Le milieu réactionnel est concentré sous pression réduite (2 kPa ; 40°C) et le résidu est repris par 50 cm³ d'acétate d'éthyle et par 20 cm³ d'eau. La phase organique est lavée avec 2 fois 20 cm³ d'eau distillée puis avec 20 cm³ d'une solution aqueuse saturée en chlorure de sodium. Après séchage sur sulfate de magnésium, filtration et concentration sous pression réduite (2 kPa ; 40°C), le résidu obtenu est purifié par chromatographie sous pression d'argon de 50 kPa, sur une colonne de gel de silice (granulométrie 40-60 µm ; diamètre 4 cm), en éluant par un mélange cyclohexane-acétate d'éthyle (60/40 en volumes). Les fractions contenant le produit attendu sont réunies puis évaporées sous pression réduite (2 kPa ; 40°C) ; on obtient après séchage (90 Pa; 40°C), 875 mg de N-[7-fluoro-6-(trifluorométhyl)-1H-indazol-3-yl)-butanamide sous forme de solide rose fondant à 220°-222°C.

Spectre de R.M.N. ¹H (300 MHz, (CD₃)₂SO d6, δ en ppm) : 0,98 (t, J = 7,5 Hz : 3H) ; 1,69 (mt : 2H) ; 2,42 (t, J = 7 Hz : 2H) ; 7,31 (dd, J = 8,5 et 6 Hz : 1H) ; 7,82 (d large, J = 8,5 Hz : 1H) ; 10,59 (mf : 1H) ; de 13,50 à 14,20 (mf étalé : 1H).

EI m/z = 289 M^{+.}

m/z = 270 [M-F]⁺

m/z = 219 [M - C₄CH₆O]^{+.}

### EXEMPLE 116

### 6-bromo-4,5,7-trifluoro-1H-indazole-3-amine:

A 2.0 g de 4-bromo-2,3,5,6-tetrafluorobenzonitrile dans 40 cm³ d'éthanol absolu, on additionne 1.14 cm³ d'hydrazine monohydratée. On chauffe au reflux pendant 18 heures puis on ajoute 30 cm³ d'eau distillée et le milieu réactionnel est concentré sous pression réduite (2 kPa ; 50°C). Le résidu est repris par 100 cm³ d'acétate d'éthyle et 10 cm³ d'eau et la phase organique est décantée, séchée sur sulfate de magnésium, filtrée et concentrée à sec sous pression réduite (2 kPa ; 50°C) ; on obtient après séchage (90 Pa; 40°C), 2.1 g de 6-bromo-4,5,7-trifluoro-1H-indazole-3-amine sous forme d'un solide beige.

Spectre de R.M.N. ¹H (300 MHz, (CD₃)₂SO d6, δ en ppm) : 5,52 (s : 2H) ; de 12,10 à 12,90 (mf étalé : 1H).

EI m/z = 265 M^{+.}

m/z = 236 [M - HN₂]⁺

m/z = 186 [M-Br]⁺

### N-(6-bromo-4,5,7-trifluoro-1H-indazol-3-yl)-butanamide

A 2.1 g de 6-bromo-4,5,7-trifluoro-1H-indazole-3-amine préparé précédemment, dans 20 cm³ de pyridine, on ajoute 0.82 cm³ de chlorure de butyryle après avoir refroidi vers 3°C puis on laisse à température ambiante pendant 76 heures. Le milieu réactionnel est concentré sous pression réduite (2 kPa ; 40°C) et le résidu est repris par 100 cm³ d'acétate d'éthyle, 100 cm³ de tétrahydrofurane et par 40 cm³ d'eau. La phase organique est lavée avec 2 fois 40 cm³ d'eau distillée puis avec 40 cm³ d'une solution aqueuse saturée en chlorure de sodium. Après séchage sur sulfate de magnésium, filtration et concentration sous pression réduite (2 kPa ; 40°C), le résidu obtenu est purifié par chromatographie sous pression d'argon de 50 kPa, sur une colonne de gel de silice (granulométrie 40-60 µm ; diamètre 4 cm), en éluant par un mélange cyclohexane-acétate d'éthyle (80/20 en volumes). Les fractions contenant le produit attendu sont réunies puis évaporées sous pression réduite (2 kPa ; 40°C) ; on obtient après séchage (90 Pa ; 40°C), 0.53 g de N-(6-bromo-4,5,7-trifluoro-1H-indazol-3-yl)-butanamide sous forme de solide rose fondant à 255°-257°C.

Spectre de R.M.N. ¹H (300 MHz, (CD₃)₂SO d6, δ en ppm): 0,97 (t, J = 7,5 Hz : 3H) ; 1,66 (mt : 2H) ; 2,36 (t, J = 7 Hz : 2H) ; 10,26 (mf : 1H) ; de 13,50 à 14,40 (mf étalé : 1H).

EI m/z = 335 M⁺.

m/z = 265 [M - C₄CH₆O]⁺.

### EXEMPLE 117

### N-[6-(6-amino-pyridin-3-yl)-1-[[2-(triméthylsilyl)éthoxy]méthyl]-1H-indazol-3-yl]-butanamide

A 1.0 g de N-[6-(4,4,5,5-tétraméthyl-[1,3,2]dioxaborolan-2-yl)-1-[[2-(triméthylsilyl)éthoxy]méthyl]-1H-indazol-3-yl]-butanamide préparé dans l'exemple 56, dans 50 cm³ de dioxane, on ajoute 0.45 g de 2-amino-5-bromopyridine et à la solution jaune pâle on ajoute 142 mg de dichloro[1,1'-bis(diphenylphosphino)ferrocene]palladium, dichlorométhane et 646 mg de carbonate de sodium dans 10 cm³ d'eau et on porte au reflux 2 heures. On dilue le milieu réactionnel avec 50 cm³ d'acétate d'éthyle et 50 cm³ d'eau puis on filtre sur verre fritté garni de célite. Le filtrat est décanté et la phase aqueuse est lavée avec 2 fois 50 cm³ d'une solution saturée en chlorure de sodium, séchée sur sulfate de magnésium, filtrée et concentrée à sec sous pression réduite (2 kPa ; 50°C). Le brut obtenu est purifié par chromatographie sous pression d'argon de 50 kPa, sur colonne de gel de silice (granulométrie 40-60 µm ; diamètre 3 cm), en éluant par un mélange gradient cyclohexane-acétate d'éthyle (70/30 à 50/50 en volumes). Les fractions contenant le produit attendu sont réunies et évaporées sous pression réduite (2 kPa ; 50°C) pour donner 0.53 mg de N-[6-(6-amino-pyridin-3-yl)-1-[[2-(triméthylsilyl)éthoxy]méthyl]-1H-indazol-3-yl]-butanamide, sous forme de solide fondant à 138°C

Spectre de R.M.N. ¹H (300 MHz, (CD₃)₂SO d6, δ en ppm) : - 0,09 (s : 9H) ; 0,83 (t, J = 8 Hz : 2H) ; 0,97 (t, J = 7,5 Hz : 3H) ; 1,67 (mt : 2H) ; 2,39 (t, J = 7 Hz : 2H) ; 3,55 (t, J = 8 Hz : 2H) ; 5,70 (s : 2H) ; 6,12 (s : 2H) ; 6,57 (d, J = 9 Hz : 1H) ; 7,37 (dd, J = 8,5 et 1,5 Hz : 1H) ; de 7,80 à 7,90 (mt : 3H) ; 8,37 (d, J = 2 Hz : 1H) ; 10,43 (mf : 1H).

EI m/z = 425 M^{+.}

m/z = 308 [M - OCH₂CH₂Si(CH₃)₃]⁺

m/z = 297 [M-C₆H₁₂OSi]^{+.}

### N-[6-(6-amino-pyridin-3-yl)-1H-indazol-3-yl]-butanamide, difluoroacétate

A 0.8 g de N-[6-(6-amino-pyridin-3-yl)-1-[[2-(triméthylsilyl)éthoxy]méthyl]-1H-indazol-3-yl]-butanamide préparé précédemment, dans 13 cm³ de tetrahydrofurane, on ajoute 3.8 cm³ de fluorure de tétrabutylamonium en solution 1M dans le tétrahydrofurane et on chauffe au reflux 6 heures puis on ajoute 50 cm³ d'acétate d'éthyle et 25 cm³ d'une solution saturée en hydrogénocarbonate de sodium. La phase organique est décantée, lavée par 25 cm³ d'eau, séchée sur sulfate de magnésium, filtrée et concentrée à sec sous pression réduite. Le brut est purifié par HPLC (colonne X Terra; C₁₈, 5µm ; longueur 50 mm, diamètre 21 mm, éluant :gradient acétonitrile-eau (5/95 à 95/5 en volumes) contenant 0.05% d'acide trifluoroacétique.; -débit 20 cm³/mn). Après concentration des fractions contenant l'attendu on obtient un solide qui est repris dans 10 cm³ d'éther diisopropylique et 2 cm³ d'acétonitrile, filtré, lavé par 5 cm³ d'éther diisopropylique et séché (90 Pa ; 45°C) pour donner 18 mg de N-[6-(6-amino-pyridin-3-yl)-1H-indazol-3-y]-butanamide difluoroacétate, sous forme de cristaux blancs fondant à 230-235°C.

Spectre de R.M.N. ¹H (300 MHz, (CD₃)₂SO d6, δ en ppm) : 0,99 (t, J = 7,5 Hz : 3H) ; 1,69 (mt : 2H) ; 2,40 (t, J = 7 Hz : 2H) ; 7,05 (d, J = 9,5 Hz : 1H) ; 7,33 (dd, J = 8,5 et 1,5 Hz : 1H) ; 7,65 (s large : 1H) ; 7,79 (mf : 2H) ; 7,90 (d, J = 8,5 Hz : 1H) ; 8,31 (dd, J = 9,5 et 1,5 Hz : 1H) ; 8,36 (d, J = 1,5 Hz : 1H) ; 10,37 (s large : 1H) ; 12,79 (mf : 1H).

EI m/z = 295 M^{+.}

m/z = 225 [M - C₄CH₆O]^{+.}

m/z = 43 [C₃H₇]⁺

### EXEMPLE 118

### 2-chloro-N-(6,7-difluoro-1H-indazol-3-yl)-acétamide

A 5 g de 6,7-difluoro-1H-indazole-3-amine, préparé dans l'exemple 40, dans 300 cm³ de toluène on ajoute 5.0 g d'anhydride chloracétique et on chauffe au reflux pendant 18 heures. Le précipité apparu est concentré à sec sous pression réduite (2 kPa ; 50°C) puis le résidu est purifié par chromatographie sous pression d'argon de 50 kPa, sur colonne de gel de silice (granulométrie 40-60 µm ; diamètre 4.5 cm), en éluant par un mélange dichlorométhane-méthanol (98/2 en volumes). Les fractions contenant le produit attendu sont réunies et évaporées sous pression réduite (2 kPa ; 50°C) puis séchées (90 Pa ; 45°C) pour donner 8.5 g de 2-chloro-N-(6,7-difluoro-1H-indazol-3-yl)-acétamide, sous forme d'une masse cristalline crème.

Spectre de R.M.N. ¹H (400 MHz, (CD₃)₂SO d6, à une température'de 353 K, δ en ppm) : 4,37 (s : 2H) ; 7,11 (ddd, J = 8,5 - 7,5 et 5 Hz : 1H) ; 7,67 (dd large, J = 7,5 et 3 Hz : 1H) ; 10,65 (s large : 1H) ; 13,30 (mf : 1H).

EI m/z = 245 M^{+.}

m/z = 169 [M-C₂ROCl]^{+.}

m/z = 140 [169-HN₂]⁺

### N-(6,7-difluoro-1H-indazol-3-yl)-1-pipéridineacétamide

On opère comme à l'exemple 75 à partir de 8.5 g de 2-chloro-N-(6,7-difluoro-1H-indazol-3-yl)-acétamide de 200 cm³ d'acétonitrile, de 8.8 cm³ de pipéridine. Le milieu réactionnel est chauffé au reflux pendant 1 heure puis le précipité apparu est filtré sur verre fritté et les cristaux repris dans 200 cm³ d'acétate d'éthyle et 100 cm³ d'eau. La phase organique est décantée, séchée sur sulfate de magnésium, filtrée et concentrée à sec sous pression réduite (2 kPa; 50°C). Le brut est purifié par chromatographie sous pression d'argon de 50 kPa, sur colonne de gel de silice ( granulométrie 40-60 µm ; diamètre 3.5 cm), en éluant par de l'acétate d'éthyle. Les fractions contenant le produit attendu, sont réunies et évaporées sous pression réduite (2 kPa ; 50°C) ; le résidu est recristallisé dans un mélange de 50 cm³ de cyclohexane et de 16 cm³ d'acétate d'éthyle , filtré et séché (90 Pa ; 45°C) pour donner 3.2 g de N-(6,7-difluoro-1H-indazol-3-yl)-1-pipéridineacétamide sous forme de cristaux blancs fondant à 158°C

Spectre de R.M.N. ¹H (300 MHz, (CD₃)₂SO d6, δ en ppm) : 1,42 (mt : 2H) ; 1,59 (mt : 4H) ; 2,52 (mt : 4H) ; 3,19 (s : 2H) ; 7,13 (ddd, J = 10,5 - 9 et 7 Hz : 1H) ; 7,66 (dd large, J = 9 et 4,5 Hz : 1H) ; 10,14 (mf : 1H) ; 13,42 (mf : 1H).

DCI m/z = 295 [M+H]⁺

Les compositions pharmaceutiques selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

L'invention a pour objet les composés et leur utilisation d'aminoindazoles de formule (I) et leurs sels pharmaceutiquement acceptables pour la préparation de compositions pharmaceutiques destinées à prévenir et traiter les maladies pouvant résulter d'une activité anormale de kinases comme par exemple celles impliquées dans les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer,

Comme activité anormale de kinase on peut citer par exemple celle de la PI3K, AkT, GSK3béta, des CDK's ...

En thérapeutique humaine, les composés selon l'invention sont particulièrement utiles pour le traitement et/ou la prévention des maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer.

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les exemples suivants illustrent des compositions selon invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

La présente invention concerne également la méthode de prévention et de traitement des maladies dans lesquelles un phosphorylation de la protéine Tau est impliquée par administration d'un composé de formule (I) et ses sels pharmaceutiquement acceptables.

## Revendications

1. Composés de formule (I) 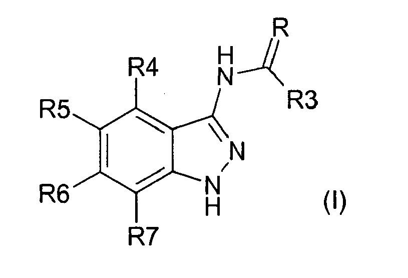 dans laquelle
R est soit O, S ou NH
R3 est un radical (1-6C)alkyle, aryle(1-6C)alkyle, hétéroaryle, hétéroaryle(1-6C)alkyle, aryle ou hétéroaryle fusionné à un cycloalkyle (1-10C), hétérocycle, cycloalkyle, adamantyle, polycycloalkyles, alkényle, alkynyle ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formule, trifluorométhyle, trifluorométhylsulfanyle, trifluorométhoxy ;
R4, R5, R6 et R7 sont indépendamment l'un de l'autre choisis parmi les radicaux suivant hydrogène, halogène, CN, N02, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle, adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR1OR11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
leurs racémiques, énantiomères, diastéréoisomères et leurs mélanges, leurs tautomères et leurs sels pharmaceutiquement acceptables à l'exception de 3-acetamido-indazole, N-(1H-indazol-3-yl)-butanamide, N-(1H-indazol-3-yl)-phenylacetamide, N-(1H-indazol-3-yl)-benzhydrylacetamide, 5-amino-3-acetamido-indazole, N-(6-chloro-1H-indazol-3-yl)-2,2,2-trifluoroacetamide, N-(6-chloro-1H-indazol-3-yl)-2-furancarboxamide, N-(6-chloro-1H-indazol-3-yl)-2-thiophenecarboxamide, N-(5-nitro-1H-indazol-3-yl)-acetamide.

2. Composés de formule (I) selon la revendication 1
dans laquelle
R est soit O, S ou NH
R3 est un radical (1-6C)alkyle, aryle(1-6C)alkyle, hétéroaryle, hétéroaryle(1-6C)alkyle, cycloalkyle, alkényle, alkynyle ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R4 et R7 sont hydrogène ;
R5, R6 sont indépendamment l'un de l'autre choisis parmi les radicaux suivant hydrogène, halogène, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, cycloalkyle, hétérocycle, alkényle, alkynyle, adamantyle, polycycloalkyle ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
leurs racémiques, énantiomères, diastéréoisomères et leurs mélanges, leurs tautomères et leurs sels pharmaceutiquement acceptables.

3. Composés de formule (I) selon la revendication 1 ou 2
dans laquelle
R est O
R4 et R7 sont H
R3 est un radical (1-6C)alkyle, aryle(1-6C)alkyle, hétéroaryle, hétéroaryle(1-6C)alkyle, hétérocycle, cycloalkyle, alkényle, ; ces radicaux étant éventuellement substitués par 1 ou plusieurs substituants choisis parmi halogène, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryle, hétéroaryle, formyle, trifluorométhyle, trifluorométhylsulfanyle, trifluorométhoxy ;
R5 et R6 sont indépendamment l'un de l'autre choisis parmi les radicaux suivant hydrogène, halogène, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, - SO₂-O-R10, aryle, hétéroaryle, formyle, trifluorométhyle, trifluorométhoxy ;
R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
leurs racémiques, énantiomères, diastéréoisomères et leurs mélanges, leurs tautomères et leurs sels pharmaceutiquement acceptables.

4. Composé choisi parmi :
Acide (2Z) 4-[(6-chloro-1H-indazol-3-yl)amino]-4-oxo-2-buténoïque
Acide (2E) 4-[(6-chloro-1H-indazol-3-yl)amino]-4-oxo-2-buténoïque
(2E) 4-[(6-chloro-1H-indazol-3-yl)amino]-4-oxo-2-buténoate d'éthyle
(2Z) 4-[(6-chloro-1H-indazol-3-yl)amino]-4-oxo-2-buténoate d'éthyle
Acide 4-[(6-chloro-1H-indazol-3-yl)amino]-4-oxo-2-butanoïque
Acide (2Z) 4-[(5-bromo-1H-indazol-3-yl)amino]-4-oxo-2-buténoïque
Acide (2E) 4-[(6-chloro-1H-indazol-3-yl)amino]-4-oxo-2-buténoïque
Acide (2E) 4-[(5-bromo-1H-indazol-3-yl)amino]-4-oxo-2-buténoïque
Acide (2Z) 4-[(6-chloro-1H-indazol-3-yl)amino]-4-oxo-2-buténoïque
Acide 4-[(5-bromo-1H-indazol-3-yl)amino]-4-oxo-2-butanoïque
(2E) N-(6-chloro-1H-indazol-3-yl)-2-buténamide
(2Z) N-(6-chtoro-1H-indazol-3-yl)-2-buténamide
N-(6-chloro-1H-indazol-3-yl)-3-buténamide, chlorhydrate
4-[(6-chloro-1H-indazol-3-yl)amino]-4-oxo-2-butanoate de méthyle
N-(6-chloro-1H-indazol-3-yl)-acétamide
N-(6-chloro-1H-indazol-3-yl)-butanamide
(2E) N-(6-bromo-1H-indazol-3-yl)-2-buténamide
(2E) N-(5-méthyl-1H-indazol-3-yl)-2-buténamide
(2Z) N-(6-bromo-1H-indazol-3-yl)-2-buténamide
(2Z) N-(5-méthyl-1H-indazol-3-yl)-2-buténamide
N-(6-chloro-1H-indazol-3-yl)-2-propanamide
(2E) N-[6-(trifluorométhyl-1H-indazol-3-yl)]-2-buténamide
(2Z) N-[6-(trifluorométhyl-1H-indazol-3-yl)]-2-buténamide
4-[[6-(trifluorométhyl)-1H-indazol-3-yl]amino]-4-oxo-butanoate d'éthyle
(2E) N-[5-(trifluorométhyl)-1H-indazol-3-yl]-2-buténamide
(2Z) N-[5-(trifluorométhyl)-1H-indazol-3-yl]-2-buténamide
N-[5-chloro-1H-indazol-3-yl]-2-butanamide
N-[4-chloro-1H-indazol-3-yl]-butanamide
N-[6-trifluorométhyl-1H-indazol-3-yl]-butanamide
N-[6-chloro-1H-indazol-3-yl]-propénamide
N-[5-(trifluorométhyl)-1H-indazol-3-yl]-butanamide
N-[5-nitro-1H-indazol-3-yl]-butanamide
N-[6-bromo-1H-indazol-3-yl]-butanamide
N-[6-(3-pyridyl)-1H-indazol-3-yl]-butanamide
N-[4-iodo-1H-indazol-3-yl]-butanamide
N-[6-phenyl-1H-indazol-3-yl]-butanamide
N-[6-bromo-5,7-dinitro-1H-indazol-3-yl]-butanamide
N-[6-bromo-7-nitro-1H-indazol-3-yl]-butanamide
N-[6-bromo-5-nitro-1H-indazol-3-yl]-butanamide
N-[(6-furan-3-yl)-1H-indazol-3-yl]-butanamide
N-[[6-(4-benzyloxy)phenyl)-1H-indazol-3-yl]]-butanamide
N-[6-(4-hydroxy-phenyl)-1H-indazol-3-yl]-butanamide
N-[6-chloro-1H-indazol-3-yl]-benzenamide
N-[[6-(3,5-difluorophenyl)-1H-indazol-3-yl]]-butanamide
N-[6-(3-thienyl)-1H-indazol-3-yl] butanamide
N-[6-chloro-1H-indazol-3-yl]-2-thiophenacétamide
N-[5-(3-fluoro-benzènesulfonylamino)-1H-indazol-3-yl]-benzamide
N-[6-(2-chlorophenyl)-1H-indazol-3-yl]-butanamide
N-[6-(2-chloro-4-hydroxyphenyl)-1H-indazol-3-yl]-butanamide
N-[6-(4-ethylphenyl)-1H-indazol-3-yl]-butanamide
N-[6-(4-ethenylphenyl)-1H-indazol-3-yl]-butanamide
N-[6-(4-pyridyl)-1H-indazol-3-yl]-butanamide
N-[6-(phenylmethyl)-1H-indazol-3-yl]-butanamide
N-[6-(4-aminophenyl)-1H-indazol-3-yl]-butanamide
N-[6-(1-morpholino)-1H-indazol-3-yl]-butanamide
N-[6-[4-phenylethynyl]-phenyl)-1H-indazol-3-yl]-butanamide
N-[6-(2-propenyl)-1H-indazol-3-yl]-butanamide
N-[5-amino-1H-indazol-3-yl]-butanamide
N-[6-bromo-5-chloro-1H-indazol-3-yl]-butanamide
N-[6-chloro-5-bromo-1H-indazol-3-yl]-butanamide
N-[6-chloro-5-nitro-1H-indazol-3-yl]-butanamide
N-[6-(4-hydroxyphenyl)-5-bromo-1H-indazol-3-yl]-butanamide
N-[6-(4-hydroxyphenyl)-5-(phenylamino)-1H-indazol-3-yl]-butanamide
N-[6-(4-hydroxy-phenyl)-5-(2-phenylethenyl)-1H-indazol-3-yl]-butanamide
N-[6-(4-hydroxyphenyl)-5-phenylcarbonyl-1H-indazol-3-yl]-butanamide
N-[6-(4-hydroxyphenyl)-5-[3-(dimethylamino)-propynyl]-1H-indazol-3-yl]-butanamide
N-[6-chloro-1H-indazol-3-yl]-3-thiophenecarboxamide
N-[6-chloro-1H-indazol-3-yl]-2-pyridineacetamide
N-[6-chloro-1H-indazol-3-yl]-3-pyridinecarboxamide
N-[6-chloro-1H-indazol-3-yl]-benzenacetamide
N-[6-chloro-1H-indazol-3-yl]-benzenepropanamide
N-[6-chloro-1H-indazol-3-yl]-3-pyridyneacétamide
N-[6-chloro-1H-indazol-3-yl]-2-chloro-acetamide
N-[6-chloro-1H-indazol-3-yl]-4-morpholineacétamide
N-[6-chloro-1H-indazol-3-yl]-1-piperazineacétamide
N-[6-chloro-1H-indazol-3-yl]-4-[(2-méthoxyéthyl)amino])-cyclohexanecarboxamide
4-amino-N-[6-chloro-1H-indazol-3-yl]-1-piperidinecarboxamide
N-[6-chloro-1H-indazol-3-yl]-4-morpholinylcarboxamide
leur racémiques, énantiomères, diastéréoisomères et leurs mélanges, leurs tautomères et leurs sels pharmaceutiquement acceptables.

5. Composé choisi parmi :
Acide (2Z) 4-[(6-chloro-1H-indazol-3-yl)amino]-4-oxo-2-buténoïque
(2E) 4-[(6-chloro-1H-indazol-3-yl)amino]-4-oxo-2-buténoate d'éthyle
Acide 4-[(6-chloro-1H-indazol-3-yl)amino]-4-oxo-2-butanoïque
Acide (2Z) 4-[(5-bromo-1H-indazol-3-yl)amino]-4-oxo-2-buténoïque
Acide (2E) 4-[(6-chloro-1H-indazol-3-yl)amino]-4-oxo-2-buténoïque
Acide 4-[(5-bromo-1H-indazol-3-yl)amino]-4-oxo-2-butanoïque
(2E) N-(6-chloro-1H-indazol-3-yl)-2-buténamide
N-(6-chloro-1H-indazol-3-yl)-3-buténamide, chlorhydrate
4-[(6-chloro-1H-indazol-3-yl)amino]-4-oxo-2-butanoate de méthyle
N-(6-chloro-1H-indazol-3-yl)-acétamide
N-(6-chloro-1H-indazol-3-yl)-butanamide
(2E) N-(6-bromo-1H-indazol-3-yl)-2-buténamide
(2E) N-(5-méthyl-1H-indazol-3-yl)-2-buténamide
N-(6-chloro-1H-indazol-3-yl)-2-propanamide
(2E) N-[6-(trifluorométhyl-1H-indazol-3-yl)]-2-buténamide
4-[[6-(trifluorométhyl)-1H-indazol-3-yl]amino]-4-oxo-butanoate d'éthyle
(2E) N-[5-(trifluorométhyl)-1H-indazol-3-yl]-2-buténamide
N-[5-chloro-1H-indazol-3-yl]-2-butanamide
N-[4-chloro-1H-indazol-3-yl]-butanamide
N-[6-trifluorométhyl-1H-indazol-3-yl]-butanamide
N-[6-chloro-1H-indazol-3-yl]-propénamide
N-[5-(trifluorométhyl)-1H-indazol-3-yl]-butanamide
N-[5-nitro-1H-indazol-3-yl]-butanamide
N-[6-bromo-1H-indazol-3-yl]-butanamide
N-[6-(3-pyridyl)-1H-indazol-3-yl]-butanamide
N-[4-iodo-1H-indazol-3-yl]-butanamide
N-[6-phenyl-1H-indazol-3-yl]-butanamide
N-[6-bromo-5,7-dinitro-1H-indazol-3-yl]-butanamide
N-[6-bromo-7-nitro-1H-indazol-3-yl]-butanamide
N-[6-bromo-5-nitro-1H-indazol-3-yl]-butanamide
N-[(6-furan-3-yl)-1H-indazol-3-yl]-butanamide
N-[[6-(4-benzyloxy)phenyl)-1H-indazol-3-yl]]-butanamide
N-[6-(4-hydroxy-phenyl)-1H-indazol-3-yl]-butanamide
N-[6-chloro-1H-indazol-3-yl]-benzenamide
N-[[6-(3,5-difluorophenyl)-1H-indazol-3-yl]]-butanamide
N-[6-(3-thienyl)-1H-indazol-3-yl] butanamide
N-[6-chloro-1H-indazol-3-yl]-2-thiophenacétamide
N-[5-(3-fluoro-benzènesulfonylamino)-1H-indazol-3-yl]-benzamide
N-[6-(2-phényléthyl)-1H-indazol-3-yl]-butanamide
N-(6,7-difluoro-1H-indazol-3-yl)-butanamide:
N-[6-(4-méthoxyphényl)-1H-indazol-3-yl]-butanamide
N-[6-(4-méthylthiophényl)-1H-indazol-3-yl]-butanamide
N-[6-(4-trifluorométhoxyphényl)-1H-indazol-3-yl]-butanamide
N-[(6-(1-propènyl)-1H-indazol-3-yl]-butanamide
N-[6-chloro-1H-indazol-3-yl]-2-pyridinecarboxamide
N-[6-(4-fluorophényl)-1H-indazol-3-yl]-butanamide
N-[6-[4-(1,1-diméthyléthyl)phényl]-1H-indazol-3-yl]-butanamide
N-[6-bromo-7-amino-1H-indazol-3-yl]-butanamide
N-[6-[4-(trifluorométhyl)phényl]-1H-indazol-3-yl]-butanamide :
N-[6-(4-méthylphényl)-1H-indazol-3-yl]-butanamide
N-[6-(3,5-dichlorophényl)-1H-indazol-3-yl]-butanamide
N-[6-chloro-1H-indazol-3-yl]-3,5-dichlorobenzamide
N-[6-(4-chlorophényl)-1H-indazol-3-yl]-butanamide
N-[6-chloro-1H-indazol-3-yl]-benzenepropanamide, trifluoroacétate
N-[6-chloro-1H-indazol-3-yl]-benzenepropanamide
N-[[6-(4-éthylphényl)-1H-indazol-3-yl]]-butanamide :
N-[6-(4-pyridinyl)-1H-indazol-3-yl]-butanamide :
N-(5-amino-1H-indazol-3-yl)-butanamide :
N-(5-bromo-6-chloro-1H-indazol-3-yl)-butanamide :
N-(6-chloro-1H-indazol-3-yl)-2-thiophenecarboxamide :
N-(6-chloro-1H-indazol-3-yl)-2-méthylpropylamide :
4-Chloro-N-(6-chloro-1H-indazol-3-yl)-butanamide :
N-(5-phényl-6-chloro-1H-indazol-3-yl)-butanamide
N-[5-bromo-6-[4-(phénylméthoxy)phényl]-1H-indazol-3-yl]-butanamide
N-[5-bromo-6-(4-hydroxyphényl)-1H-indazol-3-yl]-butanamide
N-[[6-(4-nitrophényl)-1H-indazol-3-yl]]-butanamide :
N-[6-(2-chlorophényl)-1H-indazol-3-yl]-butanamide :
N-[6-[3-(phénylméthoxy)phényl]-1H-indazol-3-yl]-butanamide
N-[6-(3-hydroxyphényl)-1H-indazol-3-yl]-butanamide
N-[6-chloro-5-(4-pyridinyl)-1H-indazol-3-yl]-butanamide
N-[6-chloro-5-(3-furanyl)-1H-indazol-3-yl]-butanamide
N-[6-[2-chloro-4-(phénylméthoxy)-phényl]-1H-indazol-3-yl]-butanamide :
N-[6-(2-chloro-4-hydroxyphényl)-1H-indazol-3-yl]-butanamide :
N-[5,6-dibromo-1H-indazol-3-yl]-butanamide :
N-[6-chloro-1H-indazol-3-yl]-2,2,3,3,4,4,4-heptafluoro-butanamide :
N-[6-chloro-5-(4-fluorophényl)-1H-indazol-3-yl]-butanamide
N-[[6-(4-aminophényl)-1H-indazol-3-yl]]-butanamide :
N-[6-[4-(diméthylamino)phényl]-1H-indazol-3-yl]-butanamide :
N-(6-chloro-1H-indazol-3-yl)-4-méthyl-1-pipérazineacétamide
N-(6-chloro-1H-indazol-3-yl)-1-pipéridineacétamide
N-(6-chloro-1H-indazol-3-yl)-4-morpholineacétamide
N-(6-chloro-1H-indazol-3-yl)-1H-1,2,4-triazo le-1-acétamide
N-(6-chloro-1H-indazol-3-yl)-2-(cyclohexylamino)-acétamide
2-[(phénylméthyl)amino]-N-(6-chloro-1H-indazol-3-yl)-acétamide
N-(6-chloro-1H-indazol-3-yl)-1H-azepine-1-acétamide
N-(6-chloro-1H-indazol-3-yl)-1-pipérazineacétamide
N-(6-chloro-1H-indazol-3-yl)-2-[[3-(diméthylamino)propyl]amino]-acétamide
N-(6-chloro-1H-indazol-3-yl)-thiomorpholine-4-acétamide
N-(6-chloro-1H-indazol-3-yl)-1-pyrrolidineacétamide
N-(6-chloro-1H-indazol-3-yl)-2-[[2-(diméthylamino)éthyl]amino]-acétamide
N-(6-chloro-1H-indazol-3-yl)-1-cyclopropylaminoacétamide, trifluoroacétate
N-(6-chloro-1H-indazol-3-yl)-1-cyclopropylaminoacétamide
N-(6-chloro-1H-indazol-3-yl)-2-(2-diéthylamino-éthylamino)-acétamide, tris trifluoroacétate
N-(6-chloro-1H-indazol-3-yl)-2-(2-diéthylamino-éthylamino)-acétamide,
N-[5,6-diphényl-1H-indazol-3-yl]-butanamide :
N-[6-chloro-5-(4-méthylphényl)-1H-indazol-3-yl]-butanamide
N-[5-phényl-6-[4-(phénylméthoxy)phényl]-1H-indazol-3-yl]-butanamide :
N-[5-phényl-6-(4-hydroxyphényl)-1H-indazol-3-yl]-butanamide
N-[6-chloro-5-(4-pyridinyl)-1H-indazol-3-yl]-butanamide :
N-[5-(4-aminophényl)-6-chloro-1H-indazol-3-yl]-butanamide :
N-[6-chloro- 5-(4-éthylphényl)-1H-indazol-3-yl]-butanamide
N-[6-chloro-5-[4-(phénylméthoxy)phényl]-1H-indazol-3-yl]-butanamide
N-[6-chloro-5-(4-hydroxyphényl)- 1H-indazol-3-yl]-butanamide
N-[5,6-bis[4-(phénylméthoxy)phényl]-1H-indazol-3-yl]-butanamide
N-[5,6-bis (4-hydroxyphényl)-1H-indazol-3-yl]-butanamide
N-[5-(3-furanyl)-6-[4-(phénylméthoxy)phényl]-1H-indazol-3-yl]-butanamide
N-[5-(3-furanyl)-6-([4-hydroxyphényl)-1H-indazol-3-yl]-butanamide
N-[5-(4-éthylphényl)-6-[4-(phénylméthoxy)phényl]-1H-indazol-3-yl]-butanamide
N-[5-(4-éthylphényl)-6-(4-hydroxyphényl)-1H-indazol-3-yl]-butanamide
N-[5-(3-pyridinyl)-6-[4-(phénylméthoxy)phényl]-1H-indazol-3-yl]-butanamide
N-[5-(3-pyridinyl)-6-(4-hydroxyphényl)-1H-indazol-3-yl]-butanamide
N-[5-(2-furanyl)-6-[4-(phénylméthoxy)phényl]-1H-indazol-3-yl]-butanamide:
N-[5-(2-furanyl)-6-(4-hydroxyphényl)-1H-indazol-3-yl]-butanamide
N-(5-bromo-6-chloro-7-nitro-1H-indazol-3-yl)-butanamide :
N-(5-bromo-6,7-difluoro-1H-indazol-3-yl)-butanamide
N-[6-(4-cyanophényl)-1H-indazol-3-yl]-butanamide :
N-(6,7-difluoro-5-nitro-1H-indazol-3-yl)-butanamide :
N-(6,7-difluoro-5-phényl-1H-indazol-3-yl)-butanamide
N-[6-(6-hydroxy-pyridin-3-yl)- 1H-indazol-3-yl]-butanamide
N-[6-(3,4-dihydroxy-phényl)-1H-indazol-3-yl]-butanamide, trifluoroacétate
N-[6-(3,4-dihydroxy-phényl)-1H-indazol-3-yl]-butanamide
N-[7-fluoro-5-nitro-6-[2-(phényléthyl)amino]-1H-indazol-3-yl]-butanamide
N-(7-fluoro-5-nitro-6-morpholino-1H-indazol-3-yl)-butanamide
N-(7-fluoro-5-amino-6-morpholino-1H-indazol-3-yl)-butanamide
N-(5-bromo-7-fluoro -6-morpholino-1H-indazol-3-yl)-butanamide
N-[7-fluoro-6-(trifluorométhyl)-1H-indazol-3-yl]-butanamide
N-(6-bromo-4,5,7-trifluoro-1H-indazol-3-yl)-butanamide
N-[6-(6-amino-pyridin-3-yl)-1H-indazol-3-yl]-butanamide, difluoroacétate
N-[6-(6-amino-pyridin-3-yl)-1H-indazol-3-yl]-butanamide
2-chloro-N-(6,7-difluoro-1H-indazol-3-yl)-acétamide
N-(6,7-difluoro-1H-indazol-3-yl)-1-pipéridineacétamide
son racémique, ses énantiomères, tautomères ainsi que leurs sels pharmaceutiquement acceptables.

6. Composition pharmaceutique **caractérisée par le fait qu'**elle comprend dans un milieu pharmaceutiquement acceptable, un composé défini selon l'une quelconque des revendications 1 à 5.

7. Médicament **caractérisé par le fait qu'**il contient au moins un composé défini selon l'une quelconque des revendications 1 à 5 pour son application thérapeutique dans le traitement des maladies dans lesquelles une phosphorylation de la protéine Tau est observée.

8. Médicament **caractérisé par le fait qu'**il contient au moins un composé défini selon l'une quelconque des revendications 1 à 5 pour son application thérapeutique dans le traitement des maladies neurodégénératives, les accidents cérébrovasculaires, les traumatismes crâniens et spinaux et neuropathies périphériques, l'obésité, les maladies du métabolisme, le diabètede type II, l'hypertension essentielle, les maladies cardiovasculaires athérosclérotiques, le syndrome des ovaires polycystiques, le syndrome X, l'imunodéficience et le cancer.

9. Médicament selon la revendication 8 **caractérisé par le fait que** la maladie neurodégérative est soit la maladie d'Alzheimer, de Parkinson, la démence frontopariétale, la dégénération corticobasale ou la maladie de Pick.

10. Procédé de préparation des composés de formule (I) tels que définis dans la revendication 1 et pour lequel R est oxygène **caractérisé en ce que** on effectue une acylation du dérivé aminé de formule (II) : 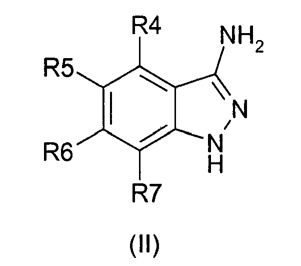 dans laquelle R4, R5, R6, R7 ont les mêmes significations que dans la revendication 1, le transforme éventuellement en sel pharmaceutiquement acceptable.

11. Procédé de préparation des composés de formule (I) tels que définis dans la revendication 1 et pour lequel R est un soufre **caractérisé en ce qu'**on effectue une thionation du dérivé où R est un oxygène et le transforme éventuellement en sel pharmaceutiquement acceptable.

12. Procédé de préparation des composés de formule (I) tels que définis dans la revendication 1 et pour lequel R est NH **caractérisé en ce qu'**on effectue une réaction du dérivé aminé de formule (II) avec un nitrile ou sel de Merwein 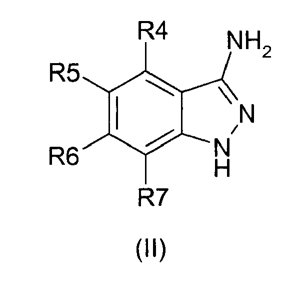 dans laquelle R4, R5, R6, R7 ont les mêmes significations que dans la revendication 1, le transforme éventuellement en sel pharmaceutiquement acceptable.

13. Procédé de préparation selon la revendication 10 ou 12 **caractérisé en ce que** le dérivé de formule (II) est obtenu à partir du dérivé de formule (III) par réaction avec de l'hydrazine

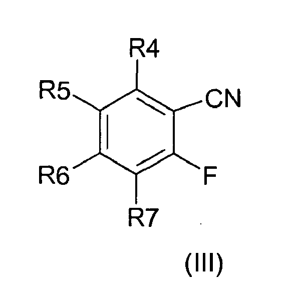

## Patentansprüche

1. Verbindungen der Formel (I): 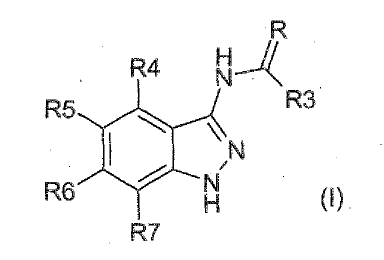 worin
R für O, S oder NH steht;
R3 für einen (1-6C)-Alkylrest, einen Aryl-(1-6C)-alkylrest, einen Heteroarylrest, einen Heteroaryl-(1-6C)-alkylrest, einen Aryl- oder Heteroarylrest, der mit einem (1-10C)-Cycloalkyl kondensiert ist, einen Heterocyclylrest, einen Cycloalkylrest, einen Adamantylrest, einen Polycycloalkylrest, einen Alkenylrest oder einen Alkinylrest steht; wobei diese Reste gegebenenfalls durch 1 oder mehrere Substituenten, die unter Halogen, CN, NO₂, NH₂, OH, OR8, COOH, C(O)OR8, -O-C(O)R8, NR8R9, NHC(O)R8, C(O)NR8R9, SR8, S(O)R8, SO₂R8 , NHSO₂R8, SO₂NR8R9, C(S)NR8R9, NHC(S)R8, -O-SO₂R8, -SO₂-O-R8, Aryl, Heteroaryl, Formyl, Trifluormethyl, Trifluormethylsulfanyl und Trifluormethoxy ausgewählt sind, substituiert sind;
R4, R5, R6 und R7 unabhängig voneinander unter den folgenden Resten ausgewählt sind: Wasserstoff, Halogen, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, Trifluormethyl, Trifluormethoxy, (1-6C)-Alkyl, (1-6C)-Alkoxy, Aryl, Aryl-(1-6C-alkyl, Heteroaryl, Heteroaryl-(1-6C)-alkyl, Heterocyclyl, Cycloalkyl, Alkenyl, Alkinyl, Adamantyl und Polycycloalkyl; wobei diese Reste gegebenenfalls durch 1 oder mehrere unter Halogen, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂,R10, SO₂NR10R11, -O-SO₂R10, -SO₂-O-R10, Aryl, Heteroaryl, Formyl, Trifluormethyl und Trifluormethoxy ausgewählte Substituenten substituiert sind; R8, R9, R10 und R11 unabhängig voneinander für Wasserstoff, (1-6C)-Alkyl, Aryl, Alkenyl, Alkinyl oder Heteroaryl, die selbst gegebenenfalls durch 1 oder mehrere unter Halogen, (1-6C-alkyl, (1-6C)-Alkoxy, CN, NO₂, NH₂, OH, COOH, COO-Alkyl, CONH₂, Formyl, Trifluormethyl und Trifluormethoxy ausgewählte Substituenten substituiert sind, stehen; Racemate, Enantiomere und Diastereoisomere davon und Gemische davon, Tautomere davon und pharmazeutisch unbedenkliche Salze davon mit Ausnahme von 3-Acetamidoindazol, N-(1H-Indazol-3-yl)butanamid, N-(1H-Indazol-3-yl)phenylacetamid, N-(1H-Indazol-3-yl)benzhydrylacetamid, 5-Amino-3-acetamidoindazol, N-(6-Chlor-1H-indazol-3-yl)-2,2,2-trifluoracetamid, N-(6-Chlor-1H-indazol-3-yl)-2-furancarboxamid, N-(6-Chlor-1H-indazol-3-yl)-2-thiophencarboxamid und N-(5-Nitro-1H-indazol-3-yl)acetamid.

2. Verbindungen der Formel (I) nach Anspruch 1, worin
R für O, S oder NH steht;
R3 für einen (1-6C)-Alkylrest, einen Aryl-(1-6C)-alkylrest, einen Heteroarylrest, einen Heteroaryl-(1-6C)-alkylrest, einen Cycloalkylrest, einen Alkenylrest oder einen Alkinylrest steht; wobei diese Reste gegebenenfalls durch 1 oder mehrere Substituenten, die unter Halogen, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, Aryl, Heteroaryl, Formyl, Trifluormethyl, Trifluormethylsulfanyl und Trifluormethoxy ausgewählt sind, substituiert sind;
R4 und R7 für Wasserstoff stehen;
R5 und R6 unabhängig voneinander unter den folgenden Resten ausgewählt sind: Wasserstoff, Halogen, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, Trifluormethyl, Trifluormethoxy, (1-6C)-Alkyl, (1-6C)-Alkoxy, Aryl, Aryl-(1-6C)-alkyl, Heteroaryl, Heteroaryl-(1-6C)-alkyl, Cycloalkyl, Heterocyclyl, Alkenyl, Alkinyl, Adamantyl und Polycycloalkyl; wobei diese Reste gegebenenfalls durch 1 oder mehrere unter Halogen, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, Aryl, Heteroaryl, Formyl, Trifluormethyl und Trifluormethoxy ausgewählte Substituenten substituiert sind;
R8, R9, R10 und R11 unabhängig voneinander für Wasserstoff, (1-6C)-Alkyl, Aryl, Alkenyl, Alkinyl oder Heteroaryl, die selbst gegebenenfalls durch 1 oder mehrere unter Halogen, (1-6C)-Alkyl, (1-6C)-Alkoxy, CN, NO₂ NH₂, OH, COOH, COO-Alkyl, CONH₂, Formyl, Trifluormethyl und Trifluormethoxy ausgewählte Substituenten substituiert sind, stehen; Racemate, Enantiomere und Diastereoisomere davon und Gemische davon, Tautomere davon und pharmazeutisch unbedenkliche Salze davon.

3. Verbindungen der Formel (I) nach Anspruch 1 oder 2,
worin
R für O steht;
R4 und R7 für H stehen;
R3 für einen (1-6C)-Alkylrest, einen Aryl-(1-6C)-alkylrest, einen Heteroarylrest, einen Heteroaryl-(1-6C)-alkylrest, einen Heterocyclylrest, einen Cycloalkylrest oder einen Alkenylrest steht; wobei diese Reste gegebenenfalls durch 1 oder mehrere Substituenten, die unter Halogen, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, Aryl, Heteroaryl, Formyl, Trifluormethyl, Trifluormethylsulfanyl und Trifluormethoxy ausgewählt sind, substituiert sind;
R5 und R6 unabhängig voneinander unter den folgenden Resten ausgewählt sind: Wasserstoff, Halogen, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, Trifluormethyl, Trifluormethoxy, (1-6C)-Alkyl, (1-6C)-Alkoxy, Aryl, Aryl-(1-6C)-alkyl, Heteroaryl, Heteroaryl-(1-6C)-alkyl, Heterocyclyl, Cycloalkyl und Alkenyl; wobei diese Reste gegebenenfalls durch 1 oder mehrere unter Halogen, CN, NO₂, NH₂, OH, OR10, COOH, C(O)OR10, -O-C(O)R10, NR10R11, NHC(O)R10, C(O)NR10R11, NHC(S)R10, C(S)NR14R11, SR10, S(O)R10, SO₂R10, NHSO₂R10, SO₂NR10R11, -O-SO₂R11, -SO₂-O-R10, Aryl, Heteroaryl, Formyl, Trifluormethyl und Trifluormethoxy ausgewählte Substituenten substituiert sind;
R8, R9, R10 und R11 unabhängig voneinander für Wasserstoff, (1-6C)-Alkyl, Aryl, Alkenyl, Alkinyl oder Heteroaryl, die selbst gegebenenfalls durch 1 oder mehrere unter Halogen, (1-6C)-Alkyl, (1-6C)-Alkoxy, CN, NO₂, NH₂, OH, COOH, COO-Alkyl, CONH₂, Formyl, Trifluormethyl und Trifluormethoxy ausgewählte Substituenten substituiert sind, stehen; Racemate, Enantiomere und Diastereoisomere davon und Gemische davon, Tautomere davon und pharmazeutisch unbedenkliche Salze davon.

4. Verbindung, ausgewählt aus:
(2Z)-4-[(6-Chlor-1H-indazol-3-yl)amino]-4-oxo-2-butensäure
(2E)-4-[(6-Chlor-1H-indazol-3-yl)a-minol-4-oxo-2-butensäure
(2E)-4-[(6-Chlor-1H-indazol-3-yl)amino]-4-oxo-2-butensäureethylester
(2Z)-4-[(6-Chlor-1H-indazol-3-y1)amino]-4-oxo-2-butensäureethylester
4-[(6-Chlor-1H-indazol-3-yl)amino]-4-oxo-2-butansäure
(2Z)-4-[(5-Brom-1H-indazol-3-yl)aminol-4-oxo-2-butensäure
(2E)-4-[(6-Chlor-1H-indazol-3-yl)amino]-4-oxo-2-butensäure
(2E)-4-[(5-Brom-1H-indazol-3-yl)amino]-4-oxo-2-butensäure
(2Z)-4-[(6-Chlor-1H-indazol-3-yl)aminol-4-oxo-2-butensäure
4-[(5-Brom-1H-indazol-3-yl)amino]-4-oxo-2-butansäure
(2E)-N-(6-Chlor-1H-indazol-3-yl)-2-butenamid (2Z)-N-(6-Chlor-1H-indazol-3-yl)-2-butenamid
N-(6-Chlor-1H-indazol-3-yl)-3-butenamid-hydrochlorid
4-[(6-Chlor-1H-indazol-3-yl)amino]-4-oxo-2-butansäuremethylester
N-(6-Chlor-1H-indazol-3-yl)acetamid
N-(6-Chlor-1H-indazol-3-yl)butanamid
(2E)-N-(6-Brom-1H-indazol-3-yl)-2-butenamid
(2E)-N-(5-Methyl-1H-indazol-3-yl)-2-butenamid
(2Z)-N-(6-Brom-1H-indazol-3-yl)-2-butenamid
(2Z)-N-(5-Methyl-1H-indazol-3-yl)-2-butenamid
N-(6-Chlor-1H-indazol-3-yl)-2-propanamid (2E)-N-[6-(Trifluormethyl)-1H-indazol-3-yl]-2-butenamid
(2Z)-N-[6-(Trifluormethyl)-1H-indazol-3-yl]-2-butenamid
4-[[6-(Trifluormethyl)-1H-indazol-3-yl]amino]-4-oxobutansäureethylester
(2E)-N-[5-(Trifluormethyl)-1H-indazol-3-yl]-2-butenamid
(2Z)-N-[5-(Trifluormethyl)-1H-indazol-3-yl]-2-butenamid
N-[5-Chlor-1H-indazol-3-yl]-2-butanamid
N-[4-Chlor-1H-indazol-3-yl]butanamid
N-[6-(Trifluormethyl)-1H-indazol-3-yl]butanamid
N-[6-Chlor-1H-indazol-3-yl]propenamid
N-[5-(Trifluormethyl)-1H-indazol-3-yl]butanamid
N-[5-Nitro-1H-indazol-3-yl]butanamid
N-[6-Brom-1H-indazol-3-yl]butanamid
N-[6-(3-Pyridyl)-1H-indazol-3-yl]butanamid
N-[4-Iod-1H-indazol-3-yl]butanamid
N-[6-Phenyl-1H-indazol-3-yl]butanamid
N-[6-Brom-5,7-dinitro-1H-indazol-3-yl]butanamid
N-[6-Brom-7-nitro-1H-indazol-3-yl]butanamid
N-[6-Brom-5-nitro-1H-indazol-3-yl]butanamid
N-[(6-Furan-3-yl)-1H-indazol-3-yl]butanamid
N-[6-[4-(Benzyloxy)phenyl]-1H-indazol-3-yl]butanamid
N-[6-(4-Hydroxyphenyl)-1H-indazol-3-yl]butanamid
N-[6-Chlor-1H-indazol-3-yl]benzenamid
N-[6-(3,5-Difluorphenyl)-1H-indazol-3-yl]butanamid
N-[6-(3-Thienyl)-1H-indazol-3-yl]butanamid
N-[6-Chlor-1H-indazol-3-yl]-2-thiophenacetamid
N-[5-(3-Fluorbenzolsulfonylamino)-1H-indazol-3-yl]benzamid
N-[6-(2-Chlorphenyl)-1H-indazol-3-yl]butanamid
N-[6-(2-Chlor-4-hydroxyphenyl)-1H-indazol-3-yl]-butanamid
N-[6-(4-Ethylphenyl)-1H-indazol-3-yl]butanamid
N-[6-(4-Ethenylphenyl)-1H-indazol-3-yl]butanamid
N-[6-(4-Pyridyl)-1H-indazol-3-yl]butanamid
N-[6-(Phenylmethyl)-1H-indazol-3-yl]butanamid
N-[6-(4-Aminophenyl)-1H-indazol-3-yl]butanamid
N-[6-(1-Morpholino)-1H-indazol-3-yl]butanamid
N-[6-[(4-Phenylethinyl)phenyl]-1H-indazol-3-yl]-butanamid
N-[6-(2-Propenyl)-1H-indazol-3-yl]butanamid
N-[5-Amino-1H-indazol-3-yl]butanamid
N-[6-Brom-5-chlor-1H-indazol-3-yl]butanamid
N-[6-Chlor-5-brom-1H-indazol-3-yl]butanamid
N-[6-Chlor-5-nitro-1H-indazol-3-yl]butanamid
N-[6-(4-Hydroxyphenyl)-5-brom-1H-indazol-3-yl]-butanamid
- N-[6-(4-Hydroxyphenyl)-5-(phenylamino)-1H-indazol-3-yl]butanamid
N-[6-(4-Hydroxyphenyl)-5-(2-phenylethenyl)-1H-indazol-3-yl]butanamid
N-[6-(4-Hydroxyphenyl)-5-phenylcarbonyl-2H-indazol-3-yl]butanamid
N-[6-(4-Hydroxyphenyl)-5-[3-(dimethylamino)-propinyl]-1H-indazol-3-yl]butanamid
N-[6-Chlor-1H-indazol-3-yl]-3-thiophencarboxamid
N-[6-Chlor-1H-indazol-3-yl]-2-pyridinacetamid
N-[6-Chlor-1H-indazol-3-yl]-3-pyridincarboxamid
N-[6-Chlor-1H-indazol-3-yl]benzolacetamid
N-[6-Chlor-1H-indazol-3-yl]benzolpropanamid
N-[6-Chlor-1H-indazol-3-yl]-3-pyridinacetamid
N-[6-Chlor-1H-indazol-3-yl]-2-chloracetamid
N-[6-Chlor-1H-indazol-3-yl]-4-morpholinacetamid
N-[6-Chlor-1H-indazol-3-yl]-1-piperazinacetamid
N-[6-Chlor-1H-indazol-3-yl]-4-[(2-methoxyethyl)-amino]cyclohexancarboxamid
4-Amino-N-[6-chlor-1H-indazol-3-yl]-1-piperidin-carboxamid
N-[6-Chlor-1H-indazol-3-yl]-4-morpholinylcarbox-amid,
Racemate, Enantiomere und Diastereomere davon und Gemische davon, Tautomere davon und pharmazeutisch unbedenkliche Salze davon.

5. Verbindung, ausgewählt aus:
(2Z)-4-[(6-Chlor-1H-indazol-3-yl)amino]-4-oxo-2-butensäure
(2E)-4-[(6-Chlor-1H-indazol-3-yl)amino]-4-oxo-2-butensäureethylester
4-[(6-Chlor-1H-indazol-3-yl)amino]-4-oxo-2-butansäure
(2Z)-4-[(5-Brom-1H-indazol-3-yl)amino]-4-oxo-2-butensäure
(2E)-4-[(6-Chlor-1H-indazol-3-yl)amino]-4-oxo-2-butensäure
4-[(5-Brom-1H-indazol-3-yl)amino]-4-oxo-2-butansäure
(2E)-N-(6-Chlor-1H-indazol-3-yl)-2-butenamid N-(6-Chlor-1H-indazol-3-yl)-3-butenamid-hydrochlorid
4-[(6-Chlor-1H-indazol-3-yl)amino]-4-oxo-2-butansäuremethylester
N-(6-Chlor-1H-indazol-3-yl)acetamid N-(6-chlor-1H-indazol-3-yl)butanamid
(2E)-N-(6-Brom-1H-indazol-3-yl)-2-butenamid
(2E)-N-(5-Methyl-1H-indazol-3-yl)-2-butenamid N-(6-Chlor-1H-indazol-3-yl)-2-propanamid
(2E)-N-[6-(Trifluormethyl)-1H-indazol-3-yl]-2-butenamid
4-[[6-(Trifluormethyl)-1H-indazol-3-yl]amino]-4-oxobutansäureethylester
(2E)-N-[5-(Trifluormethyl)-1H-indazol-3-yl]-2-butenamid
N-[5-Chlor-1H-indazol-3-yl]-2-butanamid
N-[4-Chlor-1H-indazol-3-yl]butanamid
N-[6-(Trifluormethyl)-1H-indazol-3-yl]butanamid
N-[6-Chlor-1H-indazol-3-yl]propenamid
N-[5-(Trifluormethyl)-1H-indazol-3-yl]butanamid
N-[5-Nitro-1H-indazol-3-yl]butanamid
N-[6-Brom-1H-indazol-3-yl]butanamid
N-[6-(3-Pyridyl)-1H-indazol-3-yl]butanamid
N-[4-Iod-1H-indazol-3-yl]butanamid
N-[6-Phenyl-1H-indazol-3-yl]butanamid
N-[6-Brom-5,7-dinitro-1H-indazol-3-yl]butanamid
N-[6-Brom-7-nitro-1H-indazol-3-yl]butanamid
N-[6-Brom-5-nitro-1H-indazol-3-yl]butanamid
N-[(6-Furan-3-yl)-1H-indazol-3-yl]butanamid
N-[6-[4-(Benzyloxy)phenyl]-1H-indazol-3-yl]butanamid
N-[6-(4-Hydroxyphenyl)-1H-indazol-3-yl]butanamid
N-[6-Chlor-1H-indazol-3-yl]benzenamid
N-[6-(3,5-Difluorphenyl)-1H-indazol-3-yl]butanamid
N-[6-(3-Thienyl)-1H-indazol-3-yl]butanamid N-[6-Chlor-1H-indazol-3-yl]-2-thiophenacetamid
N-[5-(3-Fluorbenzolsulfonylamino)-1H-indazol-3-yl]benzamid
N-[6-(2-Phenylethyl)-1H-indazol-3-yl]butanamid
N-(6,7-Difluor-1H-indazol-3-yl)butanamid
N-[6-(4-Methoxyphenyl)-1H-indazol-3-yl]butanamid
N-[6-(4-Methylthiophenyl)-1H-indazol-3-yl]butanamid
N-[6-(4-Trifluormethoxyphenyl)-1H-indazol-3-yl]-butanamid
N-[(6-(1-Propenyl)-1H-indazol-3-yl]butanamid
N-[6-Chlor-1H-indazol-3-yl]-2-pyridincarboxamid
N-[6-(4-Fluorphenyl)-1H-indazol-3-yl]butanamid
N-[6-[4-(1,1-Dimethylethyl)phenyl]-1H-indazol-3-yl]butanamid
N-[6-Brom-7-amino-1H-indazol-3-yl]butanamid
N-[6-[4-(Trifluormethyl)phenyl]-1H-indazol-3-yl]butanamid
N-[6-(4-Methylphenyl)-1H-indazol-3-yl]butanamid
N-[6-(3,5-Dichlorphenyl)-1H-indazol-3-yl]butanamid
N-[6-Chlor-1H-indazol-3-yl]-3,5-dichlorbenzamid
N-[6-(4-Chlorphenyl)-1H-indazol-3-yl]butanamid
N-[6-Chlor-1H-indazol-3-yl]benzolpropanamidtrifluoracetat
N-[6-Chlor-1H-indazol-3-yl]benzolpropanamid
N-[[6-(4-Ethylphenyl)-1H-indazol-3-yl]]butanamid
N-[6-(4-Pyridinyl)-1H-indazol-3-yl]butanamid
N-(5-Amino-1H-indazol-3-yl)butanamid
N-(5-Brom-6-chlor-1H-indazol-3-yl)butanamid
N-(6-Chlor-1H-indazol-3-yl)-2-thiophencarboxamid
N-(5-Chlor-1H-indazol-3-yl)-2-methylpropylamid
4-Chlor-N-(6-chlor-1H-indazol-3-yl)butanamid
N-(5-Phenyl-6-chlor-1H-indazol-3-yl)butanamid
N-[5-Brom-6-[4-(phenylmethoxy)phenyl]-1H-indazol-3-yl]butanamid
N-[5-Brom-6-(4-hydroxyphenyl)-1H-indazol-3-yl]-butanamid
N-[[6-(4-Nitrophenyl)-1H-indazol-3-yl]]butanamid
N-[6-(2-Chlorphenyl)-1H-indazol-3-yl]butanamid
N-[6-[3-(Phenylmethoxy)phenyl]-1H-indazol-3-yl]-butanamid
N-[6-(3-Hydroxyphenyl)-1H-indazol-3-yl]butanamid
N-[6-chlor-5-(4-pyridinyl)-1H-indazol-3-yl]butanamid
N-[6-Chlor-5-(3-furanyl)-1H-indazol-3-yl]butanamid
N-[6-[2-Chlor-4-(phenylmethoxy)phenyl]-1H-indazol-3-yl]butanamid
N-[6-(2-Chlor-4-hydroxyphenyl)-1H-indazol-3-yl]-butanamid
N-[5,6-Dibrom-2H-indazol-3-yl]butanamid
N-[6-Chlor-1H-indazol-3-yl]-2,2,3,3,4,4,4-heptafluorbutanamid
N-[6-Chlor-5-(4-fluorphenyl)-1H-indazol-3-yl]-butanamid
N-[[6-(4-Aminophenyl)-1H-indazol-3-yl]]butanamid
N-[6-[4-(Dimethylamino)phenyl]-1H-indazol-3-yl]-butanamid
N-(6-Chlor-1H-indazol-3-yl)-4-methyl-1-piperazinacetamid
N-(6-Chlor-1H-indazol-3-yl)-1-piperidinacetamid
N-(6-Chlor-2H-indazol-3-yl)-4-morpholinacetamid
N-(6-Chlor-1H-indazol-3-yl)-1H-1,2,4-triazol-1-acetamid
N-(6-Chlor-1H-indazol-3-yl)-2-(cyclohexylamino)-acetamid
2-[(Phenylmethyl)amino]-N-(6-chlor-1H-indazol-3-yl)acetamid
N-(6-Chlor-1H-indazol-3-yl)-1H-azepin-1-aceta-mid
N-(6-Chlor-1H-indazol-3-yl)-1-piperazinacetamid
N-(6-Chlor-1H-indazol-3-yl)-2-[[3-(dimethylamino)-propyl]amino]acetamid
N-(6-Chlor-1H-indazol-3-yl)thiomorpholin-4-acetamid
N-(6-Chlor-1H-indazol-3-yl)-1-pyrrolidinacetamid
N-(6-Chlor-1H-indazol-3-yl)-2-[[2-(dimethylamino)-ethyl]amino]acetamid
N-(6-Chlor-1H-indazol-3-yl)-1-cyclopropylamino-acetamid-trifluoracetat
N-(6-Chlor-1H-indazol-3-yl)-1-cyclopropylamino-acetamid
N-(6-Chlor-1H-indazol-3-yl)-2-(2-diethylamino-ethylamino)acetamid-tris(trifluoracetat)
N-(6-Chlor-1H-indazol-3-yl)-2-(2-diethylaminoethylamino)acetamid
N-[5,6-Diphenyl-1H-indazol-3-yl]butanamid
N-[6-chlor-5-(4-methylphenyl)-1H-indazol-3-yl]-butanamid
N-[5-Phenyl-6-[4-(phenylmethoxy)phenyl]-1H-indazol-3-yl]butanamid
N-[5-Phenyl-6-(4-hydroxyphenyl)-1H-indazol-3-yl]-butanamid
N-[6-Chlor-5-(4-pyridyl)-1H-indazol-3-yl]butanamid
N-[5-(4-Aminophenyl)-6-chlor-1H-indazol-3-yl]-butanamid
N-[6-Chlor-5-(4-ethylphenyl)-1H-indazol-3-yl]-butanamid
N-[6-Chlor-5-[4-(phenylmethoxy)phenyl]-1H-indazol-3-yl]butanamid
N-[6-Chlor-5-(4-hydroxyphenyl)-1H-indazol-3-yl]-butanamid
N-[5,6-Bis[4-(phenylmethoxy)phenyl]-1H-indazol-3-yl]butanamid
N-[5,6-Bis(4-hydroxyphenyl)-1H-indazol-3-yl]butanamid
N-[5-(3-Furanyl)-6-[4-(phenylmethoxy)phenyl]-1H-indazol-3-yl]butanamid
N-[5-(3-Furanyl)-6-([4-hydroxyphenyl)-1H-indazol-3-yl]butanamid
N-[5-(4-Ethylphenyl)-6-[4-(phenylmethoxy)phenyl]-1H-indazol-3-yl]butanamid
N-[5-(4-Ethylphenyl)-6-(4-hydroxyphenyl)-1H-indazol-3-yl]butanamid
N-[5-(3-Pyridinyl)-6-[4-(phenylmethoxy)phenyl]-1H-indazol-3-yl]butanamid
N-[5-(3-Pyridinyl)-6-(4-hydroxyphenyl)-1H-indazol-3-yl]butanamid
N-[5-(2-Furanyl)-6-[4-(phenylmethoxy)phenyl]-1H-indazol-3-yl]butanamid
N-[5-(2-Furanyl)-6-(4-hydroxyphenyl)-1H-indazol-3-yl]butanamid
N-(5-Brom-6-chlor-7-nitro-1H-indazol-3-yl)butanamid
N-(5-Brom-6,7-difluor-1H-indazol-3-yl)butanamid
N-[6-(4-Cyanophenyl)-1H-indazol-3-yl]butanamid
N-(6,7-Difluor-5-nitro-1H-indazol-3-yl)butanamid
N-(6,7-Difluor-5-phenyl-1H-indazol-3-yl)butanamid
N-[6-(6-Hydroxypyridin-3-yl)-1H-indazol-3-yl]butanamid
N-[6-(3,4-Dihydroxyphenyl)-1H-indazol-3-yl]butanamid-trifluoracetat
N-[6-(3,4-Dihydroxyphenyl)-1H-indazol-3-yl]butanamid
N-[7-Fluor-5-nitro-6-[2-(phenylethyl)aminol-1H-indazol-3-yl]butanamid
N-(7-Fluor-5-nitro-6-morpholino-1H-indazol-3-yl)-butanamid
N-(7-Fluor-5-amino-6-morpholino-1H-indazol-3-yl)-butanamid
N-(5-Brom-7-fluor-6-morpholino-1H-indazol-3-yl)-butanamid
N-[7-Fluor-6-(trifluormethyl)-1H-indazol-3-yl]-butanamid
N-(6-Brom-4,5,7-trifluor-1H-indazol-3-yl)butanamid
N-[6-(6-Aminopyridin-3-yl)-1H-indazol-3-yl]butanamid-difluoracetat
N-[6-(6-Aminopyridin-3-yl)-1H-indazol-3-yl]butanamid
2-Chlor-N-(6,7-difluor-1H-indazol-3-yl)acetamid N-(6,7-Difluor-1H-indazol-3-yl)-1-piperidinacetamid,
Racemate, Enantiomere und Tautomere davon sowie pharmazeutisch unbedenkliche Salze davon.

6. Pharmazeutische Zusammensetzung, **dadurch gekennzeichnet, dass** sie in einem pharmazeutisch unbedenklichen Medium eine Verbindung gemäß einem der Ansprüche 1 bis 5 enthält.

7. Arzneimittel, **dadurch gekennzeichnet, dass** es mindestens eine Verbindung gemäß einem der Ansprüche 1 bis 5 enthält, zur therapeutischen Anwendung bei der Behandlung von Erkrankungen, bei denen eine Phosphorylierung des Tau-Proteins beobachtet wird.

8. Arzneimittel, **dadurch gekennzeichnet, dass** es mindestens eine Verbindung gemäß einem der Ansprüche 1 bis 5 enthält, zur therapeutischen Anwendung bei der Behandlung von neurodegenerativen Erkrankungen, Schlaganfällen, Schädel- und Rückenmarkstraumata und peripheren Neuropathien, Obesitas, Stoffwechselerkrankungen, Typ-II-Diabetes, essentieller Hypertonie, atherosklerotischen Herz-Kreislauf-Erkrankungen, Syndrom der polyzystischen Ovarien, Syndrom X, Immundefekt und Krebs.

9. Medikament nach Anspruch 8, **dadurch gekennzeichnet, dass** es sich bei der neurodegenerativen Erkrankung um Alzheimer-Krankheit, Parkinson-Krankheit, frontoparietale Demenz, kortikobasale Degeneration oder Pick-Krankheit handelt.

10. Verfahren zur Herstellung der Verbindungen der Formel (I) gemäß Anspruch 1, für die R für Sauerstoff steht, **dadurch gekennzeichnet, dass** man das Aminoderivat der Formel (II): 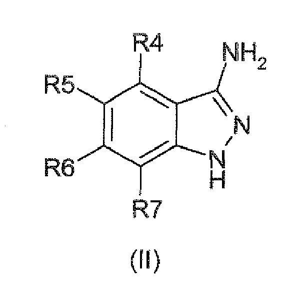 worin R4, R5, R6 und R7 die gleichen Bedeutungen wie in Anspruch 1 besitzen, acyliert und gegebenenfalls in ein pharmazeutisch unbedenkliches Salz umwandelt.

11. Verfahren zur Herstellung der Verbindungen der Formel (I) gemäß Anspruch 1, für die R für Schwefel steht, **dadurch gekennzeichnet, dass** man das Derivat, in dem R für Sauerstoff steht, thioniert und gegebenenfalls in ein pharmazeutisch unbedenkliches Salz umwandelt.

12. Verfahren zur Herstellung der Verbindungen der Formel (I) gemäß Anspruch 1, für die R für NH steht, **dadurch gekennzeichnet, dass** man das Aminderivat der Formel (II) 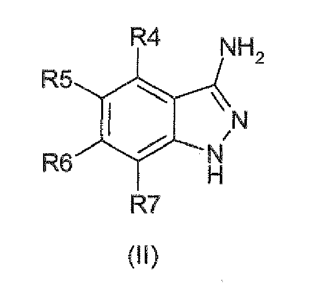 worin R4, R5, R6 und R7 die gleichen Bedeutungen wie in Anspruch 1 besitzen, mit einem Nitril oder Meerwein-Salz umsetzt und gegebenenfalls in ein pharmazeutisch unbedenkliches Salz umwandelt.

13. Herstellungsverfahren nach Anspruch 10 oder 12, **dadurch gekennzeichnet, dass** man das Derivat der Formel (II) aus dem Derivat der Formel (III) 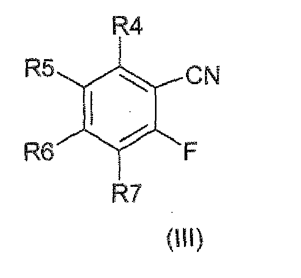 durch Umsetzung mit Hydrazin erhält.

## Claims

1. Compounds of formula (I) 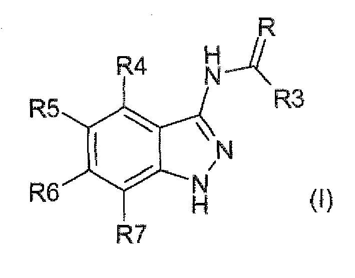 in which
R is either O, S or NH
R3 is a (1-6C)alkyl, aryl(1-6C)alkyl, heteroaryl, heteroaryl(1-6C)alkyl, aryl or heteroaryl fused to a cycloalkyl (1-10C), heterocycle, cycloalkyl, adamantyl, polycycloalkyls, alkenyl, alkynyl radical; these radicals being optionally substituted with one or more substituents chosen from halogen, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryl, heteroaryl, formyl, trifluoromethyl, trifluoromethylsulfanyl and trifluoromethoxy;
R4, R5, R6 and R7 are chosen, independently of each other, from the following radicals: hydrogen, halogen, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluoromethyl, trifluoromethoxy, (1-6C)alkyl, (1-6C)alkoxy, aryl, aryl(1-6C)alkyl, heteroaryl, heteroaryl(1-6C)alkyl, heterocycle, cycloalkyl, alkenyl, alkynyl, adamantyl, polycycloalkyls; these radicals being optionally substituted with one or more substituents chosen from halogen, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryl, heteroaryl, formyl, trifluoromethyl and trifluoromethoxy;
R8, R9, R10, R11 are, independently of each other, a hydrogen, (1-6C)alkyl, aryl, alkenyl, alkynyl or heteroaryl, themselves optionally being substituted with one or more substituents chosen from halogen, (1-6C)alkyl, (1-6C)alkoxy, CN, NO₂, NH₂, OH, COOH, COOalkyl, CONH₂, formyl, trifluoromethyl and trifluoromethoxy;
to the racemic mixtures, enantiomers, diastereoisomers and mixtures thereof, the tautomers thereof and the pharmaceutically acceptable salts thereof with the exception of 3-acetamidoindazole, N-(1H-indazol-3-yl)butanamide, N-(1H-indazol-3-yl)phenylacetamide, N-(1H-indazol-3-yl)benzhydrylacetamide, 5-amino-3-acetamidoindazole, N-(6-chloro-1H-indazol-3-yl)-2,2,2-trifluoroacetamide, N-(6-chloro-1H-indazol-3-yl)-2-furancarboxamide, N-(6-chloro-1H-indazol-3-yl)-2-thiophenecarboxamide and N-(5-nitro-1H-indazol-3-yl)acetamide.

2. Compounds of formula (I) according to Claim 1
in which
R is either O, S or NH
R3 is a (1-6C)alkyl, aryl(1-6C)alkyl, heteroaryl, heteroaryl(1-6C)alkyl, cycloalkyl, alkenyl or alkynyl radical; these radicals being optionally substituted with one or more substituents chosen from halogen, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryl, heteroaryl, formyl, trifluoromethyl,
trifluoromethylsulfanyl and trifluoromethoxy;
R4 and R7 are hydrogen;
R5 and R6 are chosen, independently of each other, from the following radicals: hydrogen, halogen, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluoromethyl, trifluoromethoxy, (1-6C)alkyl, (1-6C)alkoxy, aryl, aryl(1-6C)alkyl, heteroaryl, heteroaryl(1-6C)alkyl, cycloalkyl, heterocycle, alkenyl, alkynyl, adamantyl, polycycloalkyl; these radicals being optionally substituted with one or more substituents chosen from halogen, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryl, heteroaryl, formyl, trifluoromethyl and trifluoromethoxy;
R8, R9, R10, R11 are, independently of each other, a hydrogen, (1-6C)alkyl, aryl, alkenyl, alkynyl or heteroaryl, themselves being optionally substituted with one or more substituents chosen from halogen, (1-6C)alkyl, (1-6C)alkoxy, CN, NO₂, NH₂, OH, COOH, COOalkyl, CONH₂, formyl, trifluoromethyl and trifluoromethoxy;
to the racemic mixtures, enantiomers, diastereoisomers and mixtures thereof, the tautomers thereof and the pharmaceutically acceptable salts thereof.

3. Compounds of formula (I) according to Claim 1 or 2
in which
R is O
R4 and R7 are H
R3 is a (1-6C)alkyl, aryl, aryl(1-6C)alkyl, heteroaryl, heteroaryl(1-6C)alkyl, heterocycle, cycloalkyl or alkenyl radical; these radicals being optionally substituted with one or more substituents chosen from halogen, CN, NO₂, NH₂, OH, OR8, COOH, C(O)OR8, -O-C(O)R8, NR8R9, NHC(O)R8, C(O)NR8R9, SR8, S(O)R8, SO₂R8, NHSO₂R8, SO₂NR8R9, C(S)NR8R9, NHC(S)R8, -O-SO₂R8, -SO₂-O-R8, aryl, heteroaryl, formyl, trifluoromethyl, trifluoromethylsulfanyl and trifluoromethoxy;
R5 and R6 are chosen, independently of each other, from the following radicals: hydrogen, halogen, CN, NO₂, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, trifluoromethyl, trifluoromethoxy, (1-6C)alkyl, (1-6C)alkoxy, aryl, aryl(1-6C)alkyl, heteroaryl, heteroaryl(1-6C)alkyl, heterocycle, cycloalkyl, alkenyl; these radicals being optionally substituted with one or more substituents chosen from halogen, CN, NO₂, NH₂, OH, OR10, COOH, C(O)OR10, -0-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryl, heteroaryl, formyl, trifluoromethyl and trifluoromethoxy;
R8, R9, R10, R11 are, independently of each other, a hydrogen, (1-6C)alkyl, aryl, alkenyl, alkynyl or heteroaryl, themselves being optionally substituted with one or more substituents chosen from halogen, (1-6C)alkyl, (1-6C)alkoxy, CN, NO₂, NH₂, OH, COOH, COOalkyl, CONH₂, formyl, trifluoromethyl and trifluoromethoxy;
to the racemic mixtures, enantiomers, diastereoisomers and mixtures thereof, the tautomers thereof and the pharmaceutically acceptable salts thereof.

4. Compound chosen from:
(2Z) 4-[(6-chloro-1H-indazol-3-yl)amino]-4-oxo-2-butenoic acid
(2E) 4-[(6-chloro-1H-indazol-3-yl)amino]-4-oxo-2-butenoic acid
ethyl (2E) 4-[(6-chloro-1H-indazol-3-yl)amino]-4-oxo-2-butenoate
ethyl (2Z) 4-[(6-chloro-1H-indazol-3-yl)amino]-4-oxo-2-butenoate
4-[(6-chloro-1H-indazol-3-yl]amino]-4-oxo-2-butanoic acid
(2Z) 4-[(5-bromo-1H-indazol-3-yl)amino]-4-oxo-2-butenoic acid
(2E) 4-[(6-chloro-1H-indazol-3-yl)amino]-4-oxo-2-butenoic acid
(2E) 4-[(5-bromo-1H-indazol-3-yl)amino]-4-oxo-2-butenoic acid
(2Z) 4-[(6-chloro-1H-indazol-3-yl)amino]-4-oxo-2-butenoic acid
4-[(5-bromo-1H-indazol-3-yl)amino]-4-oxo-2-butanoic acid
(2E) N-(6-chloro-1H-indazol-3-yl)-2-butenamide
(2Z) N-(6-chloro-1H-indazol-3-yl)-2-butenamide
N-(6-chloro-1H-indazol-3-yl)-3-butenamide hydrochloride
methyl 4-[(6-chloro-1H-indazol-3-yl)amino]-4-oxo-2-butanoate
N-(6-chloro-1H-indazol-3-yl)acetamide
N-(6-chloro-1H-indazol-3-yl)butanamide
(2E) N-(6-bromo-1H-indazol-3-yl)-2-butenamide
(2E) N-(5-methyl-1H-indazol-3-yl)-2-butenamide
(2Z) N-(6-bromo-1H-indazol-3-yl)-2-butenamide
(2Z) N-(5-methyl-1H-indazol-3-yl)-2-butenamide
N-(6-chloro-1H-indazol-3-yl)-2-propanamide
(2E) N-[6-(trifluoromethyl)-1H-indazol-3-yl]-2-butenamide
(2Z) N-[6-(trifluoromethyl)-1H-indazol-3-yl]-2-butenamide
ethyl 4-[[6-(trifluoromethyl)-1H-indazol-3-yl]amino]-4-oxobutanoate
(2E) N-[5-(trifluoromethyl)-1H-indazol-3-yl]-2-butenamide
(2Z) N-[5-(trifluoromethyl)-1H-indazol-3-yl]-2-butenamide
N-[5-chloro-1H-indazol-3-yl]-2-butanamide
N-[4-chloro-1H-indazol-3-yl]butanamide
N-[6-(trifluoromethyl)-1H-indazol-3-yl]butanamide
N-[6-chloro-1H-indazol-3-yl]propenamide
N-[5-(trifluoromethyl)-1H-indazol-3-yl]butanamide
N-[5-nitro-1H-indazol-3-yl]butanamide
N-[6-bromo-1H-indazol-3-yl]butanamide
N-[6-(3-pyridyl)-1H-indazol-3-yl]butanamide
N-[4-iodo-1H-indazol-3-yl]butanamide
N-[6-phenyl-1H-indazol-3-yl]butanamide
N-[6-bromo-5,7-dinitro-1H-indazol-3-yl]butanamide
N-[6-bromo-7-nitro-1H-indazol-3-yl]butanamide
N-[6-bromo-5-nitro-1H-indazol-3-yl]butanamide
N-[6-(3-furyl)-1H-indazol-3-yl]butanamide
N-[6-[4-(benzyloxy)phenyl]-1H-indazol-3-yl]butanamide
N-[6-(4-hydroxyphenyl)-1H-indazol-3-yl]butanamide
N-[6-chloro-1H-indazol-3-yl]benzenamide
N-[6-(3,5-difluorophenyl)-1H-indazol-3-yl]butanamide
N-[6-(3-thienyl)-1H-indazol-3-yl]butanamide
N-[6-chloro-1H-indazol-3-yl]-2-thiopheneacetamide
N-[5-(3-fluorobenzenesulfonylamino)-1H-indazol-3-yl]benzamide
N-[6-(2-chlorophenyl)-1H-indazol-3-yl]butanamide
N-[6-(2-chloro-4-hydroxyphenyl)-1H-indazol-3-yl]butanamide
N-[6-(4-ethylphenyl)-1H-indazol-3-yl]butanamide
N-[6-(4-ethenylphenyl)-1H-indazol-3-yl]butanamide
N-[6-(4-pyridyl)-1H-indazol-3-yl]butanamide
N-[6-(phenylmethyl)-1H-indazol-3-yl]butanamide
N-[6-(4-aminophenyl)-1H-indazol-3-yl]butanamide
N-[6-(1-morpholino)-1H-indazol-3-yl]butanamide
N-[6-[(4-phenylethynyl)phenyl]-1H-indazol-3-yl]butanamide
N-[6-(2-propenyl)-1H-indazol-3-yl]butanamide
N-[5-amino-1H-indazol-3-yl]butanamide
N-[6-bromo-5-chloro-1H-indazol-3-yl]butanamide
N-[6-chloro-5-bromo-1H-indazol-3-yl]butanamide
N-[6-chloro-5-nitro-1H-indazol-3-yl]butanamide
N-[6-(4-hydroxyphenyl)-5-bromo-1H-indazol-3-yl]butanamide
N-[6-(4-hydroxyphenyl)-5-(phenylamino)-1H-indazol-3-yl]butanamide
N-[6-(4-hydroxyphenyl)-5-(2-phenylethenyl)-1H-indazol-3-yl]butanamide
N-[6-(4-hydroxyphenyl)-5-phenylcarbonyl-1H-indazol-3-yl]butanamide
N-[6-(4-hydroxyphenyl)-5-[3-(dimethylamino)propynyl]-1H-indazol-3-yl]butanamide
N-[6-chloro-1H-indazol-3-yl]-3-thiophenecarboxamide
N-[6-chloro-1H-indazol-3-yl]-2-pyridineacetamide
N-[6-chloro-1H-indazol-3-yl]-3-pyridinecarboxamide
N-[6-chloro-1H-indazol-3-yl]benzeneacetamide
N-[6-chloro-1H-indazol-3-yl]benzenepropanamide
N-[6-chloro-1H-indazol-3-yl]-3-pyridineacetamide
N-[6-chloro-1H-indazol-3-yl]-2-chloroacetamide
N-[6-chloro-1H-indazol-3-yl]-4-morpholineacetamide
N-[6-chloro-1H-indazol-3-yl]-1-piperazineacetamide
N-[6-chloro-1H-indazol-3-yl]-4-[(2-methoxyethyl)amino]cyclohexanecarboxamide
4-amino-N-[6-chloro-1H-indazol-3-yl]-1-piperidinecarboxamide
N-[6-chloro-1H-indazol-3-yl]-4-morpholinylcarboxamide
the racemic mixtures, enantiomers, diastereoisomers and mixtures thereof, the tautomers thereof and the pharmaceutically acceptable salts thereof.

5. Compound chosen from:
(2Z) 4-[(6-chloro-1H-indazol-3-yl)amino]-4-oxo-2-butenoic acid
ethyl (2E) 4-[(6-chloro-1H-indazol-3-yl)amino]-4-oxo-2-butenoate
4-[(6-chloro-1H-indazol-3-yl)amino]-4-oxo-2-butanoic acid
(2Z) 4-[(5-bromo-1H-indazol-3-yl)amino]-4-oxo-2-butenoic acid
(2E) 4-[(6-chloro-1H-indazol-3-yl)amino]-4-oxo-2-butenoic acid
4-[(5-bromo-1H-indazol-3-yl)amino]-4-oxo-2-butanoic acid
(2E) N-(6-chloro-1H-indazol-3-yl)-2-butenamide
N-(6-chloro-1H-indazol-3-yl)-3-butenamide hydrochloride
methyl 4-[(6-chloro-1H-indazol-3-yl)amino]-4-oxo-2-butanoate
N-(6-chloro-1H-indazol-3-yl)acetamide
N-(6-chloro-1H-indazol-3-yl)butanamide
(2E) N-(6-bromo-1H-indazol-3-yl)-2-butenamide
(2E) N-(5-methyl-1H-indazol-3-yl)-2-butenamide
N-(6-chloro-1H-indazol-3-yl)-2-propanamide
(2E) N-[6-(trifluoromethyl)-1H-indazol-3-yl]-2-butenamide
ethyl 4-[[6-(trifluoromethyl)-1H-indazol-3-yl]amino]-4-oxobutanoate
(2E) N-[5-(trifluoromethyl)-1H-indazol-3-yl]-2-butenamide
N-[5-chloro-1H-indazol-3-yl]-2-butanamide
N-[4-chloro-1H-indazol-3-yl]butanamide
N-[6-(trifluoromethyl)-1H-indazol-3-yl]butanamide
N-[6-chloro-1H-indazol-3-yl]propenamide
N-[5-(trifluoromethyl)-1H-indazol-3-yl]butanamide
N-[5-nitro-1H-indazol-3-yl]butanamide
N-[6-bromo-1H-indazol-3-yl]butanamide
N-[6-(3-pyridyl)-1H-indazol-3-yl]butanamide
N-[4-iodo-1H-indazol-3-yl]butanamide
N-[6-phenyl-1H-indazol-3-yl]butanamide
N-[6-bromo-5,7-dinitro-1H-indazol-3-yl]butanamide
N-[6-bromo-7-nitro-1H-indazol-3-yl]butanamide
N-[6-bromo-5-nitro-1H-indazol-3-yl]butanamide
N-[6-(3-furyl)-1H-indazol-3-yl]butanamide
N-[6-[4-(benzyloxy)phenyl]-1H-indazol-3-yl]butanamide
N-[6-(4-hydroxyphenyl)-1H-indazol-3-yl]butanamide
N-[6-chloro-1H-indazol-3-yl]benzenamide
N-[[6-(3,5-difluorophenyl)-1H-indazol-3-yl]]butanamide
N-[6-(3-thienyl)-1H-indazol-3-yl]butanamide
N-[6-chloro-1H-indazol-3-yl]-2-thiopheneacetamide
N-[5-(3-fluorobenzenesulfonylamino)-1H-indazol-3-yl]benzamide
N-[6-(2-phenylethyl)-1H-indazol-3-yl]butanamide
N-(6,7-difluoro-1H-indazol-3-yl)butanamide
N-[6-(4-methoxyphenyl)-1H-indazol-3-yl]butanamide
N-[6-(4-methylthiophenyl)-1H-indazol-3-yl]butanamide
N-[6-(4-trifluoromethoxyphenyl)-1H-indazol-3-yl]butanamide
N-[(6-(1-propenyl)-1H-indazol-3-yl]butanamide
N-[6-chloro-1H-indazol-3-yl]-2-pyridinecarboxamide
N-[6-(4-fluorophenyl)-1H-indazol-3-yl]butanamide
N-[6-[4-(1,1-dimethylethyl)phenyl]-1H-indazol-3-yl]butanamide
N-[6-bromo-7-amino-1H-indazol-3-yl]butanamide
N-[6-[4-(trifluoromethyl)phenyl]-1H-indazol-3-yl]butanamide
N-[6-(4-methylphenyl)-1H-indazol-3-yl]butanamide
N-[6-(3,5-dichlorophenyl)-1H-indazol-3-yl]butanamide
N-[6-chloro-1H-indazol-3-yl]-3,5-dichlorobenzamide
N-[6-(4-chlorophenyl)-1H-indazol-3-yl]butanamide
N-[6-chloro-1H-indazol-3-yl]benzenepropanamide trifluoroacetate
N-[6-chloro-1H-indazol-3-yl]benzenepropanamide
N-[[6-(4-ethylphenyl)-1H-indazol-3-yl]]butanamide
N-[6-(4-pyridyl)-1H-indazol-3-yl]butanamide
N-(5-amino-1H-indazol-3-yl)butanamide
N-(5-bromo-6-chloro-1H-indazol-3-yl)butanamide
N-(6-chloro-1H-indazol-3-yl)-2-thiophenecarboxamide
N-(6-chloro-1H-indazol-3-yl)-2-methylpropylamide
4-chloro-N-(6-chloro-1H-indazol-3-yl)butanamide
N-(5-phenyl-6-chloro-1H-indazol-3-yl)butanamide
N-[5-bromo-6-[4-(phenylmethoxy)phenyl]-1H-indazol-3-yl]butanamide
N-[5-bromo-6-(4-hydroxyphenyl)-1H-indazol-3-yl]butanamide
N-[[6-(4-nitrophenyl)-1H-indazol-3-yl]]butanamide
N-[6-(2-chlorophenyl)-1H-indazol-3-yl]butanamide
N-[6-[3-(phenylmethoxy)phenyl]-1H-indazol-3-yl]butanamide
N-[6-(3-hydroxyphenyl)-1H-indazol-3-yl]butanamide
N-[6-chloro-5-(4-pyridyl)-1H-indazol-3-yl]butanamide
N-[6-chloro-5-(3-furyl)-1H-indazol-3-yl]butanamide
N-[6-[2-chloro-4-(phenylmethoxy)phenyl]-1H-indazol-3-yl]butanamide
N-[6-(2-chloro-4-hydroxyphenyl)-1H-indazol-3-yl]butanamide
N-[5,6-dibromo-1H-indazol-3-yl]butanamide
N-[6-chloro-1H-indazol-3-yl]-2,2,3,3,4,4,4-heptafluorobutanamide
N-[6-chloro-5-(4-fluorophenyl)-1H-indazol-3-yl]butanamide
N-[[6-(4-aminophenyl)-1H-indazol-3-yl]]butanamide
N-[6-[4-(dimethylamino)phenyl]-1H-indazol-3-yl]butanamide
N-(6-chloro-1H-indazol-3-yl)-4-methyl-1-piperazineacetamide
N-(6-chloro-1H-indazol-3-yl)-1-piperidineacetamide
N-(6-chloro-1H-indazol-3-yl)-4-morpholineaceta
N-(6-chloro-1H-indazol-3-yl)-1H-1,2,4-triazole-1-acetamide
N-(6-chloro-1H-indazol-3-yl)-2-(cyclohexylamino)acetamide
2-[(phenylmethyl)amino]-N-(6-chloro-1H-indazol-3-yl)acetamide
N-(6-chloro-1H-indazol-3-yl)-1H-azepine-1-acetamide
N-(6-chloro-1H-indazol-3-yl)-1-piperazineacetamide
N-(6-chloro-1H-indazol-3-yl)-2-[[3-(dimethylamino)propyl]amino]acetamide
N-(6-chloro-1H-indazol-3-yl)thiomorpholine-4-acetamide
N-(6-chloro-1H-indazol-3-yl)-1-pyrrolidineacetamide
N-(6-chloro-1H-indazol-3-yl)-2-[[2-(dimethylamino)ethyl]amino]acetamide
N-(6-chloro-1H-indazol-3-yl)-1-cyclopropylaminoacetamide trifluoroacetate
N-(6-chloro-1H-indazol-3-yl)-1-cyclopropylaminoacetamide
N-(6-chloro-1H-indazol-3-yl)-2-(2-diethylaminoethylamino)acetamide tris(trifluoroacetate)
N-(6-chloro-1H-indazol-3-yl)-2-(2-diethylaminoethylamino)acetamide
N-[5,6-diphenyl-1H-indazol-3-yl]butanamide
N-[6-chloro-5-(4-methylphenyl)-1H-indazol-3-yl]butanamide
N-[5-phenyl-6-[4-(phenylmethoxy)phenyl]-1H-indazol-3-yl]butanamide
N-[5-phenyl-6-(4-hydroxyphenyl)-1H-indazol-3-yl]butanamide
N-[6-chloro-5-(4-pyridyl)-1H-indazol-3-yl]butanamide
N-[5-(4-aminophenyl)-6-chloro-1H-indazol-3-yl]butanamide
N-[6-chloro-5-(4-ethylphenyl)-1H-indazol-3-yl]butanamide
N-[6-chloro-5-[4-(phenylmethoxy)phenyl]-1H-indazol-3-yl]butanamide
N-[6-chloro-5-(4-hydroxyphenyl)-1H-indazol-3-yl]butanamide
N-[5,6-bis[4-(phenylmethoxy)phenyl]-1H-indazol-3-yl]butanamide
N-[5,6-bis(4-hydroxyphenyl)-1H-indazol-3-yl]butanamide
N-[5-(3-furyl)-6-[4-(phenylmethoxy)phenyl]-1H-indazol-3-yl]butanamide
N-[5-(3-furyl)-6-([4-hydroxyphenyl)-1H-indazol-3-yl]butanamide
N-[5-(4-ethylphenyl)-6-[4-(phenylmethoxy)phenyl]-1H-indazol-3-yl]butanamide
N-[5-(4-ethylphenyl)-6-(4-hydroxyphenyl)-1H-indazol-3-yl]butanamide
N-[5-(3-pyridyl)-6-[4-(phenylmethoxy)phenyl]-1H-indazol-3-yl]butanamide
N-[5-(3-pyridyl)-6-(4-hydroxyphenyl)-1H-indazol-3-yl]butanamide
N-[5-(2-furyl)-6-[4-(phenylmethoxy)phenyl]-1H-indazol-3-yl]butanamide
N-[5-(2-furyl)-6-(4-hydroxyphenyl)-1H-indazol-3-yl]butanamide
N-(5-bromo-6-chloro-7-nitro-1H-indazol-3-yl)butanamide
N-(5-bromo-6,7-difluoro-1H-indazol-3-yl)butanamide
N-[6-(4-cyanophenyl)-1H-indazol-3-yl]butanamide
N-(6,7-difluoro-5-nitro-1H-indazol-3-yl)butanamide
N-(6,7-difluoro-5-phenyl-1H-indazol-3-yl)butanamide
N-[6-(6-hydroxypyrid-3-yl)-1H-indazol-3-yl]butanamide
N-[6-(3,4-dihydroxyphenyl)-1H-indazol-3-yl]butanamide trifluoroacetate
N-[6-(3,4-dihydroxyphenyl)-1H-indazol-3-yl]butanamide
N-[7-fluoro-5-nitro-6-[2-(phenylethyl)amino]-1H-indazol-3-yl]butanamide
N-(7-fluoro-5-nitro-6-morpholino-1H-indazol-3-yl)butanamide
N-(7-fluoro-5-amino-6-morpholino-1H-indazol-3-yl)butanamide
N-(5-bromo-7-fluoro-6-morpholino-1H-indazol-3-yl)butanamide
N-[7-fluoro-6-(trifluoromethyl)-1H-indazol-3-yl]butanamide
N-(6-bromo-4,5,7-trifluoro-1H-indazol-3-yl)butanamide
N-[6-(6-aminopyrid-3-yl)-1H-indazol-3-yl]butanamide difluoroacetate
N-[6-(6-aminopyrid-3-yl)-1H-indazol-3-yl]butanamide
2-chloro-N-(6,7-difluoro-1H-indazol-3-yl)acetamide
N-(6,7-difluoro-1H-indazol-3-yl)-1-piperidineacetamide
the racemic mixtures, enantiomers, diastereoisomers and mixtures thereof, the tautomers thereof and the pharmaceutically acceptable salts thereof.

6. Pharmaceutical composition, **characterized in that** it comprises, in a pharmaceutically acceptable medium, a compound defined according to any one of Claims 1 to 5.

7. Medicinal product, **characterized in that** it contains at least one compound defined according to any one of Claims 1 to 5, for its therapeutic application in the treatment of diseases in which phosphorylation of the Tau protein is observed.

8. Medicinal product, **characterized in that** it contains at least one compound defined according to any one of Claims 1 to 5, for its therapeutic application in the treatment of neurodegenerative diseases, strokes, cranial and spinal trauma and peripheral neuropathies, obesity, metabolic diseases, type II diabetes, essential hypertension, atherosclerotic cardiovascular diseases, polycystic ovarian syndrome, syndrome X, immunodeficiency and cancer.

9. Medicinal product according to Claim 8, **characterized in that** the neurodegenerative disease is either Alzheimer's disease, Parkinson's disease, frontoparietal dementia, corticobasal degeneration or Pick's disease.

10. Process for preparing the compounds of formula (I) as defined in Claim 1 and for which R is oxygen, **characterized in that** an acylation is performed on the amine derivative of formula (II): 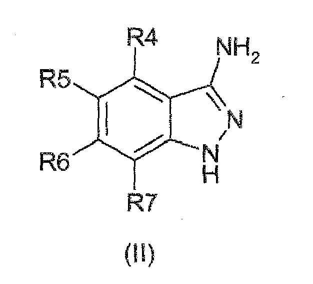 in which R4, R5, R6 and R7 have the same meanings as in Claim 1, and it is optionally converted into a pharmaceutically acceptable salt thereof.

11. Process for preparing the compounds of formula (I) as defined in Claim 1 and for which R is a sulfur, **characterized in that** a thionation is performed on the derivative in which R is an oxygen, and it is optionally converted into a pharmaceutically acceptable salt thereof.

12. Process for preparing the compounds of formula (I) as defined in Claim 1 and for which R is NH, **characterized in that** the amine derivative of formula (II) is reacted with a nitrile or a Meerwein salt 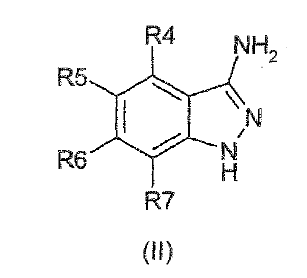 in which R4, R5, R6 and R7 have the same meanings as in Claim 1, and it is optionally converted into a pharmaceutically acceptable salt.

13. Preparation process according to Claims 10 or 12, **characterized in that** the derivative of formula (II) is obtained from the derivative of formula (III) by reaction with hydrazine
